(19) Europäisches Patentamt
European Patent Office
Office européen des brevets

(11) Veröffentlichungsnummer: **0 418 667 B1**

# (12) EUROPÄISCHE PATENTSCHRIFT

(45) Veröffentlichungstag der Patentschrift: **16.08.95**

(51) Int. Cl.[6]: **C07D 261/18**, C07D 275/03, A01N 43/80, C07D 413/04, C07D 417/04

(21) Anmeldenummer: **90117233.8**

(22) Anmeldetag: **07.09.90**

Die Akte enthält technische Angaben, die nach dem Eingang der Anmeldung eingereicht wurden und die nicht in dieser Patentschrift enthalten sind.

## (54) Carbonsäureamide.

(30) Priorität: **22.09.89 DE 3931627**
**11.10.89 DE 3933898**

(43) Veröffentlichungstag der Anmeldung:
**27.03.91 Patentblatt 91/13**

(45) Bekanntmachung des Hinweises auf die Patenterteilung:
**16.08.95 Patentblatt 95/33**

(84) Benannte Vertragsstaaten:
**BE CH DE ES FR GB IT LI NL SE**

(56) Entgegenhaltungen:
**EP-A- 0 337 623**
**US-A- 3 409 616**

**JOURNAL OF HETEROCYCLIC CHEMISTRY, Band 22, November-Dezember 1985, Seiten 1561-1563; A.CAMPARINI et al.: "Synthesis and photochemical reactivity of 3-methylisoxazolo[4,5-d]pyridazines[1]"**

(73) Patentinhaber: **BASF Aktiengesellschaft**
**Carl-Bosch-Strasse 38**
**D-67063 Ludwigshafen (DE)**

(72) Erfinder: **Maywald, Volker, Dr.**
**Berner Weg 24**
**D-6700 Ludwigshafen (DE)**
Erfinder: **Freund, Wolfgang, Dr.**
**Johann-Gottlieb-Fichte-Strasse 71**
**D-6730 Neustadt (DE)**
Erfinder: **Hamprecht, Gerhard, Dr.**
**Rote-Turm-Strasse 28**
**D-6940 Weinheim (DE)**
Erfinder: **Kuekenhoehner, Thomas, Dr.**
**Seidelstrasse 2**
**D-6710 Frankenthal (DE)**
Erfinder: **Plath, Peter, Dr.**
**Hans-Balcke-Strasse 13**
**D-6710 Frankenthal (DE)**
Erfinder: **Wuerzer, Bruno, Dr.**
**Ruedigerstrasse 13**
**D-6701 Otterstadt (DE)**
Erfinder: **Westphalen, Karl-Otto, Dr.**
**Mausbergweg 58**
**D-6720 Speyer (DE)**

**EP 0 418 667 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

JOURNAL OF THE CHEMICAL SOCIETY, 1965, Seiten 7277-7282, London, GB; A. HOLLAND et al.: "Isothiazoles. Part IX. Isothiazolopyramidines"

CHEMICAL ABSTRACTS, Band 67, Nr. 7, 14. August 1967, Seiten 3086-3087,Zusammenfassung Nr. 32624f, Columbus, Ohio, US; G. DESIMONI et al.: "3-Phenyl-4-aminoisoxazole-5-carboxylic acid", & GAZZ. CHIM. ITAL. 97(1), 25-33 1967

CHEMICAL ABSTRACTS, Band 97, Nr 3, 19. Juli 1982, Seite 701, Zusammenfassung Nr. 23671m, Columbus, Ohio, US;, G. WESTPHAL et al: "Reaction of 1,2,5-oxadiazole N-oxides with diethyl acetylenedicarboxylate"; & Y. CHEM. 1982, 22(4), 138-9

JOURNAL OF THE CHEMICAL SOCIETY PERKIN TRANSACTIONS I, 1987, Seiten 1005-1009,London, GB; R. NESI et al.: "A new spiro annellation reaction in the isoxaloze series: Applications and limits. Part 2. Reactivity of ethyl 4-nitro-3-phenylisoxazole-5-carboxylate with nitrogen binucleophiles"

JOURNAL OF THE CHEMICAL SOCIETY PERKIN TRANSACTIONS I, 1982, Seiten 2391-2394,London, GB; A. CAMPARINI et al.: "Syntheses and reactivities of 3-methylisoxazolo[4,5-b]pyridines"

JOURNAL OF THE CHEMICAL SOCIETY, 1959, Seiten 3061-3072, London, GB; A. ADAMS et al.: "Isothiazole: A new mononuclear heterocyclic system"

## Beschreibung

Die vorliegende Erfindung betrifft Carbonsäureamide der Formeln Ia, Ab, Ic und Id

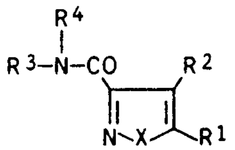
Ia

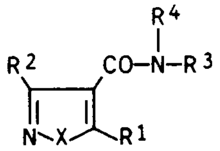
Ib

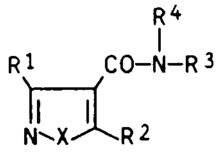
Ic

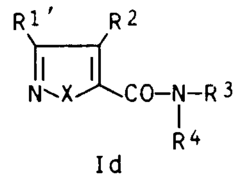
Id

in denen die Variablen folgende Bedeutung haben:

X Sauerstoff oder Schwefel;

$R^1$

- Wasserstoff, Halogen, $C_1$-$C_6$-Alkyl, welches ein bis fünf Halogenatome und/oder einen Cyanorest und/oder bis zu zwei der folgenden Reste tragen kann: $C_1$-$C_4$-Alkoxy, partiell oder vollständig halogeniertes $C_1$-$C_4$-Alkoxy, $C_1$-$C_4$-Alkylthio oder partiell oder vollständig halogeniertes $C_1$-$C_4$-Alkylthio;
- eine $C_1$-$C_4$-Alkoxy- oder $C_1$-$C_4$-Alkylthiogruppe, eine partiell oder vollständig halogenierte $C_1$-$C_4$-Alkoxygruppe, eine partiell oder vollständig halogenierte $C_1$-$C_4$-Alkylthiogruppe;
- die Benzylgruppe, die ein- bis dreimal durch $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio, partiell oder vollständig halogeniertes $C_1$-$C_4$-Alkylthio, Halogen, Cyano oder Nitro substituiert sein kann;
- die Phenylgruppe, welche noch einen bis drei der folgenden Reste tragen kann: Cyano, Nitro, Halogen, $C_1$-$C_6$-Alkyl, partiell oder vollständig halogeniertes $C_1$-$C_6$-Alkyl, $C_1$-$C_6$-Alkoxy, partiell oder vollständig halogeniertes $C_1$-$C_6$-Alkoxy, $C_1$-$C_6$-Alkylthio und/oder partiell oder vollständig halogeniertes $C_1$-$C_6$-Alkylthio;
- die Phenoxy- oder die Phenylthiogruppe, wobei beide Gruppen ein- bis dreimal durch $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio, partiell oder vollständig halogeniertes $C_1$-$C_4$-Alkylthio, Halogen, Cyano oder Nitro substituiert sein können;
- ein 5- bis 6-gliedriger gesättigter oder aromatischer heterocyclischer Rest, enthaltend ein oder zwei Heteroatome, ausgewählt aus der Gruppe Sauerstoff, Schwefel und Stickstoff, der ein oder zwei der folgenden Substituenten tragen kann: Halogen $C_1$-$C_3$-Alkyl, $C_1$-$C_3$-Alkoxy und $C_1$-$C_3$-Alkoxycarbonyl;
- eine durch $C_3$-$C_8$-Cycloalkyl substituierte $C_1$-$C_6$-Alkylgruppe;
- eine $C_2$-$C_6$-Alkenylgruppe, deren Doppelbindung epoxidiert sein kann, oder eine $C_2$-$C_6$-Alkinylgruppe, wobei beide Gruppen ein- bis dreimal durch Halogen, $C_1$-$C_3$-Alkoxy und/oder einmal durch Cyclopropyl oder Phenyl substituiert sein können, wobei der Phenylrest zusätzlich bis zu drei der folgenden Substituenten tragen kann: Halogen, Cyano, Nitro, $C_1$-$C_4$-Alkyl, das unsubstituiert oder partiell oder vollständig halogeniert sein kann, $C_1$-$C_4$-Alkoxy oder $C_1$-$C_4$-Alkylthio, die beide unsubstituiert oder partiell oder vollständig halogeniert sein können;
- eine $C_3$-$C_8$-Cycloalkyl- oder eine $C_3$-$C_6$-Cycloalkenylgruppe, wobei beide Gruppen ein- bis dreimal durch $C_1$-$C_4$-Alkyl oder Halogen substituiert sein können;

$R^{1'}$

- eine durch $C_3$-$C_8$-Cycloalkyl substituierte $C_1$-$C_6$-Alkylgruppe;
- eine $C_2$-$C_6$-Alkenylgruppe, deren Doppelbindung epoxidiert sein kann, oder eine $C_2$-$C_6$-Alkinylgruppe, wobei beide Gruppen ein- bis dreimal durch Halogen, $C_1$-$C_3$-Alkoxy und/oder einmal durch Cyclopropyl oder Phenyl substituiert sein können, wobei der Phenylrest zusätzlich bis zu drei der folgenden Substituenten tragen kann: Halogen, Cyano, Nitro, $C_1$-$C_4$-Alkyl, das unsubstituiert oder partiell oder vollständig halogeniert sein kann, $C_1$-$C_4$-Alkoxy oder $C_1$-$C_4$-Alkylthio, die beide unsubstituiert oder partiell oder vollständig halogeniert sein können;

- eine $C_3$-$C_6$-Cycloalkenylgruppe, die ein- bis dreimal durch Halogen oder $C_1$-$C_4$-Alkyl substituiert sein kann;

$R^2$

- eine Formylgruppe, eine 4,5-Dihydrooxazol-2-ylgruppe,
- ein Rest $COYR^5$ oder $CONR^6R^7$, wobei die Variablen die folgende Bedeutung haben:

Y Sauerstoff oder Schwefel;

$R^5$

- Wasserstoff;
- eine $C_1$-$C_6$-Alkylgruppe, welche ein bis fünf Halogenatome und/oder bis zu drei Hydroxy- und/oder $C_1$-$C_4$-Alkoxygruppen und/oder einen der folgenden Reste tragen kann:
  - Cyano,
  - $C_1$-$C_4$-Alkoxy-$C_2$-$C_4$-alkoxy,
  - $C_1$-$C_3$-Alkylthio,
  - $C_1$-$C_3$-Alkylamino, Di-($C_1$-$C_3$)-alkylamino, $C_3$-$C_6$-Cycloalkylamino oder Di-($C_3$-$C_6$)-cycloalkylamino,
  - Trimethylsilyl,
  - $C_1$-$C_3$-Alkylsulfinyl oder $C_1$-$C_3$-Alkylsulfonyl,
  - Carboxyl, $C_1$-$C_3$-Alkoxycarbonyl, $C_1$-$C_3$-Alkoxycarbonyl-$C_1$-$C_3$-alkoxy oder $C_1$-$C_3$-Alkoxycarbonyl-$C_1$-$C_3$-alkoxy-$C_1$-$C_3$-alkoxycarbonyl,
  - Di-($C_1$-$C_3$)-alkylaminocarbonyl,
  - Di-($C_1$-$C_3$)-alkoxyphosphonyl,
  - $C_1$-$C_6$-Alkaniminoxy oder $C_5$-$C_6$-Cycloalkaniminoxy,
  - N-Phthalimido, N-Succinimido, Benzyloxy, Benzoyl, wobei diese cyclischen Reste zusätzlich eine bis drei der folgenden Gruppen tragen können: Halogen, $C_1$-$C_3$-Alkyl oder $C_1$-$C_3$-Alkoxy,
  - einen 5- oder 6-gliedrigen gesättigten heterocyclischen Rest oder einen 5- oder 6-gliedrigen heteroaromatischen Rest mit jeweils bis zu 3 Heteroatomen, ausgewählt aus der Gruppe Sauerstoff, Schwefel und Stickstoff, wobei zwei Sauerstoff- und/oder Schwefelatome nicht direkt benachbart sein können und wobei die Heterocyclen noch einen oder zwei der folgenden Substituenten tragen können: Halogen, $C_1$-$C_3$-Alkyl, $C_1$-$C_3$-Alkoxy oder $C_1$-$C_3$-Alkoxycarbonyl;
  - Phenyl, das noch bis zu drei der folgenden Substituenten tragen kann: Halogen, Nitro, Cyano, $C_1$-$C_3$-Alkyl, partiell oder vollständig halogeniertes $C_1$-$C_3$-Alkyl, $C_1$-$C_3$-Alkoxy oder partiell oder vollständig halogeniertes $C_1$-$C_3$-Alkoxy;
  - einen Rest $-CR^{10}=N-R^{11}$, wobei $R^{10}$ und $R^{11}$ die folgende Bedeutung haben:

$R^{10}$ Wasserstoff oder $C_1$-$C_6$-Alkyl und

$R^{11}$ $C_1$-$C_6$-Alkoxy, $C_3$-$C_6$-Alkenyloxy oder $C_3$-$C_6$-Alkinyloxy, die jeweils bis zu 3 Halogenatome und/oder einen Phenylrest mit gewünschtenfalls bis zu drei der folgenden Reste tragen können: Halogen, Nitro, Cyano, $C_1$-$C_3$-Alkyl oder $C_1$-$C_3$-Alkoxy; Phenoxy, das noch bis zu drei der folgenden Substituenten tragen kann: Halogen, Nitro, Cyano, $C_1$-$C_3$-Alkyl oder $C_1$-$C_3$-Alkoxy; $C_1$-$C_6$-Alkylamino, Di-($C_1$-$C_6$)-alkylamino oder Phenylamino, wobei der Aromat zusätzlich bis zu drei der folgenden Reste tragen kann: Halogen, Nitro, Cyano, $C_1$-$C_3$-Alkyl oder $C_1$-$C_3$-Alkoxy;

- $C_3$-$C_8$-Cycloalkyl;
- $C_3$-$C_6$-Alkenyl, $C_5$-$C_6$-Cycloalkenyl, $C_3$-$C_6$-Alkinyl, wobei diese Reste eine der folgenden Gruppen tragen können: Hydroxy, Halogen, $C_1$-$C_4$-Alkoxy oder Phenyl, wobei der Aromat seinerseits eine bis drei der folgenden Gruppen tragen kann: Halogen, Nitro, Cyano, $C_1$-$C_4$-Alkyl, partiell oder vollständig halogeniertes $C_1$-$C_4$-Alkyl oder $C_1$-$C_4$-Alkoxy;
- Phenyl, das eine bis drei der folgenden Gruppen tragen kann: Halogen, Nitro, Cyano, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy oder $C_1$-$C_4$-Alkoxycarbonyl;
- einen fünf- oder sechsgliedrigen heterocyclischen Rest mit bis zu drei Heteroatomen, ausgewählt aus der Gruppe Sauerstoff, Schwefel und Stickstoff, wobei zwei Sauerstoff- und/oder Schwefelatome nicht direkt benachbart sein können und wobei die Heterocyclen noch einen oder zwei der folgenden Substituenten tragen können: Halogen, $C_1$-$C_3$-Alkyl, $C_1$-$C_3$-Alkoxy oder $C_1$-$C_3$-Alkoxycarbonyl;
- einen Benzotriazolrest;
- N-Phthalimido, Tetrahydrophthalimido, Succinimido, Maleinimido;
- die 2,2-Dimethyl-1,3-dioxolan-4-ylmethyl- oder 1,3-Dioxolan-2-on-4-ylmethylgruppe;

- im Falle Y = O: ein Äquivalent eines Kations aus der Gruppe der Alkali- und Erdalkalimetalle, Mangan, Kupfer, Eisen, Ammonium und mit bis zu 4 $C_1$-$C_3$-Alkylgruppen substituiertes Ammonium; oder
- ein Rest $-N=CR^8R^9$, wobei
  $R^8$, $R^9$ Wasserstoff; $C_1$-$C_4$-Alkyl, das unsubstituiert oder partiell oder vollständig halogeniert sein und einen $C_1$-$C_3$-Alkoxy- oder Phenylrest tragen kann, wobei der aromatische Rest seinerseits noch ein bis dreimal durch Halogen, Nitro, Cyano, $C_1$-$C_3$-Alkyl, partiell oder vollständig halogeniertes $C_1$-$C_3$-Alkyl, $C_1$-$C_3$-Alkoxy oder partiell oder vollständig halogeniertes $C_1$-$C_3$-Alkoxy substituiert sein kann; $C_3$-$C_6$-Cycloalkyl; $C_1$-$C_4$-Alkoxy; Furanyl oder Phenyl, das zusätzlich bis zu drei der folgenden Substituenten tragen kann: Halogen, Nitro, Cyano, $C_1$-$C_3$-Alkyl, partiell oder vollständig halogeniertes $C_1$-$C_3$-Alkyl, $C_1$-$C_3$-Alkoxy oder partiell oder vollständig halogeniertes $C_1$-$C_3$-Alkoxy;
  oder $R^8$ und $R^9$ gemeinsam eine Methylenkette mit 4 bis 7 Gliedern bedeuten;
- einen Rest -W-Z, wobei W eine $C_2$-$C_4$-Alkylenkette, eine Ethoxyethylenkette, eine But-2-enylen- oder eine But-2-inylenkette bedeutet und Z einen in $\omega$-Stellung an W gebundenen Molekülteil, der den gleichen Molekülteil darstellt, der in $\alpha$-Stellung von W mit W verknüpft ist, bedeutet;

$R^6$
- Wasserstoff, $C_1$-$C_6$-Alkyl oder $C_3$-$C_8$-Cycloalkyl und

$R^7$
- Wasserstoff, $C_1$-$C_6$-Alkyl, $-C(OR^{12})=N-H$ oder $-C(OR^{12})=N-(C_1-C_4)$-alkyl, wobei $R^{12}$ $C_1$-$C_4$-Alkyl bedeutet oder

$R^6$,$R^7$ gemeinsam eine Methylenkette mit 4 oder 5 Gliedern;

$R^3$
- Wasserstoff;
- $C_1$-$C_6$-Alkyl, das einen bis drei der folgenden Substituenten tragen kann: Hydroxy, Halogen, $C_1$-$C_4$-Alkoxy, $C_1$-$C_4$-Alkylthio oder Di-($C_1$-$C_4$)-alkylamino;
- $C_3$-$C_8$-Cycloalkyl, das ein- bis dreimal durch Halogen, $C_1$-$C_4$-Alkyl und partiell oder vollständig halogeniertes $C_1$-$C_4$-Alkyl substituiert sein kann;

$R^4$
- Wasserstoff, Hydroxyl, eine $C_1$-$C_4$-Alkoxygruppe;
- eine $C_1$-$C_6$-Alkylgruppe, die einen bis drei der folgenden Reste tragen kann: Halogen, Cyano, $C_1$-$C_4$-Alkoxy, partiell oder vollständig halogeniertes $C_1$-$C_4$-Alkoxy, $C_1$-$C_4$-Alkylthio, partiell oder vollständig halogeniertes $C_1$-$C_4$-Alkylthio, Di-$C_1$-$C_4$-alkylamino, $C_3$-$C_8$-Cycloalkyl oder Phenyl, wobei der Phenylring seinerseits einen bis drei der folgenden Reste tragen kann: Halogen, Cyano, Nitro, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio oder partiell oder vollständig halogeniertes $C_1$-$C_4$-Alkylthio;
- eine $C_3$-$C_8$-Cycloalkylgruppe, die einen bis drei der folgenden Reste tragen kann: Halogen, Nitro, Cyano, $C_1$-$C_6$-Alkyl, partiell oder vollständig halogeniertes $C_1$-$C_6$-Alkyl, $C_1$-$C_4$-Alkoxy oder partiell oder vollständig halogeniertes $C_1$-$C_4$-Alkoxy;
- eine $C_3$-$C_6$-Alkenyl- oder $C_3$-$C_6$-Alkinylgruppe, die jeweils ein- bis dreimal durch Halogen und/oder einmal durch Phenyl substituiert sein können, wobei der Phenylrest seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
- eine Di-($C_1$-$C_4$)-alkylaminogruppe;
- ein 5- bis 6-gliedriger heterocyclischer gesättigter oder aromatischer Rest mit einem oder zwei Heteroatomen, ausgewählt aus der Gruppe Sauerstoff, Schwefel und Stickstoff, der ein- bis dreimal durch $C_1$-$C_4$-Alkyl oder Halogen substituiert sein kann;
- eine Phenylgruppe, die eine bis vier der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro, Cyano, Formyl, $C_1$-$C_4$-Alkanoyl, $C_1$-$C_4$-Halogenalkanoyl oder $C_1$-$C_4$-Alkoxycarbonyl;
- eine Naphthylgruppe, die ein- bis dreimal durch $C_1$-$C_4$-Alkyl oder Halogen substituiert sein kann;

oder

$R^3$, $R^4$ gemeinsam eine Methylenkette mit 4 bis 7 Gliedern, welche durch Sauerstoff, Schwefel oder N-Methyl unterbrochen sein kann, oder den Rest $-(CH_2)_3-CO-$,

wobei im Falle der Verbindungen Ia bis Ic $R^3$ und $R^4$ nicht gleichzeitig Wasserstoff bedeuten, wenn

- $R^1$ Wasserstoff, Methyl oder Phenyl und $R^2$ $CONH_2$, COOH oder $COOCH_3$ bedeuten oder wenn
- X Sauerstoff, $R^1$ $CH(OCH_2CH_3)_2$ und $R^2$ $CONH_2$ bedeuten, sowie die umweltverträglichen Salze der Verbindungen Ia bis Id.

Außerdem betrifft die Erfindung herbizide Mittel, welche die Verbindungen Ia bis Id als wirksame Substanzen enthalten sowie herbizide Mittel, welche mindestens eine Verbindung Ia′, Ib′ oder Ic′ enthalten, in denen die Substituenten die vorstehend gegebene Bedeutung haben und $R^3$ und $R^4$ gleichzeitig Wasserstoff bedeuten können, wenn

- $R^1$ Wasserstoff, Methyl oder Phenyl und $R^2$ $CONH_2$, $CO_2H$ oder $CO_2CH_3$ bedeuten oder wenn
- X Sauerstoff, $R^1$ $CH(OCH_2CH_3)$ und $R^2$ $CONH_2$ bedeuten.

Isoxazol- und Isothiazol-carbonsäuren bzw. deren Derivate sind bekannt. Dies sind folgende Carbonsäureamide vom Typ Ia′, Ib′ und Ic′:

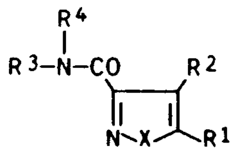

Ia′

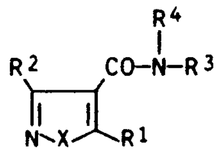

Ib′

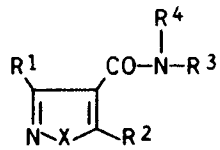

Ic′

| Ia′ | X | R1 | R2 | R3 | R4 | Lit. |
|---|---|---|---|---|---|---|
| 1. | O | $CH(OCH_2CH_3)_2$ | $CONH_2$ | H | H | 1 |
| 2. | S | H | $CONH_2$ | H | H | 2 |
| 3. | S | H | COOH | H | H | 2 |

| Ib′ | X | R1 | R2 | R3 | R4 | Lit. |
|---|---|---|---|---|---|---|
| 1. | S | H | COOH | H | H | 2 |

| Ic′ | X | R1 | R2 | R3 | R4 | Lit. |
|---|---|---|---|---|---|---|
| 1. | S | H | COOH | H | H | 3 |
| 2. | O | Ph | COOH | H | H | 4 |
| 3. | O | Ph | $COOCH_3$ | H | H | 4 |
| 4. | O | $CH_3$ | COOH | H | H | 5 |

1) K. Butler; L.H. Conover, R.B. Woodward, U.S., US 3 699 117, 17 Oct. 1972, 40 pp; CA 78(1): 4027x, CA 73(23): 120 602b, CA 73(3): 14574j, CA 70(15): 68002c

2) J. Chem. Soc., 7277 (1965)

3) J. Chem. Soc., 3061 (1959)

4) G. Desimoni, P. Gruenager, Gazz.Chim.Ital., 97(1), 25-33 (1967)

5) A. Camparini; F. Ponticelli, P. Tedeschi, J.Chem.Soc., Perkin Trans 1, (10), 2391-4 (1982)

Vom Typ der Verbindungen Id sind dies die 5-Aminocarbonyl-3-methyl-4-isoxazolcarbonsäure, der 5-Aminocarbonyl-3-methyl-4-isoxazolcarbonsaureethylester, das Isothiazol-4,5-dicarboxamid sowie die 5-Carbamoyl-4-isothiazolcarbonsäure (J. Chem. Soc. Perkin Trans. I, 1982, 2391; J. Heterocyclic. Chem. 22, 1561 (1985); J. Chem. Soc. 1959, 3061).

Eine Verwendung dieser Verbindungen als Herbizide ist jedoch unbekannt.

Aufgabe der vorliegenden Erfindung war es, neue herbizid wirksame Verbindungen bereitzustellen.

Demgemäß wurden die eingangs definierten carbonsäureamide Ia, Ib, Ic und Id gefunden.

Außerdem wurden Verfahren zu ihrer Herstellung und herbizide Mittel, die die Verbindungen Ia, Ib, Ic und/oder Id enthalten, gefunden.

Die erfindungsgemäßen carbonsäureamide Ia, Ib, Ic und Id sind auf verschiedenen Wegen herstellbar und zwar vorzugsweise nach den folgenden Verfahren:

1. Verfahren zur Herstellung von Verbindungen der Formeln Ia und Ib, in denen $R^2$ $CO_2CH_3$ und X Sauerstoff bedeuten

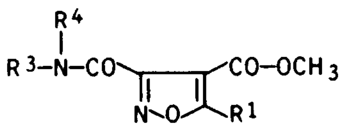

Ia

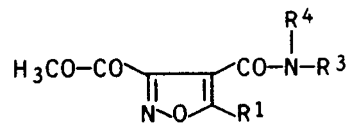

Ib

Man erhält die Carbonsäureamide Ia und Ib dadurch, daß man das Hydroxamsäurechlorid II in an sich bekannter Weise in Gegenwart einer Base mit einem β-Ketoester III umsetzt, den so erhaltenen Dimethyldiester IV anschließend zunächst mit einem Äquivalent einer wäßrigen Base zu den Monoestern Va bzw. Vb hydrolysiert und Va und Vb danach getrennt oder im Gemisch zuerst in an sich bekannter Weise in die Halogenide oder andere aktivierte Formen der Carbonsäuren überführt und diese Derivate anschließend mit einem Amin VIa amidiert.

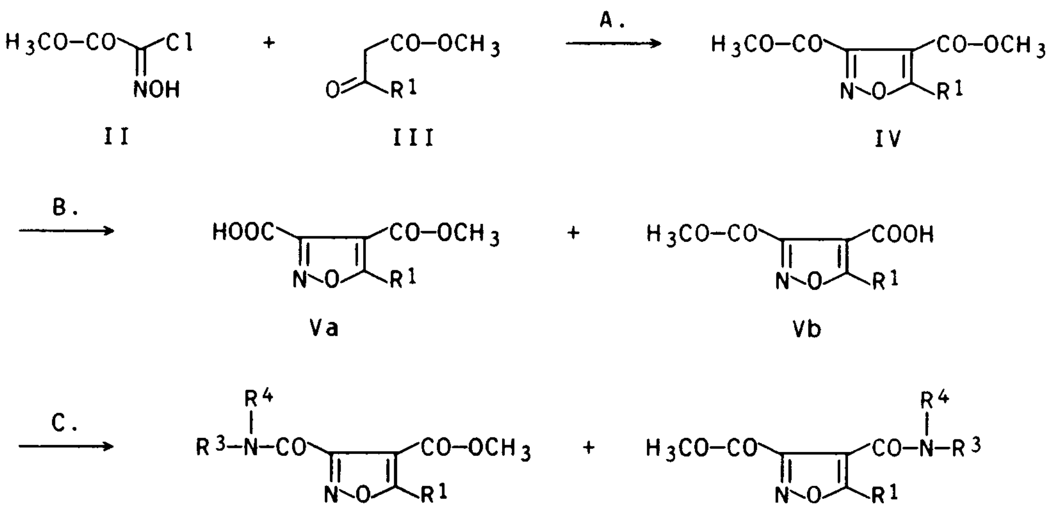


II III IV

Va Vb

Ia Ib

Die einzelnen Reaktionsschritte A, B und C dieser Synthesesequenz können wie folgt durchgeführt werden:

Reaktionsschritt A:

Die Umsetzung wird in der Regel bei Temperaturen von 0 bis 50 °C, vorzugsweise 10 bis 30 °C in einem inerten aprotisch polaren organischen Lösungsmittel in Gegenwart einer Base durchgeführt.

Zweckmäßig verwendet man als Lösungsmittel Kohlenwasserstoffe wie insbesondere Benzol, Toluol, o-, m- und p-Xylol oder Ether wie Diethylether, tert.-Butyl-methylether, Tetrahydrofuran, Dimethoxyethan, Ethylenglycoldimethylether und Dioxan.

Als Base eignet sich insbesondere Natriumhydrid.

Die Umsetzung wird üblicherweise so durchgeführt, daß zunächst der β-Ketoester III im Lösungsmittel mit 1 bis 2 mol-äq. der Base in das Anion überführt wird. Diese Lösung des Anions von III wird anschließend mit einer Lösung von II versetzt und bei der Umsetzungstemperatur belassen.

Die Umsetzung ist im allgemeinen nach 4 bis 12 Stunden beendet.

Vor der Aufarbeitung der Produkte empfiehlt es sich, das bei der Reaktion gebildete Wasser azeotrop zu entfernen.

Reaktionsschritt B:

Die partielle Verseifung des Dimethyldiesters IV zu den Monoestern Viaund Vb wird üblicherweise bei Temperaturen von (-40) bis 20 °C, vorzugsweise (-20) bis 0 °C, in einem inerten, mit Wasser mischbaren organischen Lösungsmittel in Gegenwart von 1 bis 1,1 mol-äq. einer Base durchgeführt.

Als Basen eignen sich insbesondere Hydroxyde von Alkalimetall-Kationen. Die Base wird im allgemeinen als 5 bis 10prozentige wäßrige Lösung zugesetzt.

Bevorzugte Lösungsmittel für diese Umsetzung sind beispielsweise Tetrahydrofuran und Dioxan.

Zur Aufarbeitung wird das Reaktionsgemisch üblicherweise angesäuert, wobei das gewünschte Produkt sich als Feststoff oder als Öl abscheidet. Die Isolierung erfolgt in üblicher Weise durch Filtration bzw. Extraktion.

Das Gemisch der beiden isomeren Monoester Va und Vb kann durch fraktionierte Kristallisation oder auf chromatographischem Wege getrennt werden oder es kann ohne Trennung weiter umgesetzt werden.

Reaktionsschritt C:

Man erhält die Verbindungen Ia bzw. Ib aus den Monoestern Va und Vb, in dem man Va und Vb zunächst in an sich bekannter Weise in das Halogenid oder eine andere aktivierte Form der Carbonsäurefunktion überführt und diese Derivate anschließend mit einem Amin VIa amidiert.

Aktivierte Formen der Carbonsäure sind neben Halogeniden wie insbesondere den Chloriden und den Bromiden beispielsweise auch Imidazolide. Im allgemeinen werden die Halogenide bevorzugt.

Man erhält sie durch Umsetzung der Carbonsäuren Va und Vb mit einem Halogenierungsmittel wie Phosgen, Thionylchlorid, Thionylbromid, Phosphoroxychlorid bzw. -bromid, Phosphortri- und -pentachlorid bzw. -bromid sowie elementarem Chlor und Brom.

Das Halogenierungsmittel wird in 1 bis 5 mol-äq., vorzugsweise 1 bis 2 mol.äq., eingesetzt.

Die Umsetzung verläuft bei Temperaturen von 20 °C bis zum Siedepunkt des Halogenierungsmittels bzw. sofern man in Gegenwart eines inerten organischen Lösungsmittels arbeitet, auch dessen Siedepunkt.

Als Lösungsmittel eignen sich beispielsweise Kohlenwasserstoffe wie Benzol, Toluol, o-, m- und p-Xylol, Halogenkohlenwasserstoffe wie Dichlormethan, Chloroform, 1,2-Dichlorethan, 1,1,2,2-Tetrachlorethan, Chlorbenzol und 1,2-Dichlorbenzol sowie Gemische der genannten Lösungsmittel.

Üblicherweise werden die aktivierten Carbonsäurederivate isoliert, beispielsweise durch Abdestillieren des Halogenierungsmittels und sofern vorhanden des Lösungsmittels und erst anschließend mit den Aminen VIa umgesetzt.

In diesem Fall wird die Amidierung bei Temperaturen von (-20) bis 50 °C, vorzugsweise 0 bis 30 °C in einem inerten aprotisch polaren organischen Lösungsmittel durchgeführt.

Für diese Umsetzung eignen sich insbesondere Kohlenwasserstoffe wie Benzol, Toluol, o-, m-, p-Xylol, Halogenkohlenwasserstoffe wie Dichlormethan und Ether wie Diethylether und tert.-Butylmethylether als Lösungsmittel.

Da bei der Amidierung von Säurehalogeniden Halogenwasserstoff gebildet wird, empfiehlt es sich, das Amin VIa in 2 bis 5 mol.-äq. Überschuß, vorzugsweise 2 bis 3 mol.-äq. zuzusetzen. Sofern das Amin in äquimolaren Mengen (1 bis 1,2 mol-äq.) eingesetzt wird, sollte zum Binden des Halogenwasserstoffs eine Base, insbesondere ein tertiäres Amin wie Triethylamin oder Pyridin zugegeben werden.

Sofern man von einem Gemisch der Monoester Va und Vb ausgeht, erhält man bei der Umsetzung ein Gemisch aus den isomeren Carbonsäureamiden Ia und Ib. Dieses Gemisch kann auf herkömmliche Weise, beispielsweise durch fraktionierte Kristallisation oder Chromatographie in die Einzelkomponenten aufgetrennt werden.

2. Verfahren zur Herstellung der Verbindungen Ia, in denen X Sauerstoff, $R^2$ CO-$OR^5$ und $R^5$ nicht Wasserstoff oder Methyl bedeuten:

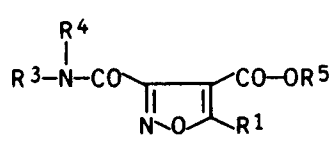

Ia ($R^5$≠H, $CH_3$)

In Analogie zu dem unter 1. geschilderten Verfahren erhält man diese Verbindungen Ia, indem man das Hydroxamsäurechlorid II in der vorstehend geschilderten Weise mit einem β-Ketoester der Formel IIIa umsetzt, den so erhaltenen Diester IVa anschließend mit einem Verseifungsreagens in den Monoester Va' spaltet und diesen aktiviert und zu Ia amidiert.

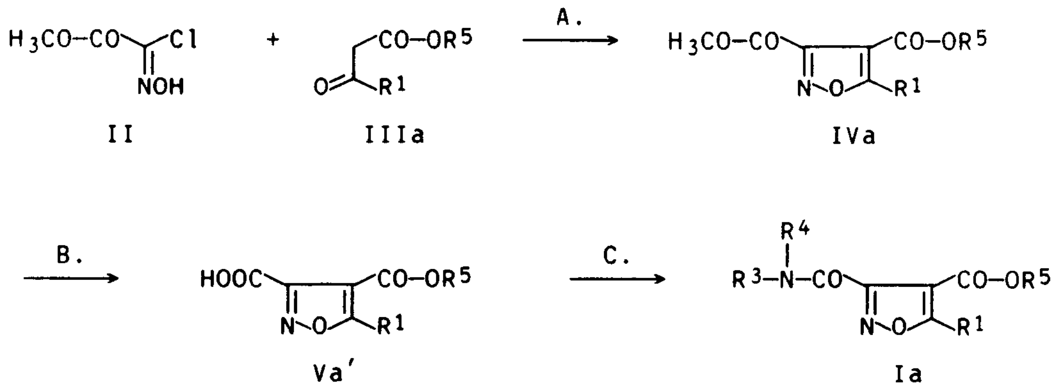


II IIIa IVa Va' Ia

Die Reaktionsschritte A und C dieser Synthesesequenz werden im allgemeinen und im besonderen entsprechend den bei Verfahren 1A und 1C geschilderten Bedingungen durchgeführt.

Reaktionsschritt B:

Die partielle Verseifung des gemischten Diesters IVa zum Monoester Va' wird üblicherweise bei Temperaturen von (-40) bis 20 ° C, vorzugsweise (-20) bis 0 ° C, in einem inerten organischen Lösungsmittel durchgeführt.

Diese Verfahren sind allgemein bekannt und können gemäß den in der Literatur beschriebenen Bedingungen durchgeführt werden.

Sofern man bei dieser Art von Synthese ein Hydroxamsäurechlorid IIa mit einem β-Ketoester III umsetzt, ist es auch möglich auf dem gleichen Weg gezielt die Verbindungen Ib herzustellen, in denen X Sauerstoff, $R^2$ $CO_2R^5$ und $R^5$ nicht Wasserstoff oder Methyl bedeuten.

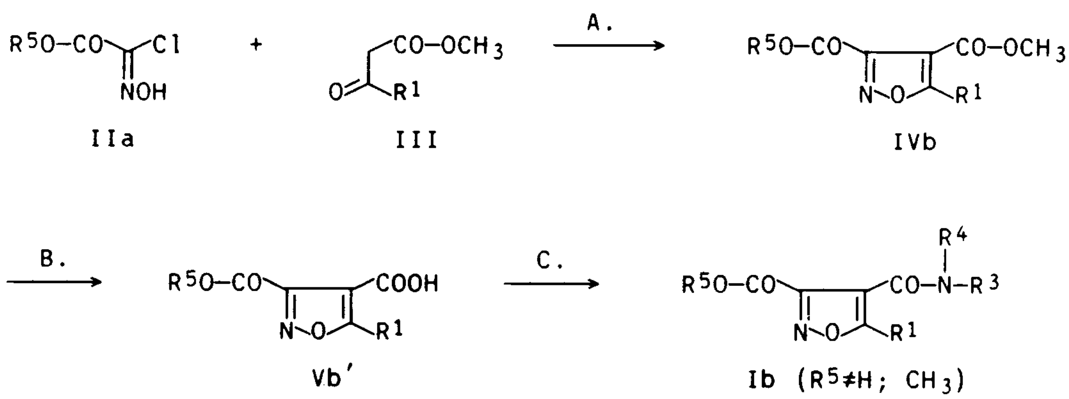


IIa III IVb Vb' Ib ($R^5 \neq H$; $CH_3$)

Die letztgenannten Verfahren zur gezielten Herstellung eines Isomeren der beiden Carbonsäureamide Ia und Ib beruhen auf der Möglichkeit bei zwei unterschiedlichen Estergruppen einer Verbindung durch die Verwendung nur eines mol-Äquivalents an Verseifungsreagens unter milden Reaktionsbedingungen selektiv die eine Estergruppe zu spalten.

Als Verseifungsreagentien eignen sich beispielsweise Hydroxide von Alkalimetall-Kationen bei unverzweigten Alkylestern, Mineralsäuren bei α-verzweigten Alkylestern und Wasserstoff bei der Hydrogenolyse von Benzyl und Allylestern.

3. Verfahren zur Herstellung der Verbindungen Ia und Ic, in denen $R^1$ nicht Wasserstoff und $R^2$ Carboxyl oder Formyl und $R^3$ Wasserstoff bedeuten:

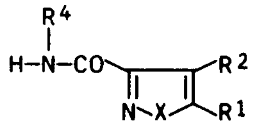

Ia ($R^1 \neq H$; $R^2 = CO_2H$, CHO)

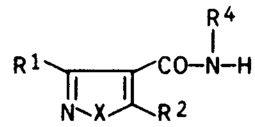

Ic ($R^1 \neq H$; $R^2 = CO_2H$, CHO)

Man erhält diese isomeren Carbonsäureamide Ia und Ic, indem man eine Carbonsäure Vc oder Vd gemäß den unter 1C geschilderten Bedingungen aktiviert und amidiert und die so erhaltenen Amide VIIa bzw. VIIb anschließend in an sich bekannter Weise in Gegenwart eines Carboxylierungsreagens oder eines Formylierungsreagens umsetzt.

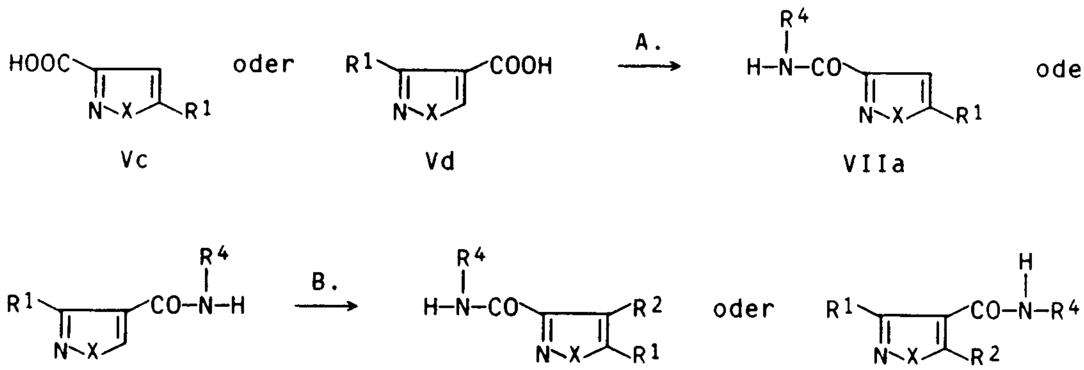


Vc oder Vd VIIa oder

VIIb Ia ($R^1 \neq H$; $R^2 = CO_2H$, CHO) oder Ic ($R^1 \neq H$; $R^2 = CO_2H$, CHO)

Der Reaktionsschritt A dieser Synthesesequenz wird im allgemeinen und im besonderen entsprechend den im Verfahren 1 unter Punkt C beschriebenen Bedingungen durchgeführt.

Reaktionsschritt B

Die Formylierung bzw. Carboxylierung der Carbonsäureamide VIIa (x = O,S) bzw. VIIb (X = S) erfolgt im allgemeinen bei Temperaturen von (-100) bis 0 °C, vorzugsweise (-80) bis (-20) °C, die Formylierung bzw. Carboxylierung der Carbonsäureamide VIIb (X = O) vorteilhaft bei Temperaturen <(-80) °C. Bevorzugt arbeitet man in einem aprotisch polaren inerten organischen Lösungsmittel unter Ausschluß von Feuchtigkeit und in Gegenwart einer Base.

Als Formylierungsreagens eignen sich insbesondere Dimethylformamid und N-Formylmorpholin, bevorzugtes Carboxylierungsmittel ist Kohlendioxid.

Geeignete Lösungsmittel sind insbesondere Diethylether, tert.-Butylmethylether, Tetrahydrofuran und Dioxan.

Als Basen finden bevorzugt Alkalimetallkohlenwasserstoffe wie Methyllithium, n-Butyllithium, tert.-Butyllithium und Phenyllithium Verwendung.

Die Umsetzung wird üblicherweise so durchgeführt, daß zunächst eine Lösung des Carbonsäureamids VIIa bzw. VIIb mit 2 bis 2,5 mol-äq. der gelösten Base versetzt wird, wobei ein im Ring metalliertes Carbonsäureamid-derivat entsteht, welches bei der anschließenden Zugabe des elektrophilen Formylierungs- bzw. Carboxylierungsreagens' zum gewünschten Produkt Ia bzw. Ic abreagiert.

Die für dieses Verfahren benötigten Carbonsäuren Vc und Vd sind literaturbekannt (Beilstein, Hauptwerk sowie 1.-5. Ergänzungswerk, Band 27; R.W. Wiley, The Chemistry of Heterocyclic Compounds, Five- and Six-Membered Compounds with Nitrogen and Oxygen, Interscience Publishers, New York, London (1962)) oder können nach allgemein literaturbekannten Methoden, z.B. durch Oxidation aus den entsprechenden Alkoholen oder Aldehyden oder durch Hydrolyse aus den entsprechenden Nitrilen, hergestellt werden.

4. Verfahren zur Herstellung von Verbindungen der Formel Id, in der $R^2$ $COOR^5$ und $R^5$ Wasserstoff oder $C_1$-$C_6$-Alkyl bedeuten, durch Hydrolyse eines Isoxazol- oder Isothiazol-4,5-dicarbonsäuredialkylesters X ($R^{5'}$ = $C_1$-$C_6$-Alkyl), Überführung des Verfahrensproduktes in ein Säurehalogenid XII und Amidierung des Säurechlorides:

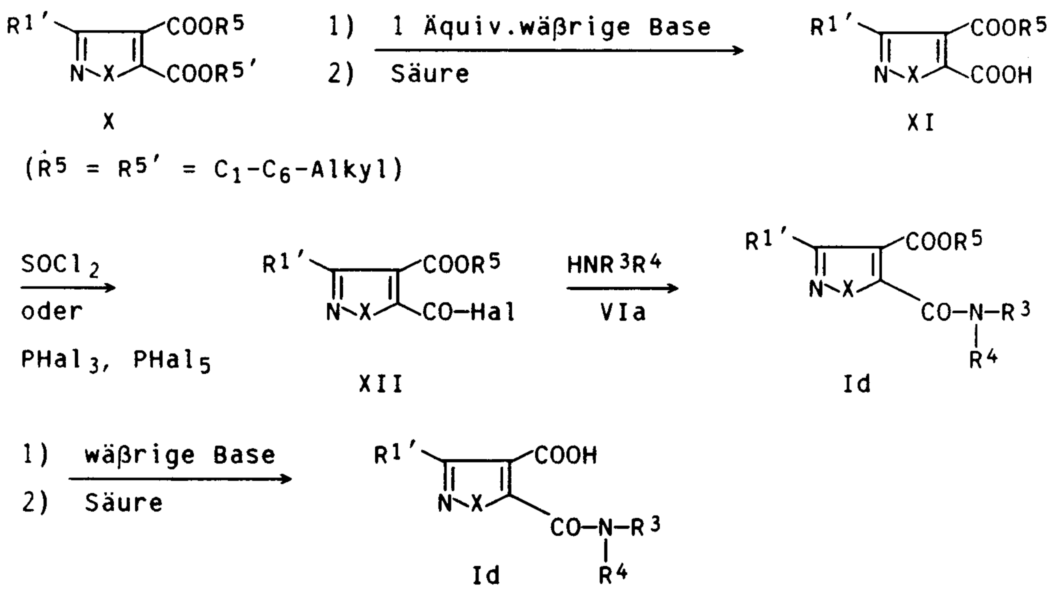


X

($R^5 = R^{5'} = C_1$-$C_6$-Alkyl)

XI

XII

Id

Id

Als Isoxazol- oder Isothiazol-4,5-dicarbonsäuredialkylester X eignen sich insbesondere Niedrigalkylester ($R^5 = R^{5'} = C_1$-$C_4$-Alkyl), wobei Dimethylester und Diethylester besonders bevorzugt sind.

Die Reaktion wird so durchgeführt, daß man einen Isoxazol- bzw. Isothiazol-4,5-dicarbonsäuredialkylester X bei Temperaturen zwischen etwa 0 und 80 °C, vorzugsweise zwischen 0 und 50 °C, in einem organischen Lösungsmittel, z.B. Methanol oder Ethanol, mit einer starken Base, z.B. NaOH, KOH oder $Ca(OH)_2$, behandelt. Im allgemeinen wird dabei etwa 1 Äquivalent der starken Base in wäßriger Lösung eingesetzt. Nach erfolgter Umsetzung wird abgekühlt und mit einer starken Mineralsäure, z.B. Salzsäure oder Schwefelsäure, angesäuert. Die entstehende Carbonsäure XI kann auf übliche Art und Weise z.B. durch Absaugen oder durch Extraktion mit einem organischen Lösungsmittel isoliert werden.

Zur Überführung der Carbonsäure XI in das Carbonsäurehalogenid XII bringt man die Säure XI in üblicher Art und Weise mit einem anorganischen Säurehalogenid wie Thionylchlorid, Phosphortri- oder Phosphorpentahalogeniden, zur Reaktion, wobei die Chloride bevorzugt sind. Dabei wird zweckmäßigerweise das anorganische Säurehalogenid in 1 bis 5 Moläquivalenten, vorzugsweise 1 bis 2 Moläquivalenten, eingesetzt. Man kann ohne Lösungsmittel oder in Gegenwart eines inerten organischen Lösungsmittels wie z.B. Benzol oder Toluol bei Temperaturen zwischen Raumtemperatur und der Siedetemperatur des anorganischen Säurehalogenids bzw. des inerten organischen Lösungsmittels arbeiten. In manchen Fällen kann der Zusatz eines Katalysators wie Dimethylformamid oder 4-Dimethylaminopyridin von Vorteil sein. Nach Beendigung der Reaktion kann das Säurehalogenids XII auf übliche Art und Weise isoliert werden, z.B. durch Abdestillation des Überschusses an anorganischem Säurehalogenid und des organischen Lösungsmittels und nachfolgende Destillation des Säurechlorids XII bei Normaldruck oder vermindertem Druck.

Die Carbonsäureamide der Formel Id, in der $R^2$ $COOR^5$ und $R^5$ $C_1$-$C_6$-Alkyl bedeuten, erhält man aus den Carbonsäurehalogeniden XII durch Umsetzung mit einem Amin VIa. Dabei geht man im allgemeinen so vor, daß man das Carbonsäurehalogenid in einem inerten organischen Lösungsmittel wie Dichlormethan, oder einem Ether wie Diethylether oder Methyl-tert.-butylether mit einem Amin VIa, ebenfalls gelöst in einem organischen Lösungsmittel, zur Reaktion bringt. Dabei setzt man das Amin VIa zweckmäßig in der 2- bis 5-fach molaren Menge, vorzugsweise 2- bis 3-fachen molaren Menge ein, um den entstehenden Halogenwasserstoff zu binden. Man kann auch in Gegenwart einer Hilfsbase wie einem tertiären Amin, z.B. Triethylamin, arbeiten. In diesem Fall genügen 1 bis 1,5 Moläquivalente Amin VIa. Die Reaktionstemperatur kann z. B. zwischen 0 und 50 °C, vorzugsweise zwischen 0 und 20 °C betragen. Die Reaktion ist im allgemeinen nach 1 bis 12 Stunden beendet. Das Gemisch kann wie üblich aufgearbeitet werden, beispielsweise durch Hydrolyse mit Wasser und anschließender Extraktion des Produktes der Formel Id ($R^2 = COOR^5$; $R^5 = C_1$-$C_6$-Alkyl) mit einem organischen Lösungsmittel und Einengen des organischen Lösungsmittels. Zur Reinigung kann das Produkt der Formel Id ($R^2 = COOR^5$; $R^5 = C_1$-$C_6$-Alkyl) beispielsweise umkristallisiert oder chromatographiert werden.

Vorteilhaft kann die Darstellung des Säureamids Id ($R^2$ = $COOR^5$, $R^5$ = $C_1$-$C_6$-Alkyl) aus der Carbonsäure XI auch in einer Stufe durchgeführt weden. Dazu wird die Carbonsäure XI mit einem Amin VIa

in Gegenwart eines wasserentziehenden Mittels, z.B. Propanphosphonsäureanhydrid (PPA) oder Dicyclohexylcarbodiimid (DCC), bei Temperaturen von 0 bis 50 °C, bevorzugt 5 bis 25 °C in einem inerten Lösungsmittel wie Dichlormethan, Tetrahydrofuran, Toluol oder Ethylacetat zur Reaktion gebracht.

Aus den 4-Alkoxycarbonyl-isoxazol-5-carbonsäureamiden bzw. 4-Alkoxycarbonyl-isothiazol-5-carbonsäureamiden Id ($R^2$ = $COOR^5$ mit $R^5$ = $C_1$-$C_6$-Alkyl) lassen sich die freien Carbonsäuren Id ($R^2$ = COOH) z.B. durch Hydrolyse mit wäßrigen Basen und anschließender Neutralisierung mit Mineralsäuren erhalten. Die Reaktion wird so durchgeführt, daß man den Ester Id ($R^2$ = $COOR^5$, $R^5$ = $C_1$-$C_6$-Alkyl) bei Temperaturen zwischen 0 und 80 °C, vorzugsweise zwischen 0 und 50 °C, in einem organischen Lösungsmittel, z.B. Methanol oder Ethanol mit einer Base, z.B. NaOH, KOH oder $Ca(OH)_2$ behandelt. Im allgemeinen werden dabei etwa 1 bis 3 Äquivalente, vorzugsweise 1 bis 1,5 Äquivalente der starken Base in wäßriger Lösung eingesetzt. Nach erfolgter Umsetzung wird unter Kühlung mit einer starken Mineralsäure, z.B. Salzsäure oder Schwefelsäure, angesäuert. Die entstehenden Carbonsäuren Id ($R^2$ = COOH) können durch Absaugen oder durch Extraktion mit einem organischen Lösungsmittel und Einengen dieses organischen Lösungsmittels isoliert werden. Die weitere Reinigung erfolgt z.B. durch Umkristallisieren oder Chromatographieren.

5. Verfahren zur Synthese von Dicarbonsäurediamiden der Formel Ic, in der $R^2 = CO\text{-}NR^3R^4$ ($R^3,R^4 \neq H$) bedeutet, durch Umsetzung der Säuren Id′ mit einem primären Amin VIa in Gegenwart eines wasserentziehenden Mittels:

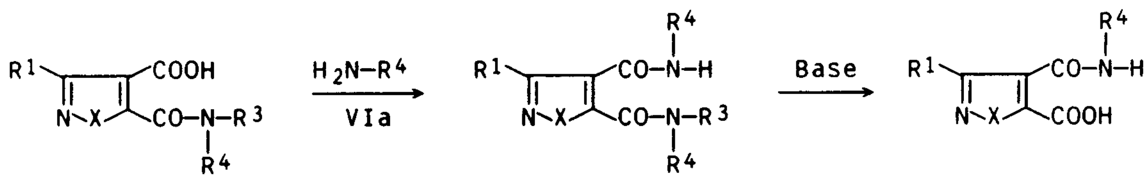


I (Id′: $R^2$=COOH) — Ic ($R^2$=CO–N$R^3R^4$) — Ic ($R^2$=COOH)

Als wasserentziehendes Mittel eignen sich z.B. Propanphosphonsäureanhydrid oder Dicyclohexylcarbodiimid. Im allgemeinen arbeitet man bei einer Temperatur zwischen (-20) und 50 °C, vorzugsweise zwischen 20 und 40 °C in einem inerten organischen Lösungsmittel, wie Dichlormethan oder einem Ether wie Diethylether oder Methyl-tert.-butylether. Die Ausgangsmaterialien werden in etwa stöchiometrischer Menge zur Reaktion gebracht. Das Gemisch kann wie üblich aufgearbeitet werden, beispielsweise durch Hydrolyse mit Wasser, Extraktion des Produkts der Formel Ic ($R^2 = CO\text{-}NR^3R^4$) mit einem organischen Lösungsmittel und Einengen dieses organischen Lösungsmittels. Zur weiteren Reinigung kann das Produkt der Formel Ic ($R^2 = CO\text{-}NR^3R^4$) beispielsweise umkristallisiert oder chromatographiert werden.

Aus den so erhaltenen Isoxazoldicarbonsäure-4,5-diamiden der Formel Im, in der X Sauerstoff und $R^2$ CO-$NR^3R^4$ ($R^3,R^4 \neq H$) bedeuten, lassen sich die Carbonsäuren Ic ($R^2$ = COOH) durch Umsetzung mit überschüssigem Kalium-tert.-butylat herstellen. Dabei geht man zweckmäßigerweise so vor, daß man das Diamid Ic ($R^2 = CO\text{-}NR^3R^4$) in einem inerten organischen Lösungsmittel wie Diethylether oder Tetrahydrofuran bei Temperaturen von 0 bis 30 °C, vorzugsweise bei Raumtemperatur, mit Kalium-tert.-butylat in Wasser (Verhältnis 3 bis 6:1) versetzt. Die Reaktion ist im allgemeinen nach 1 bis 12 Stunden beendet. Die freie Carbonsäure Ic ($R^2$ = COOH) kann nach Ansäuern mit Mineralsäure entweder durch Absaugen oder durch Extraktion mit einem organischen Lösungsmittel und Einengen dieses organischen Lösungsmittels isoliert werden. Zur weiteren Reinigung können die Säuren Ic ($R^2$ = COOH) entweder umkristallisiert oder chromatographiert werden.

Die für dieses Verfahren als Ausgangsmaterial benötigten Isothiazol-4,5-dicarbonsäureester X (X = Schwefel) sind bekannt (R. M. Paton, J. Stobie, R. M. Mortier, Phosphorus Sulfur, 15 (2), 137 (1983) oder können nach an sich bekannten Methoden hergestellt werden.

Die für dieses Verfahren als Ausgangsmaterial benötigten Isoxazol-4,5-dicarbonsäuredialkylester X (X = Sauerstoff) sind literaturbekannt [J.Org.Chem. 43, 3736 (1978); Chem.Pharm.Bull. 28, 3296 (1980); Tetrahedron 30, 1365 (1974)] oder können nach allgemein literaturbekannten Methoden hergestellt werden [vgl. z.B. DE-A 27 54 832 und Synthesis, 508 (1982)], beispielsweise aus den Aldoximen XIII und Acetylendicarbonsäurediestern XIV:

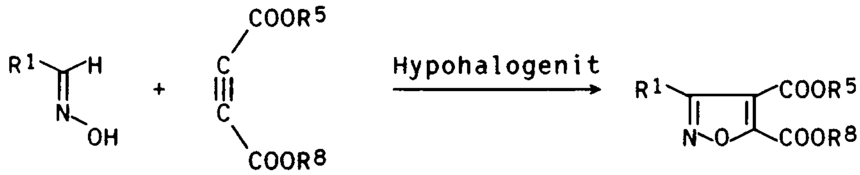


XIII XIV X

Bei diesem Verfahren wird das Aldoxim XIII im Reaktionsmedium durch das Hypohalogenit zum entsprechenden Nitriloxid oxidiert, welches ein sehr reaktiver 1,3-Dipol ist. Dieses Nitriloxid wird vom ebenfalls im Reaktionsmedium vorliegenden Acetylendicarbonsäure-diester XIV laufend, wie es entsteht, in einer 1,3-dipolaren Cycloaddition unter Bildung der Isoxazolverbindung X abgefangen.

Zweckmäßigerweise werden äquimolare Mengen des Aldoxims XIII und des Acetylendicarbonsäurediesters XIV mit dem Hypohalogenit umgesetzt. Das Hypohalogenit kann in stöchiometrischer Menge zur Reaktionsmischung gegeben werden. In der Regel wird es jedoch in leicht überschüssiger Menge, bis zu einem zweifachen Überschuß, zum Reaktionsansatz dosiert. Aus verfahrenstechnischen Gründen kann es ggf. vorteilhaft sein, den Umsatz durch Verwendung unterstöchiometrischer Mengen an Hypohalogenit - etwa 50 bis 90 mol-% Hypohalogenit pro mol XIII - zu begrenzen. Ebenso ist es möglich, mit unter- oder überstöchiometrischen Mengen der Reaktanten XIII oder XIV zu arbeiten.

Als Hypohalogenite werden im allgemeinen Hyprobromite und Hypochlorite, letztere bevorzugt, verwendet. Es können zu diesem Zweck wäßrige Lösungen der unterchlorigen oder unterbromigen Säure eingesetzt werden, vorzugsweise werden aber Alkalimetall- oder Erdalkalimetallhypochlorite oder -hypobromite, beispielsweise Natriumhypochlorit, Kaliumhypochlorit, Calciumhypochlorit, Magnesiumhypochlorit, Strontiumhypochlorit, Bariumhypochlorit oder die entsprechenden Hypobromite benutzt. Besonders bevorzugt werden Natrium-, Kalium- und Calciumhypochlorit und zwar in Form ihrer handelsüblichen, wäßrigen Lösungen angewandt.

Geeignete Lösungsmittel für das Verfahren sind z.B. Alkohole, wie Methanol, Ethanol, Propanol oder Isopropanol, Ketone wie Aceton oder Methylethylketon, Ether wie Diethylether, Methyl-tert.-butylether, Tetrahydrofuran oder Dioxan, Kohlenwasserstoffe wie Pentan, Hexan, Cyclohexan, Petrolether, Weißöle oder Ligroin, halogenierte aliphatische Kohlenwasserstoffe, wie Methylenchlorid, Chloroform, Tetrachlorkohlenstoff, Dichlorethan, Trichlorethan, Tetrachlorethan oder Perchlorethan, aromatische Verbindungen wie Benzol, Toluol, Xylole oder Chlorbenzole, Ester, wie Ethylacetat sowie Dimethylformamid, N-Methylpyrrolidon, Dimethylsulfoxid, Sulfolan usw.

Die Temperatur, bei der die Umsetzung durchgeführt wird, kann in weiten Bereichen variiert werden. In der Regel findet die Umsetzung schon bei Temperaturen von (-15) °C und tiefer statt, und nach oben wird der Temperaturbereich im Prinzip nur durch den Siedepunkt des verwendeten Lösungsmittel begrenzt, da die Umsetzung zweckmäßigerweise bei Atmosphärendruck ausgeführt wird. Vorzugsweise wird bei Temperaturen im Bereich von 0 bis 40 °C gearbeitet. Die Reaktion kann auch unter erhöhtem Druck ausgeführt werden, insbesondere unter autogen erzeugtem Druck, bevorzugt ist aber das Arbeiten bei Atmosphärendruck.

Die für dieses Verfahren benötigten Aldoxime XIII sind entweder bekannt oder können nach an sich bekannten Verfahren (z. B. Houben-Weyl, Methoden der organischen Chemie, Bd. 10/4, Seite 55 bis 56, Thieme Verlag, Stuttgart 1968) durch Umsetzung der entsprechenden Aldehyde mit Hydroxylamin, hergestellt werden. Die Aldoxime XIII können selbstverständlich sowohl in Form ihrer E- oder Z-Isomeren als auch als Gemische dieser Stereoisomeren verwendet werden. Die Acetylendicarbonsäure-diester sind im Handel erhältlich oder nach an sich bekannten Methoden (z.B. Organic Syntheses Coll. Vol. 4, Seite 329) darstellbar.

6. Verfahren zur Herstellung der Verbindungen Ia und Ib, in denen $R^2$ eine Carboxylgruppe und X Schwefel bedeuten:

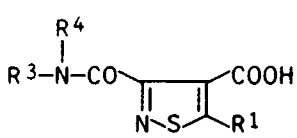

Ia (X=S; $R^2$=COOH)

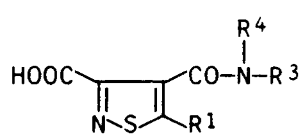

Ib (X=S; $R^2$=COOH)

Man erhält diese Carbonsäureamide Ia und Ib besonders vorteilhaft, indem man ein Isothiazoldicarbonsäureanhydrid VIII in an sich bekannter Weise mit einem Amin VIa umsetzt und das so erhaltene Gemisch der Isomeren Ia und Ib in die Einzelkomponenten trennt.

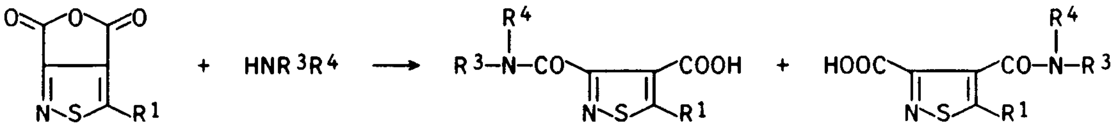

VIII VIa Ia (X=S; $R^2$=COOH) Ib (X=S; $R^2$=COOH)

Die Umsetzung wird üblicherweise bei Temperaturen von (-10) bis 50 °C, vorzugsweise 0 bis 30 °C in einem inerten aprotisch polaren organischen Lösungsmittel durchgeführt.

Insbesondere finden als Lösungsmittel Halogenkohlenwasserstoffe wie Methylenchlorid und Ether wie Diethylether, tert.-Butyl-methylether und Tetrahydrofuran Anwendung.

Das Amin VIa wird im allgemeinen in äquimolaren Mengen oder im Überschuß, vorzugsweise in Mengen von 1 bis 1,2 mol-äq. bezogen auf VIII eingesetzt.

Bei diesem Verfahren entstehen die isomeren Carbonsäureamide der Formeln Ia und Ib ($R^2$ = COOH) in unterschiedlichen Mengen. Die Auftrennung des Isomerengemischs gelingt entweder durch fraktionierte Kristallisation oder auf chromatographischem Wege.

Die für dieses Verfahren benötigten Isothiazoldicarbonsäureanhydride VIII sind bekannt oder können nach bekannten Methoden hergestellt werden (Beilstein, Hauptwerk und 1.-5. Ergänzungswerk, Band 27).

7. Verfahren zur Herstellung der Verbindungen Ia, Ib, Ic und Id, in denen $R^2$ $CO_2H$ bedeutet:

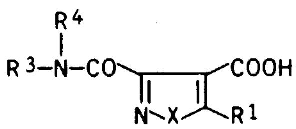

Ia

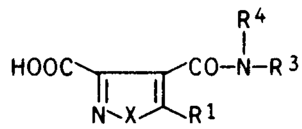

Ib

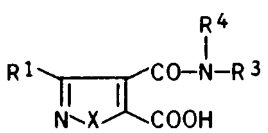

Ic

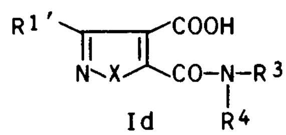

Id

Man erhält diese Verbindungen Ia, Ib, Ic und Id dadurch, daß man einen entsprechenden Ester Ia, Ib, Ic oder Id, in dem $R^2$ eine Gruppe $CO_2R^5$ und $R^5$ $C_1$-$C_4$-Alkyl bedeutet, in an sich bekannter Weise in Gegenwart einer wäßrigen Base hydrolysiert.

Diese Esterhydrolyse wird im allgemeinen und im besonderen entsprechend den bei Verfahren 2 unter Punkt 8 geschilderten Bedingungen durchgeführt.

8. Verfahren zur Herstellung von Verbindungen Ia, Ib, Ic und Id, in denen $R^2$ $COYR^5$ oder $CONR^6R^7$ bedeutet (beispielhaft für die Carbonsäureamide Ia gezeigt):

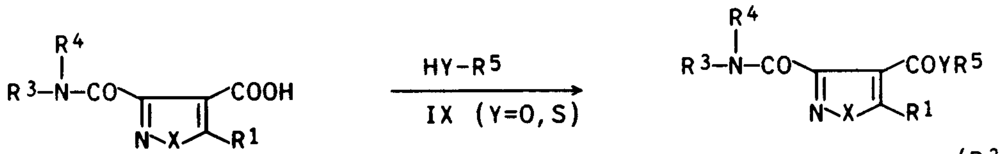


Ia

($R^2$=COYR5)

Ia ($R^2$=COOH)

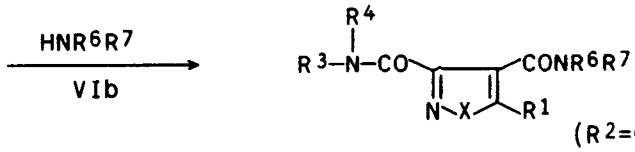


($R^2$=CONR6R7)

Zweckmäßigerweise setzt man eine Carbonsäure Ia, Ib, Ic bzw. Id ($R^2$ = COOH) mit einem Alkohol oder Thiol IX bzw. mit einem Amin VIb in Gegenwart eines wasserentziehenden Mittels, z. B. Propanphosphonsäureanhydrid (PPA) oder Dicyclohexylcarbodiimid (DCC) bei einer Temperatur zwischen (-20) und 50 ° C, vorzugsweise zwischen 0 und 40 ° C, insbesondere zwischen 20 und 30 ° C, um.

Vorteilhaft setzt man alle Ausgangsverbindungen in etwa stöchiometrischem Verhältnis ein, jedoch kann in manchen Fällen auch ein überschuß der einen oder anderen Komponente, etwa bis zu 10 mol-%, empfehlenswert sein.

Zweckmäßig verwendet man als Lösungsmittel Kohlenwasserstoffe wie Toluol und o-, m-, p-Xylol, Halogenkohlenwasserstoffe wie Dichlormethan und Ether wie Diethylether, tert.-Butyl-methylether und Tetrahydrofuran.

Eine Verfahrensvariante besteht darin, die Carbonsäuren Ia, Ib, Ic oder Id ($R^2$ = COOH) nach den für das Verfahren 1C gemachten Angaben zu aktivieren und die Verfahrensprodukte anschließend ohne Verwendung eines wasserentziehenden Mittels zu verestern oder zu amidieren.

Normalerweise arbeitet man bei Atmosphärendruck.

Die Amine VIb sind bekannt oder lassen sich nach bekannten Verfahren herstellen. Die Alkohole und Thiole HY-$R^5$ sind teilweise bekannt. Bedeutet $R^5$ eine durch einen Rest $-CR^{10}=N-R^{11}$ substituierte $C_1$-$C_6$-Alkylgruppe (Iminoalkohole XVI), so sind diese Alkohole und Thiole neu; sie lassen sich aber nach einem der folgenden bekannten Verfahren herstellen (beispielhaft für Y = O und $R^5$ = $-CH_2-CR^{10}=N-R^{11}$ mit $R^{10}$ = H und $R^{11}$ = $C_2H_5$ gezeigt):

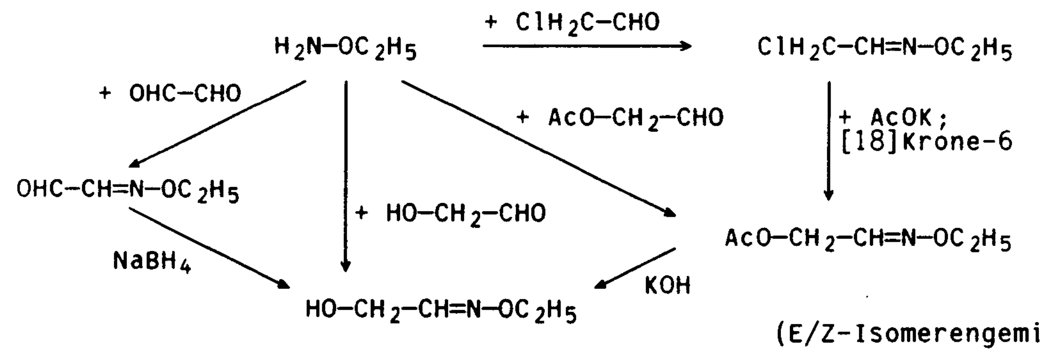


(E/Z-Isomerengemisch)

Kp (30 mbar) = 72-76°C

Ac = $CH_3CO$ $n_D^{20}$ = 1,4340

Nach den genannten Verfahren wurden z.B. die folgenden Alkohole hergestellt:

$HO-CH_2-CH=N-OCH_3$
$HO-CH_2-C(CH_3)=N-OCH_3$
$HO-CH_2-CH=N-OCH_2-CH=CHCl$
$HO-CH_2-CH=N-OCH_2-C_6H_5$
$HO-CH_2-C(CH_3)=N-OC_2H_5$
$HO-CH_2-CH=N-OCH_2-CH=CH_2$
$HO-CH_2-C(CH_3)=N-OCH_2-C_6H_5$
$HO-CH_2-C(CH_3)=N-OCH_2-CH=CH_2$

9. Verfahren zur Herstellung der Verbindungen Ia, Ib, Ic und Id, in denen $R^2$ 4,5-Dihydrooxazol-2-yl bedeutet, durch an sich bekannte Umsetzung von Carbonsäureamiden Ia, Ib, Ic bzw. Id, wobei $R^2$ COOH oder $COOR^5$ ($R^5$ = geg. subst. $C_1$-$C_4$-Alkyl wie unter den Resten $R^5$ aufgeführt) bedeutet, mit einem Aminoalkohol der Formel XV [vgl. Wehrmeister, J.Org.Chem. 26, 3821 (1961)]:

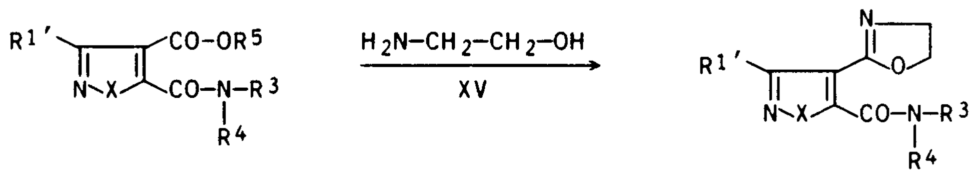


Id ($R^2$=CO-OR$^5$) Id ($R^2$=Dihydrooxazol-2-yl)

Die Reaktion wird so durchgeführt, daß man die Verbindungen bei 0 bis 180°C, vorzugsweise bei Rückflußtemperatur des verwendeten Gemisches mit einem Aminoalkohol XV, gegebenenfalls in Gegenwart eines inerten Lösungsmittels, umsetzt. Ester oder Carbonsäure und Aminoalkohol XV werden dabei im Verhältnis 1:1 bis 1:2,5, vorzugsweise 1:1 bis 1:1,5 eingesetzt.

Als Lösungsmittel verwendet man zweckmäßigerweise Halogenkohlenwasserstoffe wie Chlorbenzol und 1,2-Dichlorbenzol, Ether, z.B. Methyl-tert.-butylether, 1,2-Dimethoxyethan, Diethylenglykol-dimethylether, Tetrahydrofuran und Dioxan, Alkohole wie Methanol, Ethanol, Propanol oder Ethylenglykol, dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolin-2-on oder Aromaten, z.B. Benzol, Toluol und Xylol. Die Konzentration der Edukte im Lösungsmittel beträgt im allgemeinen 0,1 bis 5,0 mol/l, bevorzugt 0,2 bis 2,0 mol/l.

Die Umsetzung ist im allgemeinen nach 14 Stunden beendet; die Carbonsäureamide Ia, Ib, Ic bzw. Id (mit $R^2$ = 4,5-Dihydrooxazol-2-yl) werden dann gegebenenfalls durch zugabe von Wasser ausgefällt, abgesaugt oder mit einem organischen Lösungsmittel extrahiert und mit üblichen Standardmethoden wie Umkristallisation oder Chromatographie gereinigt.

Im allgemeinen arbeitet man bei Atmosphärendruck oder unter dem Eigendruck des jeweiligen Lösungsmittels.

10. Verfahren zur Herstellung der Verbindungen Ia, Ib, Ic und Id, in denen $R^2$ Formyl bedeutet:

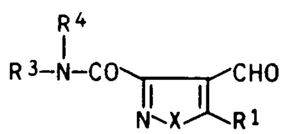

Ia

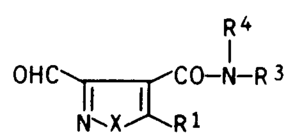

Ib

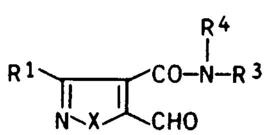

Ic

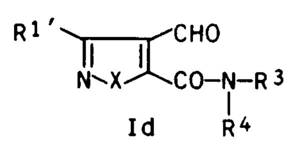

Id

Man erhält diese Verbindungen Ia, Ib, Ic und Id beispielsweise dadurch, daß man ein entsprechendes Carbonsäureamid der Formel Ia, Ib, Ic bzw. Id, wobei $R^2$ eine Gruppe $CO_2H$ bedeutet, gemäß den bei Verfahren 1 geschilderten Bedingungen aktiviert und die so erhaltene aktivierte Form der Carbonsäure in an sich bekannter Weise reduziert.

Vorzugsweise überführt man die Carbonsäure in das entsprechende Chlorid und reduziert dieses Carbonsäurechlorid bei Temperaturen von (-100) bis 0°C, insbesondere (-80) bis (-50)°C mit einem komplexen Hydrid wie insbesondere Lithium-tri-tertiärbutoxyaluminiumhydrid. Als Lösungsmittel dienen in diesem Fall besonders bevorzugt Ether wie Dimethylether, Diethylether, Tetrahydrofuran, 1,2-Dimethoxyethan, Diethylenglykoldimethylether und Dioxan (J.Am.Chem. Soc. 80, 5372 (1958); J.Am.Chem.Soc. 80, 5377 (1958)).

11. Verbindungen der Formeln Ia, Ib, Ic und Id, in denen $R^1$ bzw. $R^{1'}$ eine epoxidierte $C_2$-$C_6$-Alkenylgruppe bedeuten, die ein- bis dreimal durch Halogen, $C_1$-$C_3$-Alkoxy und/oder einmal durch Cyclopropyl oder geg. subst. Phenyl wie unter $R^1$ genannt, substituiert sein kann, erhält man beispielsweise durch Epoxidierung von Carbonsäureamiden der Formeln Ia, Ib, Ic bzw. Id, wobei $R^1$ bzw. $R^{1'}$ eine $C_2$-$C_6$-Alkenylgruppe bedeutet, die die vorstehend genannten Substituenten tragen kann, in an sich bekannter Weise mit geeigneten Oxidationsmitteln (z.B. J. March, Advanced Organic Chemistry, Third Edition, John Wiley and Sons, 1985, S. 735ff.).

Neben den vorstehend geschilderten Verfahren 1-11 zur Herstellung der Verbindungen Ia, Ib, Ic und Id gibt es weitere Synthesemöglichkeiten, die den folgenden Literaturstellen zu entnehmen sind:

Beilstein, Hauptwerk sowie 1.-5. Ergänzungswerk, Band 27;

R.W. Wiley, The Chemistry of Heterocyclic Compounds, Five- and Six-Membered Compounds with Nitrogen and Oxygen, Interscience Publishers, New York, London (1962);

A.R. Katritzky, C.W. Rees, Comphrehensive Heterocyclic Chemistry, Vol. 6, Five-membered Rings with Two or More Oxygen, Sulfur or Nitrogen Atoms, Pergamon Press, 1984;

J. March, Advanced Organic Chemistry, Third Edition, John Wiley and konus 1985;

Houben-Weyl, Methoden der organischen Chemie, 4. Auflage, Thieme Verlag, Bände IV, VI, VII, VIII, X.

Im einzelnen haben die Substituenten in den Verbindungen Ia, Ib, Im und Id die folgende Bedeutung:

$R^1$

- Wasserstoff;
- Halogen wie Fluor, Chlor, Brom, Iod, insbesondere Fluor und Chlor;
- unverzweigtes oder verzweigtes $C_1$-$C_6$-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl; 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, Propyl, 1-Methylethyl und 1,1-Dimethylethyl, welches ein bis fünf Halogenatome wie Fluor, Chlor, Brom, Iod, insbesondere Fluor und Chlor, und/oder einen Cyanorest und/oder bis zu zwei der folgenden Reste tragen kann:
  - ($C_1$-$C_4$-Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy;
  - partiell oder vollständig halogeniertes $C_1$-$C_4$-Alkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und Pentafluorethoxy;
  - $C_1$-$C_4$-Alkylthio wie Methylthio, Ethylthio, Propylthio, 1-Methylethylthio, n-Butylthio, 1-Methylpropylthio, 2-Methylpropylthio und 1,1-Dimethylethylthio, insbesondere Methylthio und Ethylthio;
  - partiell oder vollständig halogeniertes $C_1$-$C_4$-Alkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio und Pentafluorethylthio;
- eine $C_1$-$C_4$-Alkoxygruppe wie vorstehend genannt, insbesondere Methoxy und Ethoxy;
- eine partiell oder vollständig halogenierte $C_1$-$C_4$-Alkoxygruppe wie vorstehend genannt, insbesondere Trifluormethoxy, Trichlormethoxy und Pentafluorethoxy;
- eine $C_1$-$C_4$-Alkylthiogruppe wie vorstehend genannt, insbesondere Methylthio und Ethylthio;
- eine partiell oder vollständig halogenierte $C_1$-$C_4$-Alkylthiogruppe wie vorstehend genannt, insbesondere Difluormethylthio, Trifluormethylthio und Pentafluorethylthio;
- eine Benzylgruppe, die ein bis dreimal durch Alkyl mit 1 bis 4 Kohlenstoffatomen wie vorstehend genannt, insbesondere Methyl, Ethyl und 1-Methylethyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; Halogenalkylthio wie vorstehend genannt, insbesondere Difluormethylthio, Trifluormethylthio und Pentafluormethylthio; Halogen wie vorstehend genannt, insbesondere Fluor und Chlor; Cyano oder Nitro substituiert sein kann;

- die Phenylgruppe, welche noch einen bis drei der folgenden Reste tragen kann: Cyano; Nitro; Halogen wie Fluor, Chlor, Brom und Jod, insbesondere Fluor und Chlor; $C_1$-$C_6$-Alkyl wie vorstehend genannt, insbesondere Methyl, Ethyl und 1-Methylethyl; partiell oder vollständig halogeniertes $C_1$-$C_6$-Alkyl wie vorstehend genannt, insbesondere Trifluormethyl und Chlordifluormethyl; $C_1$-$C_6$-Alkoxy wie vorstehend genannt, insbesondere Methoxy und Ethoxy; partiell oder vollständig halogeniertes $C_1$-$C_6$-Alkoxy wie vorstehend genannt, insbesondere Trifluormethoxy, Trichlormethoxy und Pentafluorethoxy; $C_1$-$C_4$-Alkylthio wie vorstehend genannt, insbesondere Methylthio und Ethylthio und/oder partiell oder vollständig halogeniertes $C_1$-$C_6$-Alkylthio wie vorstehend genannt, insbesondere Difluormethylthio, Trifluormethylthio und Pentafluormethylthio;
- eine $C_3$-$C_8$-Cycloalkyl-$C_1$-$C_6$-alkylgruppe, bevorzugt eine $C_3$-$C_6$-Cycloalkyl-$C_1$-$C_4$-alkylgruppe wie Cyclopropylmethyl, Cyclopentylmethyl, Cyclohexylmethyl, 1-Cyclopropylethyl, 2-Cyclopropylethyl, 1-Cyclopropyl-1-methylethyl, 2-Cyclopropyl-1-methylethyl oder 4-Cyclohexyl-n-butyl;
- eine $C_2$-$C_6$-Alkenylgruppe, deren Doppelbindung epoxidiert sein kann, bevorzugt eine $C_2$-$C_4$-Alkenylgruppe wie Ethenyl, Prop-2-en-1-yl, 1-Methylethenyl, But-2-en-1-yl und 1-Methylprop-2-en-1-yl, welche ein- bis dreimal durch Halogen wie Fluor, Chlor, Brom oder Jod, $C_1$-$C_3$-Alkoxy wie Methoxy, Ethoxy oder Isopropoxy und/oder einmal durch Cyclopropyl oder Phenyl substituiert sein kann, wobei der Phenylrest seinerseits eine bis drei der folgenden Gruppen tragen kann: Cyano, Nitro, Alkyl wie vorstehend genannt, insbesondere Methyl, Ethyl und 1-Methylethyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; partiell oder vollständig halogeniertes Alkylthio wie vorstehend genannt, insbesondere Difluormethylthio, Trifluormethylthio und Pentafluormethylthio oder Halogen wie vorstehend genannt, insbesondere Fluor und Chlor;
- eine $C_2$-$C_6$-Alkinylgruppe, bevorzugt eine $C_2$-$C_4$-Alkinylgruppe wie Ethinyl, Propin-1-yl, 1-Methyl-2-propinyl und n-Butinyl, welche ein- bis dreimal durch Halogen wie Fluor, Chlor, Brom oder Jod, $C_1$-$C_3$-Alkoxy wie Methoxy oder Isopropoxy und/oder einmal durch Cyclopropyl oder Phenyl substituiert sein kann, wobei der Phenylrest seinerseits eine bis drei der folgenden Gruppen tragen kann: Cyano, Nitro, Alkyl wie vorstehend genannt, insbesondere Methyl, Ethyl und 1-Methylethyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; partiell oder vollständig halogeniertes Alkylthio wie vorstehend genannt, insbesondere Difluormethylthio, Trifluormethylthio und Pentafluormethylthio oder Halogen wie vorstehend genannt, insbesondere Fluor und Chlor;
- eine $C_3$-$C_8$-Cycloalkylgruppe wie Cyclopropyl, Cyclobutyl, Cyclopentyl, Cyclohexyl, Cycloheptyl und Cyclooctyl, insbesondere Cyclopropyl, Cyclopentyl und Cyclohexyl oder eine $C_3$-$C_6$-Cycloalkenylgruppe, insbesondere eine $C_5$-$C_6$-Cycloalkenylgruppe wie Cyclohexen-1-yl, wobei der Cyclus noch ein- bis dreimal durch $C_1$-$C_4$-Alkyl wie vorstehend genannt, insbesondere Methyl und Ethyl; oder Halogen wie Fluor, Chlor, Brom und Iod, insbesondere Fluor und Chlor, substituiert sein kann;
- eine Phenoxy- oder Phenylthiogruppe, wobei beide Gruppen ein- bis dreimal durch $C_1$-$C_4$-Alkyl wie vorstehend genannt, insbesondere Methyl, Ethyl und 1-Methylethyl; partiell oder vollständig halogeniertes $C_1$-$C_4$-Alkyl wie vorstehend genannt, insbesondere Trifluormethyl und Chlordifluormethyl; $C_1$-$C_4$-Alkoxy wie vorstehend genannt, insbesondere Methoxy und Ethoxy; partiell oder vollständig halogeniertes $C_1$-$C_4$-Alkoxy wie vorstehend genannt, insbesondere Trifluormethoxy, Trichlormethoxy und Pentafluorethoxy; $C_1$-$C_4$-Alkylthio wie vorstehend genannt, insbesondere Methylthio und Ethylthio; partiell oder vollständig halogeniertes $C_1$-$C_4$-Alkylthio wie vorstehend genannt, insbesondere Difluormethylthio, Trifluormethylthio und Pentafluormethylthio; Halogen wie vorstehend genannt, insbesondere Fluor und Chlor; Cyano oder Nitro; substituiert sein kann; - ein 5- bis 6-gliedriger gesättigter oder aromatischer heterocyclischer Rest, enthaltend ein oder zwei Heteroatome, ausgewählt aus

der Gruppe Sauerstoff, Schwefel und Stickstoff wie 2-Tetrahydrofuranyl, 3-Tetrahydrofuranyl, 2-Tetrahydrothienyl, 3-Tetrahydrothienyl, 2-Tetrahydropyranyl, 3-Tetrahydropyranyl, 4-Tetrahydropyranyl, 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrazolyl, 4-Pyrazolyl, 5-Pyrazolyl, 2-Pyridyl, 3-Pyridyl und 4-Pyridyl, der ein oder zwei der folgenden Substituenten tragen kann: Alkyl wie vorstehend genannt, insbesondere Methyl; Halogen wie vorstehend genannt, insbesondere Fluor und Chlor; Alkoxy wie vorstehend genannt, insbesondere Methoxy und Ethoxy; oder Alkoxycarbonyl wie Methoxycarbonyl und Ethoxycarbonyl, insbesondere Methoxycarbonyl;

$R^{1'}$

- eine $C_3$-$C_8$-Cycloalkyl-$C_1$-$C_6$-alkylgruppe, bevorzugt eine $C_3$-$C_6$-Cycloalkyl-$C_1$-$C_4$-alkylgruppe wie Cyclopropylmethyl, Cyclopentylmethyl, Cyclohexylmethyl, 1-Cyclopropylethyl, 2-Cyclopropylethyl, 1-Cyclopropyl-1-methylethyl, 2-Cyclopropyl-1-methylethyl und 4-Cyclohexyl-n-butyl;
- eine $C_2$-$C_6$-Alkenylgruppe, deren Doppelbindung epoxidiert sein kann, bevorzugt eine $C_2$-$C_4$-Alkenylgruppe wie Ethenyl, Prop-2-en-1-yl und Isopropenyl, welche ein- bis dreimal durch Halogen wie Fluor, Chlor, Brom oder Jod, $C_1$-$C_3$-Alkoxy wie Methoxy, Ethoxy oder Isopropoxy und/oder einmal durch Cyclopropyl oder Phenyl substituiert sein kann, wobei der Phenylrest seinerseits eine bis drei der folgenden Gruppen tragen kann: Cyano, Nitro, Alkyl wie vorstehend genannt, insbesondere Methyl, Ethyl und 1-Methylethyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; partiell oder vollständig halogeniertes Alkylthio wie vorstehend genannt, insbesondere Difluormethylthio, Trifluormethylthio und Pentafluormethylthio oder Halogen wie vorstehend genannt, insbesondere Fluor und Chlor;
- eine $C_2$-$C_6$-Alkinylgruppe, bevorzugt eine $C_2$-$C_4$-Alkinylgruppe wie Ethinyl, Propin-1-yl und n-Butinyl, welche ein- bis dreimal durch Halogen wie Fluor, Chlor, Brom und Jod, insbesondere Fluor und Chlor, $C_1$-$C_3$-Alkoxy wie Methoxy und Isopropoxy und/oder einmal durch Cyclopropyl oder Phenyl substituiert sein kann, wobei der Phenylrest seinerseits eine bis drei der folgenden Gruppen tragen kann: Cyano, Nitro, Alkyl wie vorstehend genannt, insbesondere Methyl, Ethyl und 1-Methylethyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; partiell oder vollständig halogeniertes Alkylthio wie vorstehend genannt, insbesondere Difluormethylthio, Trifluormethylthio und Pentafluormethylthio oder Halogen wie vorstehend genannt, insbesondere Fluor und Chlor;
- eine $C_3$-$C_6$-Cycloalkenylgruppe, bevorzugt eine $C_5$-$C_6$-Cycloalkenylgruppe, die ein- bis dreimal durch Halogen wie Fluor, Chlor, Brom und Jod, insbesondere Fluor und Chlor, oder $C_1$-$C_4$-Alkyl wie Methyl, Ethyl und tert.-Butyl substituiert sein kann;

$R^2$

- die Formylgruppe;
- die 4,5-Dihydrooxazol-2-ylgruppe;
- ein Rest $COYR^5$ oder $CONR^6R^7$ mit

$R^5$

- Wasserstoff;
- $C_1$-$C_6$-Alkyl, bevorzugt $C_1$-$C_4$-Alkyl wie Methyl, Ethyl oder tert.-Butyl, welches ein bis fünf Halogenatome wie Fluor, Chlor, Brom oder Jod, insbesondere Fluor und Chlor, und/oder bis zu drei Hydroxy- und/oder $C_1$-$C_4$-Alkoxygruppen wie Methoxy, Ethoxy, n-Propoxy, Isopropoxy, n-Butoxy und tert.-Butoxy, und/oder einen der folgenden Reste tragen kann:
  - Cyano,
  - $C_1$-$C_4$-Alkoxy-$C_2$-$C_4$-alkoxy, insbesondere Methoxy-ethoxy, Ethoxy-ethoxy und Propoxy-ethoxy,
  - $C_1$-$C_3$-Alkylthio, insbesondere Methylthio und Ethylthio,
  - $C_1$-$C_3$-Alkylamino wie Methylamino, Ethylamino und Isopropylamino,

- Di-($C_1$-$C_3$)-alkylamino wie Dimethylamino, Diethylamino, Dipropylamino, Di-(1-methylethyl)amino und Methylethylamino,
- $C_3$-$C_6$-Cycloalkylamino oder Di-($C_3$-$C_6$)-Cycloalkylamino wie Cyclopropylamino oder Dicyclopropylamino,
- Trimethylsilyl,
- $C_1$-$C_3$-Alkylsulfinyl wie Methylsulfinyl, 1-Methylethylsulfinyl und n-Propylsulfinyl,
- $C_1$-$C_3$-Alkylsulfonyl wie Methylsulfonyl, Ethylsulfonyl und Isopropylsulfonyl,
- Carboxyl,
- $C_1$-$C_3$-Alkoxycarbonyl wie Methoxycarbonyl und Isopropoxycarbonyl,
- $C_1$-$C_3$-Alkoxycarbonyl-$C_1$-$C_3$-alkoxy wie Methoxycarbonylmethoxy,
- $C_1$-$C_3$-Alkoxycarbonyl-$C_1$-$C_3$-alkoxy-$C_1$-$C_3$-alkoxycarbonyl wie Methoxycarbonylethoxymethoxycarbonyl,
- Di-($C_1$-$C_3$)-alkylaminocarbonyl wie Dimethylaminocarbonyl, Methylethylaminocarbonyl und Di-isopropylaminocarbonyl,
- Di-($C_1$-$C_3$)-alkoxyphosphonyl wie Dimethoxyphosphonyl und Diisopropoxyphosphonyl,
- $C_1$-$C_6$-Alkaniminoxy wie 2-Propaniminoxy oder $C_5$-$C_6$-Cycloalkaniminoxy wie Cyclopentaniminoxy und Cyclohexaniminoxy
- N-Phthalimido, N-Succinimido, Benzyloxy oder Benzoyl, wobei diese cyclischen Reste ein bis drei der folgenden Gruppen tragen können: Halogen wie vorstehend genannt, insbesondere Fluor und Chlor, $C_1$-$C_3$-Alkyl wie Methyl, Ethyl und Isopropyl, insbesondere Methyl, oder $C_1$-$C_3$-Alkoxy wie Methoxy, Ethoxy und Isopropoxy, insbesondere Methoxy;
- einen 5- oder 6-gliedrigen gesättigten heterocyclischen Rest oder einen 5- oder 6-gliedrigen heteroaromatischen Rest mit jeweils 1 bis 3 Heteroatomen, ausgewählt aus der Gruppe Sauerstoff, Schwefel und Stickstoff, wobei zwei Sauerstoff-oder Schwefelatome oder ein Sauerstoff und ein Schwefelatom nicht direkt benachbart sein können, insbesondere Tetrahydrofuran-2-yl, Tetrahydrofuran-3-yl, Tetrahydrothien-2-yl, Tetrahydrothien-3-yl, Tetrahydropyran-2-yl, Tetrahydropyran-3-yl, Tetrahydropyran-4-yl, Pyrrolidin-2-yl, Pyrrolidin-3-yl, Furan-2-yl, Furan-3-yl, Thien-2-yl, Thien-3-yl, Pyrrol-2-yl, Pyrrol-3-yl, Isoxazol-3-yl, Isoxazol-4-yl, Isoxazol-5-yl, Isothiazol-3-yl, Isothiazol-4-yl, Isothiazol-5-yl, Oxazol-2-yl, Oxazol-4-yl, Oxazol-5-yl, Thiazol-2-yl, Thiazol-4-yl, Thiazol-5-yl, Imidazol-2-yl, Imidazol-4-yl, Imidazol-5-yl, Pyrazol-1-yl, Pyrazol-3-yl, Pyrazol-4-yl, Pyrazol-5-yl, 1,2,3-Triazol-1-yl, 1,2,3-Triazol-4-yl, 1,2,3-Triazol-5-yl, 1,2,4-Triazol-1-yl, 1,2,4-Triazol-3-yl, 1,2,4-Triazol-5-yl, 1,2,3-Thiadiazol-4-yl, 1,2,3-Thiadiazol-5-yl, 1,2,4-Thiadiazol-3-yl, 1,2,4-Thiadiazol-5-yl, 1,2,5-Thiadiazol-3-yl, 1,2,5-Thiadiazol-4-yl, 1,3,4-Thiadiazol-2-yl, 1,3,4-Thiadiazol-5-yl, 1,2,3-Oxadiazol-3-yl, 1,2,3-Oxadiazol-5-yl, 1,2,4-Oxadiazol-3-yl, 1,2,4-Oxadiazol-5-yl, 1,2,5-Oxadiazol-3-yl, 1,2,5-Oxadiazol-4-yl, 1,3,4-Oxadiazol-2-yl, 1,3,4-Oxadiazol-5-yl, Pyrid-2-yl, Pyrid-3-yl, Pyrid-4-yl, Pyrimid-2-yl, Pyrimid-4-yl und Pyrimid-5-yl, wobei die Heterocyclen noch einen oder zwei der folgenden Substituenten tragen können: Halogen wie Fluor, Chlor, Brom und Jod, insbesondere Chlor und Brom, $C_1$-$C_3$-Alkyl wie Methyl, Ethyl, n-Propyl und iso-Propyl, $C_1$-$C_3$-Alkoxy wie Methoxy, Ethoxy, n-Propoxy und iso-Propoxy und/oder $C_1$-$C_3$-Alkoxycarbonyl wie Methoxycarbonyl, Ethoxycarbonyl und iso-Propoxycarbonyl;
- die Phenylgruppe, die noch einen bis drei der folgenden Substituenten tragen kann: Halogen wie Fluor, Chlor, Brom und Iod, insbesondere Fluor und Chlor, Nitro, Cyano, $C_1$-$C_3$-Alkyl wie Methyl oder Isopropyl, partiell oder vollständig halogeniertes $C_1$-$C_3$-Alkyl wie Trifluormethyl, 1,1,2,2-Tetrafluorethyl und Trichlormethyl, $C_1$-$C_3$-Alkoxy wie Methoxy und Isopropoxy, und/oder partiell oder vollständig halogeniertes $C_1$-$C_3$-Alkoxy, insbesondere Trifluormethoxy;
- einen Rest $-CR^{10}=N-R^{11}$ mit

$R^{10}$ Wasserstoff oder verzweigtes oder unverzweigtes $C_1$-$C_6$-Alkyl, insbesondere $C_1$-$C_4$-Alkyl wie Methyl, Ethyl, n-Propyl, iso-Propyl, n-Butyl und tert.-Butyl;

$R^{11}$ $C_1$-$C_6$-Alkoxy, $C_3$-$C_6$-Alkenyloxy oder $C_3$-$C_6$-Alkinyloxy, insbesondere $C_1$-$C_4$-Alkoxy wie Methoxy, Ethoxy, n-Propoxy, iso-Propoxy, n-Butoxy und tert.-Butoxy, sowie Prop-2-enyloxy, But-2-enyloxy, Prop-2-inyloxy und But-2-inyloxy, wobei diese Substituenten noch ein bis drei Halogenatome wie Fluor, Chlor, Brom und Iod, insbesondere Fluor und Chlor, und/oder einen Phenylrest, der unsubstituiert oder ein- bis dreifach durch Halogen wie vorstehend genannt, Nitro, Cyano, $C_1$-$C_3$-Alkyl wie Methyl, Ethyl, n-Propyl und iso-Propyl und/oder $C_1$-$C_3$-Alkoxy wie Methoxy,

Ethoxy, n-Propoxy und iso-Propoxy substituiert sein kann, tragen können; Phenoxy, das noch einen bis drei der folgenden Substituenten tragen kann: Nitro, Cyano, Halogen wie vorstehend genannt, $C_1$-$C_3$-Alkyl wie vorstehend genannt und/oder $C_1$-$C_3$-Alkyl wie vorstehend genannt; verzweigtes oder unverzweigtes $C_1$-$C_6$-Alkylamino, Di-($C_1$-$C_6$)-alkylamino oder Phenylamino, wobei der Aromat zusätzlich ein- bis dreifach durch Nitro, Cyano, Halogen wie vorstehend genannt, $C_1$-$C_3$-Alkyl wie vorstehend genannt und/oder $C_1$-$C_3$-Alkoxy wie vorstehend genannt, substituiert sein kann;

- $C_3$-$C_8$-Cycloalkyl, bevorzugt $C_3$-$C_6$-Cycloalkyl wie Cyclopropyl, Cyclopentyl und Cyclohexyl;
- $C_3$-$C_6$-Alkenyl, bevorzugt $C_3$-$C_4$-Alkenyl wie 2-Propenyl und 2-Butenyl, $C_5$-$C_6$-Cycloalkenyl wie 2-Cyclopentenyl und 2-Cyclohexenyl, $C_3$-$C_6$-Alkinyl, bevorzugt $C_3$-$C_4$-Alkenyl wie 2-Propinyl, 2-Butinyl und 3-Butinyl, wobei die 3 letztgenannten Gruppen einen der folgenden Reste tragen können: Hydroxy, Halogen wie Fluor, Chlor, Brom und Jod, $C_1$-$C_4$-Alkoxy wie Methoxy und tert.-Butoxy oder Phenyl, welches seinerseits eine bis drei der folgenden Gruppen tragen kann: Halogen wie Fluor, Chlor oder Brom, Nitro, Cyano, $C_1$-$C_4$-Alkyl wie Methyl, Ethyl oder tert.-Butyl, $C_1$-$C_4$-Halogenalkyl wie Fluormethyl, Trifluormethyl, Chlordifluormethyl, Pentafluorethyl und 2-Chlor-1,1,2-trifluorethyl oder $C_1$-$C_4$-Alkoxy wie Methoxy, Isopropoxy und tert.-Butoxy;
- Phenyl, das eine bis drei der folgenden Gruppen tragen kann:
- Halogen wie Fluor, Chlor, Brom und Jod, insbesondere Fluor und Chlor, Nitro, Cyano, $C_1$-$C_4$-Alkyl wie Methyl, Ethyl und tert.-Butyl, partiell oder vollständig halogeniertes $C_1$-$C_4$-Alkyl wie Trifluormethyl, 1,1,2,2-Tetrafluorethyl und Trichlormethyl, $C_1$-$C_4$-Alkoxy wie Methoxy, Ethoxy und Isopropoxy, partiell oder vollständig halogeniertes $C_1$-$C_4$-Alkoxy wie Trifluormethoxy, Chlordifluormethoxy, 1-Fluorethoxy, Pentafluorethoxy und 2-Chlor-1,1,2-trifluorethoxy oder $C_1$-$C_4$-Alkoxycarbonyl wie Methoxycarbonyl, n-Propoxycarbonyl und tert.-Butoxycarbonyl;
- einen 5- oder 6-gliedrigen gesättigten heterocyclischen Rest oder einen 5- oder 6-gliedrigen heteroaromatischen Rest mit jeweils 1 bis 3 Heteroatomen, ausgewählt aus der Gruppe Sauerstoff, Schwefel und Stickstoff, wobei zwei Sauerstoff-oder Schwefelatome oder ein Sauerstoff- und ein Schwefelatom nicht direkt benachbart sein können, wie vorstehend genannt, insbesondere 2-Tetrahydrofuranyl, 3-Tetrahydrothienyl, 4-Tetrahydropyranyl, 2-Furanyl, 2-Thienyl, 4-Isoxazolyl, 5-Isothiazolyl, 2-Oxazolyl, 4-Thiazolyl, 2-Imidazolyl, 2-Pyrrolyl, 3-Pyrazolyl und 4-Pyridyl, wobei die Heterocyclen noch einen oder zwei der folgenden Substituenten tragen können: Halogen wie vorstehend genannt, $C_1$-$C_3$-Alkyl wie vorstehend genannt, $C_1$-$C_3$-Alkoxy wie vorstehend genannt und/oder $C_1$-$C_3$-Alkoxycarbonyl wie vorstehend genannt;
- einen Benzotriazolrest;
- N-Phthalimido, Tetrahydrophthalimido, N-Succinimido oder Maleinimido;
- die 2,2-Dimethyl-1,3-dioxolan-4-ylmethyl- oder die 1,3-Dioxolan-2-on-4-ylmethylgruppe;
- im Falle Y = O: ein Äquivalent eines Kations aus der Gruppe der Alkali- und Erdalkalimetalle wie Natrium, Kalium und Calcium, Mangan, Kupfer, Eisen, Ammonium und mit bis zu 4 $C_1$-$C_3$-Alkylgruppen substituiertes Ammonium wie Tetramethylammonium;
- ein Rest $-N=CR^8R^9$ mit

$R^8$, $R^9$

- Wasserstoff;
- $C_1$-$C_4$-Alkyl oder partiell oder vollständig halogeniertes $C_1$-$C_4$-Alkyl wie Methyl, Ethyl, Isopropyl, tert.-Butyl, Chlormethyl, Fluormethyl, Trifluormethyl, Trichlormethyl und 1,1,2,2-Tetrafluorethyl, wobei die Alkyl-oder Halogenalkylgruppe noch einen der folgenden Reste tragen kann:
  - $C_1$-$C_3$-Alkoxy wie vorstehend genannt, insbesondere Methoxy,
  - Phenyl, das zusätzlich ein- bis dreimal durch Nitro, Cyano, Halogen wie vorstehend genannt, insbesondere Fluor und Chlor, $C_1$-$C_3$-Alkyl wie vorstehend genannt, insbesondere Methyl und tert.-Butyl, partiell oder vollständig halogeniertes $C_1$-$C_3$-Alkyl wie vorstehend genannt, insbesondere Trifluormethyl, $C_1$-$C_3$-Alkoxy wie vorstehend genannt, insbesondere Methoxy und/oder partiell oder vollständig halogeniertes $C_1$-$C_3$-Alkoxy wie vorstehend genannt, insbesondere Trifluormethoxy, substituiert sein kann;
  - $C_3$-$C_6$-Cycloalkyl wie Cyclopropyl, Cyclopentyl und Cyclohexyl;
  - $C_1$-$C_4$-Alkoxy wie vorstehend genannt, insbesondere Methoxy;
  - Furanyl oder Phenyl, das zusätzlich ein- bis dreimal durch Nitro, Cyano, Halogen wie vorstehend genannt, insbesondere Fluor und Chlor, $C_1$-$C_3$-Alkyl wie vorstehend genannt, insbesondere Methyl und tert.-Butyl, partiell oder vollständig

halogeniertes $C_1$-$C_3$-Alkyl wie vorstehend genannt, insbesondere Trifluormethyl, $C_1$-$C_3$-Alkoxy wie vorstehend genannt, insbesondere Methoxy und/oder partiell oder vollständig halogeniertes $C_1$-$C_3$-Alkoxy wie vorstehend genannt, insbesondere Trifluormethoxy, substituiert sein kann;

$R^8$ und $R^9$

- gemeinsam eine Methylenkette mit 4-7, bevorzugt 4-5 Gliedern;

- ein Rest -W-Z mit

W Ethylen-, n-Propylen- oder n-Butylenkette, Ethoxyethylenkette, But-2-enylen- oder But-2-inylenkette;

Z einen in ω-Stellung an W gebundenen Molekülteil, der den gleichen Molekülteil darstellt, der in α-Stellung von W mit W verknüpft ist, beispielsweise

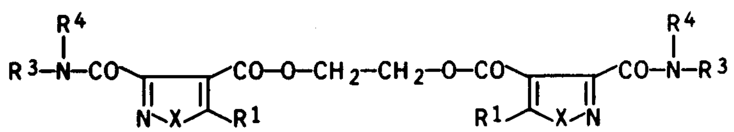

$R^6$

- Wasserstoff;
- $C_1$-$C_6$-Alkyl, bevorzugt $C_1$-$C_4$-Alkyl wie Methyl, Ethyl, Isopropyl und tert.-Butyl;
- $C_3$-$C_8$-Cycloalkyl, bevorzugt $C_3$-$C_6$-Cycloalkyl wie Cyclopropyl, Cyclopentyl und Cyclohexyl;

$R^7$

- Wasserstoff;
- $C_1$-$C_6$-Alkyl, bevorzugt $C_1$-$C_4$-Alkyl wie Methyl und tert.-Butyl;
- ein Rest $-C(OR^{12})=N-H$ oder $-C(OR^{12})=N-(C_1-C_4)$-alkyl, wobei $C_1$-$C_4$-alkyl eine Alkylgruppe wie vorstehend genannt, insbesondere Methyl, Ethyl und tert.-Butyl und $R^{12}$ ebenfalls eine $C_1$-$C_4$-Alkylgruppe wie vorstehend genannt, insbesondere Methyl, bedeutet;

$R^6$, $R^7$

- gemeinsam eine Methylenkette mit 4 bis 7, bevorzugt 4 bis 5 Gliedern;

$R^3$

- Wasserstoff
- $C_1$-$C_6$-Alkyl, bevorzugt $C_1$-$C_4$-Alkyl wie Methyl, Ethyl und tert.-Butyl, das einen bis drei der folgenden Substituenten tragen kann: Hydroxy, Halogen, $C_1$-$C_4$-Alkoxy wie Methoxy und tert.-Butoxy, $C_1$-$C_4$-Alkylthio wie Methylthio und tert.-Butylthio oder Di-($C_1$-$C_4$)-alkylamino, bevorzugt Di-($C_1$-$C_2$)-alkylamino wie Dimethylamino und Diethylamino;
- $C_3$-$C_8$-Cycloalkyl, bevorzugt $C_3$-$C_6$-Cycloalkyl wie Cyclopropyl, Cyclopentyl und Cyclohexyl, das ein- bis dreimal durch Halogen wie Fluor, Chlor und Brom, $C_1$-$C_4$-Alkyl wie Methyl und tert.-Butyl oder partiell oder vollständig halogeniertes $C_1$-$C_4$-Alkyl wie Fluormethyl, Trifluormethyl, Chlordifluormethyl, Pentafluorethyl und 2-Chlor-1,1,2-trifluorethyl substituiert sein kann;

$R^4$

- Wasserstoff, Hydroxyl;
- $C_1$-$C_4$-Alkoxy wie Methoxy, Ethoxy und tert.-Butoxy;
- verzweigtes oder unverzweigtes $C_1$-$C_6$-Alkyl, bevorzugt $C_1$-$C_4$-Alkyl wie Methyl, Ethyl, Isopropyl und tert.-Butyl, welches ein bis drei der folgenden Reste tragen kann: Halogen wie Fluor, Chlor und Brom, Cyano, $C_1$-$C_4$-Alkoxy wie Methoxy und tert.-Butoxy, partiell oder vollständig halogeniertes $C_1$-$C_4$-Alkoxy wie Fluormethoxy, Trichlormethoxy, 2-Chlor-1,1,2-trifluorethoxy und Pentafluorethoxy, $C_1$-$C_4$-Alkylthio wie Methylthio und tert.-Butylthio, $C_1$-$C_4$-Halogenalkylthio wie Fluromethylthio, Trichlormethylthio, 2-Chlor-1,1,2-trifluorethylthio und Pentafluorethylthio, Di-($C_1$-$C_4$)-alkylamino, insbesondere Di-($C_1$-$C_2$)-alkylamino wie Dimethylamino und Diethylamino, $C_3$-$C_8$-Cycloalkyl, insbesondere $C_3$-$C_6$-Cycloalkyl wie Cyclopropyl, Cyclopentyl und Cyclohexyl oder Phenyl, wobei der Phenylrest seinerseits bis zu drei der folgenden Gruppen tragen kann: Halogen wie Fluor, Chlor und Brom, Cyano, Nitro, $C_1$-$C_4$-Alkyl wie Methyl und tert.-Butyl, $C_1$-$C_4$-

Halogenalkyl wie Fluormethyl, Trichlormethyl, 2-Chlor-1,1,2-trifluorethyl und Pentafluorethyl, $C_1$-$C_4$-Alkoxy wie Methoxy und tert.-Butoxy, partiell oder vollständig halogeniertes $C_1$-$C_4$-Alkoxy wie Fluormethoxy, Trichlormethoxy, 2-Chlor-1,1,2-trifluorethoxy und Pentafluorethoxy, $C_1$-$C_4$-Alkylthio wie Methylthio und tert.-Butylthio oder $C_1$-$C_4$-Halogenalkylthio wie Fluormethylthio, Trichlormethylthio, 2-Chlor-1,1,2-trifluorethylthio und Pentafluorethylthio;

- $C_3$-$C_8$-Cycloalkyl, bevorzugt $C_3$-$C_6$-Cycloalkyl, insbesondere Cyclopropyl, Cyclopentyl und Cyclohexyl, das jeweils einen bis drei der folgenden Reste tragen kann: Halogen wie Fluor, Chlor und Brom, Nitro, Cyano, $C_1$-$C_6$-Alkyl, bevorzugt $C_1$-$C_4$-Alkyl wie Methyl und tert.-Butyl, partiell oder vollständig halogeniertes $C_1$-$C_6$-Alkyl, bevorzugt $C_1$-$C_4$-Halogenalkyl wie Fluormethyl, Trichlormethyl, 2-Chlor-1,1,2-trifluorethyl und Pentafluorethyl, $C_1$-$C_4$-Alkoxy wie Methoxy und tert.-Butoxy, oder partiell oder vollständig halogeniertes $C_1$-$C_4$-Alkoxy wie Fluormethoxy, Trichlormethoxy, 2-Chlor-1,1,2-trifluorethoxy und Pentafluorethoxy;
- $C_3$-$C_6$-Alkenyl oder $C_3$-$C_6$-Alkinyl, bevorzugt $C_3$-$C_4$-Alkenyl oder $C_3$-$C_4$-Alkinyl wie 2-Propenyl, 2-Butenyl, 2-Propinyl, 1,1-Dimethyl-2-propinyl und 3-Butinyl, welches jeweils bis zu dreifach durch Halogen wie Fluor, Chlor oder Brom und/oder einmal durch Phenyl substituiert sein kann, wobei der Phenylrest seinerseits eine bis drei der folgenden Substituenten tragen kann: Halogen, insbesondere Fluor und Chlor, Cyano, Nitro, $C_1$-$C_4$-Alkyl wie Methyl und tert.-Butyl, partiell oder vollständig halogeniertes $C_1$-$C_4$-Alkyl wie Fluormethyl, Trifluormethyl, Trichlormethyl, 2-Chlor-1,1,2-trifluorethyl und Pentafluorethyl, $C_1$-$C_4$-Alkoxy wie Methoxy und tert.-Butoxy, $C_1$-$C_4$-Halogenalkoxy wie Fluormethoxy, Trifluormethoxy, Trichlormethoxy, 2-Chlor-1,1,2-trifluorethoxy und Pentafluorethoxy, $C_1$-$C_4$-Alkylthio wie Methylthio und tert.-Butylthio, partiell oder vollständig halogeniertes $C_1$-$C_4$-Alkylthio wie Fluormethylthio, Trifluormethylthio, Trichlormethylthio, 2-Chlor-1,1,2-trifluorethylthio und Pentafluorethylthio;
- Di($C_1$-$C_4$)-alkylamino, bevorzugt Di-($C_1$-$C_2$)-alkylamino wie Dimethylamino und Diethylamino;
- ein 5- bis 6-gliedriger gesättigter oder aromatischer heterocyclischer Rest, enthaltend ein oder zwei Heteroatome, ausgewählt aus der Gruppe Sauerstoff, Schwefel und Stickstoff wie 2-Tetrahydrofuranyl, 3-Tetrahydrofuranyl, 2-Tetrahydrothienyl, 3-Tetrahydrothienyl, 2-Tetrahydropyranyl, 3-Tetrahydropyranyl, 4-Tetrahydropyranyl, 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrazolyl, 4-Pyrazolyl, 5-Pyrazolyl, 2-Pyridyl, 3-Pyridyl und 4-Pyridyl, der einen bis drei der folgenden Substituenten tragen kann: $C_1$-$C_4$-Alkyl wie vorstehend genannt, insbesondere Methyl oder Halogen wie vorstehend genannt, insbesondere Fluor und Chlor;
- Phenyl, welches eine bis vier der folgenden Gruppen tragen kann: $C_1$-$C_4$-Alkyl wie vorstehend genannt, insbesondere Methyl, Ethyl und 1-Methylethyl; partiell oder vollständig halogeniertes $C_1$-$C_4$-Alkyl wie vorstehend genannt, insbesondere Trifluormethyl und Chlordifluormethyl; $C_1$-$C_4$-Alkoxy wie vorstehend genannt, insbesondere Methoxy und Ethoxy; partiell oder vollständig halogeniertes $C_1$-$C_4$-Alkoxy wie vorstehend genannt, insbesondere Trifluormethoxy, Trichlormethoxy und Pentafluorethoxy; $C_1$-$C_4$-Alkylthio wie vorstehend genannt, insbesondere Methylthio und Ethylthio; partiell oder vollständig halogeniertes $C_1$-$C_4$-Alkylthio wie vorstehend genannt, insbesondere Difluormethylthio, Trifluormethylthio und Pentafluormethylthio, Halogen wie vorstehend genannt, insbesondere Fluor und Chlor, Cyano, Nitro, Formyl, $C_1$-$C_4$-Alkanoyl wie Acetyl, Propionyl, Butyryl, insbesondere Acetyl, partiell oder vollständig halogeniertes $C_1$-$C_4$-Alkanoyl wie Trifluoracetyl, Trichloracetyl, Pentafluorpropionyl, insbesondere Trifluoracetyl oder $C_1$-$C_4$-Alkoxycarbonyl wie Methoxycarbonyl und tert.-Butoxycarbonyl;
- Naphthyl, das ein- bis dreimal durch $C_1$-$C_4$-Alkyl wie Methyl und tert.-Butyl, insbesondere Methyl und Ethyl, oder Halogen wie Fluor und Chlor substituiert sein kann;
- $R^3$ und $R^4$ gemeinsam eine $C_4$-$C_7$-Methylenkette, welche durch Sauerstoff, Schwefel oder N-Methyl unterbrochen sein kann wie $-(CH_2)_3-$, $-(CH_2)_4-$, $-(CH_2)_5-$, $-(CH_2)_6-$, $-CH_2-O-CH_2-$, $-CH_2-CH_2-O-CH_2-CH_2-$, $-CH_2-S-CH_2-$, $-CH_2-CH_2-S-CH_2-CH_2-$, $-CH_2-CH_2-N(CH_3)-CH_2-CH_2-$, insbesondere $-(CH_2)_5-$ und $-CH_2-CH_2-O-CH_2-CH_2-$;
- oder den Rest der Formel $-(CH_2)_3-CO-$.

Bevorzugt werden Verbindungen der Formeln Ia bis Id, wobei $R^2$ einen Rest CO-Y-$R^5$ und Y Sauerstoff oder Schwefel bedeutet:

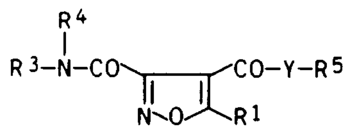

Ia (X = O)

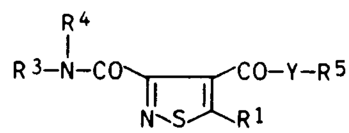

Ia (X = S)

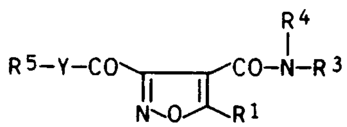

Ib (X = O)

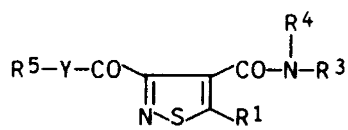

Ib (X = S)

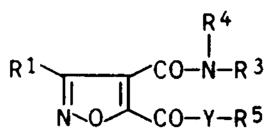

Ic (X = O)

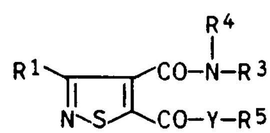

Ic (X = S)

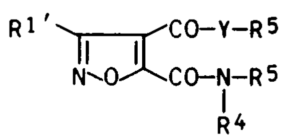

Id (X = O)

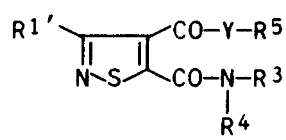

Id (X = S)

In Tabelle 1 sind beispielhaft mögliche Substituenten $R^1$, $R^3$, $R^4$ und $R^5$ der bevorzugten Verbindungen Ia-Ic ($R^2$ = CO-Y-$R^5$), in Tabelle 2 mögliche Substituenten der Verbindungen Id ($R^2$ = CO-Y-$R^5$), aufgeführt, wobei die Carbonsäureamide Ia ($R^2$ = CO-Y-$R^5$) besonders bevorzugt sind.

Tabelle 1

| $R^1$ | $R^3$ | $R^4$ | $R^5$ | Y |
|---|---|---|---|---|
| H | H | tert.-Butyl | H | O |
| F | H | tert.-Butyl | H | O |
| Cl | H | tert.-Butyl | H | O |
| Methyl | H | tert.-Butyl | H | O |
| Ethyl | H | tert.-Butyl | H | O |
| n-Propyl | H | tert.-Butyl | H | O |
| iso-Propyl | H | tert.-Butyl | H | O |
| n-Butyl | H | tert.-Butyl | H | O |
| iso-Butyl | H | tert.-Butyl | H | O |
| sek.-Butyl | H | tert.-Butyl | H | O |
| tert.-Butyl | H | tert.-Butyl | H | O |
| cyclo-Propyl | H | tert.-Butyl | H | O |
| cyclo-Butyl | H | tert.-Butyl | H | O |
| cyclo-Pentyl | H | tert.-Butyl | H | O |
| cyclo-Hexyl | H | tert.-Butyl | H | O |
| cyclo-Heptyl | H | tert.-Butyl | H | O |
| cyclo-Octyl | H | tert.-Butyl | H | O |
| 1-Methylcyclopropyl | H | tert.-Butyl | H | O |
| Chlormethyl | H | tert.-Butyl | H | O |
| 1-Chlorethyl | H | tert.-Butyl | H | O |
| Trifluormethyl | H | tert.-Butyl | H | O |
| Chlordifluormethyl | H | tert.-Butyl | H | O |
| Pentafluorethyl | H | tert.-Butyl | H | O |
| Methoxymethyl | H | tert.-Butyl | H | O |
| 1-Methylmethoxymethyl | H | tert.-Butyl | H | O |
| 1-Methylmethoxyethyl | H | tert.-Butyl | H | O |
| Ethoxymethyl | H | tert.-Butyl | H | O |
| Vinyl | H | tert.-Butyl | H | O |
| Allyl | H | tert.-Butyl | H | O |
| Methallyl | H | tert.-Butyl | H | O |
| Crotyl | H | tert.-Butyl | H | O |
| Ethinyl | H | tert.-Butyl | H | O |
| Propargyl | H | tert.-Butyl | H | O |
| Phenylethinyl | H | tert.-Butyl | H | O |
| Methoxy | H | tert.-Butyl | H | O |
| Ethoxy | H | tert.-Butyl | H | O |
| Trifluormethoxy | H | tert.-Butyl | H | O |
| Methylthio | H | tert.-Butyl | H | O |
| Trifluormethylthio | H | tert.-Butyl | H | O |

| $R^1$ | $R^3$ | $R^4$ | $R^5$ | Y |
|---|---|---|---|---|
| Phenoxy | H | tert.-Butyl | H | O |
| 4-Cl-Phenoxy | H | tert.-Butyl | H | O |
| 2,4-(Cl,Cl)-Phenoxy | H | tert.-Butyl | H | O |
| 4-$CF_3$-Phenoxy | H | tert.-Butyl | H | O |
| Phenyl | H | tert.-Butyl | H | O |
| 2-F-Phenylthio | H | tert.-Butyl | H | O |
| 3-F-Phenyl | H | tert.-Butyl | H | O |
| 2,4-(F,F)-Phenyl | H | tert.-Butyl | H | O |
| 2-Cl-Phenyl | H | tert.-Butyl | H | O |
| 3-Cl-Phenyl | H | tert.-Butyl | H | O |
| 2,4-(Cl,Cl)-Phenyl | H | tert.-Butyl | H | O |
| 2-$CH_3$-Phenyl | H | tert.-Butyl | H | O |
| 3-$CH_3$-Phenyl | H | tert.-Butyl | H | O |
| 4-$CH_3$-Phenyl | H | tert.-Butyl | H | O |
| 2,4-($CH_3$,$CH_3$)-Phenyl | H | tert.-Butyl | H | O |
| 2,4,6-($CH_3$,$CH_3$,$CH_3$)-Phenyl | H | tert.-Butyl | H | O |
| 2-$CF_3$-Phenyl | H | tert.-Butyl | H | O |
| 2-$OCH_3$-Phenyl | H | tert.-Butyl | H | O |
| 2,4-($OCH_3$,$OCH_3$)-Phenyl | H | tert.-Butyl | H | O |
| 4-$OCF_3$-Phenyl | H | tert.-Butyl | H | O |
| 4-$SCH_3$-Phenyl | H | tert.-Butyl | H | O |
| 3-$SCF_3$-Phenyl | H | tert.-Butyl | H | O |
| 2,4-($NO_2$,$NO_2$)-Phenyl | H | tert.-Butyl | H | O |
| 4-$NO_2$-Phenyl | H | tert.-Butyl | H | O |
| 2-Thienyl | H | tert.-Butyl | H | O |
| 3-Thienyl | H | tert.-Butyl | H | O |
| 2-Furanyl | H | tert.-Butyl | H | O |
| 3-Furanyl | H | tert.-Butyl | H | O |
| 2-Tetrahydrofuranyl | H | tert.-Butyl | H | O |
| 3-Tetrahydrofuranyl | H | tert.-Butyl | H | O |
| 2-Pyridyl | H | tert.-Butyl | H | O |
| 3-Pyridyl | H | tert.-Butyl | H | O |
| 4-Pyridyl | H | tert.-Butyl | H | O |
| 2-Tetrahydropyranyl | H | tert.-Butyl | H | O |
| 3-Tetrahydropyranyl | H | tert.-Butyl | H | O |
| 4-Tetrahydropyranyl | H | tert.-Butyl | H | O |
| iso-Propoxy | H | tert.-Butyl | H | O |
| H | H | cyclo-Propyl | H | O |
| F | H | cyclo-Propyl | H | O |
| Cl | H | cyclo-Propyl | H | O |
| Methyl | H | cyclo-Propyl | H | O |
| Ethyl | H | cyclo-Propyl | H | O |

| $R^1$ | $R^3$ | $R^4$ | $R^5$ | Y |
|---|---|---|---|---|
| n-Propyl | H | cyclo-Propyl | H | O |
| iso-Propyl | H | cyclo-Propyl | H | O |
| n-Butyl | H | cyclo-Propyl | H | O |
| iso-Butyl | H | cyclo-Propyl | H | O |
| sek.-Butyl | H | cyclo-Propyl | H | O |
| tert.-Butyl | H | cyclo-Propyl | H | O |
| cyclo-Propyl | H | cyclo-Propyl | H | O |
| cyclo-Butyl | H | cyclo-Propyl | H | O |
| cyclo-Pentyl | H | cyclo-Propyl | H | O |
| cyclo-Hexyl | H | cyclo-Propyl | H | O |
| cyclo-Heptyl | H | cyclo-Propyl | H | O |
| cyclo-Octyl | H | cyclo-Propyl | H | O |
| 1-Methylcyclopropyl | H | cyclo-Propyl | H | O |
| Chlormethyl | H | cyclo-Propyl | H | O |
| 1-Chlorethyl | H | cyclo-Propyl | H | O |
| Trifluormethyl | H | cyclo-Propyl | H | O |
| Chlordifluormethyl | H | cyclo-Propyl | H | O |
| Pentafluorethyl | H | cyclo-Propyl | H | O |
| Methoxymethyl | H | cyclo-Propyl | H | O |
| 1-Methylmethoxymethyl | H | cyclo-Propyl | H | O |
| 1-Methylmethoxyethyl | H | cyclo-Propyl | H | O |
| Ethoxymethyl | H | cyclo-Propyl | H | O |
| Vinyl | H | cyclo-Propyl | H | O |
| Allyl | H | cyclo-Propyl | H | O |
| Methallyl | H | cyclo-Propyl | H | O |
| Crotyl | H | cyclo-Propyl | H | O |
| Ethinyl | H | cyclo-Propyl | H | O |
| Propargyl | H | cyclo-Propyl | H | O |
| Phenylethinyl | H | cyclo-Propyl | H | O |
| Methoxy | H | cyclo-Propyl | H | O |
| Ethoxy | H | cyclo-Propyl | H | O |
| Trifluormethoxy | H | cyclo-Propyl | H | O |
| Methylthio | H | cyclo-Propyl | H | O |
| Trifluormethylthio | H | cyclo-Propyl | H | O |
| Phenoxy | H | cyclo-Propyl | H | O |
| 4-Cl-Phenoxy | H | cyclo-Propyl | H | O |
| 2,4-(Cl,Cl)-Phenoxy | H | cyclo-Propyl | H | O |
| 4-$CF_3$-Phenoxy | H | cyclo-Propyl | H | O |
| Phenyl | H | cyclo-Propyl | H | O |
| 2-F-Phenylthio | H | cyclo-Propyl | H | O |
| 3-F-Phenyl | H | cyclo-Propyl | H | O |
| 2,4-(F,F)-Phenyl | H | cyclo-Propyl | H | O |

| $R^1$ | $R^3$ | $R^4$ | $R^5$ | Y |
|---|---|---|---|---|
| 2-Cl-Phenyl | H | cyclo-Propyl | H | O |
| 3-Cl-Phenyl | H | cyclo-Propyl | H | O |
| 2,4-(Cl,Cl)-Phenyl | H | cyclo-Propyl | H | O |
| 2-$CH_3$-Phenyl | H | cyclo-Propyl | H | O |
| 3-$CH_3$-Phenyl | H | cyclo-Propyl | H | O |
| 4-$CH_3$-Phenyl | H | cyclo-Propyl | H | O |
| 2,4-($CH_3$,$CH_3$)-Phenyl | H | cyclo-Propyl | H | O |
| 2,4,6-($CH_3$,$CH_3$,$CH_3$)-Phenyl | H | cyclo-Propyl | H | O |
| 2-$CF_3$-Phenyl | H | cyclo-Propyl | H | O |
| 2-$OCH_3$-Phenyl | H | cyclo-Propyl | H | O |
| 2,4-($OCH_3$,$OCH_3$)-Phenyl | H | cyclo-Propyl | H | O |
| 4-$OCF_3$-Phenyl | H | cyclo-Propyl | H | O |
| 4-$SCH_3$-Phenyl | H | cyclo-Propyl | H | O |
| 3-$SCF_3$-Phenyl | H | cyclo-Propyl | H | O |
| 2,4-($NO_2$,$NO_2$)-Phenyl | H | cyclo-Propyl | H | O |
| 4-$NO_2$-Phenyl | H | cyclo-Propyl | H | O |
| 2-Thienyl | H | cyclo-Propyl | H | O |
| 3-Thienyl | H | cyclo-Propyl | H | O |
| 2-Furanyl | H | cyclo-Propyl | H | O |
| 3-Furanyl | H | cyclo-Propyl | H | O |
| 2-Tetrahydrofuranyl | H | cyclo-Propyl | H | O |
| 3-Tetrahydrofuranyl | H | cyclo-Propyl | H | O |
| 2-Pyridyl | H | cyclo-Propyl | H | O |
| 3-Pyridyl | H | cyclo-Propyl | H | O |
| 4-Pyridyl | H | cyclo-Propyl | H | O |
| 2-Tetrahydropyranyl | H | cyclo-Propyl | H | O |
| 3-Tetrahydropyranyl | H | cyclo-Propyl | H | O |
| 4-Tetrahydropyranyl | H | cyclo-Propyl | H | O |
| iso-Propoxy | H | cyclo-Propyl | H | O |
| H | Methyl | tert.-Butyl | H | O |
| F | Methyl | tert.-Butyl | H | O |
| Cl | Methyl | tert.-Butyl | H | O |
| Methyl | Methyl | tert.-Butyl | H | O |
| Ethyl | Methyl | tert.-Butyl | H | O |
| n-Propyl | Methyl | tert.-Butyl | H | O |
| iso-Propyl | Methyl | tert.-Butyl | H | O |
| n-Butyl | Methyl | tert.-Butyl | H | O |
| iso-Butyl | Methyl | tert.-Butyl | H | O |
| sek.-Butyl | Methyl | tert.-Butyl | H | O |
| tert.-Butyl | Methyl | tert.-Butyl | H | O |
| cyclo-Propyl | Methyl | tert.-Butyl | H | O |
| cyclo-Butyl | Methyl | tert.-Butyl | H | O |
| cyclo-Pentyl | Methyl | tert.-Butyl | H | O |

| $R^1$ | $R^3$ | $R^4$ | $R^5$ | Y |
|---|---|---|---|---|
| cyclo-Hexyl | iso-Propyl | tert.-Butyl | H | O |
| cyclo-Heptyl | iso-Propyl | tert.-Butyl | H | O |
| cyclo-Octyl | iso-Propyl | tert.-Butyl | H | O |
| 1-Methylcyclopropyl | iso-Propyl | tert.-Butyl | H | O |
| Trifluormethyl | iso-Propyl | tert.-Butyl | H | O |
| Chlordifluormethyl | iso-Propyl | tert.-Butyl | H | O |
| Pentafluorethyl | iso-Propyl | tert.-Butyl | H | O |
| Methoxymethyl | iso-Propyl | tert.-Butyl | H | O |
| 1-Methylmethoxymethyl | iso-Propyl | tert.-Butyl | H | O |
| 1-Methylmethoxyethyl | iso-Propyl | tert.-Butyl | H | O |
| Ethoxymethyl | iso-Propyl | tert.-Butyl | H | O |
| Vinyl | iso-Propyl | tert.-Butyl | H | O |
| Allyl | iso-Propyl | tert.-Butyl | H | O |
| Methallyl | iso-Propyl | tert.-Butyl | H | O |
| Crotyl | iso-Propyl | tert.-Butyl | H | O |
| Ethinyl | iso-Propyl | tert.-Butyl | H | O |
| Propargyl | iso-Propyl | tert.-Butyl | H | O |
| Phenylethinyl | iso-Propyl | tert.-Butyl | H | O |
| Methoxy | iso-Propyl | tert.-Butyl | H | O |
| Ethoxy | iso-Propyl | tert.-Butyl | H | O |
| Trifluormethoxy | iso-Propyl | tert.-Butyl | H | O |
| H | Methyl | cyclo-Propyl | H | O |
| F | Methyl | cyclo-Propyl | H | O |
| Cl | Methyl | cyclo-Propyl | H | O |
| Methyl | Methyl | cyclo-Propyl | H | O |
| Ethyl | Methyl | cyclo-Propyl | H | O |
| n-Propyl | Methyl | cyclo-Propyl | H | O |
| iso-Propyl | Methyl | cyclo-Propyl | H | O |
| n-Butyl | iso-Propyl | cyclo-Propyl | H | O |
| iso-Butyl | iso-Propyl | cyclo-Propyl | H | O |
| sek.-Butyl | iso-Propyl | cyclo-Propyl | H | O |
| tert.-Butyl | iso-Propyl | cyclo-Propyl | H | O |
| cyclo-Propyl | iso-Propyl | cyclo-Propyl | H | O |
| cyclo-Butyl | iso-Propyl | cyclo-Propyl | H | O |
| cyclo-Pentyl | iso-Propyl | cyclo-Propyl | H | O |
| cyclo-Hexyl | Methyl | cyclo-Propyl | H | O |
| cyclo-Heptyl | Methyl | cyclo-Propyl | H | O |
| cyclo-Octyl | Methyl | cyclo-Propyl | H | O |
| 1-Methylcyclopropyl | Methyl | cyclo-Propyl | H | O |
| Trifluormethyl | Methyl | cyclo-Propyl | H | O |
| Chlordifluormethyl | Methyl | cyclo-Propyl | H | O |
| Pentafluorethyl | Methyl | cyclo-Propyl | H | O |

| $R^1$ | $R^3$ | $R^4$ | $R^5$ | Y |
|---|---|---|---|---|
| Methoxymethyl | iso-Propyl | cyclo-Propyl | H | O |
| 1-Methylmethoxymethyl | iso-Propyl | cyclo-Propyl | H | O |
| 1-Methylmethoxyethyl | iso-Propyl | cyclo-Propyl | H | O |
| Ethoxymethyl | iso-Propyl | cyclo-Propyl | H | O |
| Vinyl | iso-Propyl | cyclo-Propyl | H | O |
| Allyl | iso-Propyl | cyclo-Propyl | H | O |
| Methallyl | iso-Propyl | cyclo-Propyl | H | O |
| Crotyl | Methyl | cyclo-Propyl | H | O |
| Ethinyl | Methyl | cyclo-Propyl | H | O |
| Propargyl | Methyl | cyclo-Propyl | H | O |
| Phenylethinyl | Methyl | cyclo-Propyl | H | O |
| Methoxy | Methyl | cyclo-Propyl | H | O |
| Ethoxy | Methyl | cyclo-Propyl | H | O |
| Trifluormethoxy | Methyl | cyclo-Propyl | H | O |

Tabelle 1 (Fortsetzung)

| $R^1$ | $R^3$ | $R^4$ | $R^5$ | Y |
|---|---|---|---|---|
| Chlor | H | tert.-Butyl | 4-Hydroxy-2-butinyl | O |
| Chlor | H | tert.-Butyl | $N=C(C_2H_5)_2$ | O |
| Chlor | H | tert.-Butyl | $N=C(cyclo-C_3H_5)_2$ | O |
| Chlor | H | tert.-Butyl | 2-Butanimino | O |
| Chlor | H | tert.-Butyl | Cyclohexanimino | O |
| Chlor | H | tert.-Butyl | Cyclooctanimino | O |
| Methyl | H | tert.-Butyl | $N=CH-C_6H_5$ | O |
| Methyl | H | tert.-Butyl | 2-Furyl-methanimino | O |
| Methyl | H | tert.-Butyl | $CH_2CH_2N(CH_3)_2$ | O |
| Methyl | H | tert.-Butyl | $CH_2CH_2N^+(CH_3)_3I^-$ | O |
| Methyl | H | tert.-Butyl | $CH_2CF_3$ | O |
| Methyl | H | tert.-Butyl | $CH_2CH_2Cl$ | O |
| Methyl | H | tert.-Butyl | $CH_2CH_2CN$ | O |
| Methyl | H | tert.-Butyl | Methyl | O |
| Methyl | H | tert.-Butyl | Ethyl | O |
| Methyl | H | tert.-Butyl | n-Propyl | O |
| Methyl | H | tert.-Butyl | iso-Propyl | O |
| Methyl | H | tert.-Butyl | tert.-Butyl | O |
| Methyl | H | tert.-Butyl | n-Butyl | O |
| Methyl | H | tert.-Butyl | $CH_2-CCl_3$ | O |
| Methyl | H | tert.-Butyl | $CH_2-CH(OCH_3)_2$ | O |
| Methyl | H | tert.-Butyl | $CH_2-C(OCH_3)_3$ | O |

| $R^1$ | $R^3$ | $R^4$ | $R^5$ | Y |
|---|---|---|---|---|
| Methyl | H | tert.-Butyl | $CH_2-CH(OH)-CH_2-OH$ | O |
| Methyl | H | tert.-Butyl | $CH_2-CH_2-CH_2-CH_2-OH$ | O |
| Methyl | H | tert.-Butyl | $CH_2-CH_2-O-CH_3$ | O |
| Methyl | H | tert.-Butyl | $CH_2-CH_2-S-CH_3$ | O |
| Methyl | H | tert.-Butyl | $CH_2-CH_2-NHCH_3$ | O |
| Methyl | H | tert.-Butyl | $CH_2-CH_2-NH$(cyclopropyl) | O |
| Methyl | H | tert.-Butyl | $CH_2-CH_2-N$(cyclopropyl)$_2$ | O |
| Methyl | H | tert.-Butyl | $CH_2-CH_2-Si(CH_3)_3$ | O |
| Methyl | H | tert.-Butyl | $CH_2-CH_2-SOCH_3$ | O |
| Methyl | H | tert.-Butyl | $CH_2-CH_2-SO_2CH_3$ | O |
| Methyl | H | tert.-Butyl | $CH_2-CO_2H$ | O |
| Methyl | H | tert.-Butyl | $CH_2-CO_2CH_3$ | O |
| Methyl | H | tert.-Butyl | $CH_2-CH_2-O-CH_2-CH_2-CO_2CH_3$ | O |
| Methyl | H | tert.-Butyl | $CH_2-CH_2-CO_2-CH_2-CH_2-O-CH_2-CH_2-CO_2CH_3$ | O |
| Methyl | H | tert.-Butyl | $CH_2-CON(CH_3)_2$ | O |
| Methyl | H | tert.-Butyl | $CH_2-PO(OC_2H_5)_2$ | O |
| Methyl | H | tert.-Butyl | $CH_2-CH_2-O-N=C(CH_3)_2$ | O |
| Methyl | H | tert.-Butyl | $CH_2-CH_2-O-N=$ 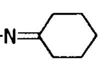 | O |
| Methyl | H | tert.-Butyl | N-Phthalimidomethyl | O |
| Methyl | H | tert.-Butyl | N-Succinimidomethyl | O |
| Methyl | H | tert.-Butyl | Benzyloxymethyl | O |
| Methyl | H | tert.-Butyl | (4-Br-benzoyl)-methyl | O |

| $R^1$ | $R^3$ | $R^4$ | $R^5$ | Y |
|---|---|---|---|---|
| Methyl | H | tert.-Butyl | (4-Methoxybenzoyl)methyl | O |
| Methyl | H | tert.-Butyl | 2-Tetrahydrofuranyl-methyl | O |
| Methyl | H | tert.-Butyl | 2-Tetrahydrothienyl-methyl | O |
| Methyl | H | tert.-Butyl | 4-Tetrahydropyranyl-methyl | O |
| Methyl | H | tert.-Butyl | 2-Furanyl-methyl | O |
| Methyl | H | tert.-Butyl | 2-Thienyl-methyl | O |
| Methyl | H | tert.-Butyl | 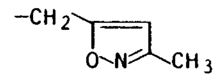 | O |
| Methyl | H | tert.-Butyl | 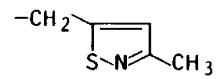 | O |
| Methyl | H | tert.-Butyl | 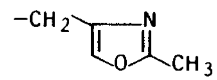 | O |
| Methyl | H | tert.-Butyl | 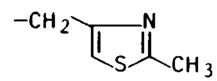 | O |
| Methyl | H | tert.-Butyl | 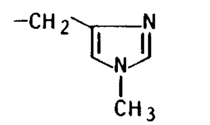 | O |
| Methyl | H | tert.-Butyl | N-Methylpyrrolidin-3-ylmethyl | O |
| Methyl | H | tert.-Butyl | N-Methylpyrrol-3-ylmethyl | O |
| Methyl | H | tert.-Butyl | 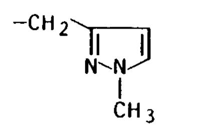 | O |

| $R^1$ | $R^3$ | $R^4$ | $R^5$ | Y |
|---|---|---|---|---|
| Methyl | H | tert.-Butyl | 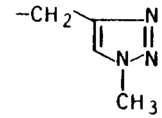 | O |
| Methyl | H | tert.-Butyl | 2-Pyridyl-methyl | O |
| Methyl | H | tert.-Butyl | 3-Pyridyl-methyl | O |
| Methyl | H | tert.-Butyl | 4-Pyridyl-methyl | O |
| Methyl | H | tert.-Butyl | 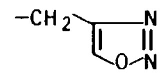 | O |
| Methyl | H | tert.-Butyl | 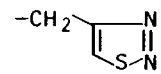 | O |
| Methyl | H | tert.-Butyl | 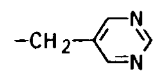 | O |
| Methyl | H | tert.-Butyl | Benzyl | O |
| Methyl | H | tert.-Butyl | 2,4-Dichlorbenzyl | O |
| Methyl | H | tert.-Butyl | 2-Phenylethyl | O |
| Methyl | H | tert.-Butyl | Cyclopentyl | O |
| Methyl | H | tert.-Butyl | Cyclohexyl | O |
| Methyl | H | tert.-Butyl | 2-Propenyl | O |
| Methyl | H | tert.-Butyl | $CH_2-CH=CH-C_6H_5$ | O |
| Methyl | H | tert.-Butyl | 2-Cyclohexenyl | O |
| Methyl | H | tert.-Butyl | 2-Propinyl | O |
| Methyl | H | tert.-Butyl | $CH_2-C\equiv C-CH_2-OH$ | O |
| Methyl | H | tert.-Butyl | 4-F-Phenyl | O |

| $R^1$ | $R^3$ | $R^4$ | $R^5$ | Y |
|---|---|---|---|---|
| Methyl | H | tert.-Butyl | 4-$NO_2$-Phenyl | O |
| Methyl | H | tert.-Butyl | 4-CN-Phenyl | O |
| Methyl | H | tert.-Butyl | 4-$CH_3$-Phenyl | O |
| Methyl | H | tert.-Butyl | 4-$CF_3$-Phenyl | O |
| Methyl | H | tert.-Butyl | 3,5-($CF_3$,$CF_3$)-Phenyl | O |
| Methyl | H | tert.-Butyl | 2-$NO_2$-4-F-Phenyl | O |
| Methyl | H | tert.-Butyl | 4-$OCH_3$-Phenyl | O |
| Methyl | H | tert.-Butyl | 4-$OCF_3$-Phenyl | O |
| Methyl | H | tert.-Butyl | 4-$CO_2CH_3$-Phenyl | O |
| Methyl | H | tert.-Butyl | 2,6(Br,Br)-4-$NO_2$-Phenyl | O |
| Methyl | H | tert.-Butyl | 2-Tetrahydrofuranyl | O |
| Methyl | H | tert.-Butyl | 2-Tetrahydropyranyl | O |
| Methyl | H | tert.-Butyl | 1-Pyrazolyl | O |
| Methyl | H | tert.-Butyl | 1-(1,2,3)-Triazolyl | O |
| Methyl | H | tert.-Butyl | 1-Benzotriazolyl | O |
| Methyl | H | tert.-Butyl | Phthalimido | O |
| Methyl | H | tert.-Butyl | Tetrahydrophthalimido | O |
| Methyl | H | tert.-Butyl | Succinimido | O |
| Methyl | H | tert.-Butyl | Maleinimido | O |
| Methyl | H | tert.-Butyl | $Na^+$ | O |
| Methyl | H | tert.-Butyl | $K^+$ | O |
| Methyl | H | tert.-Butyl | $NH_4^+$ | O |

| $R^1$ | $R^3$ | $R^4$ | $R^5$ | Y |
|---|---|---|---|---|
| Methyl | H | tert.-Butyl | $\overset{\oplus}{N}H_3$-(iso-Propyl) | O |
| Methyl | H | tert.-Butyl | $\overset{\oplus}{N}H_2$-(iso-Propyl)$_2$ | O |
| Methyl | H | tert.-Butyl | 2-Propanimino | O |
| Methyl | H | tert.-Butyl | 2-Butanimino | O |
| Methyl | H | tert.-Butyl | $-N=C(C_2H_5)_2$ | O |
| Methyl | H | tert.-Butyl | $-N=CH-CH_2-$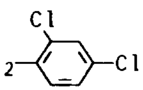 | O |
| Methyl | H | tert.-Butyl | $-N=CH-CH_2-$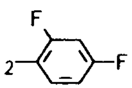 | O |
| Methyl | H | tert.-Butyl | -N=C(cyclopropyl)$_2$ | O |
| Methyl | H | tert.-Butyl | Cyclopentanimino | O |
| Methyl | H | tert.-Butyl | Cyclohexanimino | O |
| Methyl | H | tert.-Butyl | $CH_2-CH=N-OCH_3$ | O |
| Methyl | H | tert.-Butyl | $CH_2-CH=N-OC_2H_5$ | O |
| Methyl | H | tert.-Butyl | $CH_2-CH=N-O-CH_2-C=CH_2$ | O |
| Methyl | H | tert.-Butyl | $CH_2-CH=N-O-CH_2-C\equiv CH$ | O |
| Methyl | H | tert.-Butyl | $CH_2-CH=N-O-CH_2-CH_2-CH=CH-$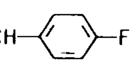 | O |
| Methyl | H | tert.-Butyl | $CH_2-CH=N-N-CH_3$ | O |
| Methyl | H | tert.-Butyl | $CH_2-CH=N-N-C_6H_5$ | O |
| iso-Propyl | H | tert.-Butyl | $CH_2CCl_3$ | O |

| $R^1$ | $R^3$ | $R^4$ | $R^5$ | Y |
|---|---|---|---|---|
| iso-Propyl | H | tert.-Butyl | $CH_2CH_2Si(CH_3)_3$ | O |
| iso-Propyl | H | tert.-Butyl | $CH_2CH_2O-N=C(CH_3)_2$ | O |
| iso-Propyl | H | tert.-Butyl | $CH_2PO(OC_2H_5)_2$ | O |
| iso-Propyl | H | tert.-Butyl | $CH(CH_3)CH(OCH_3)_2$ | O |
| iso-Propyl | H | tert.-Butyl | $CH_2-CON(C_2H_5)_2$ | O |
| iso-Propyl | H | tert.-Butyl | Benzyl | O |
| cyclo-Propyl | H | tert.-Butyl | 2,4-(Cl,Cl)-Benzyl | O |
| cyclo-Propyl | H | tert.-Butyl | 3-Pyridyl-methyl | O |
| cyclo-Propyl | H | tert.-Butyl | 2-Thienyl-methyl | O |
| cyclo-Propyl | H | tert.-Butyl | 2-Tetrahydrofuranyl-methyl | O |
| cyclo-Propyl | H | tert.-Butyl | 2-Furanyl-methyl | O |
| cyclo-Propyl | H | tert.-Butyl | 2-Pyridyl-methyl | O |
| cyclo-Propyl | H | tert.-Butyl | Phenyl | O |
| Allyl | H | tert.-Butyl | 4-F-Phenyl | O |
| Allyl | H | tert.-Butyl | 4-Trifluormethylphenyl | O |
| Allyl | H | tert.-Butyl | $2-NO_2-4-F$-Phenyl | O |
| Allyl | H | tert.-Butyl | $3,5-(CF_3,CF_3)$-Phenyl | O |
| Allyl | H | tert.-Butyl | $4-OCH_3$-Phenyl | O |
| Allyl | H | tert.-Butyl | $4-OCF_3$-Phenyl | O |
| Allyl | H | tert.-Butyl | $4-NHCOCH_3$-Phenyl | O |
| Ethinyl | H | tert.-Butyl | 2-Tetrahydropyranyl | O |
| Ethinyl | H | tert.-Butyl | 2-Tetrahydrofuranyl | O |
| Ethinyl | H | tert.-Butyl | 1-Benzotriazolyl | O |

| $R^1$ | $R^3$ | $R^4$ | $R^5$ | Y |
|---|---|---|---|---|
| Ethinyl | H | tert.-Butyl | Methyl | O |
| Ethinyl | H | tert.-Butyl | Ethyl | O |
| Ethinyl | H | tert.-Butyl | n-Propyl | O |
| Ethinyl | H | tert.-Butyl | iso-Propyl | O |
| Methoxy | H | tert.-Butyl | n-Butyl | O |
| Methoxy | H | tert.-Butyl | iso-Butyl | O |
| Methoxy | H | tert.-Butyl | sek.-Butyl | O |
| Methoxy | H | tert.-Butyl | tert.-Butyl | O |
| Methoxy | H | tert.-Butyl | cyclo-Hexyl | O |
| Methoxy | H | tert.-Butyl | Cyclopropylmethyl | O |
| Methoxy | H | tert.-Butyl | Ethoxymethyl | O |
| 4-Cl-Phenoxy | H | tert.-Butyl | 2-Methoxy-ethoxy-methyl | O |
| 4-Cl-Phenoxy | H | tert.-Butyl | Benzyloxymethyl | O |
| 4-Cl-Phenoxy | H | tert.-Butyl | (4-Brombenzoyl)-methyl | O |
| 4-Cl-Phenoxy | H | tert.-Butyl | (4-Methoxybenzoyl)-methyl | O |
| 4-Cl-Phenoxy | H | tert.-Butyl | Phthalimidomethyl | O |
| 4-Cl-Phenoxy | H | tert.-Butyl | Methylthiomethyl | O |
| 4-Cl-Phenoxy | H | tert.-Butyl | 2-Thiomethyl-ethyl | O |
| Phenylthio | H | tert.-Butyl | $CH(C_6H_5)COOCH_3$ | O |
| Phenylthio | H | tert.-Butyl | Phenylethyl | O |
| Phenylthio | H | tert.-Butyl | 4-F-Phenylethyl | O |
| Phenylthio | H | tert.-Butyl | Phthalimido | O |
| Phenylthio | H | tert.-Butyl | Tetrahydrophthalimido | O |

| $R^1$ | $R^3$ | $R^4$ | $R^5$ | Y |
|---|---|---|---|---|
| Phenylthio | H | tert.-Butyl | Maleinimido | O |
| Phenylthio | H | tert.-Butyl | Succinimido | O |
| 2,4-(Cl,Cl)-Phenyl | H | tert.-Butyl | Piperidino | O |
| 2,4-(Cl,Cl)-Phenyl | H | tert.-Butyl | $Li^+$ | O |
| 2,4-(Cl,Cl)-Phenyl | H | tert.-Butyl | $Na^+$ | O |
| 2,4-(Cl,Cl)-Phenyl | H | tert.-Butyl | $K^+$ | O |
| 2,4-(Cl,Cl)-Phenyl | H | tert.-Butyl | $NH_4^+$ | O |
| 2,4-(Cl,Cl)-Phenyl | H | tert.-Butyl | Diisopropylammonium | O |
| 2,4-(Cl,Cl)-Phenyl | H | tert.-Butyl | 2-Hydroxyethyl-ammonium | O |
| 2-Thienyl | H | tert.-Butyl | Allyl | O |
| 2-Thienyl | H | tert.-Butyl | Methallyl | O |
| 2-Thienyl | H | tert.-Butyl | 2-Chlorallyl | O |
| 2-Thienyl | H | tert.-Butyl | Propargyl | O |
| 2-Thienyl | H | tert.-Butyl | 3-Jodpropargyl | O |
| Chlor | H | cyclo-Propyl | 4-Hydroxy-2-butinyl | O |
| Chlor | H | cyclo-Propyl | $N=C(C_2H_5)_2$ | O |
| Chlor | H | cyclo-Propyl | $N=C(cyclo-C_3H_5)_2$ | O |
| Chlor | H | cyclo-Propyl | 2-Butanimino | O |
| Chlor | H | cyclo-Propyl | Cyclohexanimino | O |
| Chlor | H | cyclo-Propyl | Cyclooctanimino | O |
| Methyl | H | cyclo-Propyl | $N=CH-C_6H_5$ | O |
| Methyl | H | cyclo-Propyl | 2-Furyl-methanimino | O |
| Methyl | H | cyclo-Propyl | $CH_2CH_2N(CH_3)_2$ | O |

| $R^1$ | $R^3$ | $R^4$ | $R^5$ | Y |
|---|---|---|---|---|
| Methyl | H | cyclo-Propyl | $CH_2CH_2N^+(CH_3)_3I^-$ | O |
| Methyl | H | cyclo-Propyl | $CH_2CF_3$ | O |
| Methyl | H | cyclo-Propyl | $CH_2CH_2Cl$ | O |
| Methyl | H | cyclo-Propyl | $CH_2CH_2CN$ | O |
| iso-Propyl | H | cyclo-Propyl | $CH_2CCl_3$ | O |
| iso-Propyl | H | cyclo-Propyl | $CH_2CH_2Si(CH_3)_3$ | O |
| iso-Propyl | H | cyclo-Propyl | $CH_2CH_2O-N=C(CH_3)_2$ | O |
| iso-Propyl | H | cyclo-Propyl | $CH_2PO(OC_2H_5)_2$ | O |
| iso-Propyl | H | cyclo-Propyl | $CH(CH_3)CH(OCH_3)_2$ | O |
| iso-Propyl | H | cyclo-Propyl | $CH_2-CON(C_2H_5)_2$ | O |
| iso-Propyl | H | cyclo-Propyl | Benzyl | O |
| cyclo-Propyl | H | cyclo-Propyl | 2,4-(Cl,Cl)-Benzyl | O |
| cyclo-Propyl | H | cyclo-Propyl | 3-Pyridyl-methyl | O |
| cyclo-Propyl | H | cyclo-Propyl | 2-Thienyl-methyl | O |
| cyclo-Propyl | H | cyclo-Propyl | 2-Tetrahydrofuranyl-methyl | O |
| cyclo-Propyl | H | cyclo-Propyl | 2-Furanyl-methyl | O |
| cyclo-Propyl | H | cyclo-Propyl | 2-Pyridyl-methyl | O |
| cyclo-Propyl | H | cyclo-Propyl | Phenyl | O |
| Allyl | H | cyclo-Propyl | 4-F-Phenyl | O |
| Allyl | H | cyclo-Propyl | 4-Trifluormethylphenyl | O |
| Allyl | H | cyclo-Propyl | $2-NO_2-4-F$-Phenyl | O |
| Allyl | H | cyclo-Propyl | $3,5-(CF_3,CF_3)$-Phenyl | O |
| Allyl | H | cyclo-Propyl | $4-OCH_3$-Phenyl | O |

| $R^1$ | $R^3$ | $R^4$ | $R^5$ | Y |
|---|---|---|---|---|
| Allyl | H | cyclo-Propyl | 4-$OCF_3$-Phenyl | O |
| Allyl | H | cyclo-Propyl | 4-$NHCOCH_3$-Phenyl | O |
| Ethinyl | H | cyclo-Propyl | 2-Tetrahydropyranyl | O |
| Ethinyl | H | cyclo-Propyl | 2-Tetrahydrofuranyl | O |
| Ethinyl | H | cyclo-Propyl | 1-Benzotriazolyl | O |
| Ethinyl | H | cyclo-Propyl | Methyl | O |
| Ethinyl | H | cyclo-Propyl | Ethyl | O |
| Ethinyl | H | cyclo-Propyl | n-Propyl | O |
| Ethinyl | H | cyclo-Propyl | iso-Propyl | O |
| Methoxy | H | cyclo-Propyl | n-Butyl | O |
| Methoxy | H | cyclo-Propyl | iso-Butyl | O |
| Methoxy | H | cyclo-Propyl | sek.-Butyl | O |
| Methoxy | H | cyclo-Propyl | tert.-Butyl | O |
| Methoxy | H | cyclo-Propyl | cyclo-Hexyl | O |
| Methoxy | H | cyclo-Propyl | Cyclopropylmethyl | O |
| Methoxy | H | cyclo-Propyl | Ethoxymethyl | O |
| 4-Cl-Phenoxy | H | cyclo-Propyl | 2-Methoxy-ethoxy-methyl | O |
| 4-Cl-Phenoxy | H | cyclo-Propyl | Benzyloxymethyl | O |
| 4-Cl-Phenoxy | H | cyclo-Propyl | (4-Brombenzoyl)-methyl | O |
| 4-Cl-Phenoxy | H | cyclo-Propyl | (4-Methoxybenzoyl)-methyl | O |
| 4-Cl-Phenoxy | H | cyclo-Propyl | Phthalimidomethyl | O |
| 4-Cl-Phenoxy | H | cyclo-Propyl | Methylthiomethyl | O |
| 4-Cl-Phenoxy | H | cyclo-Propyl | 2-Thiomethyl-ethyl | O |

| $R^1$ | $R^3$ | $R^4$ | $R^5$ | Y |
|---|---|---|---|---|
| Phenylthio | H | cyclo-Propyl | $CH(C_6H_5)COOCH_3$ | O |
| Phenylthio | H | cyclo-Propyl | Phenylethyl | O |
| Phenylthio | H | cyclo-Propyl | 4-F-Phenylethyl | O |
| Phenylthio | H | cyclo-Propyl | Phthalimido | O |
| Phenylthio | H | cyclo-Propyl | Tetrahydrophthalimido | O |
| Phenylthio | H | cyclo-Propyl | Maleinimido | O |
| Phenylthio | H | cyclo-Propyl | Succinimido | O |
| 2,4-(Cl,Cl)-Phenyl | H | cyclo-Propyl | Piperidino | O |
| 2,4-(Cl,Cl)-Phenyl | H | cyclo-Propyl | $Li^+$ | O |
| 2,4-(Cl,Cl)-Phenyl | H | cyclo-Propyl | $Na^+$ | O |
| 2,4-(Cl,Cl)-Phenyl | H | cyclo-Propyl | $K^+$ | O |
| 2,4-(Cl,Cl)-Phenyl | H | cyclo-Propyl | $NH_4^+$ | O |
| 2,4-(Cl,Cl)-Phenyl | H | cyclo-Propyl | Diisopropylammonium | O |
| 2,4-(Cl,Cl)-Phenyl | H | cyclo-Propyl | 2-Hydroxyethyl-ammonium | O |
| 2-Thienyl | H | cyclo-Propyl | Allyl | O |
| 2-Thienyl | H | cyclo-Propyl | Methallyl | O |
| 2-Thienyl | H | cyclo-Propyl | 2-Chlorallyl | O |
| 2-Thienyl | H | cyclo-Propyl | Propargyl | O |
| 2-Thienyl | H | cyclo-Propyl | 3-Jodpropargyl | O |
| H | H | tert.-Butyl | $-N=C(CH_3)_2$ | O |
| F | H | tert.-Butyl | $-N=C(CH_3)_2$ | O |
| Cl | H | tert.-Butyl | $-N=C(CH_3)_2$ | O |
| Methyl | H | tert.-Butyl | $-N=C(CH_3)_2$ | O |

| $R^1$ | $R^3$ | $R^4$ | $R^5$ | Y |
|---|---|---|---|---|
| Ethyl | H | tert.-Butyl | $-N=C(CH_3)_2$ | O |
| n-Propyl | H | tert.-Butyl | $-N=C(CH_3)_2$ | O |
| iso-Propyl | H | tert.-Butyl | $-N=C(CH_3)_2$ | O |
| n-Butyl | H | tert.-Butyl | $-N=C(CH_3)_2$ | O |
| iso-Butyl | H | tert.-Butyl | $-N=C(CH_3)_2$ | O |
| sek.-Butyl | H | tert.-Butyl | $-N=C(CH_3)_2$ | O |
| tert.-Butyl | H | tert.-Butyl | $-N=C(CH_3)_2$ | O |
| cyclo-Propyl | H | tert.-Butyl | $-N=C(CH_3)_2$ | O |
| cyclo-Butyl | H | tert.-Butyl | $-N=C(CH_3)_2$ | O |
| cyclo-Pentyl | H | tert.-Butyl | $-N=C(CH_3)_2$ | O |
| cyclo-Hexyl | H | tert.-Butyl | $-N=C(CH_3)_2$ | O |
| cyclo-Heptyl | H | tert.-Butyl | $-N=C(CH_3)_2$ | O |
| cyclo-Octyl | H | tert.-Butyl | $-N=C(CH_3)_2$ | O |
| 1-Methylcyclopropyl | H | tert.-Butyl | $-N=C(CH_3)_2$ | O |
| Chlormethyl | H | tert.-Butyl | $-N=C(CH_3)_2$ | O |
| 1-Chlorethyl | H | tert.-Butyl | $-N=C(CH_3)_2$ | O |
| Trifluormethyl | H | tert.-Butyl | $-N=C(CH_3)_2$ | O |
| Chlordifluormethyl | H | tert.-Butyl | $-N=C(CH_3)_2$ | O |
| Pentafluormethyl | H | tert.-Butyl | $-N=C(CH_3)_2$ | O |
| iso-Propoxy | H | tert.-Butyl | $-N=C(CH_3)_2$ | O |
| Methoxymethyl | H | tert.-Butyl | $-N=C(CH_3)_2$ | O |
| 1-Methylmethoxymethyl | H | tert.-Butyl | $-N=C(CH_3)_2$ | O |
| 1-Methylmethoxyethyl | H | tert.-Butyl | $-N=C(CH_3)_2$ | O |

| $R^1$ | $R^3$ | $R^4$ | $R^5$ | Y |
|---|---|---|---|---|
| Ethoxymethyl | H | tert.-Butyl | $-N=C(CH_3)_2$ | O |
| Vinyl | H | tert.-Butyl | $-N=C(CH_3)_2$ | O |
| Allyl | H | tert.-Butyl | $-N=C(CH_3)_2$ | O |
| Methallyl | H | tert.-Butyl | $-N=C(CH_3)_2$ | O |
| Crotyl | H | tert.-Butyl | $-N=C(CH_3)_2$ | O |
| Ethinyl | H | tert.-Butyl | $-N=C(CH_3)_2$ | O |
| Propargyl | H | tert.-Butyl | $-N=C(CH_3)_2$ | O |
| Phenylethinyl | H | tert.-Butyl | $-N=C(CH_3)_2$ | O |
| Methoxy | H | tert.-Butyl | $-N=C(CH_3)_2$ | O |
| Ethoxy | H | tert.-Butyl | $-N=C(CH_3)_2$ | O |
| Trifluormethoxy | H | tert.-Butyl | $-N=C(CH_3)_2$ | O |
| Methylthio | H | tert.-Butyl | $-N=C(CH_3)_2$ | O |
| Trifluormethylthio | H | tert.-Butyl | $-N=C(CH_3)_2$ | O |
| Phenoxy | H | tert.-Butyl | $-N=C(CH_3)_2$ | O |
| 4-Cl-Phenoxy | H | tert.-Butyl | $-N=C(CH_3)_2$ | O |
| 2,4-(Cl,Cl)-Phenoxy | H | tert.-Butyl | $-N=C(CH_3)_2$ | O |
| 4-$CF_3$-Phenoxy | H | tert.-Butyl | $-N=C(CH_3)_2$ | O |
| Phenyl | H | tert.-Butyl | $-N=C(CH_3)_2$ | O |
| 2-F-Phenylthio | H | tert.-Butyl | $-N=C(CH_3)_2$ | O |
| 3-F-Phenyl | H | tert.-Butyl | $-N=C(CH_3)_2$ | O |
| 2,4-(F,F)-Phenyl | H | tert.-Butyl | $-N=C(CH_3)_2$ | O |
| 2-Cl-Phenyl | H | tert.-Butyl | $-N=C(CH_3)_2$ | O |
| 3-Cl-Phenyl | H | tert.-Butyl | $-N=C(CH_3)_2$ | O |

| $R^1$ | $R^3$ | $R^4$ | $R^5$ | Y |
|---|---|---|---|---|
| 2,4-(Cl,Cl)-Phenyl | H | tert.-Butyl | $-N=C(CH_3)_2$ | O |
| $2-CH_3$-Phenyl | H | tert.-Butyl | $-N=C(CH_3)_2$ | O |
| $3-CH_3$-Phenyl | H | tert.-Butyl | $-N=C(CH_3)_2$ | O |
| $4-CH_3$-Phenyl | H | tert.-Butyl | $-N=C(CH_3)_2$ | O |
| $2,4-(CH_3,CH_3)$-Phenyl | H | tert.-Butyl | $-N=C(CH_3)_2$ | O |
| $2,4,6-(CH_3,CH_3,CH_3)$-Phenyl | H | tert.-Butyl | $-N=C(CH_3)_2$ | O |
| $2-CF_3$-Phenyl | H | tert.-Butyl | $-N=C(CH_3)_2$ | O |
| $2-OCH_3$-Phenyl | H | tert.-Butyl | $-N=C(CH_3)_2$ | O |
| $2,4-(OCH_3,OCH_3)$-Phenyl | H | tert.-Butyl | $-N=C(CH_3)_2$ | O |
| $4-OCF_3$-Phenyl | H | tert.-Butyl | $-N=C(CH_3)_2$ | O |
| $4-SCH_3$-Phenyl | H | tert.-Butyl | $-N=C(CH_3)_2$ | O |
| $3-SCF_3$-Phenyl | H | tert.-Butyl | $-N=C(CH_3)_2$ | O |
| $2,4-(NO_2,NO_2)$-Phenyl | H | tert.-Butyl | $-N=C(CH_3)_2$ | O |
| $4-NO_2$-Phenyl | H | tert.-Butyl | $-N=C(CH_3)_2$ | O |
| 2-Thienyl | H | tert,-Butyl | $-N=C(CH_3)_2$ | O |
| 3-Thienyl | H | tert.-Butyl | $-N=C(CH_3)_2$ | O |
| 2-Furanyl | H | tert.-Butyl | $-N=C(CH_3)_2$ | O |
| 3-Furanyl | H | tert.-Butyl | $-N=C(CH_3)_2$ | O |
| 2-Tetrahydrofuranyl | H | tert.-Butyl | $-N=C(CH_3)_2$ | O |
| 3-Tetrahydrofuranyl | H | tert.-Butyl | $-N=C(CH_3)_2$ | O |
| 2-Pyridyl | H | tert.-Butyl | $-N=C(CH_3)_2$ | O |
| 3-Pyridyl | H | tert.-Butyl | $-N=C(CH_3)_2$ | O |
| 4-Pyridyl | H | tert.-Butyl | $-N=C(CH_3)_2$ | O |

| $R^1$ | $R^3$ | $R^4$ | $R^5$ | Y |
|---|---|---|---|---|
| 2-Tetrahydropyranyl | H | tert.-Butyl | $-N=C(CH_3)_2$ | O |
| 3-Tetrahydropyranyl | H | tert.-Butyl | $-N=C(CH_3)_2$ | O |
| 4-Tetrahydropyranyl | H | tert.-Butyl | $-N=C(CH_3)_2$ | O |
| H | H | cyclo-Propyl | $-N=C(CH_3)_2$ | O |
| F | H | cyclo-Propyl | $-N=C(CH_3)_2$ | O |
| Cl | H | cyclo-Propyl | $-N=C(CH_3)_2$ | O |
| Methyl | H | cyclo-Propyl | $-N=C(CH_3)_2$ | O |
| Ethyl | H | cyclo-Propyl | $-N=C(CH_3)_2$ | O |
| n-Propyl | H | cyclo-Propyl | $-N=C(CH_3)_2$ | O |
| iso-Propyl | H | cyclo-Propyl | $-N=C(CH_3)_2$ | O |
| n-Butyl | H | cyclo-Propyl | $-N=C(CH_3)_2$ | O |
| iso-Butyl | H | cyclo-Propyl | $-N=C(CH_3)_2$ | O |
| sek.-Butyl | H | cyclo-Propyl | $-N=C(CH_3)_2$ | O |
| tert.-Butyl | H | cyclo-Propyl | $-N=C(CH_3)_2$ | O |
| cyclo-Propyl | H | cyclo-Propyl | $-N=C(CH_3)_2$ | O |
| cyclo-Butyl | H | cyclo-Propyl | $-N=C(CH_3)_2$ | O |
| cyclo-Pentyl | H | cyclo-Propyl | $-N=C(CH_3)_2$ | O |
| cyclo-Hexyl | H | cyclo-Propyl | $-N=C(CH_3)_2$ | O |
| cyclo-Heptyl | H | cyclo-Propyl | $-N=C(CH_3)_2$ | O |
| cyclo-Octyl | H | cyclo-Propyl | $-N=C(CH_3)_2$ | O |
| 1-Methylcyclopropyl | H | cyclo-Propyl | $-N=C(CH_3)_2$ | O |
| Chlormethyl | H | cyclo-Propyl | $-N=C(CH_3)_2$ | O |
| 1-Chlorethyl | H | cyclo-Propyl | $-N=C(CH_3)_2$ | O |

| $R^1$ | $R^3$ | $R^4$ | $R^5$ | Y |
|---|---|---|---|---|
| Trifluormethyl | H | cyclo-propyl | $-N=C(CH_3)_2$ | O |
| Chlordifluormethyl | H | cyclo-Propyl | $-N=C(CH_3)_2$ | O |
| Pentafluorethyl | H | cyclo-Propyl | $-N=C(CH_3)_2$ | O |
| iso-Propoxy | H | cyclo-Propyl | $-N=C(CH_3)_2$ | O |
| Methoxymethyl | H | cyclo-Propyl | $-N=C(CH_3)_2$ | O |
| 1-Methylmethoxymethyl | H | cyclo-Propyl | $-N=C(CH_3)_2$ | O |
| 1-Methylmethoxyethyl | H | cyclo-Propyl | $-N=C(CH_3)_2$ | O |
| Ethoxymethyl | H | cyclo-Propyl | $-N=C(CH_3)_2$ | O |
| Vinyl | H | cyclo-Propyl | $-N=C(CH_3)_2$ | O |
| Allyl | H | cyclo-Propyl | $-N=C(CH_3)_2$ | O |
| Methallyl | H | cyclo-Propyl | $-N=C(CH_3)_2$ | O |
| Crotyl | H | cyclo-Propyl | $-N=C(CH_3)_2$ | O |
| Ethinyl | H | cyclo-Propyl | $-N=C(CH_3)_2$ | O |
| Propargyl | H | cyclo-Propyl | $-N=C(CH_3)_2$ | O |
| Phenylethinyl | H | cyclo-Propyl | $-N=C(CH_3)_2$ | O |
| Methoxy | H | cyclo-Propyl | $-N=C(CH_3)_2$ | O |
| Ethoxy | H | cyclo-Propyl | $-N=C(CH_3)_2$ | O |
| Trifluormethoxy | H | cyclo-Propyl | $-N=C(CH_3)_2$ | O |
| Methylthio | H | cyclo-Propyl | $-N=C(CH_3)_2$ | O |
| Trifluormethylthio | H | cyclo-Propyl | $-N=C(CH_3)_2$ | O |
| Phenoxy | H | cyclo-Propyl | $-N=C(CH_3)_2$ | O |
| 4-Cl-Phenoxy | H | cyclo-Propyl | $-N=C(CH_3)_2$ | O |
| 2,4-(Cl,Cl)-Phenoxy | H | cyclo-Propyl | $-N=C(CH_3)_2$ | O |

| $R^1$ | $R^3$ | $R^4$ | $R^5$ | Y |
|---|---|---|---|---|
| 4-$CF_3$-Phenoxy | H | cyclo-Propyl | $-N=C(CH_3)_2$ | O |
| Phenyl | H | cyclo-Propyl | $-N=C(CH_3)_2$ | O |
| 2-F-Phenylthio | H | cyclo-Propyl | $-N=C(CH_3)_2$ | O |
| 3-F-Phenyl | H | cyclo-Propyl | $-N=C(CH_3)_2$ | O |
| 2,4-(F,F)-Phenyl | H | cyclo-Propyl | $-N=C(CH_3)_2$ | O |
| 2-Cl-Phenyl | H | cyclo-Propyl | $-N=C(CH_3)_2$ | O |
| 3-Cl-Phenyl | H | cyclo-Propyl | $-N=C(CH_3)_2$ | O |
| 2,4-(Cl,Cl)-Phenyl | H | cyclo-Propyl | $-N=C(CH_3)_2$ | O |
| 2-$CH_3$-Phenyl | H | cyclo-Propyl | $-N=C(CH_3)_2$ | O |
| 3-$CH_3$-Phenyl | H | cyclo-Propyl | $-N=C(CH_3)_2$ | O |
| 4-$CH_3$-Phenyl | H | cyclo-Propyl | $-N=C(CH_3)_2$ | O |
| 2,4-($CH_3$,$CH_3$)-Phenyl | H | cyclo-Propyl | $-N=C(CH_3)_2$ | O |
| 2,4,6-($CH_3$,$CH_3$,$CH_3$)-Phenyl | H | cyclo-Propyl | $-N=C(CH_3)_2$ | O |
| 2-$CF_3$-Phenyl | H | cyclo-Propyl | $-N=C(CH_3)_2$ | O |
| 2-$OCH_3$-Phenyl | H | cyclo-Propyl | $-N=C(CH_3)_2$ | O |
| 2,4-($OCH_3$,$OCH_3$)-Phenyl | H | cyclo-Propyl | $-N=C(CH_3)_2$ | O |
| 4-$OCF_3$-Phenyl | H | cyclo-Propyl | $-N=C(CH_3)_2$ | O |
| 4-$SCH_3$-Phenyl | H | cyclo-Propyl | $-N=C(CH_3)_2$ | O |
| 3-$SCF_3$-Phenyl | H | cyclo-Propyl | $-N=C(CH_3)_2$ | O |
| 2,4-($NO_2$,$NO_2$)-Phenyl | H | cyclo-Propyl | $-N=C(CH_3)_2$ | O |
| 4-$NO_2$-Phenyl | H | cyclo-Propyl | $-N=C(CH_3)_2$ | O |
| 2-Thienyl | H | cyclo-Propyl | $-N=C(CH_3)_2$ | O |
| 3-Thienyl | H | cyclo-Propyl | $-N=C(CH_3)_2$ | O |

| $R^1$ | $R^3$ | $R^4$ | $R^5$ | Y |
|---|---|---|---|---|
| 2-Furanyl | H | cyclo-Propyl | $-N=C(CH_3)_2$ | O |
| 3-Furanyl | H | cyclo-Propyl | $-N=C(CH_3)_2$ | O |
| 2-Tetrahydrofuranyl | H | cyclo-Propyl | $-N=C(CH_3)_2$ | O |
| 3-Tetrahydrofuranyl | H | cyclo-Propyl | $-N=C(CH_3)_2$ | O |
| 2-Pyridyl | H | cyclo-Propyl | $-N=C(CH_3)_2$ | O |
| 3-Pyridyl | H | cyclo-Propyl | $-N=C(CH_3)_2$ | O |
| 4-Pyridyl | H | cyclo-Propyl | $-N=C(CH_3)_2$ | O |
| 2-Tetrahydropyranyl | H | cyclo-Propyl | $-N=C(CH_3)_2$ | O |
| 3-Tetrahydropyranyl | H | cyclo-Propyl | $-N=C(CH_3)_2$ | O |
| 4-Tetrahydropyranyl | H | cyclo-Propyl | $-N=C(CH_3)_2$ | O |
| Chlor | H | Methyl | H | O |
| Chlor | H | Ethyl | H | O |
| Chlor | H | n-Propyl | H | O |
| Chlor | H | iso-Propyl | H | O |
| Chlor | H | n-Butyl | H | O |
| Chlor | H | iso-Butyl | H | O |
| Methyl | H | sek.-Butyl | H | O |
| Methyl | H | n-Pentyl | H | O |
| Methyl | H | 2-Pentyl | H | O |
| Methyl | H | 3-Pentyl | H | O |
| Methyl | H | n-Hexyl | H | O |
| Methyl | H | 2-Hexyl | H | O |
| iso-Propyl | H | 3-Hexyl | H | O |

| $R^1$ | $R^3$ | $R^4$ | $R^5$ | Y |
|---|---|---|---|---|
| iso-Propyl | H | 2-Methyl-2-pentyl | H | O |
| iso-Propyl | H | cyclo-Propylmethyl | H | O |
| iso-Propyl | H | cyclo-Butyl | H | O |
| iso-Propyl | H | cyclo-Pentyl | H | O |
| iso-Propyl | H | cyclo-Hexyl | H | O |
| cyclo-Propyl | H | 1-Methylcyclohexyl | H | O |
| cyclo-Propyl | H | 3-Trifluormethylcyclohexyl | H | O |
| cyclo-Propyl | H | Allyl | H | O |
| cyclo-Propyl | H | 1-Buten-3-yl | H | O |
| cyclo-Propyl | H | Crotyl | H | O |
| cyclo-Propyl | H | Propargyl | H | O |
| Allyl | H | 1-Butin-3-yl | H | O |
| Allyl | H | 3-Methyl-1-butin-3-yl | H | O |
| Allyl | H | 2-Pentin-4-yl | H | O |
| Allyl | H | Benzyl | H | O |
| Allyl | H | 2-Phenylethyl | H | O |
| Allyl | H | 2-Methylthioethyl | H | O |
| Ethinyl | H | 2-Chlorethyl | H | O |
| Ethinyl | H | 2-Methoxyethyl | H | O |
| Ethinyl | H | 2-(N,N-Dimethylamino)ethyl | H | O |
| Ethinyl | H | Phenyl | H | O |
| Ethinyl | H | 2-$CH_3$-Phenyl | H | O |
| Ethinyl | H | 4-$CH_3$-Phenyl | H | O |

| $R^1$ | $R^3$ | $R^4$ | $R^5$ | Y |
|---|---|---|---|---|
| Methoxy | H | 2,4-($CH_3$,$CH_3$)-Phenyl | H | O |
| Methoxy | H | 2,3,5-($CH_3$,$CH_3$,$CH_3$)-Phenyl | H | O |
| Methoxy | H | 3-$CF_3$-Phenyl | H | O |
| Methoxy | H | 3-F-Phenyl | H | O |
| Methoxy | H | 2-Cl-Phenyl | H | O |
| Methoxy | H | 4-Cl-Phenyl | H | O |
| 4-Cl-Phenoxy | H | 2,4-(F,F)-Phenyl | H | O |
| 4-Cl-Phenoxy | H | 2,3,5-(Cl,Cl,Cl)-Phenyl | H | O |
| 4-Cl-Phenoxy | H | 2-CN-Phenyl | H | O |
| 4-Cl-Phenoxy | H | 2-$OCH_3$-Phenyl | H | O |
| 4-Cl-Phenoxy | H | 2,3-($OCH_3$,$OCH_3$)-Phenyl | H | O |
| 4-Cl-Phenoxy | H | 3,4,5-($OCH_3$,$OCH_3$,$OCH_3$)-Phenyl | H | O |
| Phenylthio | H | 3-$OCF_3$-Phenyl | H | O |
| Phenylthio | H | 4-$OCF_2CHF_2$-Phenyl | H | O |
| Phenylthio | H | 2-$SCH_3$-Phenyl | H | O |
| Phenylthio | H | 2,4-($SCH_3$,$SCH_3$)-Phenyl | H | O |
| Phenylthio | H | 2-$SCF_3$-Phenyl | H | O |
| Phenylthio | H | 4-$NO_2$-Phenyl | H | O |
| 2,4-(Cl,Cl)-Phenyl | H | 2,4-($NO_2$,$NO_2$)-Phenyl | H | O |
| 2,4-(Cl,Cl)-Phenyl | H | 2-CHO-Phenyl | H | O |
| 2,4-(Cl,Cl)-Phenyl | H | 3-$COCH_3$-Phenyl | H | O |
| 2,4-(Cl,Cl)-Phenyl | H | 3-$COCF_3$-Phenyl | H | O |
| 2,4-(Cl,Cl)-Phenyl | H | 1-Naphthyl | H | O |

| $R^1$ | $R^3$ | $R^4$ | $R^5$ | Y |
|---|---|---|---|---|
| 2,4-(Cl,Cl)-Phenyl | H | 2-Naphthyl | H | O |
| 2-Thienyl | H | Piperidino | H | O |
| 2-Thienyl | H | 3-Tetrahydrofuranyl | H | O |
| 2-Thienyl | H | 4-Tetrahydropyranyl | H | O |
| 2-Thienyl | H | 2-Thiazolyl | H | O |
| 2-Thienyl | H | 5-$CH_3$-2-Thiazolyl | H | O |
| 2-Thienyl | H | 4-$CH_3$-5-COOH-2-Thiazolyl | H | O |
| 3-Pyridyl | H | Methyl | H | O |
| 3-Pyridyl | H | Ethyl | H | O |
| 3-Pyridyl | H | n-Propyl | H | O |
| 3-Pyridyl | H | iso-Propyl | H | O |
| 3-Pyridyl | H | n-Butyl | H | O |
| 3-Pyridyl | H | iso-Butylyl | H | O |
| iso-Propyl | Methyl | sek.-Butyl | H | O |
| iso-Propyl | Methyl | n-Pentyl | H | O |
| iso-Propyl | Methyl | 2-Pentyl | H | O |
| iso-Propyl | Methyl | 3-Pentyl | H | O |
| iso-Propyl | Methyl | n-Hexyl | H | O |
| iso-Propyl | Methyl | 2-Hexyl | H | O |
| iso-Propyl | Methyl | 3-Hexyl | H | O |
| Chlor | H | Methyl | $-N=C(CH_3)_2$ | O |
| Chlor | H | Ethyl | $-N=C(CH_3)_2$ | O |
| Chlor | H | n-Propyl | $-N=C(CH_3)_2$ | O |

| $R^1$ | $R^3$ | $R^4$ | $R^5$ | Y |
|---|---|---|---|---|
| Chlor | H | iso-Propyl | $-N=C(CH_3)_2$ | O |
| Chlor | H | n-Butyl | $-N=C(CH_3)_2$ | O |
| Chlor | H | iso-Butyl | $-N=C(CH_3)_2$ | O |
| Methyl | H | sek.-Butyl | $-N=C(CH_3)_2$ | O |
| Methyl | H | n-Pentyl | $-N=C(CH_3)_2$ | O |
| Methyl | H | 2-Pentyl | $-N=C(CH_3)_2$ | O |
| Methyl | H | 3-Pentyl | $-N=C(CH_3)_2$ | O |
| Methyl | H | n-Hexyl | $-N=C(CH_3)_2$ | O |
| Methyl | H | 2-Hexyl | $-N=C(CH_3)_2$ | O |
| iso-Propyl | H | 3-Hexyl | $-N=C(CH_3)_2$ | O |
| iso-Propyl | H | 2-Methyl-2-pentyl | $-N=C(CH_3)_2$ | O |
| iso-Propyl | H | cyclo-Proplymethyl | $-N=C(CH_3)_2$ | O |
| iso-Propyl | H | cyclo-Butyl | $-N=C(CH_3)_2$ | O |
| iso-Propyl | H | cyclo-Pentyl | $-N=C(CH_3)_2$ | O |
| iso-Propyl | H | cyclo-Hexyl | $-N=C(CH_3)_2$ | O |
| cyclo-Propyl | H | 1-Methylcyclohexyl | $-N=C(CH_3)_2$ | O |
| cyclo-Propyl | H | 3-Trifluormethylcyclohexyl | $-N=C(CH_3)_2$ | O |
| cyclo-Propyl | H | Allyl | $-N=C(CH_3)_2$ | O |
| cyclo-Propyl | H | 1-Buten-3-yl | $-N=C(CH_3)_2$ | O |
| cyclo-Propyl | H | Crotyl | $-N=C(CH_3)_2$ | O |
| cyclo-Propyl | H | Propargyl | $-N=C(CH_3)_2$ | O |
| Allyl | H | 1-Butin-3-yl | $-N=C(CH_3)_2$ | O |
| Allyl | H | 3-Methyl-1-butin-3-yl | $-N=C(CH_3)_2$ | O |

| $R^1$ | $R^3$ | $R^4$ | $R^5$ | Y |
|---|---|---|---|---|
| Allyl | H | 2-Pentin-4-yl | $-N=C(CH_3)_2$ | O |
| Allyl | H | Benzyl | $-N=C(CH_3)_2$ | O |
| Allyl | H | 2-Phenylethyl | $-N=C(CH_3)_2$ | O |
| Allyl | H | 2-Methylthioethyl | $-N=C(CH_3)_2$ | O |
| Ethinyl | H | 2-Chlorethyl | $-N=C(CH_3)_2$ | O |
| Ethinyl | H | 2-Methoxyethyl | $-N=C(CH_3)_2$ | O |
| Ethinyl | H | 2-(n,N-Dimethylamino)ethyl | $-N=C(CH_3)_2$ | O |
| Ethinyl | H | Phenyl | $-N=C(CH_3)_2$ | O |
| Ethinyl | H | 2-$CH_3$-Phenyl | $-N=C(CH_3)_2$ | O |
| Ethinyl | H | 4-$CH_3$-Phenyl | $-N=C(CH_3)_2$ | O |
| Methoxy | H | 2,4-($CH_3$,$CH_3$)-Phenyl | $-N=C(CH_3)_2$ | O |
| Methoxy | H | 2,3,5-($CH_3$,$CH_3$,$CH_3$)-Phenyl | $-N=C(CH_3)_2$ | O |
| Methoxy | H | 3-$CF_3$-Phenyl | $-N=C(CH_3)_2$ | O |
| Methoxy | H | 3-F-Phenyl | $-N=C(CH_3)_2$ | O |
| Methoxy | H | 2-Cl-Phenyl | $-N=C(CH_3)_2$ | O |
| Methoxy | H | 4-Cl-Phenyl | $-N=C(CH_3)_2$ | O |
| 4-Cl-Phenoxy | H | 2,4-(F,F)-Phenyl | $-N=C(CH_3)_2$ | O |
| 4-Cl-Phenoxy | H | 2,3,5-(Cl,Cl,Cl)-Phenyl | $-N=C(CH_3)_2$ | O |
| 4-Cl-Phenoxy | H | 2-CN-Phenyl | $-N=C(CH_3)_2$ | O |
| 4-Cl-Phenoxy | H | 2-$OCH_3$-Phenyl | $-N=C(CH_3)_2$ | O |
| 4-Cl-Phenoxy | H | 2,3-($OCH_3$,$OCH_3$)-Phenyl | $-N=C(CH_3)_2$ | O |
| 4-Cl-Phenoxy | H | 3,4,5-($OCH_3$,$OCH_3$,$OCH_3$)-Phenyl | $-N=C(CH_3)_2$ | O |
| Phenylthio | H | 3-$OCF_3$-Phenyl | $-N=C(CH_3)_2$ | O |

| $R^1$ | $R^3$ | $R^4$ | $R^5$ | Y |
|---|---|---|---|---|
| Phenylthio | H | 4-$OCF_2CHF_2$-Phenyl | $-N=C(CH_3)_2$ | O |
| Phenylthio | H | 2-$SCH_3$-Phenyl | $-N=C(CH_3)_2$ | O |
| Phenylthio | H | 2,4-($SCH_3$, $SCH_3$)-Phenyl | $-N=C(CH_3)_2$ | O |
| Phenylthio | H | 2-$SCF_3$-Phenyl | $-N=C(CH_3)_2$ | O |
| Phenylthio | H | 4-$NO_2$-Phenyl | $-N=C(CH_3)_2$ | O |
| 2,4-(Cl,Cl)-Phenyl | H | 2,4-($NO_2$,$NO_2$)-Phenyl | $-N=C(CH_3)_2$ | O |
| 2,4-(Cl,Cl)-Phenyl | H | 2-CHO-Phenyl | $-N=C(CH_3)_2$ | O |
| 2,4-(Cl,Cl)-Phenyl | H | 3-$COCH_3$-Phenyl | $-N=C(CH_3)_2$ | O |
| 2,4-(Cl,Cl)-Phenyl | H | 3-$COCF_3$-Phenyl | $-N=C(CH_3)_2$ | O |
| 2,4-(Cl,Cl)-Phenyl | H | 1-Naphthyl | $-N=C(CH_3)_2$ | O |
| 2,4-(Cl,Cl)-Phenyl | H | 2-Naphthyl | $-N=C(CH_3)_2$ | O |
| 2-Thienyl | H | Piperidino | $-N=C(CH_3)_2$ | O |
| 2-Thienyl | H | 3-Tetrahydrofuranyl | $-N=C(CH_3)_2$ | O |
| 2-Thienyl | H | 4-Tetrahydropyranyl | $-N=C(CH_3)_2$ | O |
| 2-Thienyl | H | 2-Thiazolyl | $-N=C(CH_3)_2$ | O |
| 2-Thienyl | H | 5-$CH_3$-2-Thiazolyl | $-N=C(CH_3)_2$ | O |
| 2-Thienyl | H | 4-$CH_3$-5-COOH-2-Thiazolyl | $-N=C(CH_3)_2$ | O |
| 3-Pyridyl | H | Methyl | $-N=C(CH_3)_2$ | O |
| 3-Pyridyl | H | Ethyl | $-N=C(CH_3)_2$ | O |
| 3-Pyridyl | H | n-Propyl | $-N=C(CH_3)_2$ | O |
| 3-Pyridyl | H | iso-Propyl | $-N=C(CH_3)_2$ | O |
| 3-Pyridyl | H | n-Butyl | $-N=C(CH_3)_2$ | O |
| 3-Pyridyl | H | iso-Butyl | $-N=C(CH_3)_2$ | O |

| $R^1$ | $R^3$ | $R^4$ | $R^5$ | Y |
|---|---|---|---|---|
| iso-Propyl | Methyl | sek.-Butyl | $-N=C(CH_3)_2$ | O |
| iso-Propyl | Methyl | n-Pentyl | $-N=C(CH_3)_2$ | O |
| iso-Propyl | Methyl | 2-Pentyl | $-N=C(CH_3)_2$ | O |
| iso-Propyl | Methyl | 3-Pentyl | $-N=C(CH_3)_2$ | O |
| iso-Propyl | Methyl | n-Hexyl | $-N=C(CH_3)_2$ | O |
| iso-Propyl | Methyl | 2-Hexyl | $-N=C(CH_3)_2$ | O |
| iso-Propyl | Methyl | 3-Hexyl | $-N=C(CH_3)_2$ | O |
| Methyl | H | tert.-Butyl | 2,4-(Cl,Cl)-Phenyl | S |
| Methyl | H | tert.-Butyl | 2-Pyridyl | S |
| Methyl | H | tert.-Butyl | Ethyl | S |
| Methyl | H | tert.-Butyl | iso-Propyl | S |
| Methyl | H | tert.-Butyl | Butyl | S |
| Methyl | H | tert.-Butyl | tert.-Butyl | S |
| Methyl | H | tert.-Butyl | Phenyl | S |
| iso-Propyl | H | tert.-Butyl | 4-F-Phenyl | S |
| iso-Propyl | H | tert.-Butyl | $3-CF_3$-Phenyl | S |
| iso-Propyl | H | tert.-Butyl | 2,4-(Cl,Cl)-Phenyl | S |
| iso-Propyl | H | tert.-Butyl | 2-Pyridyl | S |
| iso-Propyl | H | tert.-Butyl | Methyl | S |
| iso-Propyl | H | tert.-Butyl | Ethyl | S |
| iso-Propyl | H | tert.-Butyl | iso-Propyl | S |
| cyclo-Propyl | H | tert.-Butyl | Butyl | S |
| cyclo-Propyl | H | tert.-Butyl | tert.-Butyl | S |

| $R^1$ | $R^3$ | $R^4$ | $R^5$ | Y |
|---|---|---|---|---|
| cyclo-Propyl | H | tert.-Butyl | Phenyl | S |
| cyclo-Propyl | H | tert.-Butyl | 4-F-Phenyl | S |
| cyclo-Propyl | H | tert.-Butyl | 3-$CF_3$-Phenyl | S |
| cyclo-Propyl | H | tert.-Butyl | 2,4-(Cl,Cl)-Phenyl | S |
| cyclo-Propyl | H | tert.-Butyl | 2-Pyridyl | S |
| Allyl | H | tert.-Butyl | Methyl | S |
| Allyl | H | tert.-Butyl | Ethyl | S |
| Ally | H | tert.-Butyl | iso-Propyl | S |
| Allyl | H | tert.-Butyl | Butyl | S |
| Allyl | H | tert.-Butyl | tert.-Butyl | S |
| Allyl | H | tert.-Butyl | Phenyl | S |
| Methoxy | H | tert.-Butyl | Methyl | S |
| Methoxy | H | tert.-Butyl | Ethyl | S |
| Methoxy | H | tert.-Butyl | iso-Propyl | S |
| Methoxy | H | tert.-Butyl | Butyl | S |
| Methoxy | H | tert.-Butyl | tert.-Butyl | S |
| Methoxy | H | tert.-Butyl | Phenyl | S |
| Methoxy | H | tert.-Butyl | 4-F-Phenyl | S |
| 4-Cl-Phenoxy | H | tert.-Butyl | 3-$CF_3$-Phenyl | S |
| 4-Cl-Phenoxy | H | tert.-Butyl | 2,4-(Cl,Cl)-Phenyl | S |
| 4-Cl-Phenoxy | H | tert.-Butyl | 2-Pyridyl | S |
| 4-Cl-Phenoxy | H | tert.-Butyl | Methyl | S |
| 4-Cl-Phenoxy | H | tert.-Butyl | Ethyl | S |

| $R^1$ | $R^3$ | $R^4$ | $R^5$ | Y |
|---|---|---|---|---|
| 4-Cl-Phenoxy | H | tert.-Butyl | iso-Propyl | S |
| 4-Cl-Phenoxy | H | tert.-Butyl | Butyl | S |
| 2,4-(Cl,Cl)-Phenyl | H | tert.-Butyl | tert.-Butyl | S |
| 2,4-(Cl,Cl)-Phenyl | H | tert.-Butyl | Phenyl | S |
| 2,4-(Cl,Cl)-Phenyl | H | tert.-Butyl | 4-F-Phenyl | S |
| 2,4-(Cl,Cl)-Phenyl | H | tert.-Butyl | 3-$CF_3$-Phenyl | S |
| 2,4-(Cl,Cl)-Phenyl | H | tert.-Butyl | 2,4-(Cl,Cl)-Phenyl | S |
| 2,4-(Cl,Cl)-Phenyl | H | tert.-Butyl | 2-Pyridyl | S |
| 2,4-(Cl,Cl)-Phenyl | H | tert.-Butyl | Ethyl | S |
| 2-Thienyl | H | tert.-Butyl | iso-Propyl | S |
| 2-Thienyl | H | tert.-Butyl | Butyl | S |
| 2-Thienyl | H | tert.-Butyl | tert.-Butyl | S |
| 3-Pyridyl | H | tert.-Butyl | Phenyl | S |
| 3-Pyridyl | H | tert.-Butyl | 4-F-Phenyl | S |
| 3-Pyridyl | H | tert.-Butyl | 3-$CF_3$-Phenyl | S |
| Methyl | H | cyclo-Propyl | 2,4-(Cl,Cl)-Phenyl | S |
| Methyl | H | cyclo-Propyl | 2-Pyridyl | S |
| Methyl | H | cyclo-Propyl | Ethyl | S |
| Methyl | H | cyclo-Propyl | iso-Propyl | S |
| Methyl | H | cyclo-Propyl | Butyl | S |
| Methyl | H | cyclo-Propyl | tert.-Butyl | S |
| Methyl | H | cyclo-Propyl | Phenyl | S |
| iso-Propyl | H | cyclo-Propyl | 4-F-Phenyl | S |

| $R^1$ | $R^3$ | $R^4$ | $R^5$ | Y |
|---|---|---|---|---|
| iso-Propyl | H | cyclo-Propyl | 3-$CF_3$-Phenyl | S |
| iso-Propyl | H | cyclo-Propyl | 2,4-(Cl,Cl)-Phenyl | S |
| iso-Propyl | H | cyclo-Propyl | 2-Pyridyl | S |
| iso-Propyl | H | cyclo-Propyl | Methyl | S |
| iso-Propyl | H | cyclo-Propyl | Ethyl | S |
| iso-Propyl | H | cyclo-Propyl | iso-Propyl | S |
| cyclo-Propyl | H | cyclo-Propyl | Butyl | S |
| cyclo-Propyl | H | cyclo-Propyl | tert.-Butyl | S |
| cyclo-Propyl | H | cyclo-Propyl | Phenyl | S |
| cyclo-Propyl | H | cyclo-Propyl | 4-F-Phenyl | S |
| cyclo-Propyl | H | cyclo-Propyl | 3-$CF_3$-Phenyl | S |
| cyclo-Propyl | H | cyclo-Propyl | 2,4-(Cl,Cl)-Phenyl | S |
| cyclo-Propyl | H | cyclo-Propyl | 2-Pyridyl | S |
| Allyl | H | cyclo-Propyl | Methyl | S |
| Allyl | H | cyclo-Propyl | Ethyl | S |
| Allyl | H | cyclo-Propyl | iso-Propyl | S |
| Allyl | H | cyclo-Propyl | Butyl | S |
| Allyl | H | cyclo-Propyl | tert.-Butyl | S |
| Allyl | H | cyclo-Propyl | Phenyl | S |
| Methoxy | H | cyclo-Propyl | Methyl | S |
| Methoxy | H | cyclo-Propyl | Ethyl | S |
| Methoxy | H | cyclo-Propyl | iso-Propyl | S |
| Methoxy | H | cyclo-Propyl | Butyl | S |

| $R^1$ | $R^3$ | $R^4$ | $R^5$ | Y |
|---|---|---|---|---|
| Methoxy | H | cyclo-Propyl | tert.-Butyl | S |
| Methoxy | H | cyclo-Propyl | Phenyl | S |
| Methoxy | H | cyclo-Propyl | 4-F-Phenyl | S |
| 4-Cl-Phenoxy | H | cyclo-Propyl | 3-$CF_3$-Phenyl | S |
| 4-Cl-Phenoxy | H | cyclo-Propyl | 2,4-(Cl,Cl)-Phenyl | S |
| 4-Cl-Phenoxy | H | cyclo-Propyl | 2-Pyridyl | S |
| 4-Cl-Phenoxy | H | cyclo-Propyl | Methyl | S |
| 4-Cl-Phenoxy | H | cyclo-Propyl | Ethyl | S |
| 4-Cl-Phenoxy | H | cyclo-Propyl | iso-Propyl | S |
| 4-Cl-Phenoxy | H | cyclo-Propyl | Butyl | S |
| 2,4-(Cl,Cl)-Phenyl | H | cyclo-Propyl | tert.-Butyl | S |
| 2,4-(Cl,Cl)-Phenyl | H | cyclo-Propyl | Phenyl | S |
| 2,4-(Cl,Cl)-Phenyl | H | cyclo-Propyl | 4-F-Phenyl | S |
| 2,4-(Cl,Cl)-Phenyl | H | cyclo-Propyl | 3-$CF_3$-Phenyl | S |
| 2,4-(Cl,Cl)-Phenyl | H | cyclo-Propyl | 2,4-(Cl,Cl)-Phenyl | S |
| 2,4-(Cl,Cl)-Phenyl | H | cyclo-Propyl | 2-Pyridyl | S |
| 2,4-(Cl,Cl)-Phenyl | H | cyclo-Propyl | Ethyl | S |
| 2-Thienyl | H | cyclo-Propyl | iso-Propyl | S |
| 2-Thienyl | H | cyclo-Propyl | Butyl | S |
| 2-Thienyl | H | cyclo-Propyl | tert.-Butyl | S |
| 3-Pyridyl | H | cyclo-Propyl | Phenyl | S |
| 3-Pyridyl | H | cyclo-Propyl | 4-F-Phenyl | S |
| 3-Pyridyl | H | cyclo-Propyl | 3-$CF_3$-Phenyl | S |

Tabelle 2

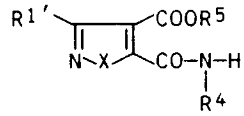

$R^2$ = COOH

$R^3$ = H

| $R^{1'}$ | $R^4$ | $R^5$ | X |
|---|---|---|---|
| Cyclopropyl-$CH(CH_3)$- | tert.-Butyl | H | O |
| Cyclopropyl-$CH(C_2H_5)$- | tert.-Butyl | H | O |
| $CH_2$=CH- | tert.-Butyl | H | O |
| E-$CH_3$-CH=CH- | tert.-Butyl | H | O |
| Z-$CH_3$-CH=CH- | tert.-Butyl | H | O |
| $CH_2$=CH-$CH_2$- | tert.-Butyl | H | O |
| $CH_2$=C($CH_3$)- | tert.-Butyl | H | O |
| $C_2H_5$-CH=CH- | tert.-Butyl | H | O |
| $CH_3$-CH=C($CH_3$)- | tert.-Butyl | H | O |
| $CH_2$=C($C_2H_5$)- | tert.-Butyl | H | O |
| $CH_3$-CH=C($C_2H_5$)- | tert.-Butyl | H | O |
| $(CH_3)_2$C=CH- | tert.-Butyl | H | O |
| $CH_2$=C($CH_3$)-CH($CH_3$)- | tert.-Butyl | H | O |
| $(CH_3)_2$C=CH-$CH_2$- | tert.-Butyl | H | O |
| $CH_3$-CCl=CH- | tert.-Butyl | H | O |
| $Cl_2$C=CH- | tert.-Butyl | H | O |
| Cyclopent-1-en | tert.-Butyl | H | O |
| Cyclohex-1-en | tert.-Butyl | H | O |
| 1-$CH_3$-Cyclohex-2-en | tert.-Butyl | H | O |
| E-Phenyl-CH=CH- | tert.-Butyl | H | O |
| E-4-($CH_3$O)-$C_6H_5$-CH=CH- | tert.-Butyl | H | O |
| HC≡C- | tert.-Butyl | H | O |
| $CH_3$-C≡C- | tert.-Butyl | H | O |
| HC≡C-$CH_2$- | tert.-Butyl | H | O |
| Phenyl-C≡C- | tert.-Butyl | H | O |
| 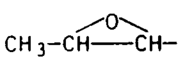 | tert.-Butyl | H | O |
| Cyclopropyl-$CH(CH_3)$- | Cyclopropyl | H | O |
| Cyclopropyl-$CH(C_2H_5)$- | Cyclopropyl | H | O |
| $CH_2$=CH- | Cyclopropyl | H | O |
| E-$CH_3$-CH=CH- | Cyclopropyl | H | O |
| Z-$CH_3$-CH=CH- | Cyclopropyl | H | O |
| $CH_2$=CH-$CH_2$- | Cyclopropyl | H | O |
| $CH_2$=C($CH_3$)- | Cyclopropyl | H | O |
| $C_2H_5$-CH=CH- | Cyclopropyl | H | O |

Tabelle 2 (Fortsetzung)

| R1' | $R^4$ | $R^5$ | X |
|---|---|---|---|
| $CH_3$-CH=C($CH_3$)- | Cyclopropyl | H | O |
| $CH_2$=C($C_2H_5$)- | Cyclopropyl | H | O |
| $CH_3$-CH=C($C_2H_5$)- | Cyclopropyl | H | O |
| $(CH_3)_2$C=CH- | Cyclopropyl | H | O |
| $CH_2$=C($CH_3$)-CH($CH_3$)- | Cyclopropyl | H | O |
| $(CH_3)_2$C=CH-$CH_2$- | Cyclopropyl | H | O |
| $CH_3$-CCl=CH- | Cyclopropyl | H | O |
| $Cl_2$C=CH- | Cyclopropyl | H | O |
| Cyclopent-1-en | Cyclopropyl | H | O |
| Cyclohex-1-en | Cyclopropyl | H | O |
| 1-$CH_3$-Cyclohex-2-en | Cyclopropyl | H | O |
| E-Phenyl-CH=CH- | Cyclopropyl | H | O |
| E-4-($CH_3$O)-$C_6H_5$-CH=CH- | Cyclopropyl | H | O |
| HC≡C- | Cyclopropyl | H | O |
| $CH_3$-C≡C- | Cyclopropyl | H | O |
| HC≡C-$CH_2$- | Cyclopropyl | H | O |
| Phenyl-C≡C- | Cyclopropyl | H | O |
| 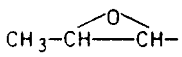 | Cyclopropyl | H | O |
| Cyclopropyl-CH($CH_3$)- | C$(CH_3)_2$C≡CH | H | O |
| Cyclopropyl-CH($C_2H_5$)- | C$(CH_3)_2$C≡CH | H | O |
| $CH_2$=CH- | C$(CH_3)_2$C≡CH | H | O |
| E-$CH_3$-CH=CH- | C$(CH_3)_2$C≡CH | H | O |
| Z-$CH_3$-CH=CH- | C$(CH_3)_2$C≡CH | H | O |
| $CH_2$=CH-$CH_2$- | C$(CH_3)_2$C≡CH | H | O |
| $CH_2$=C($CH_3$)- | C$(CH_3)_2$C≡CH | H | O |
| $C_2H_5$-CH=CH- | C$(CH_3)_2$C≡CH | H | O |
| $CH_3$-CH=C($CH_3$)- | C$(CH_3)_2$C≡CH | H | O |
| $CH_2$=C($C_2H_5$)- | C$(CH_3)_2$C≡CH | H | O |
| $CH_3$-CH=C($C_2H_5$)- | C$(CH_3)_2$C≡CH | H | O |
| $(CH_3)_2$C=CH- | C$(CH_3)_2$C≡CH | H | O |
| $CH_2$=C($CH_3$)-CH($CH_3$)- | C$(CH_3)_2$C≡CH | H | O |
| $(CH_3)_2$C=CH-$CH_2$- | C$(CH_3)_2$C≡CH | H | O |
| $CH_3$-CCl=CH- | C$(CH_3)_2$C≡CH | H | O |
| $Cl_2$C=CH- | C$(CH_3)_2$C≡CH | H | O |
| Cyclopent-1-en | C$(CH_3)_2$C≡CH | H | O |
| Cyclohex-1-en | C$(CH_3)_2$C≡CH | H | O |
| 1-$CH_3$-Cyclohex-2-en | C$(CH_3)_2$C≡CH | H | O |
| E-Phenyl-CH=CH- | C$(CH_3)_2$C≡CH | H | O |

Tabelle 2 (Fortsetzung)

| R1' | $R^4$ | $R^5$ | X |
|---|---|---|---|
| E-4-$(CH_3O)$-$C_6H_5$-CH=CH- | $C(CH_3)_2C\equiv CH$ | H | O |
| HC≡C- | $C(CH_3)_2C\equiv CH$ | H | O |
| $CH_3$-C≡C- | $C(CH_3)_2C\equiv CH$ | H | O |
| HC≡C-$CH_2$- | $C(CH_3)_2C\equiv CH$ | H | O |
| Phenyl-C≡C- | $C(CH_3)_2C\equiv CH$ | H | O |
| 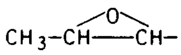 | $C(CH_3)_2C\equiv CH$ | H | O |
| Cyclopropyl-CH$(CH_3)$- | $C(CH_3)_2C\equiv N$ | H | O |
| Cyclopropyl-CH$(C_2H_5)$- | $C(CH_3)_2C\equiv N$ | H | O |
| $CH_2$=CH- | $C(CH_3)_2C\equiv N$ | H | O |
| E-$CH_3$-CH=CH- | $C(CH_3)_2C\equiv N$ | H | O |
| Z-$CH_3$-CH=CH- | $C(CH_3)_2C\equiv N$ | H | O |
| $CH_2$=CH-$CH_2$- | $C(CH_3)_2C\equiv N$ | H | O |
| $CH_2$=C$(CH_3)$- | $C(CH_3)_2C\equiv N$ | H | O |
| $C_2H_5$-CH=CH- | $C(CH_3)_2C\equiv N$ | H | O |
| $CH_3$-CH=C$(CH_3)$- | $C(CH_3)_2C\equiv N$ | H | O |
| $CH_2$=C$(C_2H_5)$- | $C(CH_3)_2C\equiv N$ | H | O |
| $CH_3$-CH=C$(C_2H_5)$- | $C(CH_3)_2C\equiv N$ | H | O |
| $(CH_3)_2$C=CH- | $C(CH_3)_2C\equiv N$ | H | O |
| $CH_2$=C$(CH_3)$-CH$(CH_3)$- | $C(CH_3)_2C\equiv N$ | H | O |
| $(CH_3)_2$C=CH-$CH_2$- | $C(CH_3)_2C\equiv N$ | H | O |
| $CH_3$-CCl=CH- | $C(CH_3)_2C\equiv N$ | H | O |
| $Cl_2$C=CH- | $C(CH_3)_2C\equiv N$ | H | O |
| Cyclopent-1-en | $C(CH_3)_2C\equiv N$ | H | O |
| Cyclohex-1-en | $C(CH_3)_2C\equiv N$ | H | O |
| 1-$CH_3$-Cyclohex-2-en | $C(CH_3)_2C\equiv N$ | H | O |
| E-Phenyl-CH=CH- | $C(CH_3)_2C\equiv N$ | H | O |
| E-4-$(CH_3O)$-$C_6H_5$-CH=CH- | $C(CH_3)_2C\equiv N$ | H | O |
| HC≡C- | $C(CH_3)_2C\equiv N$ | H | O |
| $CH_3$-C≡C- | $C(CH_3)_2C\equiv N$ | H | O |
| HC≡C-$CH_2$- | $C(CH_3)_2C\equiv N$ | H | O |
| Phenyl-C≡C- | $C(CH_3)_2C\equiv N$ | H | O |
| 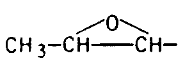 | $C(CH_3)_2C\equiv N$ | H | O |
| $CH_2$=CH- | tert.-Butyl | H | S |
| E-$CH_3$-CH=CH- | tert.-Butyl | H | S |
| E-$C_6H_5$-CH=CH- | tert.-Butyl | H | S |
| $CH_2$=C$(CH_3)$- | tert.-Butyl | H | S |

Tabelle 2 (Fortsetzung)

| $R^{1'}$ | $R^4$ | $R^5$ | X |
|---|---|---|---|
| $CH_2$=CH- | Cyclopropyl | H | S |
| E-$CH_3$-CH=CH- | Cyclopropyl | H | S |
| E-$C_6H_5$-CH=CH- | Cyclopropyl | H | S |
| $CH_2$=C($CH_3$)- | Cyclopropyl | H | S |
| Cyclopropyl-CH($CH_3$)- | tert.-Butyl | -N=C$(CH_3)_2$ | O |
| Cyclopropyl-CH($C_2H_5$)- | tert.-Butyl | -N=C$(CH_3)_2$ | O |
| $CH_2$=CH- | tert.-Butyl | -N=C$(CH_3)_2$ | O |
| E-$CH_3$-CH=CH- | tert.-Butyl | -N=C$(CH_3)_2$ | O |
| Z-$CH_3$-CH=CH- | tert.-Butyl | -N=C$(CH_3)_2$ | O |
| $CH_2$=CH-$CH_2$- | tert.-Butyl | -N=C$(CH_3)_2$ | O |
| $CH_2$=C($CH_3$)- | tert.-Butyl | -N=C$(CH_3)_2$ | O |
| $C_2H_5$-CH=CH- | tert.-Butyl | -N=C$(CH_3)_2$ | O |
| $CH_3$-CH=C($CH_3$)- | tert.-Butyl | -N=C$(CH_3)_2$ | O |
| $CH_2$=C($C_2H_5$)- | tert.-Butyl | -N=C$(CH_3)_2$ | O |
| $CH_3$-CH=C($C_2H_5$)- | tert.-Butyl | -N=C$(CH_3)_2$ | O |
| $(CH_3)_2$C=CH- | tert.-Butyl | -N=C$(CH_3)_2$ | O |
| $CH_2$=C($CH_3$)-CH($CH_3$)- | tert.-Butyl | -N=C$(CH_3)_2$ | O |
| $(CH_3)_2$C=CH-$CH_2$- | tert.-Butyl | -N=C$(CH_3)_2$ | O |
| $CH_3$-CCl=CH- | tert.-Butyl | -N=C$(CH_3)_2$ | O |
| $Cl_2$C=CH- | tert.-Butyl | -N=C$(CH_3)_2$ | O |
| Cyclopent-1-en | tert.-Butyl | -N=C$(CH_3)_2$ | O |
| Cyclohex-1-en | tert.-Butyl | -N=C$(CH_3)_2$ | O |
| 1-$CH_3$-Cyclohex-2-en | tert.-Butyl | -N=C$(CH_3)_2$ | O |
| E-Phenyl-CH=CH- | tert.-Butyl | -N=C$(CH_3)_2$ | O |
| E-4-($CH_3$O)-$C_6H_5$-CH=CH- | tert.-Butyl | -N=C$(CH_3)_2$ | O |
| HC≡C- | tert.-Butyl | -N=C$(CH_3)_2$ | O |
| $CH_3$-C≡C- | tert.-Butyl | -N=C$(CH_3)_2$ | O |
| HC≡C-$CH_2$- | tert.-Butyl | -N=C$(CH_3)_2$ | O |
| Phenyl-C≡C- | tert.-Butyl | -N=C$(CH_3)_2$ | O |
| 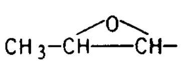 | tert.-Butyl | -N=C$(CH_3)_2$ | O |
| Cyclopropyl-CH($CH_3$)- | Cyclopropyl | -N=C$(CH_3)_2$ | O |
| Cyclopropyl-CH($C_2H_5$)- | Cyclopropyl | -N=C$(CH_3)_2$ | O |
| $CH_2$=CH- | Cyclopropyl | -N=C$(CH_3)_2$ | O |
| E-$CH_3$-CH=CH- | Cyclopropyl | -N=C$(CH_3)_2$ | O |
| Z-$CH_3$-CH=CH- | Cyclopropyl | -N=C$(CH_3)_2$ | O |
| $CH_2$=CH-$CH_2$- | Cyclopropyl | -N=C$(CH_3)_2$ | O |
| $CH_2$=C($CH_3$)- | Cyclopropyl | -N=C$(CH_3)_2$ | O |
| $C_2H_5$-CH=CH- | Cyclopropyl | -N=C$(CH_3)_2$ | O |

Tabelle 2 (Fortsetzung)

| $R^{1'}$ | $R^4$ | $R^5$ | X |
|---|---|---|---|
| $CH_3-CH=C(CH_3)-$ | Cyclopropyl | $-N=C(CH_3)_2$ | O |
| $CH_2=C(C_2H_5)-$ | Cyclopropyl | $-N=C(CH_3)_2$ | O |
| $CH_3-CH=C(C_2H_5)-$ | Cyclopropyl | $-N=C(CH_3)_2$ | O |
| $(CH_3)_2C=CH-$ | Cyclopropyl | $-N=C(CH_3)_2$ | O |
| $CH_2=C(CH_3)-CH(CH_3)-$ | Cyclopropyl | $-N=C(CH_3)_2$ | O |
| $(CH_3)_2C=CH-CH_2-$ | Cyclopropyl | $-N=C(CH_3)_2$ | O |
| $CH_3-CCl=CH-$ | Cyclopropyl | $-N=C(CH_3)_2$ | O |
| $Cl_2C=CH-$ | Cyclopropyl | $-N=C(CH_3)_2$ | O |
| Cyclopent-1-en | Cyclopropyl | $-N=C(CH_3)_2$ | O |
| Cyclohex-1-en | Cyclopropyl | $-N=C(CH_3)_2$ | O |
| 1-$CH_3$-Cyclohex-2-en | Cyclopropyl | $-N=C(CH_3)_2$ | O |
| E-Phenyl-CH=CH- | Cyclopropyl | $-N=C(CH_3)_2$ | O |
| E-4-$(CH_3O)-C_6H_5-CH=CH-$ | Cyclopropyl | $-N=C(CH_3)_2$ | O |
| $HC\equiv C-$ | Cyclopropyl | $-N=C(CH_3)_2$ | O |
| $CH_3-C\equiv C-$ | Cyclopropyl | $-N=C(CH_3)_2$ | O |
| $HC\equiv C-CH_2-$ | Cyclopropyl | $-N=C(CH_3)_2$ | O |
| Phenyl-$C\equiv C-$ | Cyclopropyl | $-N=C(CH_3)_2$ | O |
| 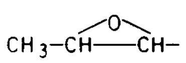 | Cyclopropyl | $-N=C(CH_3)_2$ | O |
| $CH_2=CH-$ | tert.-Butyl | $-N=C(CH_3)_2$ | S |
| E-$CH_3-CH=CH-$ | tert.-Butyl | $-N=C(CH_3)_2$ | S |
| E-$C_6H_5-CH=CH-$ | tert.-Butyl | $-N=C(CH_3)_2$ | S |
| $CH_2=C(CH_3)-$ | tert.-Butyl | $-N=C(CH_3)_2$ | S |
| $CH_2=CH-$ | Cyclopropyl | $-N=C(CH_3)_2$ | S |
| E-$CH_3-CH=CH-$ | Cyclopropyl | $-N=C(CH_3)_2$ | S |
| E-$C_6H_5-CH=CH-$ | Cyclopropyl | $-N=C(CH_3)_2$ | S |
| $CH_2=C(CH_3)-$ | Cyclopropyl | $-N=C(CH_3)_2$ | S |

Ganz besonders bevorzugt ist 3-Cyclopropylaminocarbonyl-5-isopropyl-isoxazol-4-carbonsäure.

Die Carbonsäureamide Ia, Ib, Ic und Id bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen Ia, Ib, Ic und Id eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden.

Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Beispiele für solche Zubereitungen sind:

I. eine Lösung aus 90 Gew.-Teilen der Verbindung Nr. 3.002 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 3.006, 80 Gew.-Teilen Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine Dispersion, die 0,02 Gew.-% des Wirkstoffs enthält.

III. eine wäßrige Dispersion aus 20 Gew.-Teilen der Verbindung Nr. 3.016, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.

IV. eine wäßrige Dispersion aus 20 Gew.-Teilen der Verbindung Nr. 3.020, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;

V. eine in einer Hammermühle vermahlene Mischung aus 80 Gew.-Teilen der Verbindung Nr. 3.029, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;

VI. eine innige Mischung aus 3 Gew.-Teilen der Verbindung Nr. 3.047 und 97 Gew.-Teilen feinteiligem Kaolin. Dieses Stäubemittel enthält 3 Gew.-% Wirkstoff;

VII. eine innige Mischung aus 30 Gew.-Teilen der Verbindung Nr. 3.048, 92 Gew.-Teilen pulverförmigem Kieselsäuregel und 8 Gew.-Teilen Paraffinöl, das auf die Oberfläche dieses Kieselsäuregels gesprüht wurde. Diese Aufbereitung gibt dem Wirkstoff eine gute Haftfähigkeit;

VIII. eine stabile wäßrige Dispersion aus 40 Gew.-Teilen der Verbindung Nr. 6.04, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 6.06, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoffformaldehyd-Kondensates und 68 Gew.-Teilen eines paraffinischen Mineralöls;

X. eine in einer Hammermühle vermahlene Mischung aus 10 Gew.-Teilen der Verbindung Nr. 6.08, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5, vorzugsweise 0,01 bis 1 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |

| Botanischer Name | Deutscher Name |
|---|---|
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |

| Botanischer Name | Deutscher Name |
|---|---|
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna.sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Carbonsäureamide Ia, Ib, Ic und Id mit zahlreichen Vertretern anderer herbizider oder wachstumsregulierender Wirkstoffgruppen, gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen Ia, Ib, Ic und Id allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Herstellungsbeispiele zur Synthese der Wirkstoffe Ia, Ib, Ic und Id

Beispiel 1

3-tert.-Butylaminocarbonyl-5-n-propyl-isoxazol-4-carbonsäure (Nr. 3.013 in Tabelle 3)

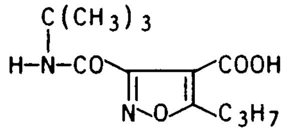

Zu einer Lösung von 10,0 g (47,6 mmol) 5-n-Propyl-isoxazol-3-carbonsäure-tert.-butylamid in 250 ml trockenem Tetrahydrofuran tropfte man unter Stickstoffatmosphäre bei (-70)°C 104,6 mmol n-Butyllithium (67,7 ml einer 1,5 molaren Lösung in Hexan) und rührte 30 min bei dieser Temperatur. Anschließend goß

man das Reaktionsgemisch auf 500 g festes $CO_2$ und ließ über Nacht stehen. Man engte ein, nahm den Rückstand in 300 ml Wasser und 20 ml 2N NaOH auf, extrahierte zweimal mit je 100 ml Diethylether, säuerte die wäßrige Phase mit 6N Salzsäure auf pH 2 an und extrahierte viermal mit je 200 ml Ethylacetat. Man trocknete über Magnesiumsulfat und zog das Solvens im Vakuum ab. Ausbeute: 80 %.

Vorstufe 1α

5-n-Propyl-isoxazol-3-carbonsäure-tert.-butylamid

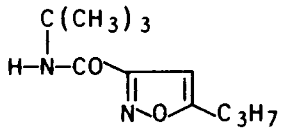

Zu 10,0 g (57,6 mmol) 5-n-Propyl-isoxazol-3-carbonsäurechlorid in 250 ml trockenem Dichlormethan, tropfte man bei 5 °C 9,3 g (126,8 mmol) tert.-Butylamin in 20 ml Dichlormethan. Man rührte 12 h bei Raumtemperatur, gab 200 ml Wasser zu, trennte die Phasen, wusch die organische Phase je einmal mit 150 ml gesättigter Natriumhydrogencarbonatlösung und 150 ml gesättigter Natriumchloridlösung, trocknete über Magnesiumsulfat und zog das Solvens im Vakuum ab. Man erhielt 11,5 g (95 %) 5-n-Propyl-isoxazol-3-carbonsäure-tert.-butylamid als Feststoff vom Smp. 34 - 37 °C.

Auf analoge Weise wurden beispielsweise die folgenden Isoxazol-3-carbonsäureamide synthetisiert:

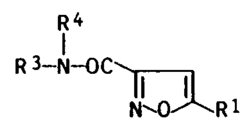

| $R^1$ | $R^3$ | $R^4$ | Fp(°C)/$^1$H-NMR (250 MHz; $CDCl_3$), $\delta$ in ppm |
|---|---|---|---|
| Methyl | H | iso-Propyl | 68- 70 |
| Methyl | H | tert.-Butyl | 79- 82 |
| Methyl | H | Cyclopropyl | 100-105 |
| Methyl | H | Phenyl | 132-135 |
| Methyl | H | Methyl | 122-126 |
| Methyl | H | Ethyl | 61- 67 |
| n-Propyl | H | tert.-Butyl | 34- 37 |
| iso-Propyl | H | tert.-Butyl | 78- 79 |
| iso-Propyl | H | Cyclopropyl | 58- 59 |
| n-Butyl | H | tert.-Butyl | 0,93 (t;3H), 1,40 (m;2H), 1,46 (s;9H), 1,70 (m;2H), 2,77 (t;3H), 6,40 (s,1H), 6,68 (bs;1H, NH) |
| n-Butyl | H | Cyclopropyl | 58- 61 |
| sek.-Butyl | H | tert.-Butyl | 84- 87 |
| sek.-Butyl | H | Cyclopropyl | 54- 60 |
| tert.-Butyl | H | tert.-Butyl | 132-134 |
| tert.-Butyl | H | Cyclopropyl | 75- 80 |
| Phenyl | H | tert.-Butyl | 131-133 |
| Phenyl | H | Cyclopropyl | 146-147 |

| $R^1$ | $R^3$ | $R^4$ | Fp(°C)/$^1$H-NMR (250 MHz; $CDCl_3$), δ in ppm |
|---|---|---|---|
| 2,4-(Cl,Cl)-Phenyl | H | tert.-Butyl | 1,49 (s;9H), 6,70 (bs;1H,NH), 7,30 (s;1H), 7,25-7,90 (m;3H) |
| Methoxyeth-1-yl | H | tert.-Butyl | 1,48 (s;9H), 1,56 (d;3H), 3,34 (s;3H), 4,52 (q;1H), 6,63 (s;1H), 6,64 (bs;1H,NH) |
| Methoxyeth-1-yl | H | Cyclopropyl | 0,60-0,93 (m;4H), 1,54 (d;3H), 2,91 (m;1H), 3,38 (s;3H), 4,54 (q;1H), 6,68 (s;1H), 7,04 (bs;1H,NH) |
| Methoxyeth-1-yl | H | sek.-Butyl | 37- 40 |
| Methoxyeth-1-yl | H | Cyclopropyl-methyl | 0,24-0,60 (m;4H), 1,09 (m;1H), 1,56 (d;3H), 3,34 (m;2H), 3,38 (s;3H), 4,58 (q;1H), 6,70 (s;1H), 7,30 (bs;1H,NH) |
| Methoxymethyl | H | tert.-Butyl | 1,46 (s;9H), 3,44 (s;3H), 4,60 (s;2H), 6,68 (s;1H), 6,68 (bs;1H,NH) |
| Methoxymethyl | H | sek.-Butyl | 0,96 (t;3H), 1,26 (d;3H), 1,58 (m;2H), 3,43 (s;3H), 4,10 (m;1H), 4,56 (s;2H), 6,70 (bs;1H,NH), 6,74 (s;1H) |
| 2-Methyltetrahydro-pyran-2-yl | H | Cyclopropyl | 60- 62 |
| 2-Methyltetrahydro-pyran-2-yl | H | 3-$CF_3$-Phenyl | 1,54 (s;3H), 1,40-2,46 (m;6H), 3,56 (m;2H), 6,74 (s;1H), 6,75-8,00 (m;4H), 8,68 (bs;1H,NH) |
| 2-Methyl-norbornan-2-yl* | H | Cyclopropyl | 0,60 -0,94 (m;4H), 1,18-2,42 (m;13H), 2,90 (m;1H), 6,42 (s;1H), 6,97 (bs;1H,NH) |
| 4-F-Phenyl | H | tert.-Butyl | 115-118 |
| 2,4,6-Trimethylphenyl | H | tert.-Butyl | 83- 90 |
| Cyclopropyl | H | neo-Pentyl | 85- 90 |
| Cyclopropyl | H | 1,1-Dimethyl--2-propenyl | 48- 54 |
| Cyclopropyl | H | Benzyl | 89- 93 |

* Isomerengemisch: exo:endo 1:1

| $R^1$ | $R^3$ | $R^4$ | Fp(°C)/$^1$H-NMR (250 MHz; $CDCl_3$), δ in ppm |
|---|---|---|---|
| Cyclopropyl | H | 2-Methoxyethyl | 0,92-1,14 (m;4H), 2,09 (m;1H), 3,37 (s;1H), 3,58 (m;4H), 6,33 (s;1H), 7,11 (bs;1H,NH) |
| Cyclopropyl | H | 2-Propenyl | 42- 46 |
| Cyclopropyl | H | Cyclopentyl | 86- 92 |
| Cyclohexyl | H | tert.-Butyl | 98-100 |
| Cyclohexyl | H | Cyclopropyl | 134-136 |
| Cyclohexyl | H | 1-Cyclopropyl-ethyl | 117-122 |
| Cyclopropyl | H | tert.-Butyl | 80- 84 |
| Cyclopropyl | H | Cyclopropyl | 92- 98 |
| Cyclopropyl | H | 1-Cyclopropyl-ethyl | 60- 64 |
| 1-Methylcyclohexyl | H | tert.-Butyl | 84- 87 |
| 1-Methylcyclohexyl | H | Cyclopropyl | 0,60-0,91 (m;4H), 1,27 (s;3H), 1,29-2,10 (m;10H), 2,86 (m;1H), 6,47 (s;1H), 6,92 (bs;1H,NH) |
| Cyclopropyl | H | sek.-Butyl | 70- 77 |
| Cyclopropyl | H | Cyclopropyl-methyl | 62- 66 |
| Methyl | H | 1-Cyclopropyl-ethyl | 75 |
| Cyclopropyl | H | Cyclohexyl | 133-136 |
| 2-Methyltetrahydro-pyran-2-yl | H | tert.-Butyl | 72- 76 |
| Methyl | H | Cyclobutyl | 100-103 |
| Methyl | H | 1,1-Dimethyl-propyl | 0,91 (t;3H), 1,41 (s;6H), 1,83 (q;2H), 2,48 (s;3H), 6,40 (s;1H), 6,56 (bs;1H,NH) |
| Methyl | H | neo-Pentyl | 108-111 |

| $R^1$ | $R^3$ | $R^4$ | Fp(°C)/$^1H$-NMR (250 MHz; $CDCl_3$), δ in ppm |
|---|---|---|---|
| 2-Furanyl | H | tert.-Butyl | 65- 70 |
| 2-Furanyl | H | Cyclopropyl | 152-153 |
| Methyl | H | sek.-Butyl | 79- 84 |
| Methyl | H | 2-Methoxy-ethyl | 2,48 (s;3H), 3,37 (s;3H), 3,57 (m;4H), 6,44 (s;1H), 7,17 (bs;1H,NH), 5,14 (m;2H), 6,09 (m;1H), 6,40 (s;1H), 6,77 (bs;1H,NH) |
| Methyl | H | 1,1-Dimethyl--2-propenyl | 1,54 (s;6H), 2,47 (s;3H) |
| Methyl | H | Cyclopentyl | 93- 97 |

Beispiel 2

3-tert.-Butylaminocarbonyl-5-n-propyl-isoxazol-4-carbonsäure-acetonoximester (Nr. 3.014 in Tabelle 3)

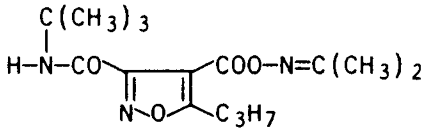

Zu einer Lösung von 3,3 g (13,0 mmol) 3-tert.-Butylaminocarbonyl-5-n-propyl-isoxazol-4-carbonsäure (hergestellt nach Beispiel 1) und 1,2 g (16,9 mmol) Acetonoxim in 100 ml Dichlormethan tropfte man bei Raumtemperatur 4,9 g (48,1 mmol) 4-Methylmorpholin sowie 1,6 g (13,0 mmol) 4-Dimethylaminopyridin und rührte 5 min. Anschließend fügte man 11,3 g einer 50 %igen Lösung von Propanphosphonsäureanhydrid in Dichlormethan (= 17,8 mmol) zu und erhitzte 12 h unter Rückfluß. Man engte ein, nahm den Rückstand in 100 ml Ethylacetat auf, extrahierte zweimal mit gesättigter Natriumhydrogencarbonatlösung sowie je einmal mit 5 %iger Zitronensäurelösung, gesättigter Natriumcarbonatlösung und gesättigter Natriumchloridlösung. Die organische Phase wurde über Magnesiumsulfat getrocknet und das Solvens im Vakuum abgezogen. Ausbeute: 95 %.

Beispiel 3

3-tert.-Butylaminocarbonyl-5-phenyl-isothiazol-4-carbonsäure (Nr. 3.045 in Tabelle 3)

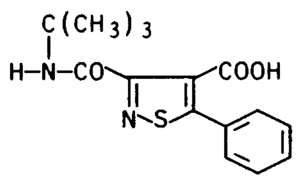

Zu 2,0 g (8,7 mmol) 5-Phenyl-isothiazol-3,4-dicarbonsäureanhydrid in 10 ml Dichlormethan tropfte man unter Eiskühlung 0,64 g (8,8 mmol) tert.-Butylamin und rührte 3 h bei Raumtemperatur. Danach engte man die Lösung ein, versetzte mit 25 ml Wasser, säuerte mit 6N HCl auf pH 2 an und extrahieren dreimal mit je 30 ml Ethylacetat. Die organische Phase wurde mit 20 ml gesättigter Natriumchloridlösung gewaschen, über Magnesiumsulfat getrocknet und eingeengt. Der Rückstand wurde an Kieselgel (Lösungsmittel: Ethanol/Toluol 2:3) chromatographiert. Ausbeute: 49 %.

Beispiel 4

5-Piperidinocarbonyl-3-methyl-isoxazol-4-carbonsäure-tert.-butylamid (Nr. 5.001 in Tabelle 5)

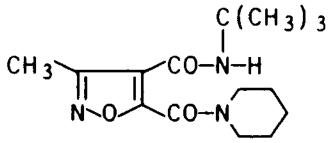

Zu 4,6 g (19,3 mmol) 5-Piperidinocarbonyl-3-methyl-isoxazol-4-carbonsäure in 100 ml Dichlormethan tropfte man bei (-5)°C nacheinander 1,8 g (25,1 mmol) tert.-Butylamin, 7,2 g (71,6 mmol) Methylmorpholin, 0,8 g (6,4 mmol) Dimethylaminopyridin und 16,8 g einer 50 %igen Lösung von Propanphosphonsäureanhydrid in Dichlormethan (= 26,4 mmol) und rührte 12 h bei Raumtemperatur. Man zog das Solvens im Vakuum ab, nahm den Rückstand in 200 ml Ethylacetat auf und extrahierte zweimal mit gesättigter Natriumhydrogencarbonatlösung sowie je einmal mit 5 %iger Zitronensäurelösung, gesättigter Natriumcarbonatlösung und

gesättigter Natriumchloridlösung. Die organische Phase wurde über Magnesiumsulfat getrocknet und das Solvens im Vakuum abgezogen. Ausbeute: 90 %.

Vorstufe 4α

5-Piperidinocarbonyl-3-methyl-isoxazol-4-carbonsäuremethylester

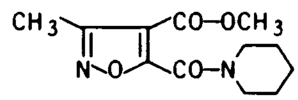

Zu 5,0 g (27,0 mmol) 4-Methoxycarbonyl-3-methyl-isoxazol-5-carbonsäure in 100 ml Dichlormethan tropfte man bei (-5)° C nacheinander 3,0 g (35,1 mmol) Piperidin, 10,1 g (100,0 mmol) Methylmorpholin, 1,1 g (9 mmol) Dimethylaminopyridin und 22,9 g einer 50 %igen Lösung von Propanphosphonsäureanhydrid in Dichlormethan (36,0 mmol) und rührte 12 h bei Raumtemperatur Man zog das Solvens im Vakuum ab, nahm den Rückstand in 200 ml Ethylacetat auf und extrahierte zweimal mit gesättigter Natriumhydrogencarbonatlösung sowie je einmal mit 5 %iger Zitronensäurelösung und gesättigter Natriumchloridlösung. Die organische Phase wurde über Magnesiumsulfat getrocknet und das Solvens im Vakuum abgezogen. Ausbeute: 90 %; $^{1}$H-NMR (250 MHz; $CDCl_3$): δ = 1,65 ppm (m; 6H), 2,49 ppm (s; 3H), 3,18 ppm (m; 2H), 3,73 ppm (m; 2H), 3,87 ppm (s; 3H).

Vorstufe 4β

5-Piperidinocarbonyl-3-methyl-isoxazol-4-carbonsäure

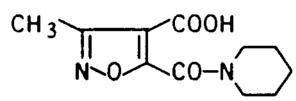

Zu einer Lösung von 5,7 g (22,6 mmol) 5-Piperidinocarbonyl-3-methyl-isoxazol-4-carbonsäuremethylester in 20 ml Methanol tropfte man unter $N_2$ bei (-15) bis (-10)° C innerhalb von 4 h 1,0 g (25,0 mmol) Natriumhydroxid in 20 ml Wasser und rührte 12 h bei Raumtemperatur. Man engte die Lösung ein, nahm den Rückstand in 100 ml Wasser auf, stellte auf pH 8 - 9 ein und extrahierte einmal mit 100 ml Diethylether. Anschließend säuerte man mit 6N HCl auf pH 2 an und extrahierte viermal mit je 100 ml Dichlormethan. Die vereinigten organischen Phasen wurden über Magnesiumsulfat getrocknet und das Solvens im Vakuum abgezogen. Ausbeute: 91 %; Smp.: 128 - 130° C.

Beispiel 5

4-tert.-Butylaminocarbonyl-3-methyl-isoxazol-5-carbonsäure (Nr. 4.001 in Tabelle 4)

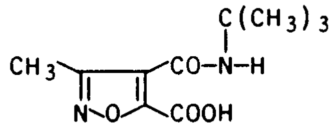

Zu 3,3 g (11,3 mmol) 5-Piperidinocarbonyl-3-methyl-isoxazol-4-carbonsäure-tert.-butylamid (hergestellt nach Beispiel 4) in 150 ml Diethylether und 0,4 g (22,2 mmol) Wasser gab man portionsweise 7,6 g (67,8 mmol) Kalium-tert.-butylat und rührte 6 h bei Raumtemperatur. Nach Zugabe von 50 ml Wasser und Phasentrennung wurde die wäßrige Phase mit 6N HCl auf pH 2 angesäuert und viermal mit je 100 ml Ethylacetat extrahiert. Die vereinigten organischen Phasen wurden wie üblich auf das Produkt hin aufgearbeitet. Das Rohprodukt wurde aus Cyclohexan/Ethylacetat 5 : 1 umkristallisiert. Ausbeute: 42 %; farblose Kristalle.

Beispiel 6

4-Cyclopropylamino-3-methyl-isothiazol-5-carbonsäure (Nr. 4.006 in Tabelle 4)

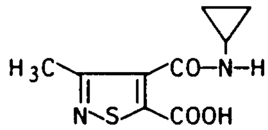

Zu einer Lösung von 5,2 g (28,6 mmol) 3-Methyl-isothiazol-4-carbonsäurecyclopropylamid in 250 ml Tetrahydrofuran tropfte man unter Stickstoffatmosphäre bei -70 °C 60,0 mmol n-Butyllithium (40,0 ml einer 1,5 molaren Lösung in Hexan) und rührte 30 min bei dieser Temperatur. Anschließend goß man das Reaktionsgemisch auf 500 g festes $CO_2$ und ließ über Nacht stehen. Man engte ein, nahm den Rückstand in 300 ml Wasser und 15 ml 2N NaOH auf, extrahierte zweimal mit je 100 ml Diethylether, säuerte die wäßrige Phase mit 6N Salzsäure auf pH 2 an und extrahierte viermal mit je 200 ml Ethylacetat. Man trocknete über Magnesiumsulfat und zog das Solvens im Vakuum ab. Ausbeute: 74 %.

Vorstufe 6α

3-Methyl-isothiazol-4-carbonsäure-cyclopropylamid

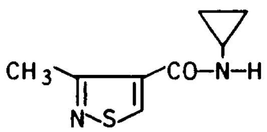

Zu 7,2 g (50,0 mmol) 3-Methyl-isothiazol-4-carbonsäure in 200 ml Dichlormethan tropfte man bei -5 °C nacheinander 3,7 g (65,0 mmol) Cyclopropylamin, 18,7 g (185,0 mmol) Methylmorpholin, 2,0 g (16,7 mmol) Dimethylaminopyridin und 43,5 g einer 50 %igen Lösung von Propanphosphonsäureanhydrid in Dichlormethan (=68,4 mmol) und rührte 12 h bei Raumtemperatur. Man zog das Solvens im Vakuum ab, nahm den Rückstand in 250 ml Ethylacetat auf und extrahierte zweimal mit gesättigter Natriumhydrogencarbonatlösung sowie je einmal mit 5 %iger Zitronensäurelösung, gesättigter Natriumcarbonatlösung und gesättigter Natriumchloridlösung. Die organische Phase wurde über Magnesiumsulfat getrocknet und das Solvens im Vakuum abgezogen. Ausbeute: 84 %; Smp.: 106 - 108 °C.

Auf analoge Weise wurden beispielsweise die folgenden Isothiazol-4-carbonsäureamide synthetisiert:

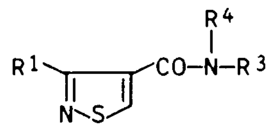

| $R^1$ | $R^3$ | $R^4$ | Fp(°C) |
|---|---|---|---|
| Methyl | H | Cyclopropyl | 106-108 |
| Methyl | H | tert.-Butyl | 96- 97 |
| Methyl | H | iso-Propyl | 100-102 |
| iso-Propyl | H | 3-$CF_3$-Phenyl | 114-115 |
| iso-Propyl | H | tert.-Butyl | 158-159 |
| Phenyl | H | Cyclopropyl | 172-173 |
| Phenyl | H | 4-Cl-Phenyl | 202-203 |

Beispiel 7

Beispiel 7

4-Ethoxy-carbonyl-5-methyl-isoxazol-3-carbonsäure-tert.-butylamid (Nr. 3.036 in Tabelle 3)

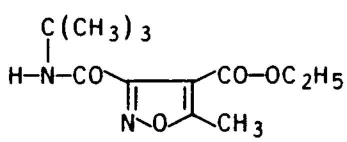

Zu 10,6 g (53,3 mmol) 4-Ethoxycarbonyl-5-methyl-isoxazol-3-carbonsäure in 150 ml Toluol und 2 ml Dimethylformamid tropfte man bei Raumtemperatur 12,7 g (106,8 mmol) Thionylchlorid und rührte 1 h bei 80 °C. Man zog die Solventien im Vakuum ab, löste den Rückstand in 200 ml trockenem Dichlormethan und tropfte 10,0 g (137,0 mmol) tert.-Butylamin in 20 ml trockenem Dichlormethan zu. Man rührte 12 h bei Raumtemperatur, gab 200 ml Wasser zu, trennte die Phasen, wusch die organische Phase je einmal mit 150 ml gesättigter Natriumhydrogencarbonatlösung und 150 ml gesättigter Natriumchloridlösung, trocknete über Magnesiumsulfat und zog das Solvens im Vakuum ab. Ausbeute: 73 %.

Vorstufe 7α

4-Ethoxycarbonyl-5-methyl-isoxazol-3-carbonsäuremethylester

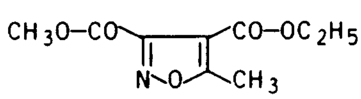

Zu 9,9 g (0,33 mol) NaH (80 %ige Suspension in Weißöl) in 1 l trockenem Toluol tropfte man bei Raumtemperatur 39,0 g (0,3 mol) Acetessigsäureethylester in 100 ml Toluol und rührte 3 h. Anschließend gab man 41,3 g (0,3 mol) Methyl-α-chloro-α-oximinoacetat in 100 ml Toluol zu und rührte 12 h bei

Raumtemperatur. Danach überführte man das Reaktionsgemisch in eine Soxhletapparatur (Extraktionshülse gefüllt mit Molekularsieb 4 Å), fügte 1 g Methansulfonsäure hinzu und erhitzte 1,5 h unter Rückfluß. Man ließ abkühlen, wusch die organische Phase je einmal mit 200 ml Dinatriumhydrogenphosphatlösung und 200 ml gesättigter Natriumchloridlösung, trocknete über Magnesiumsulfat und zog das Solvens im Vakuum ab. Ausbeute: 63 %; gelbes Öl; $^1$H-NMR (250 MHz; $CDCl_3$): δ = 1,34 ppm (t; 3H), 2,73 ppm (s; 3H), 4,00 ppm (s; 3H), 4,32 ppm (q; 2H).

Auf analoge Weise wurden beispielsweise die folgenden Isoxazoldicarbonsäurediester synthetisiert:

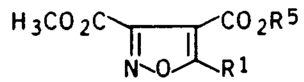

| $R^1$ | $R^5$ | $^1$H-NMR (250 MHz; $CDCl_3$) δ in ppm |
|---|---|---|
| $CH_3$ | $CH_3$ | 2,72 (s;3H), 3,87 (s;3H), 4.00 (s;3H) |
| $CH_3$ | $C(CH_3)_3$ | 1,52 (s;9H), 2,70 (s;3H), 4,00 (s;3H) |
| $CH_3$ | $C_2H_5$ | 1,34 (t;3H), 2,73 (s;3H), 4,00 (s;3H), 4,32 (q;2H) |
| $CF_3$ | $C_2H_5$ | 1,39 (t;3H), 4,06 (s;3H), 4,42 (q;2H) |
| $(CH_3)_2CH$ | $C_2H_5$ | 1,34 (t;3H), 1,37 (d;6H), 3,73 (sp;1H), 4,00 (s;3H), 4,29 (q;2H) |

Vorstufe 7β

4-Ethoxycarbonyl-5-methyl-isoxazol-3-carbonsäure

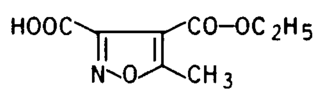

Zu 25,0 g (0,117 mol) 4-Ethoxycarbonyl-5-methyl-isoxazol-3-carbonsäuremethylester in 200 ml trockenem Tetrahydrofuran tropfte man unter $N_2$-Atmosphäre bei -15 bis -10 °C 4,7 g (0,117 mol) Natriumhydroxid in 80 ml Wasser und rührte 12 h. Man zog die Solventien am Rotationsverdampfer ab (Badtemperatur 30 - 35 °C), nahm den Rückstand in 250 ml Wasser auf, stellte mit Salzsäure auf pH = 8 - 9 ein und extrahierte zweimal mit je 150 ml Diethylether. Anschließend säuerte man die wäßrige Phase mit 6N HCl auf pH = 2 an und extrahierte viermal mit je 250 ml Ethylacetat. Die vereinigten organischen Phasen wurden über Magnesiumsulfat getrocknet und das Solvens im Vakuum abgezogen. Ausbeute: 70 %; $^1$H-NMR (250 MHz; ($CDCl_3$): δ = 1,42 ppm (t; 3H), 2,80 ppm (s; 3H), 4,59 ppm (q; 2H).

Auf analoge Weise wurden beispielsweise die folgenden Isoxazol-3-carbonsäuren synthetisiert:

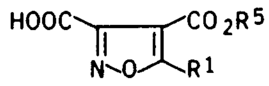

| $R^1$ | $R^5$ | $^1H$-NMR (250 MHz; $CDCl_3$) δ in ppm |
|---|---|---|
| $CH_3$ | $C_2H_5$ | 1,42 (t;3H), 2,80 (s;3H), 4,59 (q;2H) |
| $CH_3$ | $C(CH_3)_3$ | 1,54 (s;9H), 2,34 (s;3H), 13,80 (bs;1H) |
| $CF_3$ | $C_2H_5$ | 1,29 (t;3H), 4,15 (q;2H) |

Beispiel 8

3-tert.-Butylaminocarbonyl-5-methyl-isoxazol-4-carbonsäure (Nr. 3.002 in Tabelle 3)

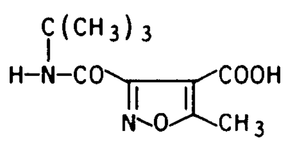

Zu einer Lösung von 5,4 g (21,3 mmol) 4-Ethoxycarbonyl-5-methyl-isoxazol-3-carbonsäure-tert.-butylamid in 100 ml Ethanol tropfte man unter $N_2$ bei 5 bis 10 °C 1,0 g (25,0 mmol) Natriumhydroxid in 50 ml Wasser und rührte 12 h bei Raumtemperatur. Man engte die Lösung ein, nahm den Rückstand in 150 ml Wasser auf, stellte auf pH = 8 - 9 ein und extrahierte zweimal mit je 100 ml Diethylether. Danach säuerte man die wäßrige Phase mit 6N HCl auf pH = 2 an und extrahierte viermal mit je 200 ml Ethylacetat. Die vereinigten organischen Phasen wurden über Magnesiumsulfat getrocknet und das Solvens im Vakuum abgezogen. Ausbeute: 95 %; gelber Farbstoff.

Beispiel 9

5-Cyclopropylaminocarbonyl-3-isopropenyl-isoxazol-4-carbonsäuremethylester (Nr. 6.02 in Tabelle 6)

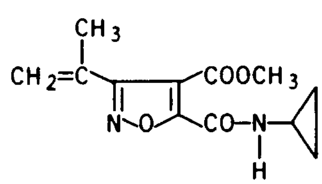

Zu einer Lösung von 9,0 g (0,043 mol) 3-Isopropenyl-4-methoxycarbonyl-isoxazol-5-carbonsäure in 200 ml Dichlormethan wurden bei 5 °C nacheinander 3,1 g (0,055 mol) Cyclopropylamin, 16,0 g (0,158 mol) 4-Methylmorpholin, 1,73 g (0,014 mol) 4-Dimethylaminopyridin und 37,0 g (0,058 mol) einer 50 %igen Lösung aus Propanphosphonsäureanhydrid in Dichlormethan getropft. Nach 12-stündigem Rühren bei Raumtemperatur wurde das Lösungsmittel entfernt, der Rückstand in 250 ml Ethylacetat aufgenommen, zweimal mit gesättigter Natriumhydrogencarbonatlösung und je einmal mit 5 %iger Zitronensäurelösung, gesättigter Natriumcarbonat- und Natriumchloridlösung extrahiert. Nach Trocknen über Magnesiumsulfat wurde das

Solvens bei reduziertem Druck abdestilliert. Ausbeute: 54 %.

Vorstufe 9α

E-3-Styryl-isoxazol-4,5-dicarbonsäuredimethylester

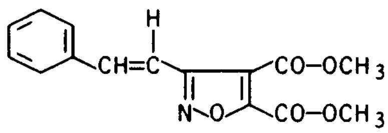

Eine auf 0 °C gekühlte Mischung aus 14,7 g (0,1 mol) E-Zimtaldehydoxim, 1,8 g (0,01 mol) Dinatriumhydrogenphosphat-Dihydrat, 1,6 g (0,01 mol) Natriumdihydrogenphosphat-Dihydrat, 50 ml Dichlormethan und 50 ml Wasser, wurde mit Salzsäure/Natronlauge auf pH = 6,3 eingestellt. Nach Zugabe von 14,2 g (0,1 mol) Acetylendicarbonsäuredimethylester wurden innerhalb einer Stunde bei 0-10 °C 61,2 g (0,11 mol) einer 13,4 %igen wäßrigen Natriumhypochlorid-Lösung zugetropft und gleichzeitig der pH-Wert durch Zugabe von Salzsäure oder Natronlauge konstant gehalten. Anschließend wurde 1 Std. bei Raumtemperatur nachgerührt, die Phasen getrennt und die wäßrige Phase zweimal mit je 100 ml Dichlormethan extrahiert. Nach Trocknen der vereinigten organischen Phasen über Natriumsulfat wurde das Lösungsmittel im Vakuum entfernt und die leichter flüchtigen Komponenten bei 120 °C (0,1 Torr) abdestilliert. Ausbeute: 56 %; $^1$H-NMR (in $CDCl_3$): δ = 3,9 ppm (s; 3H), 4,0 ppm (s; 3H), 7,2 ppm (d; 1H), 7,3-7,7 ppm (m; 6H).

Analog Vorstufe 9α wurde auch der 3-Isopropenyl-isoxazol-4,5-dicarbonsäuredimethylester synthetisiert. Ausbeute: 26 %; $^1$H-NMR (in $CDCl_3$): δ = 2,2 ppm (s; 3H), 3,9 ppm (s; 3H), 4,0 ppm (s; 3H), 5,45 ppm (m; 2H).

Vorstufe 9β

3-Isopropenyl-4-methoxycarbonyl-isoxazol-5-carbonsäure

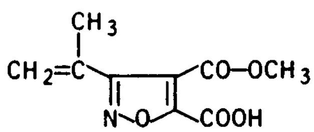

Zu 20,0 g (0,09 mol) 3-Isopropenylisoxazol-4,5-dicarbonsäuredimethylester (aus Stufe A.1) in 150 ml Methanol wurde bei 0 °C eine Lösung aus 3,6 g (0,09 mol) Natriumhydroxid in 75 ml Wasser getropft und anschließend 14 Std. bei Raumtemperatur gerührt. Nach Zugabe von 250 ml Wasser wurde auf pH = 8 eingestellt, mit 200 ml Dichlormethan extrahiert, die wäßrige Phase mit 6N-Salzsäure auf pH = 1-2 eingestellt und dreimal mit je 250 ml Dichlormethan extrahiert. Die vereinigten organischen Extrakte wurden über Magnesiumsulfat getrocknet und das Lösungsmittel unter Vakuum abdestilliert. Ausbeute: 97 %; $^1$H-NMR (in $CDCl_3$): δ = 2,12 ppm (s; 3H), 4,07 ppm (s; 3H), 5,41 ppm (m; 2H); 8,00 ppm (bs; 1H).

Beispiel 10

5- Cyclopropylaminocarbonyl-3-isopropenyl-isoxazol-4-carbonsäure (Nr. 6.05 in Tabelle 6)

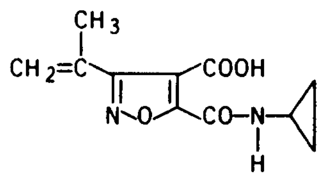

Zu einer Lösung von 4,0 g (0,016 mol) 5-Cyclopropylaminocarbonyl-3-isopropenyl-4-carbonsäuremethylester (hergestellt nach Beispiel 9) in 50 ml Methanol wurden bei 5 bis 10 °C 0,68 g (0,017 mol) Natriumhydroxid gegeben. Nach 12-stündigem Rühren bei Raumtemperatur wurde das Lösungsmittel entfernt, der Rückstand in 150 ml Wasser aufgenommen, die Mischung auf pH = 8-9 eingestellt und zweimal mit je 100 ml Diethylether extrahiert. Danach säuerte man die wäßrige Phase mit 6N HCl auf pH = 2 an, extrahierte viermal mit je 200 ml Ethylacetat, trocknete die vereinigten organischen Phasen über Magnesiumsulfat und destillierte das Lösungsmittel bei reduziertem Druck ab. Ausbeute: 74 %.

Beispiel 11

5-tert.-Butylaminocarbonyl-3-isopropenyl-isoxazol-4-carbonsäureacetonoximester (Nr. 6.08 in Tabelle 6)

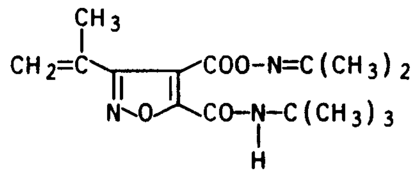

Zu einer Lösung von 4,0 g (0,016 mol) 5-tert.-Butylaminocarbonyl-3-isopropenyl-isoxazol-4-carbonsäure (Nr.6.06 in Tabelle 6) und 1,5 g (0,021 mol) Acetonoxim in 200 ml Dichlormethan wurden bei Raumtemperatur 5,9 g (0,059 mol) 4-Methylmorpholin und 1,94 g (0,016 mol) 4-Dimethylaminopyridin getropft und nach 5-minütigem Rühren 13,7 g (0,0215 mol) einer 50 %igen Lösung von Propanphosphonsäureanhydrid in Dichlormethan zugegeben. Nach 8-stündigem Erhitzen auf Rückflußtemperatur wurde das Lösungsmittel entfernt, der Rückstand in 100 ml Ethylacetat gelöst und analog Beispiel 9 auf das Produkt hin aufgearbeitet. Ausbeute: 55%.

Die physikalischen Daten der Endprodukte (Beispiele 1 bis 10) sind den folgenden Tabellen 3 bis 7 zu entnehmen, in denen noch weitere Verbindungen Ia, Ib, Ic und Id aufgeführt sind, welche auf die gleichen Weisen hergestellt wurden oder herstellbar sind.

Tabelle 3

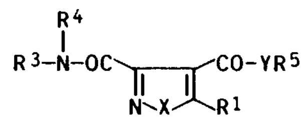

Ia ($R^2$ = CO–$YR^5$)

| Nr. | $R^1$ | $R^3$ | $R^4$ | $R^5$ | X | Y | Fp. [°C]/$^1$H-NMR (250 MHz, $CDCl_3$), δ in ppm |
|---|---|---|---|---|---|---|---|
| 3.001 | Methyl | H | iso-Propyl | H | O | O | 134-137 |
| 3.002 | Methyl | H | tert.-Butyl | H | O | O | 90- 93 |
| 3.003 | Methyl | H | cyclo-Propyl | H | O | O | 120-125 |
| 3.004 | Methyl | H | Phenyl | H | O | O | 178-181 |
| 3.005 | Methyl | H | tert.-Butyl | $-N=C(CH_3)_2$ | O | O | 110-113 |
| 3.006 | Methyl | H | tert.-Butyl | Succinimido | O | O | 172-178 |
| 3.007 | Methyl | H | cyclo-Propyl | $-N=C(CH_3)_2$ | O | O | 160-163 |
| 3.008 | Methyl | H | Methyl | H | O | O | 180-184 |
| 3.009 | Methyl | H | Ethyl | H | O | O | 130-134 |
| 3.010 | Methyl | H | tert.-Butyl | 2,4-Cl,Cl-Phenyl | O | O | 134-137 |
| 3.011 | Methyl | H | tert.-Butyl | 2,3-$OCH_3$, $OCH_3$-Phenyl | O | O | 170-173 |
| 3.012 | Methyl | H | tert.-Butyl | 4-$OCH_3$-Phenyl | O | O | 141-143 |
| 3.013 | n-Propyl | H | tert.-Butyl | H | O | O | 1.00 (t; 3H), 1,52 (s; 9H), 1,80 (m; 2H), 3,25 (t; 2H), 7,17 (bs; 1H, NH) |

Tabelle 3 (Fortsetzung)

| Nr. | $R^1$ | $R^3$ | $R^4$ | $R^5$ | X | Y | Fp. [°C]/$^1$H-NMR (250 MHz, $CDCl_3$), δ in ppm |
|---|---|---|---|---|---|---|---|
| 3.014 | n-Propyl | H | tert.-Butyl | $-N=C(CH_3)_2$ | O | O | 95- 96 |
| 3.015 | n-Propyl | H | tert.-Butyl | Propargyl | O | O | 60- 66 |
| 3.016 | iso-Propyl | H | cyclo-Propyl | H | O | O | 70- 71 |
| 3.017 | iso-Propyl | H | cyclo-Propyl | $-N=C(CH_3)_2$ | O | O | 105-107 |
| 3.018 | iso-Propyl | H | tert.-Butyl | $-N=C(CH_3)_2$ | O | O | 139-143 |
| 3.019 | iso-Propyl | H | tert.-Butyl | H | O | O | 32- 35 |
| 3.020 | n-Butyl | H | tert.-Butyl | H | O | O | 0,94 (t; 3H), 1,41 (m; 2H), 1,49 (s; 9H), 1,74 (m; 2H), 3,27 (t; 2H), 7,25 (bs;1H,NH) |
| 3.021 | n-Butyl | H | cyclo-Propyl | H | O | O | 74- 77 |
| 3.022 | n-Butyl | H | cyclo-Propyl | $-N=C(CH_3)_2$ | O | O | 62- 67 |
| 3.023 | tert.-Butyl | H | tert.-Butyl | H | O | O | 95- 99 |
| 3.024 | tert.-Butyl | H | tert.-Butyl | $-N=C(CH_3)_2$ | O | O | 101-104 |
| 3.025 | tert.-Butyl | H | cyclo-Propyl | H | O | O | 70- 72 |
| 3.026 | tert.-Butyl | H | cyclo-Propyl | $-N=C(CH_3)_2$ | O | O | 122-125 |
| 3.027 | sek.-Butyl | H | tert.-Butyl | H | O | O | 40 |
| 3.028 | sek.-Butyl | H | cyclo-Propyl | H | O | O | 0,80 (m; 2H), 0,92 (m; 2H), 0,90 (t; 3H), 1,30 (d; 3H), 1,77 (m;2H), 3,00 (m; 1H), 4,00 (m; 1H), 7,83 (bs;1H,NH) |
| 3.029 | sek.-Butyl | H | tert.-Butyl | $-N=C(CH_3)_2$ | O | O | 97-101 |

Tabelle 3 (Fortsetzung)

| Nr. | $R^1$ | $R^3$ | $R^4$ | $R^5$ | X | Y | Fp. [°C]/$^1$H-NMR (250 MHz, $CDCl_3$), δ in ppm |
|---|---|---|---|---|---|---|---|
| 3.030 | sek.-Butyl | H | cyclo-Propyl | $-N=C(CH_3)_2$ | O | O | 0,66 (m; 2H), 0,89 (m; 2H), 0,90 (t; 3H), 1,36 (d; 3H), 1,72 (m; 2H), 2,08 und 2,09 (2s; 6H), 2,92 (m; 1H), 3,58 (m; 1H), 7.25 (bs;1H,NH) |
| 3.031 | Phenyl | H | tert.-Butyl | H | O | O | 133-135 |
| 3.032 | Phenyl | H | cyclo-Propyl | H | O | O | 144-146 |
| 3.033 | Phenyl | H | cyclo-Propyl | $-N=C(CH_3)_2$ | O | O | 153-155 |
| 3.034 | Phenyl | H | tert.-Butyl | $-N=C(CH_3)_2$ | O | O | 131 |
| 3.035 | 2,4-Cl,Cl-Phenyl | H | tert.-Butyl | H | O | O | 1,54 (s; 9H), 7,25 (bs;1H,NH), 7,20-7,90 (m; 3H) |
| 3.036 | Methyl | H | tert.-Butyl | Ethyl | O | O | 1,40 (t; 3H), 1,48 (s; 9H), 2,70 (s; 3H), 4,37 (q; 2H), 7,89 (bs; 1H, NH) |
| 3.037 | iso-Propyl | H | tert.-Butyl | $-N=C(C_2H_5)(CH_3)$ | O | O | 107-112 |
| 3.038 | iso-Propyl | H | tert.-Butyl | 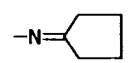 | O | O | 137-141 |
| 3.039 | iso-Propyl | H | tert.-Butyl | 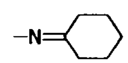 | O | O | 114-117 |

Tabelle 3 (Fortsetzung)

| Nr. | $R^1$ | $R^3$ | $R^4$ | $R^5$ | X | Y | Fp. [°C]/$^1H$-NMR (250 MHz, $CDCl_3$), δ in ppm |
|---|---|---|---|---|---|---|---|
| 3.040 | iso-Propyl | H | tert.-Butyl | $CH_2CH=CH-C_6H_5$ | O | O | 1,38 (d; 6H), 1,44 (s; 9H), 3,72 (sp; 1H), 4,94 (m; 2H), 6,20-6,90 (m; 2H), 7,22-7,40 (m; 5H) |
| 3.041 | iso-Propyl | H | tert.-Butyl | $CH_2CCl_3$ | O | O | 68- 71 |
| 3.042 | iso-Propyl | H | tert.-Butyl | $CH_2-CH_2-CN$ | O | O | 93- 95 |
| 3.043 | iso-Propyl | H | tert.-Butyl | $CH_2CF_3$ | O | O | 73- 76 |
| 3.044 | iso-Propyl | H | tert.-Butyl | 3-Jod-propargyl | O | O | 1,38 (d; 6H), 1,44 (s; 9H), 3,70 (sp; 1H), 4,40 (s; 2H), 6,70 (bs; 1H, NH) |
| 3.045 | Phenyl | H | tert.-Butyl | H | S | O | > 190 (Zers.) |
| 3.046 | iso-Propyl | H | tert.-Butyl | Methyl | O | O | 123-124 |
| 3.047 | 1-Methoxy-eth-1-yl | H | tert.-Butyl | H | O | O | 1,55 (d; 3H); 1,55 (s; 9H); 3,35 (s; 3H); 5,46 (q; 1H); 7,30 (bs; 1H, NH) |
| 3.048 | 1-Methoxy-eth-1-yl | H | Cyclopropyl | H | O | O | 97- 98 |
| 3.049 | 1-Methoxy-eth-1-yl | H | sek.-Butyl | H | O | O | 83- 87 |

Tabelle 3 (Fortsetzung)

| Nr. | $R^1$ | $R^3$ | $R^4$ | $R^5$ | X | Y | Fp. [°C]/$^1$H-NMR (250 MHz, $CDCl_3$), δ in ppm |
|---|---|---|---|---|---|---|---|
| 3.050 | 1-Methoxy-eth-1-yl | H | Cyclopropyl-methyl | H | O | O | 0,38 (m; 2H); 0,63 (m; 2H); 1,10 (m; 1H); 1,57 (d; 3H); 3,38 (s; 3H); 3,40 (m; 2H); 5,46 (q; 1H); 7,64 (bs, 1H, NH) |
| 3.051 | 1-Methoxy-eth-1-yl | H | Cyclopropyl-methyl | $-N=C(CH_3)_2$ | O | O | 96- 99 |
| 3.052 | 1-Methoxy-eth-1-yl | H | tert.-Butyl | $-N=C(CH_3)_2$ | O | O | 99-101 |
| 3.053 | 1-Methoxy-eth-1-yl | H | tert.-Butyl | Propargyl | O | O | 1,46 (s; 9H); 1,58 (d; 3H); 2,53 (t; 1H); 3,36 (s; 3H); 4,91 (d; 2H); 5,08 (q; 1H); 6,67 (bs,1H,NH) |
| 3.054 | 1-Methoxy-eth-1-yl | H | Cyclopropyl | $-N=C(CH_3)_2$ | O | O | 89- 92 |
| 3.055 | 1-Methoxy-eth-1-yl | H | Cyclopropyl | Propargyl | O | O | 94- 98 |
| 3.056 | 1-Methoxy-eth-1-yl | H | sek.-Butyl | $-N=C(CH_3)_2$ | O | O | 76- 79 |
| 3.057 | Methoxymethyl | H | tert.-Butyl | H | O | O | 1,52 (s; 9H); 3,50 (s; 3H); 5,05 (s; 2H); 7,26 (bs; 1H, NH) |
| 3.058 | Methoxymethyl | H | sek.-Butyl | H | O | O | 50- 56 |
| 3.059 | Methoxymethyl | H | Cyclopropyl | H | O | O | 56- 62 |

Tabelle 3 (Fortsetzung)

| Nr. | $R^1$ | $R^3$ | $R^4$ | $R^5$ | X | Y | Fp. [°C]/$^1$H-NMR (250 MHz, $CDCl_3$), δ in ppm |
|---|---|---|---|---|---|---|---|
| 3.060 | Ethyl | H | Cyclopropyl | Me | O | O | 0,58-0,96 (m; 4H); 1,32 (t; 3H); 2,90 (m; 1H); 3,09 (q; 2H); 3,91 (s; 3H); 7,91 (bs, 1H, NH) |
| 3.061 | Ethyl | H | Cyclopropyl | H | O | O | 0,80-1,10 (m; 4H); 1,30 (t; 3H); 3,00 (m; 1H); 3,26 (q; 2H); 8,06 (bs, 1H, NH) |
| 3.062 | Cyclohexyl | H | tert.-Butyl | H | O | O | 126-129 |
| 3.063 | Cyclohexyl | H | Cyclopropyl | H | O | O | 140-144 |
| 3.064 | Cyclohexyl | H | 1-Cyclo-propyl-ethyl | H | O | O | 125-130 |
| 3.065 | Cyclopropyl | H | tert.-Butyl | H | O | O | 88- 91 |
| 3.066 | Cyclopropyl | H | Cyclopropyl | H | O | O | 98-103 |
| 3.067 | Cyclopropyl | H | 1-Cyclo-propyl-ethyl | H | O | O | 55- 60 |
| 3.068 | 1-Methylcyclo-hexyl | H | tert.-Butyl | H | O | O | 108-112 |
| 3.069 | 1-Methylcyclo-hexyl | H | Cyclopropyl | H | O | O | 102-107 |
| 3.070 | Cyclopropyl | H | tert.-Butyl | $-N=C(CH_3)_2$ | O | O | 150-152 |
| 3.071 | iso-Propyl | H | tert.-Butyl | Ethyl | O | O | 69- 72 |
| 3.072 | Ethyl | H | Cyclopropyl | $-N=C(CH_3)_2$ | O | O | 87- 88 |

Tabelle 3 (Fortsetzung)

| Nr. | $R^1$ | $R^3$ | $R^4$ | $R^5$ | X | Y | Fp. [°C]/$^1$H-NMR (250 MHz, $CDCl_3$), δ in ppm |
|---|---|---|---|---|---|---|---|
| 3.073 | Cyclopropyl | H | Cyclopropyl-methyl | H | O | O | 102-110 |
| 3.074 | Cyclopropyl | H | sek.-Butyl | H | O | O | 92- 98 |
| 3.075 | Cyclopropyl | H | Cyclopentyl | H | O | O | 78- 84 |
| 3.076 | Cyclopropyl | H | Cyclohexyl | H | O | O | 80- 87 |
| 3.077 | 2-Methyltetra-hydropyran-2-yl | H | tert.-Butyl | H | O | O | 93- 96 |
| 3.078 | 2-Methyltetra-hydropyran-2-yl | H | Cyclopropyl | H | O | O | 104-112 |
| 3.079 | 2-Methyl-norbornan-2-yl | H | Cyclopropyl | H | O | O | 133-136 |
| 3.080 | 4-F-Phenyl | H | tert.-Butyl | H | O | O | 93- 96 |
| 3.081 | Cyclopropyl | H | 1,1-Dimethyl--2-propenyl | H | O | O | 1,24 (m; 2H); 1,56 (s; 6H); 3,20 (m; 1H); 5,20 (m; 2H); 6,09 (m; 1H); 7,26 (bs; 1H; NH) |
| 3.082 | Cyclopropyl | H | Benzyl | H | O | O | 145-159 |
| 3.083 | Cyclopropyl | H | 2-Methoxy-eth-1-yl | H | O | O | 91- 96 |
| 3.084 | Cyclopropyl | H | 2-Propenyl | H | O | O | 109-114 |
| 3.085 | Methyl | H | tert.-Butyl | 2,4-(Br,Br) 4-CN-Phenyl | O | O | 192-203 |

Tabelle 3 (Fortsetzung)

| Nr. | R1 | R3 | R4 | R5 | X | Y | Fp. [°C]/$^1$H-NMR (250 MHz, $CDCl_3$), δ in ppm |
|---|---|---|---|---|---|---|---|
| 3.086* | Methyl | H | tert.-Butyl | $-CH_2-CH=N-OC_2H_5$ | O | O | Hauptisomer: 1,24 (t; 3H); 1,46 (s; 3H); 2,68 (s; 3H); 4,16 (m; 2H); 4,84 (d; 2H); 7,08 (bs; 1H, NH); 7,49 (t; 1H) |
| 3.087 | Methyl | H | tert.-Butyl | $Na^⊕$ | O | O | 298-300 |
| 3.088 | Methyl | H | tert.-Butyl | $K^⊕$ | O | O | 256-257 |
| 3.089 | Methyl | H | Cyclobutyl | H | O | O | 119-124 |
| 3.090 | Methyl | H | 1,1-Dimethyl-propyl | H | O | O | 61- 66 |
| 3.091 | Methyl | H | tert.-Butyl | $NH_4^⊕$ | O | O | 226-230 |
| 3.092 | Methyl | H | tert.-Butyl | $-CH_2-CH=N-OCH_3$ | O | O | 83- 90 |
| 3.093 | Methyl | H | tert.-Butyl | 4-CN-Phenyl | O | O | 119-125 |
| 3.094 | Methyl | H | tert.-Butyl | Propargyl | O | O | 93- 96 |
| 3.095 | Methyl | H | 2,2-Dimethyl-propyl | H | O | O | 125-129 |
| 3.096 | Methyl | H | tert.-Butyl | -N 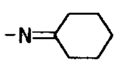 | O | O | 124-127 |
| 3.097 | Methyl | H | tert.-Butyl | $H_3N^⊕-CH(CH_3)_2$ | O | O | 175-180 |
| 3.098 | 2-Furanyl | H | tert.-Butyl | H | O | O | 114-117 |

*) Isomerengemisch

Tabelle 3 (Fortsetzung)

| Nr. | $R^1$ | $R^3$ | $R^4$ | $R^5$ | X | Y | Fp. [°C]/$^1$H-NMR (250 MHz, $CDCl_3$), δ in ppm |
|---|---|---|---|---|---|---|---|
| 3.099 | 2-Furanyl | H | Cyclopropyl | H | O | O | 124-127 |
| 3.100 | Methyl | H | tert.-Butyl | $-CH_2-CH=CH-C_6H_5$ | O | O | |
| 3.101 | Methyl | H | sek.-Butyl | H | O | O | 78- 83 |
| 3.102 | Methyl | H | 2-Methoxy-eth-1-yl | H | O | O | 74- 77 |
| 3.103 | iso-Propyl | H | Cyclopropyl | $Na^{\oplus}$ | O | O | 241 |
| 3.104 | iso-Propyl | H | Cyclopropyl | $K^{\oplus}$ | O | O | 208-209 |
| 3.105 | Methyl | H | 1,1-Dimethyl-2-propenyl | H | O | O | 58- 82 |
| 3.106 | iso-Propyl | H | Cyclopropyl | $H_3N^{\oplus}-CH(CH_3)_2$ | O | O | 170-176 |
| 3.107 | iso-Propyl | H | Cyclopropyl | $NH_4^{\oplus}$ | O | O | 221-223 |
| 3.108 | Methyl | H | tert.-Butyl | 2-Cl-Phenyl | O | O | 168-170 |
| 3.109 | Methyl | H | tert.-Butyl | 3-Cl-Phenyl | O | O | 125-128 |
| 3.110 | Methyl | H | Cyclopentyl | H | O | O | 99-104 |
| 3.111 | Methyl | H | tert.-Butyl | 4-Cl-Phenyl | O | O | 112-117 |
| 3.112 | Methyl | H | tert.-Butyl | 4-F-Phenyl | O | O | 173-176 |
| 3.113 | Methyl | H | tert.-Butyl | $4-CH_3$-Phenyl | O | O | 132-134 |
| 3.114 | Methyl | H | tert.-Butyl | 2-F-Phenyl | O | O | 148-150 |
| 3.115 | Methyl | H | tert.-Butyl | Phenyl | O | O | 152-157 |
| 3.116 | Methyl | H | tert.-Butyl | Methyl | O | O | 129-132 |
| 3.117 | Methyl | H | tert.-Butyl | iso-Propyl | O | O | 108-112 |
| 3.118 | Methyl | H | tert.-Butyl | tert.-Butyl | O | S | 1,46 (s; 9H); 1,60 (s; 9H); 2,71 (s; 3H); 6,75 (bs; 1H, NH) |
| 3.119 | Methyl | H | tert.-Butyl | Cyclohexyl | O | O | 86- 90 |

Tabelle 3 (Fortsetzung)

| Nr. | $R^1$ | $R^3$ | $R^4$ | $R^5$ | X | Y | Fp. [°C]/$^1$H-NMR (250 MHz, $CDCl_3$), δ in ppm |
|---|---|---|---|---|---|---|---|
| 3.120 | 2,4,6-Trimethylphenyl | H | tert.-Butyl | H | O | O | 1,53 (s;9H), 2,08 (s;3H), 2,10 (s; 6H), 6,94 (s;2H), 7,30 (bs;1H,NH) |
| 3.121 | Ethyl | Methyl | Ethyl | Methyl | O | O | 1,17 und 1,27 (2t; 3H+3H); 1,35 (t; 3H); 2,89 und 3,12 (2s; 3H+3H); 3,18 (q; 2H); 3,25 und 3,63 (2q; 2H+2H); 3,84 (s; 3H)*) |
| 3.122 | Ethyl | Methyl | Ethyl | H | O | O | 1,27 und 1,30 (2t; 3H+3H); 1,34 (t; 3H), 3,18 und 3,28 (2s; 3H+3H); 3,24 (q; 2H), 3,67 und 3,68 (2q; 2H+2H)*) |
| 3.123 | Methyl | H | Cyclopropyl | Methyl | O | O | 0,60-0,92 (m; 4H); 2,70 (s; 3H); 2,93 (m; 1H); 3,92 (s; 3H); 8,27 (bs; 1H, NH) |
| 3.124 | iso-Propyl | H | Cyclopropyl | Methyl | O | O | 0,60-0,93 (m; 4H); 1,36 (d; 6H); 2,92 (m; 1H); 3,70 (sp; 1H); 3,89 (s; 3H); 7,84 (bs; 1H, NH) |
| 3.125 | Ethyl | H | tert.-Butyl | Methyl | O | O | 1,32 (t; 3H); 1,48 (s; 9H); 3,09 (q; 2H); 3,88 (s; 3H); 7,23 (bs; 1H, NH) |
| 3.126 | Ethyl | H | tert.-Butyl | H | O | O | 1,36 (t; 3H); 1,52 (s; 9H); 3,28 (q; 2H); 7,22 (bs; 1H, NH) |

*) Mischung aus 2 Rotameren

Tabelle 4

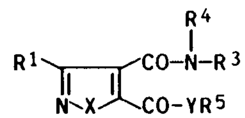

Ic ($R^2$ = CO–Y$R^5$)

| Nr. | $R^1$ | $R^3$ | $R^4$ | $R^5$ | X | Y | Fp. [°C]/$^1$H-NMR (250 MHz, $CDCl_3$), δ in ppm |
|---|---|---|---|---|---|---|---|
| 4.001 | Methyl | H | tert.-Butyl | H | O | O | 142-147 |
| 4.002 | Ethyl | H | tert.-Butyl | H | O | O | 124-127 |
| 4.003 | iso-Propyl | H | tert.-Butyl | H | O | O | 142-145 |
| 4.004 | iso-Propyl | H | Cyclopropyl | H | O | O | 136-138 |
| 4.005 | Methyl | H | tert.-Butyl | H | S | O | 134 |
| 4.006 | Methyl | H | Cyclopropyl | H | S | O | 131 |
| 4.007 | Methyl | H | iso-Propyl | H | S | O | 111 |
| 4.008 | Methyl | H | tert.-Butyl | $-N=C(CH_3)_2$ | S | O | 116-117 |
| 4.009 | iso-Propyl | H | 3-$CF_3$-Phenyl | H | S | O | 144 |
| 4.010 | iso-Propyl | H | tert.-Butyl | H | S | O | 164 |
| 4.011 | iso-Propyl | H | tert.-Butyl | $-N=C(CH_3)_2$ | S | O | 166-167 |
| 4.012 | Phenyl | H | 4-Cl-Phenyl | H | S | O | 169 |
| 4.013 | Phenyl | H | Cyclopropyl | H | S | O | 167 |
| 4.014 | Phenyl | H | Cyclopropyl | $-N=C(CH_3)_2$ | S | O | 148-149 |
| 4.015 | iso-Propyl | H | tert.-Butyl | Methyl | S | O | 133-134 |
| 4.016 | Methyl | H | 2,4,6-Trimethylphenyl | $-N=C(CH_3)_2$ | O | O | 135 |
| 4.017 | iso-Propyl | H | tert.-Butyl | Methyl | O | O | 121-123 |

Tabelle 5

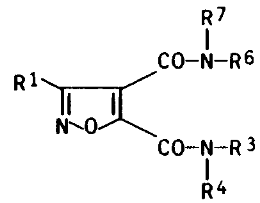

Ic 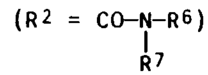

| Nr. | $R^1$ | $R^3$ | $R^4$ | $R^6$ | $R^7$ | Fp. [°C]/$^1$H-NMR (250 MHz, $CDCl_3$), δ in ppm |
|---|---|---|---|---|---|---|
| 5.01 | Methyl | $-(CH_2)_5-$ | | H | tert.-Butyl | 74-75 |
| 5.02 | 4-F-Phenyl | $-(CH_2)_5-$ | | H | tert.-Butyl | 1,40 (s; 9H), 1,73 (m; 6H), 3,40 (m; 2H), 3,73 (m; 2H), 7,14 (m; 2H), 7,59 (bs;1H,NH), 7,73 (m; 2H) |
| 5.03 | iso-Propyl | H | tert.-Butyl | H | tert.-Butyl | 111-114 |
| 5.04 | Methyl | H | tert.-Butyl | H | H | 155-158 |
| 5.05 | Ethyl | $-(CH_2)_5-$ | | H | tert.-Butyl | 1,34 (t; 3H), 1,41 (s; 9H), 1,50-1,80 (m; 6H), 3,00 (q; 2H), 3,34-3,43 (m; 2H), 3,68-3,76 (m; 2H), 8,10 (bs; 1H, NH) |
| 5.06 | iso-Propyl | $-(CH_2)_5-$ | | H | tert.-Butyl | 76- 77 |
| 5.07 | iso-Propyl | $-(CH_2)_5-$ | | H | Cyclopropyl | 50- 52 |
| 5.08 | Cyclopropyl | $-(CH_2)_5-$ | | H | tert.-Butyl | 65 |
| 5.09 | Methyl | H | Cyclopropyl | H | Cyclopropyl | 110-114 |

Tabelle 6

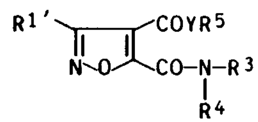

Id (R2 = CO-YR5)

| Nr. | R1' | R3 | R4 | R5 | Y | Fp. [°C]/1H-NMR (250 MHz, $CDCl_3$), δ [ppm] |
|---|---|---|---|---|---|---|
| 6.01 | $CH_2$=C($CH_3$)- | H | iso-Propyl | Methyl | O | 1.29 (d; 6H), 2.12 (bs; 3H), 3.94 (s; 3H) 4.26 (sp; 1H) 5.42 (m; 2H), 8.05 (bs; 1H,NH) |
| 6.02 | $CH_2$=C($CH_3$)- | H | Cyclopropyl | Methyl | O | 0.63-0.92 (m; 4H), 2.10 (bs; 3H), 2.93 (m; 1H), 3.93 (s; 3H) 5.40 (m; 2H), 8.40 (bs; 1H, NH) |
| 6.03 | $CH_2$=C($CH_3$)- | H | tert.-Butyl | Methyl | O | 1.48 (s; 9H), 2.1 (bs; 3H), 3.93 (s; 3H) 5.40 (m; 2H), 8.07 (bs; 1H, NH) |
| 6.04 | $CH_2$=C($CH_3$)- | H | iso-Propyl | H | O | 84- 87 |
| 6.05 | $CH_2$=C($CH_3$)- | H | Cyclopropyl | H | O | 96-100 |
| 6.06 | $CH_2$=C($CH_3$)- | H | tert.-Butyl | H | O | 1.55 (s; 9H), 2.15 (bs; 3H), 5.40 (m; 2H) 7.15 (bs; 1H, NH) |
| 6.07 | $CH_2$=C($CH_3$)- | H | -C($CH_3$)$_2$C≡CH | H | O | 1.82 (s; 6H), 2.17 (bs; 3H), 2.53 (s; 1H), 5.40 (m; 2H), 7.43 (bs; 1H, NH) |
| 6.08 | $CH_2$=C($CH_3$)- | H | tert.-Butyl | -N=C($CH_3$)$_2$ | O | 1.44 (s; 9H), 2.07 und 2.10 (2s; 6H) 2.17 (s; 3H), 5.42 (m; 2H), 8.17 (bs; 1H, NH) |
| 6.09 | E-$C_6H_5$-CH=CH- | H | tert.-Butyl | Methyl | O | 1.48 (s; 9H), 4.00 (s; 3H), 7.12-7.61 (m; 7H), 9.13 (bs; 1H, NH) |
| 6.10 | E-$C_6H_5$-CH=CH | H | tert.-Butyl | H | O | 75- 80 |
| 6.11 | $CH_2$=C($CH_3$)- | H | -C($CH_3$)$_2$C≡CH | Methyl | O | 1.78 (s; 6H), 2.10 (s; 3H), 2.41 (s; 1H) 3.93 (s; 3H), 5.41 (m; 2H), 8.63 (bs; 1H, NH) |

Tabelle 6 (Fortsetzung)

| Nr. | R1' | R3 | R4 | R5 | Y | Fp. [°C]/$^1$H-NMR (250 MHz, $CDCl_3$), $\delta$ [ppm] |
|---|---|---|---|---|---|---|
| 6.12 | $CH_2=C(C_2H_5)-$ | H | tert.-Butyl | H | O | 80- 81 |
| 6.13 | $CH_2=C(C_2H_5)-$ | H | $-C(CH_3)_2C\equiv CH$ | H | O | 1,08 (t;3H), 1,80 (s;6H), 2,48 (m;2H), 2,50 (s;1H), 5,30 und 5,44 (2s;2H), 7,50 (bs;1H,NH) |
| 6.14 | $CH_2=C(C_2H_5)-$ | H | Cyclopropyl | H | O | 73- 74 |
| 6.15 | $CH_2=C(C_2H_5)-$ | H | Cyclopropyl | $-N=C(CH_3)_2$ | O | 48- 52 |
| 6.16 | $CH_2=C(i-C_3H_7)-$ | H | tert.-Butyl | H | O | 114-117 |
| 6.17 | $E-CH_3-CH=CH-$ | H | tert.-Butyl | H | O | 64- 69 |
| 6.18 | $CH_2=C(i-C_3H_7)-$ | H | tert.-Butyl | $-N=C(CH_3)_2$ | O | 1,10 (d;6H), 1,48 (s;9H), 2,02 und 2,12 (2s;6H), 2,70(m;1H), 5,34 und 5,41((2s;2H), 8,36 (bs;1H,NH) |
| 6.19 | $E-CH_3-CH=CH-$ | H | tert.-Butyl | $-N=C(CH_3)_2$ | O | 78- 82 |
| 6.20 | $CH_2=C(i-C_3H_7)-$ | H | tert.-Butyl | Methyl | O | 1,12 (d;6H), 1,48 (s;9H), 2,66 (m;1H), 3,90 (s;3H), 5,24 und 5,39 (2s;2H), 8,50 (bs;1H,NH) |
| 6.21 | $CH_2=C(i-C_3H_7)-$ | H | Cyclopropyl | Methyl | O | 0,60-0,94 (m;4H), 1,11 (d;6H), 2,64 (m;1H), 3,00 (m;1H), 3,88 (s;3H), 5,24 und 5,39 (2s;2H), 8,87 (bs;1H,NH) |
| 6.22 | $E-CH_3-CH=CH-$ | H | tert.-Butyl | Methyl | O | 1,48 (s;9H), 1,95 (dd;3H), 3,94 (s;3H), 6,43-6,79 (m;2H), 9,07 (bs;1H,NH) |

Tabelle 7

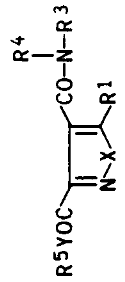

Ib ($R^2$ = CO–YR5)

| Nr. | $R^1$ | $R^3$ | $R^4$ | $R^5$ | X | Y | Fp. [°C]/$^1$H-NMR (250 MHz, $CDCl_3$), δ in ppm |
|---|---|---|---|---|---|---|---|
| 7.01 | Methyl | H | tert.-Butyl | Methyl | O | O | 97- 99 |
| 7.02 | Methyl | H | tert.-Butyl | H | O | O | 152-154 |
| 7.03 | iso-Propyl | H | tert.-Butyl | $-N=C(CH_3)_2$ | O | O | 58- 61 |

Anwendungsbeispiele

Die herbizide Wirkung der Carbonsäureamide der Formeln Ia′, Ib′, Ic′ und Id ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und

Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 1,0 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Cassia tora | - |
| Chenopodium album | Weißer Gänsefuß |
| Chrysanthemum coronarium | Kronenwucherblume |
| Ipomoea spp. | Prunkwindearten |
| Polygonum persicaria | Flohknöterich |
| Stellaria media | Vogelsternmiere |
| Triticum aestivum | Winterweizen |

Mit 1,0 kg/ha a.S. im Nachauflaufverfahren eingesetzt, lassen sich mit den Verbindungen 3.002, 3.005, 3.006, 3.008, 3.016, 3.017, 3.018, 3.019, 3.020, 3.023, 3.028, 3.029, 3.037, 3.052, 3.053, 6.04, 6.05, 6.06 und 6.08 breitblättrige unerwünschte Pflanzen sehr gut bekämpfen. Die Carbonsäureamide 3.006, 3.016 bis 3.020, 3.028, 3.029, 3.037, 3.052, 3.053, 6.04, 6.05, 6.06 und 6.08 sind gleichzeitig verträglich für Gramineen-Kulturen wie Weizen, Mais und Reis.

**Patentansprüche**

**Patentansprüche für folgende Vertragsstaaten : BE, CH, DE, FR, GB, IT, LI, NL, SE**

**1.** Carbonsäureamide der Formeln Ia, Ib, Ic und Id

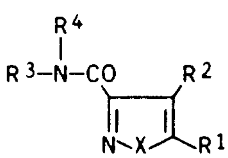
Ia

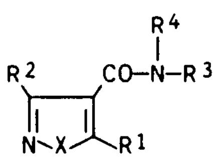
Ib

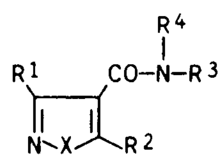
Ic

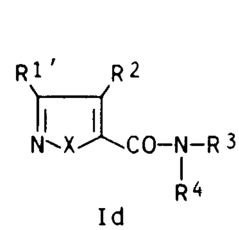
Id

in denen die Variablen folgende Bedeutung haben:

X Sauerstoff oder Schwefel;

$R^1$

- Wasserstoff, Halogen, $C_1$-$C_6$-Alkyl, welches ein bis fünf Halogenatome und/oder einen Cyanorest und/oder bis zu zwei der folgenden Reste tragen kann: $C_1$-$C_4$-Alkoxy, partiell oder vollständig halogeniertes $C_1$-$C_4$-Alkoxy, $C_1$-$C_4$-Alkylthio oder partiell oder vollständig halogeniertes $C_1$-$C_4$-Alkylthio;
- eine $C_1$-$C_4$-Alkoxy- oder $C_1$-$C_4$-Alkylthiogruppe, eine partiell oder vollständig halogenierte $C_1$-$C_4$-Alkoxygruppe, eine partiell oder vollständig halogenierte $C_1$-$C_4$-Alkylthiogruppe;
- die Benzylgruppe, die ein- bis dreimal durch $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio, partiell oder vollständig halogeniertes $C_1$-$C_4$-Alkylthio, Halogen, Cyano oder Nitro substituiert sein kann;

- die Phenylgruppe, welche noch einen bis drei der folgenden Reste tragen kann: Cyano, Nitro, Halogen, $C_1$-$C_6$-Alkyl, partiell oder vollständig halogeniertes $C_1$-$C_6$-Alkyl, $C_1$-$C_6$-Alkoxy, partiell oder vollständig halogeniertes $C_1$-$C_6$-Alkoxy, $C_1$-$C_6$-Alkylthio und/oder partiell oder vollständig halogeniertes $C_1$-$C_6$-Alkylthio;
- die Phenoxy- oder die Phenylthiogruppe, wobei beide Gruppen ein- bis dreimal durch $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio, partiell oder vollständig halogeniertes $C_1$-$C_4$-Alkylthio, Halogen, Cyano oder Nitro substituiert sein können;
- ein 5- bis 6-gliedriger gesättigter oder aromatischer heterocyclischer Rest, enthaltend ein oder zwei Heteroatome, ausgewählt aus der Gruppe Sauerstoff, Schwefel und Stickstoff, der ein oder zwei der folgenden Substituenten tragen kann: Halogen, $C_1$-$C_3$-Alkyl, $C_1$-$C_3$-Alkoxy und $C_1$-$C_3$-Alkoxycarbonyl;
- eine durch $C_3$-$C_8$-Cycloalkyl substituierte $C_1$-$C_6$-Alkylgruppe,
- eine $C_2$-$C_6$-Alkenylgruppe, deren Doppelbindung epoxidiert sein kann, oder eine $C_2$-$C_6$-Alkinylgruppe, wobei beide Gruppen ein- bis dreimal durch Halogen, $C_1$-$C_3$-Alkoxy und/oder einmal durch Cyclopropyl oder Phenyl substituiert sein können, wobei der Phenylrest zusätzlich bis zu drei der folgenden Substituenten tragen kann: Halogen, Cyano, Nitro, $C_1$-$C_4$-Alkyl, das unsubstituiert oder partiell oder vollständig halogeniert sein kann, $C_1$-$C_4$-Alkoxy oder $C_1$-$C_4$-Alkylthio, die beide unsubstituiert oder partiell oder vollständig halogeniert sein können,
- eine $C_3$-$C_8$-Cycloalkyl- oder eine $C_3$-$C_6$-Cycloalkenylgruppe, wobei beide Gruppen ein- bis dreimal durch $C_1$-$C_4$-Alkyl oder Halogen substituiert sein können;

$R^{1'}$

- eine durch $C_3$-$C_8$-Cycloalkyl substituierte $C_1$-$C_6$-Alkylgruppe;
- eine $C_2$-$C_6$-Alkenylgruppe, deren Doppelbindung epoxidiert sein kann, oder eine $C_2$-$C_6$-Alkinylgruppe, wobei beide Gruppen ein- bis dreimal durch Halogen, $C_1$-$C_3$-Alkoxy und/oder einmal durch Cyclopropyl oder Phenyl substituiert sein können, wobei der Phenylrest zusätzlich bis zu drei der folgenden Substituenten tragen kann: Halogen, Cyano, Nitro, $C_1$-$C_4$-Alkyl, das unsubstituiert oder partiell oder vollständig halogeniert sein kann, $C_1$-$C_4$-Alkoxy oder $C_1$-$C_4$-Alkylthio, die beide unsubstituiert oder partiell oder vollständig halogeniert sein können;
- eine $C_3$-$C_6$-Cycloalkenylgruppe, die ein- bis dreimal durch Halogen oder $C_1$-$C_4$-Alkyl substituiert sein kann;

$R^2$

- eine Formylgruppe, eine 4,5-Dihydrooxazol-2-ylgruppe,
- ein Rest $COYR^5$ oder $CONR^6R^7$, wobei die Variablen die folgende Bedeutung haben:

Y Sauerstoff oder Schwefel;

$R^5$

- Wasserstoff;
- eine $C_1$-$C_6$-Alkylgruppe, welche ein bis fünf Halogenatome und/oder bis zu drei Hydroxy- und/oder $C_1$-$C_4$-Alkoxygruppen und/oder einen der folgenden Reste tragen kann:
  - Cyano,
  - $C_1$-$C_4$-Alkoxy-$C_2$-$C_4$-alkoxy,
  - $C_1$-$C_3$-Alkylthio,
  - $C_1$-$C_3$-Alkylamino, Di-($C_1$-$C_3$)-alkylamino, $C_3$-$C_6$-Cycloalkylamino oder Di-($C_3$-$C_6$)-cycloalkylamino,
  - Trimethylsilyl,
  - $C_1$-$C_3$-Alkylsulfinyl oder $C_1$-$C_3$-Alkylsulfonyl, Carboxyl, $C_1$-$C_3$-Alkoxycarbonyl, $C_1$-$C_3$-Alkoxycarbonyl-$C_1$-$C_3$-alkoxy oder $C_1$-$C_3$-Alkoxycarbonyl-$C_1$-$C_3$-alkoxy-$C_1$-$C_3$-alkoxycarbonyl,
  - Di-($C_1$-$C_3$)-alkylaminocarbonyl,
  - Di-($C_1$-$C_3$)-alkoxyphosphonyl,
  - $C_1$-$C_6$-Alkaniminoxy oder $C_5$-$C_6$-Cycloalkaniminoxy,
  - N-Phthalimido, N-Succinimido, Benzyloxy, Benzoyl, wobei diese cyclischen Reste zusätzlich eine bis drei der folgenden Gruppen tragen können: Halogen, $C_1$-

$C_3$-Alkyl oder $C_1$-$C_3$-Alkoxy,

- einen 5- oder 6-gliedrigen gesättigten heterocyclischen Rest oder einen 5- oder 6-gliedrigen heteroaromatischen Rest mit jeweils bis zu 3 Heteroatomen, ausgewählt aus der Gruppe Sauerstoff, Schwefel und Stickstoff, wobei zwei Sauerstoff- und/oder Schwefelatome nicht direkt benachbart sein können und wobei die Heterocyclen noch einen oder zwei der folgenden Substituenten tragen können: Halogen, $C_1$-$C_3$-Alkyl, $C_1$-$C_3$-Alkoxy oder $C_1$-$C_3$-Alkoxycarbonyl;
- Phenyl, das noch bis zu drei der folgenden Substituenten tragen kann: Halogen, Nitro, Cyano, $C_1$-$C_3$-Alkyl, partiell oder vollständig halogeniertes $C_1$-$C_3$-Alkyl, $C_1$-$C_3$-Alkoxy oder partiell oder vollständig halogeniertes $C_1$-$C_3$-Alkoxy;
- einen Rest $-CR^{10}=N-R^{11}$, wobei $R^{10}$ und $R^{11}$ die folgende Bedeutung haben:

$R^{10}$ Wasserstoff oder $C_1$-$C_6$-Alkyl und

$R^{11}$ $C_1$-$C_6$-Alkoxy, $C_3$-$C_6$-Alkenyloxy oder $C_3$-$C_6$-Alkinyloxy, die jeweils bis zu 3 Halogenatome und/oder einen Phenylrest mit gewünschtenfalls bis zu drei der folgenden Reste tragen können: Halogen, Nitro, Cyano, $C_1$-$C_3$-Alkyl oder $C_1$-$C_3$-Alkoxy;
Phenoxy, das noch bis zu drei der folgenden Substituenten tragen kann: Halogen, Nitro, Cyano, $C_1$-$C_3$-Alkyl oder $C_1$-$C_3$-Alkoxy;
$C_1$-$C_6$-Alkylamino, Di-($C_1$-$C_6$)-alkylamino oder Phenylamino, wobei der Aromat zusätzlich bis zu drei der folgenden Reste tragen kann: Halogen, Nitro, Cyano, $C_1$-$C_3$-Alkyl oder $C_1$-$C_3$-Alkoxy;

- $C_3$-$C_8$-Cycloalkyl;
- $C_3$-$C_6$-Alkenyl, $C_5$-$C_6$-Cycloalkenyl, $C_3$-$C_6$-Alkinyl, wobei diese Reste eine der folgenden Gruppen tragen können: Hydroxy, Halogen, $C_1$-$C_4$-Alkoxy oder Phenyl, wobei der Aromat seinerseits eine bis drei der folgenden Gruppen tragen kann: Halogen, Nitro, Cyano, $C_1$-$C_4$-Alkyl, partiell oder vollständig halogeniertes $C_1$-$C_4$-Alkyl oder $C_1$-$C_4$-Alkoxy;
- Phenyl, das eine bis drei der folgenden Gruppen tragen kann: Halogen, Nitro, Cyano, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy oder $C_1$-$C_4$-Alkoxycarbonyl;
- einen fünf- oder sechsgliedrigen heterocyclischen Rest mit bis zu drei Heteroatomen, ausgewählt aus der Gruppe Sauerstoff, Schwefel und Stickstoff, wobei zwei Sauerstoff- und/oder Schwefelatome nicht direkt benachbart sein können, und wobei die Heterocyclen noch einen oder zwei der folgenden Substituenten tragen können: Halogen, $C_1$-$C_3$-Alkoxy oder $C_1$-$C_3$-Alkoxycarbonyl;
- einen Benzotriazolrest;
- N-Phthalimido, Tetrahydrophthalimido, Succinimido, Maleinimido;
- die 2,2-Dimethyl-1,3-dioxolan-4-ylmethyl- oder 1,3-Dioxolan-2-on-4-ylmethylgruppe;
- im Falle Y = O: ein Äquivalent eines Kations aus der Gruppe der Alkali- und Erdalkalimetalle, Mangan, Kupfer, Eisen, Ammonium und mit bis zu 4 $C_1$-$C_3$-Alkylgruppen substituiertes Ammonium; oder
- ein Rest $-N=CR^8R^9$, wobei

$R^8$, $R^9$ Wasserstoff; $C_1$-$C_4$-Alkyl, das unsubstituiert oder partiell oder vollständig halogeniert sein kann und einen $C_1$-$C_3$-Alkoxy- oder Phenylrest tragen kann, wobei der aromatische Rest seinerseits noch ein- bis dreimal durch Halogen, Nitro, Cyano, $C_1$-$C_3$-Alkyl, partiell oder vollständig halogeniertes $C_1$-$C_3$-Alkyl, $C_1$-$C_3$-Alkoxy oder partiell oder vollständig halogeniertes $C_1$-$C_3$-Alkoxy substituiert sein kann; $C_3$-$C_6$-Cycloalkyl; $C_1$-$C_4$-Alkoxy; Furanyl oder Phenyl, das zusätzlich bis zu 3 der folgenden Substituenten tragen kann: Halogen, Nitro, Cyano, $C_1$-$C_3$-Alkyl, partiell oder vollständig halogeniertes $C_1$-$C_3$-Alkyl, $C_1$-$C_3$-Alkoxy oder partiell oder vollständig halogeniertes $C_1$-$C_3$-Alkoxy; oder $R^8$ und $R^9$ gemeinsam eine Methylenkette mit 4 bis 7 Gliedern bedeuten;

- einen Rest -W-Z, wobei W eine $C_2$-$C_4$-Alkylenkette, eine Ethoxyethylenkette, eine But-2-enylen- oder eine But-2-inylenkette bedeutet und Z einen in $\omega$-Stellung an W gebundenen Molekülteil, der den gleichen Molekülteil darstellt, der in $\alpha$-Stellung von W mit W verknüpft ist, bedeutet;

$R^6$

- Wasserstoff, $C_1$-$C_6$-Alkyl oder $C_3$-$C_8$-Cycloalkyl und

$R^7$

- Wasserstoff, $C_1$-$C_6$-Alkyl, -C($OR^{12}$) = N-H oder -C($OR^{12}$) = N-($C_1$-$C_4$)-alkyl, wobei $R^{12}$ $C_1$-$C_4$-Alkyl bedeutet oder

$R^6$,$R^7$ gemeinsam eine Methylenkette mit 4 oder 5 Gliedern;

$R^3$

- Wasserstoff;
- $C_1$-$C_6$-Alkyl, das einen bis drei der folgenden Substituenten tragen kann: Hydroxy, Halogen, $C_1$-$C_4$-Alkoxy, $C_1$-$C_4$-Alkylthio oder Di-($C_1$-$C_4$)-alkylamino;
- $C_3$-$C_8$-Cycloalkyl, das ein- bis dreimal durch Halogen, $C_1$-$C_4$-Alkyl und partiell oder vollständig halogeniertes $C_1$-$C_4$-Alkyl substituiert sein kann;

$R^4$

- Wasserstoff, Hydroxyl, eine $C_1$-$C_4$-Alkoxygruppe;
- eine $C_1$-$C_6$-Alkylgruppe, die einen bis drei der folgenden Reste tragen kann: Halogen, Cyano, $C_1$-$C_4$-Alkoxy, partiell oder vollständig halogeniertes $C_1$-$C_4$-Alkoxy, $C_1$-$C_4$-Alkylthio, partiell oder vollständig halogeniertes $C_1$-$C_4$-Alkylthio, Di-$C_1$-$C_4$-alkylamino, $C_3$-$C_8$-Cycloalkyl oder Phenyl, wobei der Phenylring seinerseits einen bis drei der folgenden Reste tragen kann: Halogen, Cyano, Nitro, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio oder partiell oder vollständig halogeniertes $C_1$-$C_4$-Alkylthio;
- eine $C_3$-$C_8$-Cycloalkylgruppe, die einen bis drei der folgenden Reste tragen kann: Halogen, Nitro, Cyano, $C_1$-$C_6$-Alkyl, partiell oder vollständig halogeniertes $C_1$-$C_6$-Alkyl, $C_1$-$C_4$-Alkoxy oder partiell oder vollständig halogeniertes $C_1$-$C_4$-Alkoxy;
- eine $C_3$-$C_6$-Alkenyl- oder $C_3$-$C_6$-Alkinylgruppe, die jeweils ein- bis dreimal durch Halogen und/oder einmal durch Phenyl substituiert sein können, wobei der Phenylrest seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
- eine Di-($C_1$-$C_4$)-alkylaminogruppe;
- ein 5- bis 6-gliedriger heterocyclischer gesättigter oder aromatischer Rest mit einem oder zwei Heteroatomen, ausgewählt aus der Gruppe Sauerstoff, Schwefel und Stickstoff, der ein- bis dreimal durch $C_1$-$C_4$-Alkyl oder Halogen substituiert sein kann;
- eine Phenylgruppe, die eine bis vier der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro, Cyano, Formyl, $C_1$-$C_4$-Alkanoyl, $C_1$-$C_4$-Halogenalkanoyl oder $C_1$-$C_4$-Alkoxycarbonyl;
- eine Naphthylgruppe, die ein- bis dreimal durch $C_1$-$C_4$-Alkyl oder Halogen substituiert sein kann;

oder

$R^3$, $R^4$ gemeinsam eine Methylenkette mit 4 bis 7 Gliedern, welche durch Sauerstoff, Schwefel oder N-Methyl unterbrochen sein kann, oder den Rest -$(CH_2)_3$-CO-,

wobei im Falle der Verbindungen Ia bis Ic $R^3$ und $R^4$ nicht gleichzeitig Wasserstoff bedeuten, wenn

- $R^1$ Wasserstoff, Methyl oder Phenyl und $R^2$ $CONH_2$, $CO_2H$ oder $CO_2CH_3$ bedeuten oder wenn
- X Sauerstoff, $R^1$ $CH(OCH_2CH_3)_2$ und $R^2$ $CONH_2$ bedeuten,

sowie deren umweltverträglichen Salze.

**2.** Carbonsäureamide der Formeln Ia, Ib, Ic, Id nach Anspruch 1, wobei $R^3$ Wasserstoff bedeutet.

**3.** Carbonsäureamide der Formeln Ia, Ib, Ic nach Anspruch 1, in denen

$R^1$ Wasserstoff, $C_1$-$C_4$-Alkyl oder $C_3$-$C_6$-Cycloalkyl;

$R^2$ $COYR^5$, wobei Y für Sauerstoff und $R^5$ für Wasserstoff oder den Rest -N = $CR^8R^9$ stehen, wobei

$R^8$, $R^9$ dabei unabhängig voneinander Wasserstoff, $C_1$-$C_4$-Alkyl und $C_3$-$C_6$-Cycloalkyl bedeuten oder zusammen eine $C_4$-$C_7$-Alkylenkette bilden;

$R^3$ Wasserstoff und

$R^4$ $C_1$-$C_4$-Alkyl oder $C_3$-$C_8$-Cycloalkyl

bedeuten.

4. Carbonsäureamide der Formel Ia nach den Ansprüchen 1 bis 3.

5. Verfahren zur Herstellung der Verbindungen Ia und Ib gemäß Anspruch 1, in denen $R^2$ $CO_2CH_3$ und X Sauerstoff bedeutet, dadurch gekennzeichnet, daß man das Hydroxamsäurechlorid der Formel II

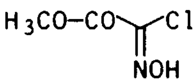
II

in an sich bekannter Weise in Gegenwart einer Base mit einem β-Ketoester der Formel III

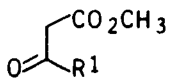
III

umsetzt, den so erhaltenen Dimethyldiester IV

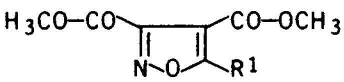
IV

anschließend zunächst mit einem Äquivalent einer wäßrigen Base zu den Monoestern Va und Vb

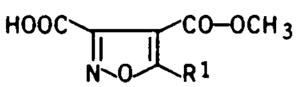
Va

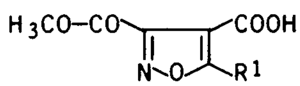
Vb

hydrolysiert und Va und Vb danach getrennt oder im Gemisch zuerst in an sich bekannter Weise in die Halogenide oder andere aktivierte Formen der Carbonsäuren überführt und diese Derivate anschließend mit einem substituierten Amin der Formel VIa

$HNR^3R^4$ VIa

amidiert.

6. Verfahren zur Herstellung der Verbindungen Ia gemäß Anspruch 1, in denen $R^2$ $CO_2R^5$, X Sauerstoff und $R^5$ nicht Wasserstoff oder Methyl bedeuten, dadurch gekennzeichnet, daß man das Hydroxamsäurechlorid II in an sich bekannter Weise in Gegenwart einer Base mit einem β-Ketoester der Formel IIIa

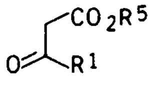
IIIa

umsetzt, den so erhaltenen Diester IVa

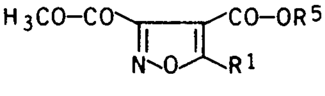
IVa

anschließend mit einem Verseifungsreagens zum Monoester Va′

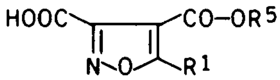

Va′

hydrolysiert, Va′ in an sich bekannter Weise in die Halogenide oder andere aktivierte Formen der Carbonsäuren überführt und diese Derivate anschließend mit einem substituierten Amin VIa amidiert.

**7.** Verfahren zur Herstellung der Verbindungen Ia und Ic gemäß Anspruch 1, in denen $R^1$ nicht Wasserstoff und $R^2$ Carboxyl oder Formyl und $R^3$ Wasserstoff bedeuten, dadurch gekennzeichnet, daß man eine Carbonsäure Vc oder Vd

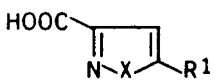

Vc

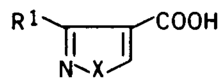

Vd

in an sich bekannter Weise in die Halogenide oder andere aktivierte Formen der Carbonsäuren überführt, diese Derivate mit einem substituierten Amin VIa amidiert und das so erhaltene Amid VIIa bzw. VIIb

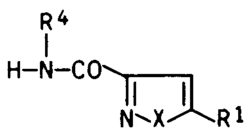

VIIa

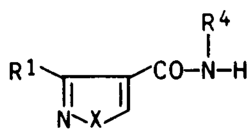

VIIb

anschließend in an sich bekannter Weise in Gegenwart einer Base mit einem Carboxylierungs- oder einem Formylierungsreagens umsetzt.

**8.** Verfahren zur Herstellung der Verbindungen Ia und Ib gemäß Anspruch 1, in denen $R^2$ eine Carboxylgruppe und X Schwefel bedeutet, dadurch gekennzeichnet, daß man ein Isothiazoldicarbonsäureanhydrid VIII

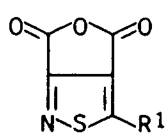

VIII

in an sich bekannter Weise mit einem Amin VIa zu den Isomeren Ia und Ib umsetzt und anschließend die Isomeren trennt.

**9.** Verfahren zur Herstellung der Verbindungen Ia, Ib, Ic und Id gemäß Anspruch 1, in denen $R^2$ $CO_2H$ bedeutet, dadurch gekennzeichnet, daß man einen entsprechenden Ester Ia, Ib, Ic oder Id, in dem $R^2$ $CO_2R^5$ und $R^5$ $C_1$-$C_4$-Alkyl bedeutet, in an sich bekannter Weise in Gegenwart einer wäßrigen Base hydrolysiert.

**10.** Verfahren zur Herstellung der Verbindungen Ia, Ib, Ic und Id gemäß Anspruch 1, in denen $R^2$ eine Gruppe $COYR^5$ oder $CONR^6R^7$ bedeutet, dadurch gekennzeichnet, daß man eine entsprechende Carbonsäure Ia, Ib, Ic bzw. Id ($R^2 = CO_2H$) zunächst in an sich bekannter Weise in die Halogenide oder

andere aktivierte Derivate der Carbonsäuren überführt und diese Derivate anschließend in an sich bekannter Weise mit einer Verbindung IX

$HYR^5$ IX

oder mit einem Amin VIb

$HNR^6R^7$ VIb

derivatisiert.

**11.** Verfahren zur Herstellung der Verbindungen Ia, Ib, Ic und Id gemäß Anspruch 1, in denen $R^2$ 4,5-Dihydrooxazol-2-yl bedeutet, dadurch gekennzeichnet, daß man ein Carbonsäureamid der Formel Ia, Ib, Ic oder Id gemäß Anspruch 1, in der $R^2$ eine Gruppe $CO_2H$ oder $CO_2R^5$ und $R^5$ $C_1$-$C_4$-Alkyl bedeuten, in an sich bekannter Weise mit einem Aminoalkohol XV

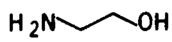

XV

cyclisiert.

**12.** Verfahren zur Herstellung der Verbindungen Ia, Ib, Ic und Id gemäß Anspruch 1, in denen $R^2$ Formyl bedeutet, dadurch gekennzeichnet, daß man eine entsprechende Carbonsäure der Formel Ia, Ib, Ic oder Id ($R^2 = CO_2H$) in das Halogenid oder eine andere aktivierte Form der Carbonsäure überführt und dieses Derivat anschließend in an sich bekannter Weise reduziert.

**13.** Verfahren zur Herstellung der Verbindungen Ia, Ib, Ic und Id gemäß Anspruch 1, in denen $R^1$ bzw. $R^{1'}$ eine epoxidierte $C_2$-$C_6$-Alkenylgruppe bedeutet, dadurch gekennzeichnet, daß man ein Carbonsäureamid der Formel Ia, Ib, Ic oder Id gemäß Anspruch 1, wobei $R^1$ bzw. $R^{1'}$ eine $C_2$-$C_6$-Alkenylgruppe bedeutet, in an sich bekannter Weise mit einem Oxidationsmittel epoxidiert.

**14.** Mittel, enthaltend inerte Trägerstoffe und eine herbizid wirksame Menge mindestens eines Carbonsäureamides der Formel Ia, Ib, Ic oder Id gemäß den Ansprüchen 1 bis 4.

**15.** Verfahren zur Bekämpfung unerwünschten Pflanzenwuchses, dadurch gekennzeichnet, daß man eine herbizid wirksame Menge eines Carbonsäureamids der Formel Id gemäß Anspruch 1 oder eines Carbonsäureamids der Formel Ia′, Ib′ oder Ic′, wobei Ia′, Ib′ und Ic′ die Bedeutung von Ia, Ib bzw. Ic ohne die Ausnahmebestimmung hat, auf Pflanzen, deren Lebensraum oder auf Saatgut einwirken läßt.

**Patentansprüche für folgenden Vertragsstaat : ES**

**1.** Mittel, enthaltend inerte Trägerstoffe und eine herbizid wirksame Menge mindestens eines Carbonsäureamides der Formel Ia, Ib, Ic oder Id

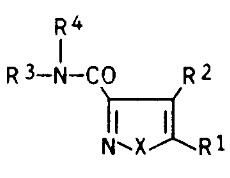
Ia

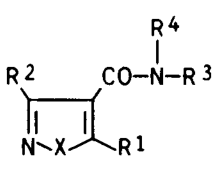
Ib

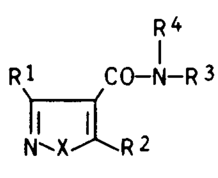
Ic

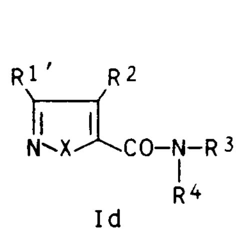
Id

in denen die Variablen folgende Bedeutung haben:

X Sauerstoff oder Schwefel;

$R^1$

- Wasserstoff, Halogen, $C_1$-$C_6$-Alkyl, welches ein bis fünf Halogenatome und/oder einen Cyanorest und/oder bis zu zwei der folgenden Reste tragen kann: $C_1$-$C_4$-Alkoxy, partiell oder vollständig halogeniertes $C_1$-$C_4$-Alkoxy, $C_1$-$C_4$-Alkylthio oder partiell oder vollständig halogeniertes $C_1$-$C_4$-Alkylthio;
- eine $C_1$-$C_4$-Alkoxy- oder $C_1$-$C_4$-Alkylthiogruppe, eine partiell oder vollständig halogenierte $C_1$-$C_4$-Alkoxygruppe, eine partiell oder vollständig halogenierte $C_1$-$C_4$-Alkylthiogruppe;
- die Benzylgruppe, die ein- bis dreimal durch $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio, partiell oder vollständig halogeniertes $C_1$-$C_4$-Alkylthio, Halogen, Cyano oder Nitro substituiert sein kann;
- die Phenylgruppe, welche noch einen bis drei der folgenden Reste tragen kann: Cyano, Nitro, Halogen, $C_1$-$C_6$-Alkyl, partiell oder vollständig halogeniertes $C_1$-$C_6$-Alkyl, $C_1$-$C_6$-Alkoxy, partiell oder vollständig halogeniertes $C_1$-$C_6$-Alkoxy, $C_1$-$C_6$-Alkylthio und/oder partiell oder vollständig halogeniertes $C_1$-$C_6$-Alkylthio;
- die Phenoxy- oder die Phenylthiogruppe, wobei beide Gruppen ein- bis dreimal durch $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio, partiell oder vollständig halogeniertes $C_1$-$C_4$-Alkylthio, Halogen, Cyano oder Nitro substituiert sein können;
- ein 5- bis 6-gliedriger gesättigter oder aromatischer heterocyclischer Rest, enthaltend ein oder zwei Heteroatome, ausgewählt aus der Gruppe Sauerstoff, Schwefel und Stickstoff, der ein oder zwei der folgenden Substituenten tragen kann: Halogen, $C_1$-$C_3$-Alkyl, $C_1$-$C_3$-Alkoxy und $C_1$-$C_3$-Alkoxycarbonyl;
- eine durch $C_3$-$C_8$-Cycloalkyl substituierte $C_1$-$C_6$-Alkylgruppe,
- eine $C_2$-$C_6$-Alkenylgruppe, deren Doppelbindung epoxidiert sein kann, oder eine $C_2$-$C_6$-Alkinylgruppe, wobei beide Gruppen ein- bis dreimal durch Halogen, $C_1$-$C_3$-Alkoxy und/oder einmal durch Cyclopropyl oder Phenyl substituiert sein können, wobei der Phenylrest zusätzlich bis zu drei der folgenden Substituenten tragen kann: Halogen, Cyano, Nitro, $C_1$-$C_4$-Alkyl, das unsubstituiert oder partiell oder vollständig halogeniert sein kann, $C_1$-$C_4$-Alkoxy oder $C_1$-$C_4$-Alkylthio, die beide unsubstituiert oder partiell oder vollständig halogeniert sein können,
- eine $C_3$-$C_8$-Cycloalkyl- oder eine $C_3$-$C_6$-Cycloalkenylgruppe, wobei beide Gruppen ein- bis dreimal durch $C_1$-$C_4$-Alkyl oder Halogen substituiert sein können;

$R^{1'}$

- eine durch $C_3$-$C_8$-Cycloalkyl substituierte $C_1$-$C_6$-Alkylgruppe;
- eine $C_2$-$C_6$-Alkenylgruppe, deren Doppelbindung epoxidiert sein kann, oder eine $C_2$-$C_6$-Alkinylgruppe, wobei beide Gruppen ein- bis dreimal durch Halogen, $C_1$-$C_3$-Alkoxy und/oder einmal durch Cyclopropyl oder Phenyl substituiert sein können, wobei der Phenylrest zusätzlich bis zu drei der folgenden Substituenten tragen kann: Halogen, Cyano, Nitro, $C_1$-$C_4$-Alkyl, das unsubstituiert oder partiell oder vollständig halogeniert sein kann, $C_1$-$C_4$-Alkoxy oder $C_1$-$C_4$-Alkylthio, die beide unsubstituiert oder partiell oder vollständig halogeniert sein können;
- eine $C_3$-$C_6$-Cycloalkenylgruppe, die ein- bis dreimal durch Halogen oder $C_1$-$C_4$-Alkyl substituiert sein kann;

$R^2$

- eine Formylgruppe, eine 4,5-Dihydrooxazol-2-ylgruppe,
- ein Rest $COYR^5$ oder $CONR^6R^7$, wobei die Variablen die folgende Bedeutung haben:

Y Sauerstoff oder Schwefel;

$R^5$

- Wasserstoff;
- eine $C_1$-$C_6$-Alkylgruppe, welche ein bis fünf Halogenatome und/oder bis zu drei Hydroxy- und/oder $C_1$-$C_4$-Alkoxygruppen und/oder einen der folgenden Reste tragen kann:
  - Cyano,
  - $C_1$-$C_4$-Alkoxy-$C_2$-$C_4$-alkoxy,
  - $C_1$-$C_3$-Alkylthio,

- $C_1$-$C_3$-Alkylamino, Di-($C_1$-$C_3$)-alkylamino, $C_3$-$C_6$-Cycloalkylamino oder Di-($C_3$-$C_6$)-cycloalkylamino,
- Trimethylsilyl,
- $C_1$-$C_3$-Alkylsulfinyl oder $C_1$-$C_3$-Alkylsulfonyl, Carboxyl, $C_1$-$C_3$-Alkoxycarbonyl, $C_1$-$C_3$-Alkoxycarbonyl-$C_1$-$C_3$-alkoxy oder $C_1$-$C_3$-Alkoxycarbonyl-$C_1$-$C_3$-alkoxy-$C_1$-$C_3$-alkoxycarbonyl,
- Di-($C_1$-$C_3$)-alkylaminocarbonyl,
- Di-($C_1$-$C_3$)-alkoxyphosphonyl,
- $C_1$-$C_6$-Alkaniminoxy oder $C_5$-$C_6$-Cycloalkaniminoxy,
- N-Phthalimido, N-Succinimido, Benzyloxy, Benzoyl, wobei diese cyclischen Reste zusätzlich eine bis drei der folgenden Gruppen tragen können: Halogen, $C_1$-$C_3$-Alkyl oder $C_1$-$C_3$-Alkoxy,
- einen 5- oder 6-gliedrigen gesättigten heterocyclischen Rest oder einen 5- oder 6-gliedrigen heteroaromatischen Rest mit jeweils bis zu 3 Heteroatomen, ausgewählt aus der Gruppe Sauerstoff, Schwefel und Stickstoff, wobei zwei Sauerstoff- und/oder Schwefelatome nicht direkt benachbart sein können und wobei die Heterocyclen noch einen oder zwei der folgenden Substituenten tragen können: Halogen, $C_1$-$C_3$-Alkyl, $C_1$-$C_3$-Alkoxy oder $C_1$-$C_3$-Alkoxycarbonyl;

- Phenyl, das noch bis zu drei der folgenden Substituenten tragen kann: Halogen, Nitro, Cyano, $C_1$-$C_3$-Alkyl, partiell oder vollständig halogeniertes $C_1$-$C_3$-Alkyl, $C_1$-$C_3$-Alkoxy oder partiell oder vollständig halogeniertes $C_1$-$C_3$-Alkoxy;
- einen Rest $-CR^{10}=N-R^{11}$, wobei $R^{10}$ und $R^{11}$ die folgende Bedeutung haben:

$R^{10}$ Wasserstoff oder $C_1$-$C_6$-Alkyl und

$R^{11}$ $C_1$-$C_6$-Alkoxy, $C_3$-$C_6$-Alkenyloxy oder $C_3$-$C_6$-Alkinyloxy, die jeweils bis zu 3 Halogenatome und/oder einen Phenylrest mit gewünschtenfalls bis zu drei der folgenden Reste tragen können: Halogen, Nitro, Cyano, $C_1$-$C_3$-Alkyl oder $C_1$-$C_3$-Alkoxy;
Phenoxy, das noch bis zu drei der folgenden Substituenten tragen kann: Halogen, Nitro, Cyano, $C_1$-$C_3$-Alkyl oder $C_1$-$C_3$-Alkoxy;
$C_1$-$C_3$-Alkylamino, Di-($C_1$-$C_6$)-alkylamino oder Phenylamino, wobei der Aromat zusätzlich bis zu drei der folgenden Reste tragen kann: Halogen, Nitro, Cyano, $C_1$-$C_3$-Alkyl oder $C_1$-$C_3$-Alkoxy;

- $C_3$-$C_8$-Cycloalkyl;
- $C_3$-$C_6$-Alkenyl, $C_5$-$C_6$-Cycloalkenyl, $C_3$-$C_6$-Alkinyl, wobei diese Reste eine der folgenden Gruppen tragen können: Hydroxy, Halogen, $C_1$-$C_4$-Alkoxy oder Phenyl, wobei der Aromat seinerseits eine bis drei der folgenden Gruppen tragen kann: Halogen, Nitro, Cyano, $C_1$-$C_4$-Alkyl, partiell oder vollständig halogeniertes $C_1$-$C_4$-Alkyl oder $C_1$-$C_4$-Alkoxy;
- Phenyl, das eine bis drei der folgenden Gruppen tragen kann: Halogen, Nitro, Cyano, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy oder $C_1$-$C_4$-Alkoxycarbonyl;
- einen fünf- oder sechsgliedrigen heterocyclischen Rest mit bis zu drei Heteroatomen, ausgewählt aus der Gruppe Sauerstoff, Schwefel und Stickstoff, wobei zwei Sauerstoff- und/oder Schwefelatome nicht direkt benachbart sein können, und wobei die Heterocyclen noch einen oder zwei der folgenden Substituenten tragen können: Halogen, $C_1$-$C_3$-Alkoxy oder $C_1$-$C_3$-Alkoxycarbonyl;
- einen Benzotriazolrest;
- N-Phthalimido, Tetrahydrophthalimido, Succinimido, Maleinimido;
- die 2,2-Dimethyl-1,3-dioxolan-4-ylmethyl- oder 1,3-Dioxolan-2-on-4-ylmethylgruppe;
- im Falle Y = O: ein Äquivalent eines Kations aus der Gruppe der Alkali- und Erdalkalimetalle, Mangan, Kupfer, Eisen, Ammonium und mit bis zu 4 $C_1$-$C_3$-Alkylgruppen substituiertes Ammonium; oder
- ein Rest $-N=CR^8R^9$, wobei

$R^8$, $R^9$ Wasserstoff; $C_1$-$C_4$-Alkyl, das unsubstituiert oder partiell oder vollständig halogeniert sein kann und einen $C_1$-$C_3$-Alkoxy-oder Phenylrest tragen kann, wobei der aromatische Rest seinerseits noch ein- bis dreimal durch Halogen, Nitro, Cyano, $C_1$-$C_3$-Alkyl, partiell oder vollständig halogeniertes $C_1$-$C_3$-Alkyl, $C_1$-$C_3$-Alkoxy oder partiell oder vollständig halogeniertes $C_1$-$C_3$-Alkoxy substituiert sein kann; $C_3$-$C_6$-Cycloalkyl; $C_1$-$C_4$-Alkoxy; Furanyl oder

Phenyl, das zusätzlich bis zu 3 der folgenden Substituenten tragen kann: Halogen, Nitro, Cyano, $C_1$-$C_3$-Alkyl, partiell oder vollständig halogeniertes $C_1$-$C_3$-Alkyl, $C_1$-$C_3$-Alkoxy oder partiell oder vollständig halogeniertes $C_1$-$C_3$-Alkoxy; oder $R^8$ und $R^9$ gemeinsam eine Methylenkette mit 4 bis 7 Gliedern bedeuten;

- einen Rest -W-Z, wobei W eine $C_2$-$C_4$-Alkylenkette, eine Ethoxyethylenkette, eine But-2-enylen- oder eine But-2-inylenkette bedeutet und Z einen in ω-Stellung an W gebundenen Molekülteil, der den gleichen Molekülteil darstellt, der in α-Stellung von W mit W verknüpft ist, bedeutet;

$R^6$
- Wasserstoff, $C_1$-$C_6$-Alkyl oder $C_3$-$C_8$-Cycloalkyl und

$R^7$
- Wasserstoff, $C_1$-$C_6$-Alkyl, $-C(OR^{12})=N-H$ oder $-C(OR^{12})=N-(C_1-C_4)$-alkyl, wobei $R^{12}$ $C_1$-$C_4$-Alkyl bedeutet oder

$R^6$,$R^7$ gemeinsam eine Methylenkette mit 4 oder 5 Gliedern;

$R^3$
- Wasserstoff;
- $C_1$-$C_6$-Alkyl, das einen bis drei der folgenden Substituenten tragen kann: Hydroxy, Halogen, $C_1$-$C_4$-Alkoxy, $C_1$-$C_4$-Alkylthio oder Di-($C_1$-$C_4$)-alkylamino;
- $C_3$-$C_8$-Cycloalkyl, das ein- bis dreimal durch Halogen, $C_1$-$C_4$-Alkyl und partiell oder vollständig halogeniertes $C_1$-$C_4$-Alkyl substituiert sein kann;

$R^4$
- Wasserstoff, Hydroxyl, eine $C_1$-$C_4$-Alkoxygruppe;
- eine $C_1$-$C_6$-Alkylgruppe, die einen bis drei der folgenden Reste tragen kann: Halogen, Cyano, $C_1$-$C_4$-Alkoxy, partiell oder vollständig halogeniertes $C_1$-$C_4$-Alkoxy, $C_1$-$C_4$-Alkylthio, partiell oder vollständig halogeniertes $C_1$-$C_4$-Alkylthio, Di-$C_1$-$C_4$-alkylamino, $C_3$-$C_8$-Cycloalkyl oder Phenyl, wobei der Phenylring seinerseits einen bis drei der folgenden Reste tragen kann: Halogen, Cyano, Nitro, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio oder partiell oder vollständig halogeniertes $C_1$-$C_4$-Alkylthio;
- eine $C_3$-$C_8$-Cycloalkylgruppe, die einen bis drei der folgenden Reste tragen kann: Halogen, Nitro, Cyano, $C_1$-$C_6$-Alkyl, partiell oder vollständig halogeniertes $C_1$-$C_6$-Alkyl, $C_1$-$C_4$-Alkoxy oder partiell oder vollständig halogeniertes $C_1$-$C_4$-Alkoxy;
- eine $C_3$-$C_6$-Alkenyl- oder $C_3$-$C_6$-Alkinylgruppe, die jeweils ein- bis dreimal durch Halogen und/oder einmal durch Phenyl substituiert sein können, wobei der Phenylrest seinerseits eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro;
- eine Di-($C_1$-$C_4$)-alkylaminogruppe;
- ein 5- bis 6-gliedriger heterocyclischer gesättigter oder aromatischer Rest mit einem oder zwei Heteroatomen, ausgewählt aus der Gruppe Sauerstoff, Schwefel und Stickstoff, der ein- bis dreimal durch $C_1$-$C_4$-Alkyl oder Halogen substituiert sein kann;
- eine Phenylgruppe, die eine bis vier der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Nitro, Cyano, Formyl, $C_1$-$C_4$-Alkanoyl, $C_1$-$C_4$-Halogenalkanoyl oder $C_1$-$C_4$-Alkoxycarbonyl;
- eine Naphthylgruppe, die ein- bis dreimal durch $C_1$-$C_4$-Alkyl oder Halogen substituiert sein kann;

oder

$R^3$, $R^4$ gemeinsam eine Methylenkette mit 4 bis 7 Gliedern, welche durch Sauerstoff, Schwefel oder N-Methyl unterbrochen sein kann, oder den Rest $-(CH_2)_3-CO-$,

wobei im Falle der Verbindungen Ia bis Ic $R^3$ und $R^4$ nicht gleichzeitig Wasserstoff bedeuten, wenn

- $R^1$ Wasserstoff, Methyl oder Phenyl und $R^2$ $CONH_2$, $CO_2H$ oder $CO_2CH_3$ bedeuten oder wenn
- X Sauerstoff, $R^1$ $CH(OCH_2CH_3)_2$ und $R^2$ $CONH_2$ bedeuten,

sowie deren umweltverträglichen Salze.

2. Mittel, enthaltend inerte Trägerstoffe und eine herbizid wirksame Menge mindestens eines Carbonsäureamides der Formel Ia, Ib, Ic oder Id nach Anspruch 1, wobei $R^3$ Wasserstoff bedeutet.

3. Mittel, enthaltend inerte Trägerstoffe und eine herbizid wirksame Menge mindestens eines Carbonsäureamides der Formel Ia, Ib oder Ic nach Anspruch 1, wobei die Variablen die folgende Bedeutung haben:
   - $R^1$ Wasserstoff, $C_1$-$C_4$-Alkyl oder $C_3$-$C_6$-Cycloalkyl;
   - $R^2$ $COYR^5$, wobei Y für Sauerstoff und $R^5$ für Wasserstoff oder den Rest $-N=CR^8R^9$ stehen, wobei $R^8$, $R^9$ dabei unabhängig voneinander Wasserstoff, $C_1$-$C_4$-Alkyl und $C_3$-$C_6$-Cycloalkyl bedeuten oder zusammen eine $C_4$-$C_7$-Alkylenkette bilden;
   - $R^3$ Wasserstoff und
   - $R^4$ $C_1$-$C_4$-Alkyl oder $C_3$-$C_8$-Cycloalkyl.

4. Mittel, enthaltend inerte Trägerstoffe und eine herbizid wirksame Menge mindestens eines Carbonsäureamides der Formel Ia gemäß den Ansprüchen 1 bis 3.

5. Verfahren zur Herstellung der Verbindungen Ia und Ib gemäß Anspruch 1, in denen $R^2$ $CO_2CH_3$ und X Sauerstoff bedeutet, dadurch gekennzeichnet, daß man das Hydroxamsäurechlorid der Formel II

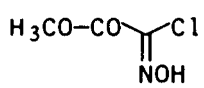

II

in an sich bekannter Weise in Gegenwart einer Base mit einem β-Ketoester der Formel III

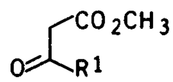

III

umsetzt, den so erhaltenen Dimethyldiester IV

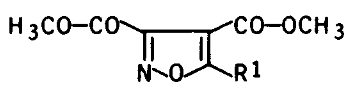

IV

anschließend zunächst mit einem Äquivalent einer wäßrigen Base zu den Monoestern Va und Vb

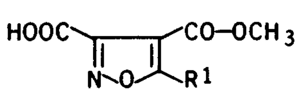

Va

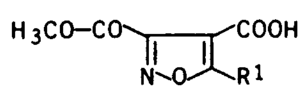

Vb

hydrolysiert und Va und Vb danach getrennt oder im Gemisch zuerst in an sich bekannter Weise in die Halogenide oder andere aktivierte Formen der Carbonsäuren überführt und diese Derivate anschließend mit einem substituierten Amin der Formel VIa

$HNR^3R^4$ VIa

amidiert.

6. Verfahren zur Herstellung der Verbindungen Ia gemäß Anspruch 1, in denen $R^2$ $CO_2R^5$, X Sauerstoff und $R^5$ nicht Wasserstoff oder Methyl bedeuten, dadurch gekennzeichnet, daß man das Hydroxamsäurechlorid II in an sich bekannter Weise in Gegenwart einer Base mit einem β-Ketoester der Formel IIIa

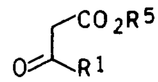

IIIa

umsetzt, den so erhaltenen Diester IVa

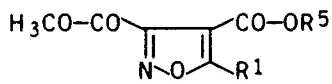

IVa

anschließend mit einem Verseifungsreagens zum Monoester Va'

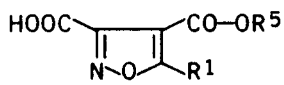

Va'

hydrolysiert, Va' in an sich bekannter Weise in die Halogenide oder andere aktivierte Formen der Carbonsäuren überführt und diese Derivate anschließend mit einem substituierten Amin VIa amidiert.

7. Verfahren zur Herstellung der Verbindungen Ia und Ic gemäß Anspruch 1, in denen $R^1$ nicht Wasserstoff und $R^2$ Carboxyl oder Formyl und $R^3$ Wasserstoff bedeuten, dadurch gekennzeichnet, daß man eine Carbonsäure Vc oder Vd

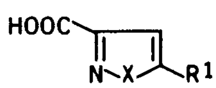

Vc

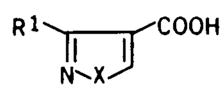

Vd

in an sich bekannter Weise in die Halogenide oder andere aktivierte Formen der Carbonsäuren überführt, diese Derivate mit einem substituierten Amin VIa amidiert und das so erhaltene Amid VIIa bzw. VIIb

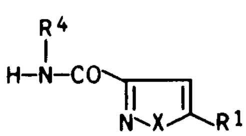

VIIa

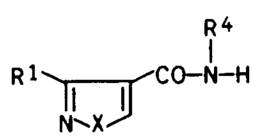

VIIb

anschließend in an sich bekannter Weise in Gegenwart einer Base mit einem Carboxylierungs- oder einem Formylierungsreagens umsetzt.

8. Verfahren zur Herstellung der Verbindungen Ia und Ib gemäß Anspruch 1, in denen $R^2$ eine Carboxylgruppe und X Schwefel bedeutet, dadurch gekennzeichnet, daß man ein Isothiazoldicarbonsäureanhydrid VIII

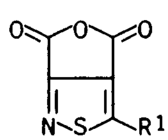

VIII

in an sich bekannter Weise mit einem Amin VIa zu den Isomeren Ia und Ib umsetzt und anschließend die Isomeren trennt.

**9.** Verfahren zur Herstellung der Verbindungen Ia, Ib, Ic und Id gemäß Anspruch 1, in denen $R^2$ $CO_2H$ bedeutet, dadurch gekennzeichnet, daß man einen entsprechenden Ester Ia, Ib, Ic oder Id, in dem $R^2$ $CO_2R^5$ und $R^5$ $C_1$-$C_4$-Alkyl bedeutet, in an sich bekannter Weise in Gegenwart einer wäßrigen Base hydrolysiert.

**10.** Verfahren zur Herstellung der Verbindungen Ia, Ib, Ic und Id gemäß Anspruch 1, in denen $R^2$ eine Gruppe $COYR^5$ oder $CONR^6R^7$ bedeutet, dadurch gekennzeichnet, daß man eine entsprechende Carbonsäure Ia, Ib, Ic bzw. Id ($R^2 = CO_2H$) zunächst in an sich bekannter Weise in die Halogenide oder andere aktivierte Derivate der Carbonsäuren überführt und diese Derivate anschließend in an sich bekannter Weise mit einer Verbindung IX

$HYR^5$ IX

oder mit einem Amin VIb

$HNR^6R^7$ VIb

derivatisiert.

**11.** Verfahren zur Herstellung der Verbindungen Ia, Ib, Ic und Id gemäß Anspruch 1, in denen $R^2$ 4,5-Dihydrooxazol-2-yl bedeutet, dadurch gekennzeichnet, daß man ein Carbonsäureamid der Formel Ia, Ib, Ic oder Id gemäß Anspruch 1, in der $R^2$ eine Gruppe $CO_2H$ oder $CO_2R^5$ und $R^5$ $C_1$-$C_4$-Alkyl bedeuten, in an sich bekannter Weise mit einem Aminoalkohol XV

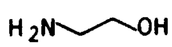

XV

cyclisiert.

**12.** Verfahren zur Herstellung der Verbindungen Ia, Ib, Ic und Id gemäß Anspruch 1, in denen $R^2$ Formyl bedeutet, dadurch gekennzeichnet, daß man eine entsprechende Carbonsäure der Formel Ia, Ib, Ic oder Id ($R^2 = CO_2H$) in das Halogenid oder eine andere aktivierte Form der Carbonsäure überführt und dieses Derivat anschließend in an sich bekannter Weise reduziert.

**13.** Verfahren zur Herstellung der Verbindungen Ia, Ib, Ic und Id gemäß Anspruch 1, in denen $R^1$ bzw. $R^{1'}$ eine epoxidierte $C_2$-$C_6$-Alkenylgruppe bedeutet, dadurch gekennzeichnet, daß man ein Carbonsäureamid der Formel Ia, Ib, Ic oder Id gemäß Anspruch 1, wobei $R^1$ bzw. $R^{1'}$ eine $C_2$-$C_6$-Alkenylgruppe bedeutet, in an sich bekannter Weise mit einem Oxidationsmittel epoxidiert.

**14.** Verfahren zur Bekämpfung unerwünschten Pflanzenwuchses, dadurch gekennzeichnet, daß man eine herbizid wirksame Menge eines Carbonsäureamids der Formel Id gemäß Anspruch 1 oder eines Carbonsäureamids der Formel Ia', Ib' oder Ic', wobei Ia', Ib' und Ic' die Bedeutung von Ia, Ib bzw. Ic ohne die Ausnahmebestimmung hat, auf Pflanzen, deren Lebensraum oder auf Saatgut einwirken läßt.

## Claims

### Claims for the following Contracting States : BE, CH, DE, FR, GB, IT, LI, NL, SE

**1.** A carboxamide of the formula Ia, Ib, Ic or Id

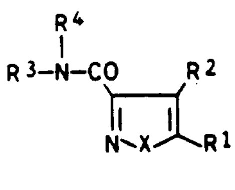
Ia

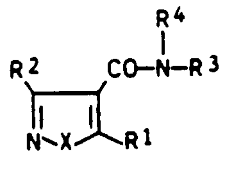
Ib

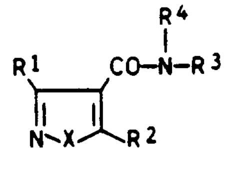
Ic

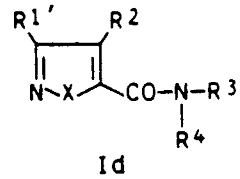
Id

where

X is oxygen or sulfur;

$R^1$ is

- hydrogen, halogen, $C_1$-$C_6$-alkyl which may carry from one to five halogen atoms and/or a cyano radical and/or up to two of the following radicals: $C_1$-$C_4$-alkoxy, partially or fully halogenated $C_1$-$C_4$-alkoxy, $C_1$-$C_4$-alkylthio or partially or fully halogenated $C_1$-$C_4$-alkylthio;
- $C_1$-$C_4$-alkoxy or $C_1$-$C_4$-alkylthio, partially or fully halogenated $C_1$-$C_4$-alkoxy, partially or fully halogenated $C_1$-$C_4$-alkylthio;
- benzyl which may be monosubstituted, disubstituted, or trisubstituted by $C_1$-$C_4$-alkyl, partially or fully halogenated $C_1$-$C_4$-alkyl, $C_1$-$C_4$-alkoxy, partially or fully halogenated $C_1$-$C_4$-alkoxy, $C_1$-$C_4$-alkylthio, partially or fully halogenated $C_1$-$C_4$-alkylthio, halogen, cyano or nitro;
- phenyl which may also carry from one to three of the following radicals: cyano, nitro, halogen, $C_1$-$C_6$-alkyl, partially or fully halogenated $C_1$-$C_6$-alkyl, $C_1$-$C_6$-alkoxy, partially or fully halogenated $C_1$-$C_6$-alkoxy $C_1$-$C_6$-alkylthio and/or partially or fully halogenated $C_1$-$C_6$-alkylthio;
- phenoxy or phenylthio, each of which may be monosubstituted, disubstituted or trisubstituted by $C_1$-$C_4$-alkyl, partially or fully halogenated $C_1$-$C_4$-alkyl, $C_1$-$C_4$-alkoxy, partially or fully halogenated $C_1$-$C_4$-alkoxy, $C_1$-$C_4$-alkylthio, partially or fully halogenated $C_1$-$C_4$-alkylthio, halogen, cyano or nitro;
- a 5- to 6-membered saturated or aromatic heterocyclic radical containing one or two heteroatoms selected from the group comprising oxygen, sulfur and nitrogen which may carry one or two of the following substituents: halogen, $C_1$-$C_3$-alkyl, $C_1$-$C_3$-alkoxy and $C_1$-$C_3$-alkoxycarbonyl;
- $C_3$-$C_8$-cycloalkyl-substituted $C_1$-$C_6$-alkyl;
- $C_2$-$C_6$-alkenyl whose double bond may be epoxidized, or $C_2$-$C_6$-alkynyl, it being possible for both groups to be monosubstituted, disubstituted or trisubstituted by halogen, $C_1$-$C_3$-alkoxy and/or monosubstituted by cyclopropyl or phenyl, it also being possible for the phenyl radical to carry up to three of the following substituents: halogen, cyano, nitro, $C_1$-$C_4$-alkyl, which may be unsubstituted or partially or fully halogenated, $C_1$-$C_4$-alkoxy or $C_1$-$C_4$-alkylthio, both of which may be unsubstituted or partially or fully halogenated;
- $C_3$-$C_8$-cycloalkyl or $C_3$-$C_6$-cycloalkenyl, it being possible for both groups to be monosubstituted, disubstituted or trisubstituted by $C_1$-$C_4$-alkyl or halogen;

$R^{1'}$ is

- $C_3$-$C_8$-cycloalkyl-substituted $C_1$-$C_6$-alkyl;
- $C_2$-$C_6$-alkenyl whose double bond may be epoxidized, or $C_2$-$C_6$-alkynyl, it being possible for both groups to be monosubstituted, disubstituted or trisubstituted by halogen, $C_1$-$C_3$-alkoxy and/or monosubstituted by cyclopropyl or phenyl, it also being possible for the phenyl radical to carry up to three of the following susbtituents: halogen, cyano, nitro, $C_1$-$C_4$-alkyl, which may be unsubstituted or partially or fully halogenated, $C_1$-$C_4$-alkoxy or $C_1$-$C_4$-alkylthio, both of which may be unsubstituted or partially or fully halogenated;

- $C_3$-$C_6$-cycloalkenyl, which may be monosubstituted, disubstituted or trisubstituted by halogen or $C_1$-$C_4$-alkyl;

$R^2$ is

- formyl, 4,5-dihydrooxazol-2-yl,
- $COYR^5$ or $CONR^6R^7$, where

Y is oxygen or sulfur;

$R^5$ is

- hydrogen;
- $C_1$-$C_6$-alkyl which may carry from one to five halogen atoms and/or up to three hydroxyl and/or $C_1$-$C_4$-alkoxy groups and/or one of the following radicals:
- cyano,
- $C_1$-$C_4$-alkoxy-$C_2$-$C_4$-alkoxy,
- $C_1$-$C_3$-alkylthio,
- $C_1$-$C_3$-alkylamino, di($C_1$-$C_3$)-alkylamino, $C_3$-$C_6$-cycloalkylamino or di($C_3$-$C_6$)-cycloalkylamino,
- trimethylsilyl,
- $C_1$-$C_3$-alkylsulphinyl or $C_1$-$C_3$-alkylsulfonyl,
- carboxyl, $C_1$-$C_3$-alkoxycarbonyl, $C_1$-$C_3$-alkoxycarbonyl-$C_1$-$C_3$-alkoxy or $C_1$-$C_3$-alkoxycarbonyl-$C_1$-$C_3$-alkoxy-$C_1$-$C_3$-alkoxycarbonyl,
- di($C_1$-$C_3$)-alkylaminocarbonyl,
- di($C_1$-$C_3$)-alkoxyphosphonyl,
- $C_1$-$C_6$-alkaneiminoxy or $C_5$-$C_6$-cycloalkaneiminoxy,
- N-phthalimido, N-succinimido, benzyloxy, benzoyl, it being possible for these cyclic radicals to additionally carry from one to three of the following groups: halogen, $C_1$-$C_3$-alkyl or $C_1$-$C_3$-alkoxy,
- a 5- or 6-membered saturated heterocyclic radical or a 5- or 6-membered heteroaromatic radical, each of which has up to three heteroatoms selected from the group comprising oxygen, sulfur and nitrogen, where two oxygen and/or sulfur atoms must not be directly adjacent and where the heterocyclic rings may also carry one or two of the following substituents: halogen, $C_1$-$C_3$-alkyl, $C_1$-$C_3$-alkoxy or $C_1$-$C_3$-alkoxycarbonyl;
- phenyl, which may also carry up to three of the following substituents: halogen, nitro, cyano, $C_1$-$C_3$-alkyl, partially or fully halogenated $C_1$-$C_3$-alkyl, $C_1$-$C_3$-alkoxy or partially or fully halogenated $C_1$-$C_3$-alkoxy;
- $-CR^{10}=N-R^{11}$, where

$R^{10}$ is hydrogen or $C_1$-$C_6$-alkyl and

$R^{11}$ is $C_1$-$C_6$-alkoxy, $C_3$-$C_6$-alkenyloxy or $C_3$-$C_6$-alkynyloxy, each of which may carry up to 3 halogen atoms and/or a phenyl radical with, if desired, up to three of the following radicals: halogen, nitro, cyano, $C_1$-$C_3$-alkyl or $C_1$-$C_3$-alkoxy; phenoxy, which may also carry up to three of the following substituents: halogen, nitro, cyano, $C_1$-$C_3$-alkyl or $C_1$-$C_3$-alkoxy; $C_1$-$C_6$-alkylamino, di($C_1$-$C_6$)alkylamino or phenylamino, it being possible for the aromatic ring to additionally carry up to three of the following radicals: halogen, nitro, cyano, $C_1$-$C_3$-alkyl or $C_1$-$C_3$-alkoxy;

- $C_3$-$C_8$-cycloalkyl;
- $C_3$-$C_6$-alkenyl, $C_5$-$C_6$-cycloalkenyl, $C_3$-$C_6$-alkynyl, it being possible for these radicals to carry one of the following groups: hydroxyl, halogen, $C_1$-$C_4$-alkoxy or phenyl, it being possible for the aromatic radical to itself carry from one to three of the following groups: halogen, nitro, cyano, $C_1$-$C_4$-alkyl, partially or fully halogenated $C_1$-$C_4$-alkyl or $C_1$-$C_4$-alkoxy;
- phenyl, which may carry from one to three of the following groups: halogen, nitro, cyano, $C_1$-$C_4$-alkyl, partially or fully halogenated $C_1$-$C_4$-alkyl, $C_1$-$C_4$-alkoxy, partially or fully halogenated $C_1$-$C_4$-alkoxy or $C_1$-$C_4$-alkoxycarbonyl;
- a five- or six-membered heterocyclic radical having up to three heteroatoms selected from the group comprising oxygen, sulfur and nitrogen, where two oxygen and/or sulfur atoms must not be directly adjacent and where the heterocyclic rings may also carry one or two of the following substituents: halogen, $C_1$-$C_3$-alkoxy or $C_1$-$C_3$-alkoxycarbonyl;
- benzotriazolyl;

- N-phthalimido, tetrahydrophthalimido, succinimido, maleiimido;
- 2,2-dimethyl-1,3-dioxolan-4-ylmethyl or 1,3-dioxolan-2-on-4-ylmethyl;
- in the case where Y = 0: one equivalent of a cation from the group comprising the alkali and alkaline earth metals, manganese, copper, iron, ammonium and ammonium which is substituted by up to 4 $C_1$-$C_3$-alkyl groups; or
- $-N=CR^8R^9$ where

$R^8$ and $R^9$ are hydrogen; $C_1$-$C_4$-alkyl, which may be unsubstituted or partially or fully halogenated and may carry a $C_1$-$C_3$-alkoxy or phenyl radical, it being possible for the aromatic radical to itself also be monosubstituted, disubstituted or trisubstituted by halogen, nitro, cyano, $C_1$-$C_3$-alkyl, partially or fully halogenated $C_1$-$C_3$-alkyl, $C_1$-$C_3$-alkoxy or partially or fully halogenated $C_1$-$C_3$-alkoxy; $C_3$-$C_6$-cycloalkyl; $C_1$-$C_4$-alkoxy; furanyl or phenyl, which may additionally carry up to three of the following substituents: halogen, nitro, cyano, $C_1$-$C_3$-alkyl, partially or fully halogenated $C_1$-$C_3$-alkyl, $C_1$-$C_3$-alkoxy or partially or fully halogenated $C_1$-$C_3$-alkoxy; or $R^8$ and $R^9$ together are a methylene chain having from 4 to 7 members;

- -W-Z, where W is $C_2$-$C_4$-alkylene, ethoxyethylene, but-2-enylene or but-2-ynylene, and Z is a molecular moiety which is bonded to W in the $\omega$-position and is the same as that linked to W in the $\alpha$-position of W;

$R^6$ is

- hydrogen, $C_1$-$C_6$-alkyl or $C_3$-$C_8$-cycloalkyl, and

$R^7$ is

- hydrogen, $C_1$-$C_6$-alkyl, $-C(OR^{12})=N-H$ or $-C(OR^{12})=N-(C_1-C_4)$-alkyl, where $R^{12}$ is $C_1$-$C_4$-alkyl, or

$R^6$ and $R^7$ together are methylene having 4 or 5 members;

$R^3$ is

- hydrogen;
- $C_1$-$C_6$-alkyl, which may carry from one to three of the following substituents: hydroxyl, halogen, $C_1$-$C_4$-alkoxy, $C_1$-$C_4$-alkylthio or di($C_1$-$C_4$)-alkylamino;
- $C_3$-$C_8$-cycloalkyl, which may be monosubstituted, disubstituted or trisubstituted by halogen, $C_1$-$C_4$-alkyl or partially or fully halogenated $C_1$-$C_4$-alkyl;

$R^4$ is

- hydrogen, hydroxyl, $C_1$-$C_4$-alkoxy;
- $C_1$-$C_6$-alkyl, which may carry from one to three of the following radicals: halogen, cyano, $C_1$-$C_4$-alkoxy, partially or fully halogenated $C_1$-$C_4$-alkoxy, $C_1$-$C_4$-alkylthio, partially or fully halogenated $C_1$-$C_4$-alkylthio, di-$C_1$-$C_4$-alkylamino, $C_3$-$C_8$-cycloalkyl or phenyl, it being possible for the phenyl ring to itself carry from one to three of the following radicals: halogen, cyano, nitro, $C_1$-$C_4$-alkyl, partially or fully halogenated $C_1$-$C_4$-alkyl, $C_1$-$C_4$-alkoxy, partially or fully halogenated $C_1$-$C_4$-alkoxy, $C_1$-$C_4$-alkylthio or partially or fully halogenated $C_1$-$C_4$-alkylthio;
- $C_3$-$C_8$-cycloalkyl, which may carry from one to three of the following radicals: halogen, nitro, cyano, $C_1$-$C_6$-alkyl, partially or fully halogenated $C_1$-$C_6$-alkyl, $C_1$-$C_4$-alkoxy or partially or fully halogenated $C_1$-$C_4$-alkoxy;
- $C_3$-$C_6$-alkenyl or $C_3$-$C_6$-alkynyl, each of which may be monosubstituted, disubstituted or trisubstituted by halogen and/or monosubstituted by phenyl, it being possible for the phenyl radical to itself carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
- di($C_1$-$C_4$)-alkylamino;
- a 5- or 6-membered heterocyclic saturated or aromatic radical having one or two heteroatoms selected from the group comprising oxygen, sulfur and nitrogen, which may be monosubstituted, disubstituted or trisubstituted by $C_1$-$C_4$-alkyl or halogen;
- phenyl, which may carry from one to four of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, nitro, cyano, formyl, $C_1$-$C_4$-alkanoyl, $C_1$-$C_4$-haloalkanoyl or $C_1$-$C_4$-alkoxycarbonyl;

- naphthyl, which may be monosubstituted, disubstituted or trisubstituted by $C_1$-$C_4$-alkyl or halogen;

or

$R^3$ and $R^4$ together are methylene having from 4 to 7 members which may be interrupted by oxygen, sulfur or N-methyl, or are $-(CH_2)_3$-CO-,

$R^3$ and $R^4$ in the compounds Ia to Ic not simultaneously being hydrogen if

- $R^1$ is hydrogen, methyl or phenyl and $R^2$ is $CONH_2$, $CO_2H$ or $CO_2CH_3$, or if
- X is oxygen, $R^1$ is $CH(OCH_2CH_3)_2$ and $R^2$ is $CONH_2$,

and the environmentally acceptable salts thereof.

**2.** A carboxamide of the formula Ia, Ib, Ic or Id as claimed in claim 1, where $R^3$ is hydrogen.

**3.** A carboxamide of the formula Ia, Ib or Ic as claimed in claim 1, where

$R^1$ is hydrogen, $C_1$-$C_4$-alkyl or $C_3$-$C_6$-cycloalkyl;

$R^2$ is $COYR^5$, where Y is oxygen and $R^5$ is hydrogen or $-N=CR^8R^9$, where $R^8$ and $R^9$ are each independently of the other hydrogen, $C_1$-$C_4$-alkyl or $C_3$-$C_6$-cycloalkyl, or together are $C_4$-$C_7$-alkylene;

$R^3$ is hydrogen and

$R^4$ is $C_1$-$C_4$-alkyl or $C_3$-$C_6$-cycloalkyl.

**4.** A carboxamide of the formula Ia as claimed in any of claims 1 to 3.

**5.** A process for the preparation of a compound Ia or Ib as claimed in claim 1 in which $R^2$ is $CO_2CH_3$ and X is oxygen, which comprises reacting the hydroxamyl chloride of the formula II

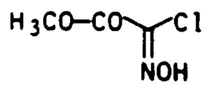

II

in a conventional manner with a $\beta$-keto ester of the formula III

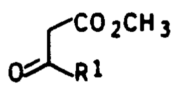

III

in the presence of a base, subsequently first hydrolyzing the resultant dimethyl diester IV

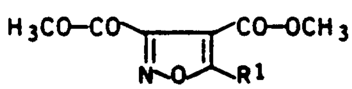

IV

using one equivalent of an aqueous base to give the monoester Va or Vb

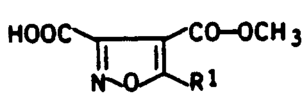

Va

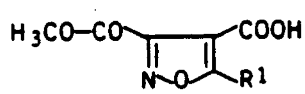

Vb

and then converting Va or Vb, separately or as a mixture, first into the halide or another activated form of the carboxylic acid in a conventional manner and subsequently amidating this derivative using a substituted amine of the formula VIa

$HNR^3R^4$ VIa.

**6.** A process for the preparation of a compound Ia as claimed in claim 1 in which $R^2$ is $CO_2R_5$, X is oxygen and $R^5$ is not hydrogen or methyl, which comprises reacting the hydroxamyl chloride II in a conventional manner with a $\beta$-keto ester of the formula IIIa

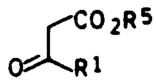

IIIa

in the presence of a base, subsequently hydrolyzing the resultant diester IVa

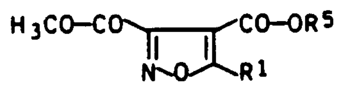

IVa ,

using a hydrolyzing agent to give the monoester Va'

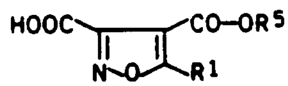

Va',

converting Va' into the halide or another activated form of the carboxylic acid in a conventional manner, and subsequently amidating this derivative using a substituted amine VIa.

**7.** A process for the preparation of a compound Ia or Ic as claimed in claim 1 in which $R^1$ is not hydrogen, and $R^2$ is carboxyl or formyl and $R^3$ is hydrogen, which comprises converting a carboxylic acid Vc or Vd

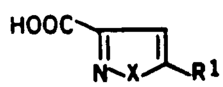

Vc

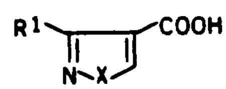

Vd

into the halide or another activated form of the carboxylic acid in a conventional manner, amidating this derivative using a substituted amine VIa and subsequently reacting the resultant amide VIIa or VIIb

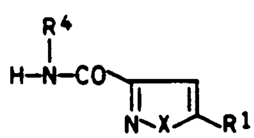

VIIa

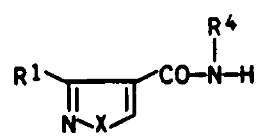

VIIb

in a conventional manner with a carboxylating or formylating agent in the presence of a base.

**8.** A process for the preparation of compounds Ia and Ib as claimed in claim 1 in which $R^2$ is carboxyl and X is sulfur, which comprises reacting an isothiazoledicarboxylic anhydride VIII

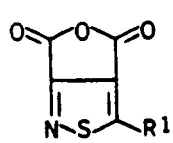

VIII

in a conventional manner with an amine VIa to give the isomers Ia and Ib, and subsequently separating the isomers.

9. A process for the preparation of a compound Ia, Ib, Ic or Id as claimed in claim 1 in which $R^2$ is $CO_2H$, which comprises hydrolyzing the corresponding ester Ia, Ib, Ic or Id in which $R^2$ is $CO_2R^5$ and $R^5$ is $C_1$-$C_4$-alkyl in a conventional manner in the presence of an aqueous base.

10. A process for the preparation of a compound Ia, Ib, Ic or Id as claimed in claim 1 in which $R^2$ is $COYR^5$ or $CONR^6R^7$, which comprises first converting the corresponding carboxylic acid 1a, 1b, 1c or Id ($R^2 = CO_2H$) into the halide or another activated derivative of the carboxylic acid in a conventional manner, and subsequently derivatizing this derivative in a conventional manner with a compound IX

$HYR^5$ IX

or with an amine VIb

$HNR^6R^7$ VIb.

11. A process for the preparation of a compound Ia, Ib, Ic or Id as claimed in claim 1 in which $R^2$ is 4,5-dihydrooxazol-2-yl, which comprises cyclizing a carboxamide of the formula Ia, Ib, Ic or Id as claimed in claim 1, in which $R^2$ is $CO_2H$ or $CO_2R^5$ and $R^5$ is $C_1$-$C_4$-alkyl, in a conventional manner with an aminoalcohol XV

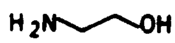

XV.

12. A process for the preparation of a compound Ia, Ib, Ic or Id as claimed in claim 1 in which $R^2$ is formyl, which comprises converting the corresponding carboxylic acid of the formula Ia, Ib, Ic or Id ($R^2$ = $CO_2H$) into the halide or another activated form of the carboxylic acid, and thus reducing this derivative in a conventional manner.

13. A process for the preparation of a compound Ia, Ib, Ic or Id as claimed in claim 1, in which $R^1$ or $R^{1'}$ is epoxidized $C_2$-$C_6$-alkenyl, which comprises epoxidizing a carboxamide of the formula Ia, Ib, Ic or Id as claimed in claim 1, where $R^1$ or $R^{1'}$ is $C_2$-$C_6$-alkenyl, in a conventional manner using an oxidant.

14. An agent containing inert carriers and a herbicidally effective amount of at least one carboxamide of the formula Ia, Ib, Ic or Id as claimed in any of claims 1 to 4.

15. A method of controlling undesirable plant growth, which comprises allowing a herbicidally effective amount of a carboxamide of the formula Id as claimed in claim 1 or of a carboxamide of the formula Ia', Ib' or Ic', where Ia', Ib' and Ic' have the meanings of .Ia, Ib and Ic without the disclaimer, to act on plants or their habitat, or on seed.

**Claims for the following Contracting State : ES**

1. An agent containing inert carriers and a herbicidally effective amount of at least one carboxamide of the formula Ia, Ib, Ic or Id

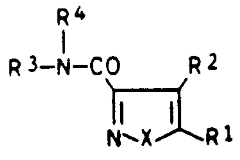

Ia

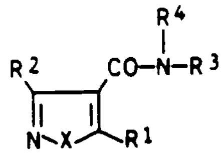

Ib

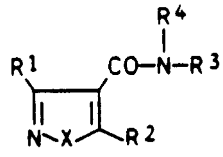

Ic

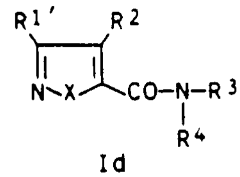

Id

where

X is oxygen or sulfur;

$R^1$ is

- hydrogen, halogen, $C_1$-$C_6$-alkyl which may carry from one to five halogen atoms and/or a cyano radical and/or up to two of the following radicals: $C_1$-$C_4$-alkoxy, partially or fully halogenated $C_1$-$C_4$-alkoxy, $C_1$-$C_4$-alkylthio or partially or fully halogenated $C_1$-$C_4$-alkylthio;
- $C_1$-$C_4$-alkoxy or $C_1$-$C_4$-alkylthio, partially or fully halogenated $C_1$-$C_4$-alkoxy, partially or fully halogenated $C_1$-$C_4$-alkylthio;
- benzyl which may be monosubstituted, disubstituted, or trisubstituted by $C_1$-$C_4$-alkyl, partially or fully halogenated $C_1$-$C_4$-alkyl, $C_1$-$C_4$-alkoxy, partially or fully halogenated $C_1$-$C_4$-alkoxy, $C_1$-$C_4$-alkylthio, partially or fully halogenated $C_1$-$C_4$-alkylthio, halogen, cyano or nitro;
- phenyl which may also carry from one to three of the following radicals: cyano, nitro, halogen, $C_1$-$C_6$-alkyl, partially or fully halogenated $C_1$-$C_6$-alkyl, $C_1$-$C_6$-alkoxy, partially or fully halogenated $C_1$-$C_6$-alkoxy $C_1$-$C_6$-alkylthio and/or partially or fully halogenated $C_1$-$C_6$-alkylthio;
- phenoxy or phenylthio, each of which may be monosubstituted, disubstituted or trisubstituted by $C_1$-$C_4$-alkyl, partially or fully halogenated $C_1$-$C_4$-alkyl, $C_1$-$C_4$-alkoxy, partially or fully halogenated $C_1$-$C_4$-alkoxy, $C_1$-$C_4$-alkylthio, partially or fully halogenated $C_1$-$C_4$-alkylthio, halogen, cyano or nitro;
- a 5- to 6-membered saturated or aromatic heterocyclic radical containing one or two heteroatoms selected from the group comprising oxygen, sulfur and nitrogen which may carry one or two of the following substituents: halogen, $C_1$-$C_3$-alkyl, $C_1$-$C_3$-alkoxy and $C_1$-$C_3$-alkoxycarbonyl;
- $C_3$-$C_8$-cycloalkyl-substituted $C_1$-$C_6$-alkyl;
- $C_2$-$C_6$-alkenyl whose double bond may be epoxidized, or $C_2$-$C_6$-alkynyl, it being possible for both groups to be monosubstituted, disubstituted or trisubstituted by halogen, $C_1$-$C_3$-alkoxy and/or monosubstituted by cyclopropyl or phenyl, it also being possible for the phenyl radical to carry up to three of the following substituents: halogen, cyano, nitro, $C_1$-$C_4$-alkyl, which may be unsubstituted or partially or fully halogenated, $C_1$-$C_4$-alkoxy or $C_1$-$C_4$-alkylthio, both of which may be unsubstituted or partially or fully halogenated;
- $C_3$-$C_8$-cycloalkyl or $C_3$-$C_6$-cycloalkenyl, it being possible for both groups to be monosubstituted, disubstituted or trisubstituted by $C_1$-$C_4$-alkyl or halogen;

$R^{1'}$ is

- $C_3$-$C_8$-cycloalkyl-substituted $C_1$-$C_6$-alkyl;
- $C_2$-$C_6$-alkenyl whose double bond may be epoxidized, or $C_2$-$C_6$-alkynyl, it being possible for both groups to be monosubstituted, disubstituted or trisubstituted by halogen, $C_1$-$C_3$-alkoxy and/or monosubstituted by cyclopropyl or phenyl, it also being possible for the phenyl radical to carry up to three of the following susbtituents: halogen, cyano, nitro, $C_1$-$C_4$-alkyl, which may be unsubstituted or partially or fully halogenated, $C_1$-$C_4$-alkoxy or $C_1$-$C_4$-alkylthio, both of which may be unsubstituted or partially or fully halogenated;
- $C_3$-$C_6$-cycloalkenyl, which may be monosubstituted, disubstituted or trisubstituted by halogen or $C_1$-$C_4$-alkyl;

$R^2$ is

- formyl, 4,5-dihydrooxazol-2-yl,

- $COYR^5$ or $CONR^6R^7$, where

Y is oxygen or sulfur;

$R^5$ is

- hydrogen;
- $C_1$-$C_6$-alkyl which may carry from one to five halogen atoms and/or up to three hydroxyl and/or $C_1$-$C_4$-alkoxy groups and/or one of the following radicals:
- cyano,
- $C_1$-$C_4$-alkoxy-$C_2$-$C_4$-alkoxy,
- $C_1$-$C_3$-alkylthio,
- $C_1$-$C_3$-alkylamino, di($C_1$-$C_3$)-alkylamino, $C_3$-$C_6$-cycloalkylamino or di($C_3$-$C_6$)-cycloalkylamino,
- trimethylsilyl,
- $C_1$-$C_3$-alkylsulphinyl or $C_1$-$C_3$-alkylsulfonyl,
- carboxyl, $C_1$-$C_3$-alkoxycarbonyl, $C_1$-$C_3$-alkoxycarbonyl-$C_1$-$C_3$-alkoxy or $C_1$-$C_3$-alkoxycarbonyl-$C_1$-$C_3$-alkoxy$C_1$-$C_3$-alkoxycarbonyl,
- di($C_1$-$C_3$)-alkylaminocarbonyl,
- di($C_1$-$C_3$)-alkoxyphosphonyl,
- $C_1$-$C_6$-alkaneiminoxy or $C_5$-$C_6$-cycloalkaneiminoxy,
- N-phthalimido, N-succinimido, benzyloxy, benzoyl, it being possible for these cyclic radicals to additionally carry from one to three of the following groups: halogen, $C_1$-$C_3$-alkyl or $C_1$-$C_3$-alkoxy,
- a 5- or 6-membered saturated heterocyclic radical or a 5- or 6-membered heteroaromatic radical, each of which has up to three heteroatoms selected from the group comprising oxygen, sulfur and nitrogen, where two oxygen and/or sulfur atoms must not be directly adjacent and where the heterocyclic rings may also carry one or two of the following substituents: halogen, $C_1$-$C_3$-alkyl, $C_1$-$C_3$-alkoxy or $C_1$-$C_3$-alkoxycarbonyl;
- phenyl, which may also carry up to three of the following substituents: halogen, nitro, cyano, $C_1$-$C_3$-alkyl, partially or fully halogenated $C_1$-$C_3$-alkyl, $C_1$-$C_3$-alkoxy or partially or fully halogenated $C_1$-$C_3$-alkoxy;
- $-CR^{10}=N-R^{11}$, where

$R^{10}$ is hydrogen or $C_1$-$C_6$-alkyl and

$R^{11}$ is $C_1$-$C_6$-alkoxy, $C_3$-$C_6$-alkenyloxy or $C_3$-$C_6$-alkynyloxy, each of which may carry up to 3 halogen atoms and/or a phenyl radical with, if desired, up to three of the following radicals: halogen, nitro, cyano, $C_1$-$C_3$-alkyl or $C_1$-$C_3$-alkoxy; phenoxy, which may also carry up to three of the following substituents: halogen, nitro, cyano, $C_1$-$C_3$-alkyl or $C_1$-$C_3$-alkoxy; $C_1$-$C_6$-alkylamino, di($C_1$-$C_6$)alkylamino or phenylamino, it being possible for the aromatic ring to additionally carry up to three of the following radicals: halogen, nitro, cyano, $C_1$-$C_3$-alkyl or $C_1$-$C_3$-alkoxy;

- $C_3$-$C_8$-cycloalkyl;
- $C_3$-$C_6$-alkenyl, $C_5$-$C_6$-cycloalkenyl, $C_3$-$C_6$-alkynyl, it being possible for these radicals to carry one of the following groups: hydroxyl, halogen, $C_1$-$C_4$-alkoxy or phenyl, it being possible for the aromatic radical to itself carry from one to three of the following groups: halogen, nitro, cyano, $C_1$-$C_4$-alkyl, partially or fully halogenated $C_1$-$C_4$-alkyl or $C_1$-$C_4$-alkoxy;
- phenyl, which may carry from one to three of the following groups: halogen, nitro, cyano, $C_1$-$C_4$-alkyl, partially or fully halogenated $C_1$-$C_4$-alkyl, $C_1$-$C_4$-alkoxy, partially or fully halogenated $C_1$-$C_4$-alkoxy or $C_1$-$C_4$-alkoxycarbonyl;
- a five- or six-membered heterocyclic radical having up to three heteroatoms selected from the group comprising oxygen, sulfur and nitrogen, where two oxygen and/or sulfur atoms must not be directly adjacent and where the heterocyclic rings may also carry one or two of the following substituents: halogen, $C_1$-$C_3$-alkoxy or $C_1$-$C_3$-alkoxycarbonyl;
- benzotriazolyl;
- N-phthalimido, tetrahydrophthalimido, succinimido, maleiimido;
- 2,2-dimethyl-1,3-dioxolan-4-ylmethyl or 1,3-dioxolan-2-on-4-ylmethyl;
- in the case where Y = 0: one equivalent of a cation from the group comprising the alkali and alkaline earth metals, manganese, copper, iron, ammonium and

ammonium which is substituted by up to 4 $C_1$-$C_3$-alkyl groups; or

- $-N=CR^8R^9$ where

$R^8$ and $R^9$ are hydrogen; $C_1$-$C_4$-alkyl, which may be unsubstituted or partially or fully halogenated and may carry a $C_1$-$C_3$-alkoxy or phenyl radical, it being possible for the aromatic radical to itself also be monosubstituted, disubstituted or trisubstituted by halogen, nitro, cyano, $C_1$-$C_3$-alkyl, partially or fully halogenated $C_1$-$C_3$-alkyl, $C_1$-$C_3$-alkoxy or partially or fully halogenated $C_1$-$C_3$-alkoxy; $C_3$-$C_6$-cycloalkyl; $C_1$-$C_4$-alkoxy; furanyl or phenyl, which may additionally carry up to three of the following substituents: halogen, nitro, cyano, $C_1$-$C_3$-alkyl, partially or fully halogenated $C_1$-$C_3$-alkyl, $C_1$-$C_3$-alkoxy or partially or fully halogenated $C_1$-$C_3$-alkoxy; or $R^8$ and $R^9$ together are a methylene chain having from 4 to 7 members;

- -W-Z, where W is $C_2$-$C_4$-alkylene, ethoxyethylene, but-2-enylene or but-2-ynylene, and Z is a molecular moiety which is bonded to W in the $\omega$-position and is the same as that linked to W in the $\alpha$-position of W;

$R^6$ is

- hydrogen, $C_1$-$C_6$-alkyl or $C_3$-$C_8$-cycloalkyl, and

$R^7$ is

- hydrogen, $C_1$-$C_6$-alkyl, $-C(OR^{12})=N-H$ or $-C(OR^{12})=N-(C_1-C_4)$-alkyl, where $R^{12}$ is $C_1$-$C_4$-alkyl, or

$R^6$ and $R^7$ together are methylene having 4 or 5 members;

$R^3$ is

- hydrogen;
- $C_1$-$C_6$-alkyl, which may carry from one to three of the following substituents: hydroxyl, halogen, $C_1$-$C_4$-alkoxy, $C_1$-$C_4$-alkylthio or di($C_1$-$C_4$)-alkylamino;
- $C_3$-$C_8$-cycloalkyl, which may be monosubstituted, disubstituted or trisubstituted by halogen, $C_1$-$C_4$-alkyl or partially or fully halogenated $C_1$-$C_4$-alkyl;

$R^4$ is

- hydrogen, hydroxyl, $C_1$-$C_4$-alkoxy;
- $C_1$-$C_6$-alkyl, which may carry from one to three of the following radicals: halogen, cyano, $C_1$-$C_4$-alkoxy, partially or fully halogenated $C_1$-$C_4$-alkoxy, $C_1$-$C_4$-alkylthio, partially or fully halogenated $C_1$-$C_4$-alkylthio, di-$C_1$-$C_4$-alkylamino, $C_3$-$C_8$-cycloalkyl or phenyl, it being possible for the phenyl ring to itself carry from one to three of the following radicals: halogen, cyano, nitro, $C_1$-$C_4$-alkyl, partially or fully halogenated $C_1$-$C_4$-alkyl, $C_1$-$C_4$-alkoxy, partially or fully halogenated $C_1$-$C_4$-alkoxy, $C_1$-$C_4$-alkylthio or partially or fully halogenated $C_1$-$C_4$-alkylthio;
- $C_3$-$C_8$-cycloalkyl, which may carry from one to three of the following radicals: halogen, nitro, cyano, $C_1$-$C_6$-alkyl, partially or fully halogenated $C_1$-$C_6$-alkyl, $C_1$-$C_4$-alkoxy or partially or fully halogenated $C_1$-$C_4$-alkoxy;
- $C_3$-$C_6$-alkenyl or $C_3$-$C_6$-alkynyl, each of which may be monosubstituted, disubstituted or trisubstituted by halogen and/or monosubstituted by phenyl, it being possible for the phenyl radical to itself carry from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;
- di($C_1$-$C_4$)-alkylamino;
- a 5- or 6-membered heterocyclic saturated or aromatic radical having one or two heteroatoms selected from the group comprising oxygen, sulfur and nitrogen, which may be monosubstituted, disubstituted or trisubstituted by $C_1$-$C_4$-alkyl or halogen;
- phenyl, which may carry from one to four of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, nitro, cyano, formyl, $C_1$-$C_4$-alkanoyl, $C_1$-$C_4$-haloalkanoyl or $C_1$-$C_4$-alkoxycarbonyl;
- naphthyl, which may be monosubstituted, disubstituted or trisubstituted by $C_1$-$C_4$-alkyl or halogen;

or

$R^3$ and $R^4$ together are methylene having from 4 to 7 members which may be interrupted by oxygen, sulfur or N-methyl, or are $-(CH_2)_3-CO-$,

$R^3$ and $R^4$ in the compounds Ia to Ic not simultaneously being hydrogen if
- $R^1$ is hydrogen, methyl or phenyl and $R^2$ is $CONH_2$, $CO_2H$ or $CO_2CH_3$, or if
- X is oxygen, $R^1$ is $CH(OCH_2CH_3)_2$ and $R^2$ is $CONH_2$,

and the environmentally acceptable salts thereof.

**2.** An agent containing inert carriers and a herbicidally effective amount of at least one carboxamide of the formula Ia, Ib, Ic or Id as claimed in claim 1, where $R^3$ is hydrogen.

**3.** An agent containing inert carriers and a herbicidally effective amount of at least one carboxamide of the formula Ia, Ib or Ic as claimed in claim 1, where
- $R^1$ is hydrogen, $C_1$-$C_4$-alkyl or $C_3$-$C_6$-cycloalkyl;
- $R^2$ is $COYR^5$, where Y is oxygen and $R^5$ is hydrogen or $-N=CR^8R^9$, where $R^8$ and $R^9$ are each independently of the other hydrogen, $C_1$-$C_4$-alkyl or $C_3$-$C_6$-cycloalkyl, or together are $C_4$-$C_7$-alkylene;
- $R^3$ is hydrogen and
- $R^4$ is $C_1$-$C_4$-alkyl or $C_3$-$C_8$-cycloalkyl.

**4.** An agent containing inert carriers and a herbicidally effective amount of at least one carboxamide of the formula Ia as claimed in any of claims 1 to 3.

**5.** A process for the preparation of a compound Ia or Ib as claimed in claim 1 in which $R^2$ is $CO_2CH_3$ and X is oxygen, which comprises reacting the hydroxamyl chloride of the formula II

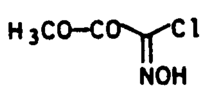

II

in a conventional manner with a β-keto ester of the formula III

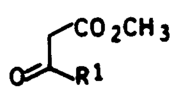

III

in the presence of a base, subsequently first hydrolyzing the resultant dimethyl diester IV

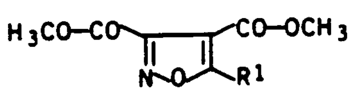

IV

using one equivalent of an aqueous base to give the monoester Va or Vb

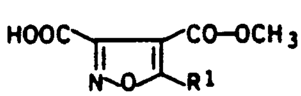

Va

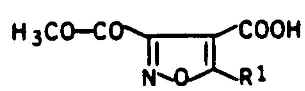

Vb

and then converting Va or Vb, separately or as a mixture, first into the halide or another activated form of the carboxylic acid in a conventional manner and subsequently amidating this derivative using a substituted amine of the formula VIa

$HNR^3R^4$ VIa.

**6.** A process for the preparation of a compound Ia as claimed in claim 1 in which $R^2$ is $CO_2R_5$, X is oxygen and $R^5$ is not hydrogen or methyl, which comprises reacting the hydroxamyl chloride II in a conventional manner with a $\beta$-keto ester of the formula IIIa

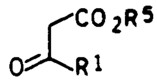

IIIa

in the presence of a base, subsequently hydrolyzing the resultant diester IVa

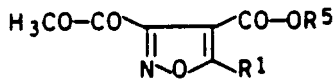

IVa

using a hydrolysing agent to give the monoester Va'

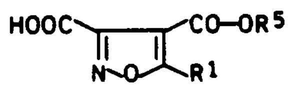

Va',

converting Va' into the halide or another activated form of the carboxylic acid in a conventional manner, and subsequently amidating this derivative using a substituted amine VIa.

**7.** A process for the preparation of a compound Ia or Ic as claimed in claim 1 in which $R^1$ is not hydrogen, and $R^2$ is carboxyl or formyl and $R^3$ is hydrogen, which comprises converting a carboxylic acid Vc or Vd

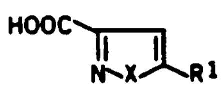

Vc

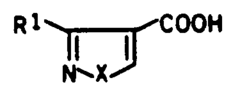

Vd

into the halide or another activated form of the carboxylic acid in a conventional manner, amidating this derivative using a substituted amine VIa and subsequently reacting the resultant amide VIIa or VIIb

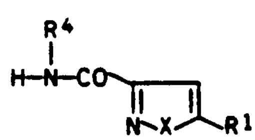

VIIa

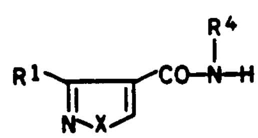

VIIb

in a conventional manner with a carboxylating or formylating agent in the presence of a base.

**8.** A process for the preparation of compounds Ia and Ib as claimed in claim 1 in which $R^2$ is carboxyl and X is sulfur, which comprises reacting an isothiazoledicarboxylic anhydride VIII

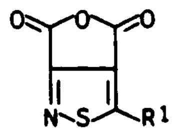

VIII

in a conventional manner with an amine VIa to give the isomers Ia and Ib, and subsequently separating the isomers.

**9.** A process for the preparation of a compound Ia, Ib, Ic or Id as claimed in claim 1 in which $R^2$ is $CO_2H$, which comprises hydrolyzing the corresponding ester Ia, Ib, Ic or Id in which $R^2$ is $CO_2R^5$ and $R^5$ is $C_1$-$C_4$-alkyl in a conventional manner in the presence of an aqueous base.

**10.** A process for the preparation of a compound Ia, Ib, Ic or Id as claimed in claim 1 in which $R^2$ is $COYR^5$ or $CONR^6R^7$, which comprises first converting the corresponding carboxylic acid 1a, 1b, 1c or Id ($R^2 = CO_2H$) into the halide or another activated derivative of the carboxylic acid in a conventional manner, and subsequently derivatizing this derivative in a conventional manner with a compound IX

$HYR^5$ IX

or with an amine VIb

$HNR^6R^7$ VIb.

**11.** A process for the preparation of a compound Ia, Ib, Ic or Id as claimed in claim 1 in which $R^2$ is 4,5-dihydrooxazol-2-yl, which comprises cyclizing a carboxamide of the formula Ia, Ib, Ic or Id as claimed in claim 1, in which $R^2$ is $CO_2H$ or $CO_2R^5$ and $R^5$ is $C_1$-$C_4$-alkyl, in a conventional manner with an aminoalcohol XV

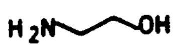

XV.

**12.** A process for the preparation of a compound Ia, Ib, Ic or Id as claimed in claim 1 in which $R^2$ is formyl, which comprises converting the corresponding carboxylic acid of the formula Ia, Ib, Ic or Id ($R^2$ = $CO_2H$) into the halide or another activated form of the carboxylic acid, and thus reducing this derivative in a conventional manner.

**13.** A process for the preparation of a compound Ia, Ib, Ic or Id as claimed in claim 1, in which $R^1$ or $R^{1'}$ is epoxidized $C_2$-$C_6$-alkenyl, which comprises epoxidizing a carboxamide of the formula Ia, Ib, Ic or Id as claimed in claim 1, where $R^1$ or $R^{1'}$ is $C_2$-$C_6$-alkenyl, in a conventional manner using an oxidant.

**14.** A method of controlling undesirable plant growth, which comprises allowing a herbicidally effective amount of a carboxamide of the formula Id as claimed in claim 1 or of a carboxamide of the formula Ia', Ib' or Ic', where Ia', Ib' and Ic' have the meanings of Ia, Ib and Ic without the disclaimer, to act on plants or their habitat, or on seed.

**Revendications**

**Revendications pour les Etats contractants suivants : BE, CH, DE, FR, GB, IT, LI, NL, SE**

**1.** Carboxamides de formules Ia, Ib, Ic et Id

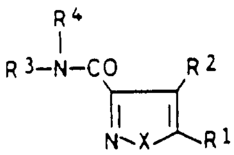

Ia

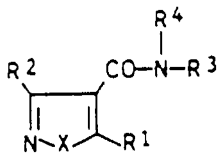

Ib

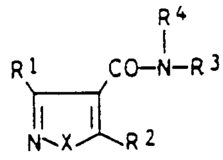

Ic

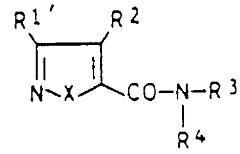

Id

dans lesquelles les symboles ont les significations suivantes :

X

- l'oxygène ou le soufre;

$R^1$

- l'hydrogène, un halogène, un groupe alkyle en C1-C6 qui peut porter un à cinq atomes d'halogènes et/ou un groupe cyano et/ou jusqu'à deux des substituants suivants : alcoxy en C1-C4, alcoxy halogéné en totalité ou en partie, alkylthio en C1-C4 ou alkylthio en C1-C4 halogéne en totalité ou en partie ;
- un groupe alcoxy en C1-C4 ou alkylthio en C1-C4, un groupe alcoxy en C1-C4 partiellement ou totalement halogéné, un groupe alkylthio en C1-C4 partiellement ou totalement halogéné ;
- le groupe benzyle qui peut être porter un à trois substituants choisis parmi les groupes alkyle en C1-C4, alkyle en C1-C4 partiellement ou totalement halogéné, alcoxy en C1-C4, alcoxy en C1-C4 partiellement ou totalement halogéne, alkylthio en C1-C4, alkylthio en C1-C4 partiellement ou totalement halogéné, les halogènes, les groupes cyano ou nitro ;
- le groupe phényle qui peut encore porter un à trois des substituants suivants : les groupes cyano, nitro, les halogènes, les groupes alkyle en C1-C6, alkyle en C1-C6 partiellement ou totalement halogéné, alcoxy en C1-C6, alcoxy en C1-C6 partiellement ou totalement halogéné, alkylthio en C1-C6 et/ou alkylthio en C1-C6 partiellement ou totalement halogéné ;
- le groupe phénoxy ou le groupe phénylthio qui peuvent tous deux porter un à trois substituants choisis parmi les groupes alkyle en C1-C4, alkyle en C1-C4 partiellement ou totalement halogénén, alcoxy en C1-C4, alcoxy en C1-C4 partiellement ou totalement halogéné, alkylthio en C1-C4, alkylthio en C1-C4 partiellement ou totalement halogéné, les halogènes, les groupes cyano ou nitro ;
- un radical hétérocyclique saturé ou aromatique de 5 à 6 chaînons contenant un ou deux hétéroatomes choisis parmi l'oxygène, le soufre et l'azote et qui peut porter un ou deux des substituants suivants : les halogènes, les groupes alkyle en C1-C3, alcoxy en C1-C3 et (alcoxy en C1-C3)-carbonyle ;
- un groupe alkyle en C1-C6 substitué par un groupe cycloalkyle en C3-C8 ;
- un groupe alcényle en C2-C6 dont la double liaison peut être époxydée ou un groupe alcynyle en C2-C6, ces deux groupes pouvant porter un à trois substituants choisis parmi les halogènes, les groupes alcoxy en C1-C3 et/ou un substituant cyclopropyle ou phényle, ce dernier pouvant en outre porter jusqu'à trois des substituants suivants : les halogènes, les groupes cyano, nitro, alkyle en C1-C4 lui-même non substitué ou partiellement ou totalement halogéné, alcoxy en C1-C4 ou alkylthio en C1-C4, tous deux non substitués ou partiellement ou totalement halogénés,
- un groupe cycloalkyle en C3-C8 ou cycloalcényle en C3-C6 qui peuvent tous deux porter un à trois substituants alkyle en C1-C4 ou halogéno ;

$R^{1'}$

- un groupe alkyle en C1-C6 substitué par un groupe cycloalkyle en C3-C8 ;
- un groupe alcényle en C2-C6 dont la double liaison peut être époxydée, ou un groupe alcynyle en C2-C6, qui peuvent tous deux porter un à trois substituants halogéno,

alcoxy en C1-C3 et/ou un substituant cyclopropyle ou phényle, ce dernier pouvant en outre porter jusqu'à trois des substituants suivants : les halogènes, les groupes cyano, nitro, alkyle en C1-C4 lui-même non substitué ou partiellement ou totalement halogéné, alcoxy en C1-C4 ou alkylthio en C1-C4 tous deux non substitués ou partiellement ou totalement halogénés ;

- un groupe cycloalcényle en C3-C6 qui peut porter un à trois substituants halogéno ou alkyle en C1-C4 ;

$R^2$

- un groupe formyle, un groupe 4,5-dihydrooxazole-2-yle,
- un groupe $COYR^5$ ou $CONR^6R^7$, dans lesquels les symboles ont les significations suivantes :

Y représente l'oxygène ou le soufre ;

$R^5$

- l'hydrogène ;
- un groupe alkyle en C1-C6 qui peut porter un à cinq atomes d'halogènes et/ou jusqu'à trois groupes hydroxy et/ou alcoxy en C1-C4 et/ou l'un des substituants suivants :
- cyano,
- (alcoxy en C1-C4)-alcoxy en C2-C4,
- alkylthio en C1-C3,
- alkylamino en C1-C3, di-(alkyle en C1-C3)-amino, cycloalkylamino en C2-C6 ou di-(cycloalkyle en C3-C6)-amino,
- triméthylsilyle,
- alkylsulfinyle en C1-C3 ou alkylsulfonyle en C1-C3, carboxyle, (alcoxy en C1-C3)-carbonyle, (alcoxy en C1-C3)-carbonyl-alcoxy en C1-C3 ou (alcoxy en C1-C3)-carbonyl-(alcoxy en C1-C3)-(alcoxy en C1-C3)-carbonyle,
- di-(alkyle en C1-C3)-aminocarbonyle,
- di-(alcoxy en C1-C3)-phosphonyle,
- alcaniminoxy en C1-C6 ou cycloalcaniminoxy en C5-C6,
- N-phtalimido, N-succinimido, benzyloxy, benzoyle, ces radicaux cycliques pouvant en outre porter un à trois des substituants suivants : halogéno, alkyle en C1-C3 ou alcoxy en C1-C3,
- un radical hétérocyclique saturé de cinq ou six chaînons ou un radical hétéroaromatique de cinq ou six chaînons contenant chacun jusqu'à trois hétéroatomes choisis parmi l'oxygène, le soufre et l'azote, sous réserve que deux atomes d'oxygène et/ou de soufre ne peuvent pas être directement voisins, les hétérocycles peuvent encore porter un ou deux des substituants suivants : halogéno, alkyle en C1-C3, alcoxy en C1-C3 ou (alcoxy en C1-C3)-carbonyle ;
- un groupe phényle qui peut encore porter jusqu'à trois des substituants suivants : halogéno, nitro, cyano, alkyle en C1-C3, alkyle en C1-C3 partiellement ou totalement halogéné, alcoxy en C1-C3 ou alcoxy en C1-C3 partiellement ou totalement halogéné ;
- un groupe $CR^{10}=N-R^{11}$ dans lequel $R^{10}$ et $R^{11}$ ont les significations suivantes :

  $R^{10}$ : l'hydrogène ou un groupe alkyle en C1-C6 et

  $R^{11}$ : un groupe alcoxy en C1-C6, alcényloxy en C3-C6 ou alcynyloxy en C3-C6 qui peuvent porter chacun jusqu'à trois atomes d'halogènes et/ou un groupe phényle qui peut lui-même porter jusqu'à trois des substituants suivants : les halogènes, les groupes nitro, cyano, alkyle en C1-C3 ou alcoxy en C1-C3 ;

  un groupe phénoxy qui peut encore porter jusqu'à trois des substituants suivants : les halogènes, les groupes nitro, cyano, alkyl en C1-C3 ou alcoxy en C1-C3 ;

  un groupe alkylamino en C1-C6, di-(alkyle en C1-C6)-amino ou phénylamino, la partie aromatique pouvant en outre porter jusqu'à trois des substituants suivants : halogéno, nitro, cyano, alkyle en C1-C3 ou alcoxy en C1-C3 ;
- un groupe cycloalkyle en C3-C8 ;
- un groupe alcényle en C3-C6, cycloalcényle en C5-C6, alcynyle en C3-C6, ces radicaux pouvant porter l'un des substituants suivants : hydroxy, halogéno, alcoxy en C1-C4 ou phényle qui peut lui-même porter un à trois des substituants suivants : halogéno, nitro, cyano, alkyle en C1-C4, alkyle en C1-C4 partiellement ou totalement halogéné ou alcoxy en C1-C4 ;

- un groupe phényle qui peut porter un à trois des substituants suivants : halogéno, nitro, cyano, alkyle en C1-C4, alkyle en C1-C4 partiellement ou totalement halogéné, alcoxy en C1-C4, alcoxy en C1-C4 partiellement ou totalement halogéné ou (alcoxy en C1-C4)-carbonyle ;
- un radical hétérocyclique de cinq ou six chaînons contenant jusqu'à trois hétéroatomes choisis parmi l'oxygène, le soufre et l'azote, sous réserve que deux atomes d'oxygène et/ou de soufre ne peuvent être directement voisins, les hétérocycles pouvant porter un ou deux des substituants suivants : halogéno, alcoxy en C1-C3 ou (alcoxy en C1-C3)-carbonyle ;
- un groupe benzotriazole ;
- un groupe N-phtalimido, tétrahydrophtalimido, succinimido, maléimido ;
- un groupe 2,2-diméthyl-1,3-dioxolanne-4-ylméthyle ou 1,3-dioxolanne-2-one-ylméthyle ;
- lorsque Y = 0 : un équivalent d'un cation choisi parmi les métaux alcalins et alcalino-terreux, le manganèse, le cuivre, le fer, l'ammonium contenant le cas échéant jusqu'à quatre substituants groupes alkyle en C1-C3 ; ou bien
- un groupe $-N=CR^8R^9$ dans lequel
  $R^8$, $R^9$ représentent l'hydrogène ; un groupe alkyle en C1-C4 qui peut être non substitué ou partiellement ou totalement halogéné et peut porter un groupe alcoxy en C1-C3 ou phényle, ce dernier pouvant encore lui-même porter un à trois substituants halogéno, nitro, cyano, alkyle en C1-C3, alkyle en C1-C3 partiellement ou totalement halogéné, alcoxy en C1-C3 ou alcoxy en C1-C3 partiellement ou totalement halogéné ; un groupe cycloalkyle en C3-C6 ; alcoxy en C1-C4 ; furannyle ou phényle qui peut en outre porter jusqu'à trois des substituants suivants : halogéno, nitro, cyano, alkyle en C1-C3, alkyle en C1-C3 partiellement ou totalement halogéné, alcoxy en C1-C3 ou alcoxy en C1-C3 partiellement ou totalement halogéné ; ou bien $R^8$ et $R^9$ forment ensemble une chaîne méthylène de 4 à 7 chaînons ;
- un groupe -W-Z dans lequel W représente une chaîne alkylène en C2-C4, une chaîne éthoxyéthylène, une chaîne buta-2-énylène ou buta-2-ynylène et Z représente une partie de la molécule reliée à W en position $\omega$ et qui est identique à la partie molécule reliée à W en position $\alpha$ de W ;
  $R^6$ - l'hydrogène, un groupe alkyle en C1-C6 ou cycloalkyle en C3-C8 et
  $R^7$ - l'hydrogène, un groupe alkyle en C1-C6, $-C(OR^{12})=N-H$ ou $-C(OR^{12})=N$-alkyle en C1-C4, $R^{12}$ représentant un groupe alkyle en C1-C4, ou bien
  $R^6$, $R^7$ forment ensemble une chaîne méthylène à 4 ou 5 chaînons ;

$R^3$

- l'hydrogène ;
- un groupe alkyle en C1-C6 qui peut porter un à trois des substituants suivants : hydroxy, halogéno, alcoxy en C1-C4, alkylthio en C1-C4 ou di-(alkyle en C1-C4)-amino ;
- cycloalkyle en C3-C8 qui peut porter un à trois substituants halogéno, alkyle en C1-C4 ou alkyle en C1-C4 partiellement ou totalement halogéné ;

$R^4$

- l'hydrogène, un groupe hydroxy, un groupe alcoxy en C1-C4 ;
- un groupe alkyle en C1-C6 qui peut porter un à trois des substituants suivants : halogéno, cyano, alcoxy en C1-C4, alcoxy en C1-C4 partiellement ou totalement halogéné, alkylthio en C1-C4, alkylthio en C1-C4 partiellement ou totalement halogéné, di-(alkyle en C1-C4)-amino, cycloalkyle en C3-C8 ou phényle dont le noyau phényle peut lui-même porter un à trois des substituants suivants : halogéno, cyano, nitro, alkyle en C1-C4, alkyle en C1-C4 partiellement ou totalement halogéné, alcoxy en C1-C4, alcoxy en C1-C4 partiellement ou totalement halogéné, alkylthio en C1-C4 ou alkylthio en C1-C4 partiellement ou totalement halogéné ;
- un groupe cycloalkyle en C3-C8 qui peut porter un à trois des substituants suivants : halogéno, nitro, cyano, alkyle en C1-C6, alkyle en C1-C6 partiellement ou totalement halogéné, alcoxy en C1-C4 ou alcoxy en C1-C4 partiellement ou totalement halogéné ;
- un groupe alcényle en C3-C6 ou alcynyle en C3-C6 qui peuvent tous deux porter un à trois substituants halogéno et/ou un substituant phényle, lequel peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4,

halogéno, cyano ou nitro ;

- un groupe -di-(alkyle en C1-C4)-amino ;
- un radical hétérocyclique saturé ou aromatique de 5 à 6 chaînons contenant un ou deux hétéroatomes choisis parmi l'oxygène, le soufre et l'azote et qui peut porter un à trois substituants alkyle en C1-C4 ou halogéno ;
- un groupe phényle qui peut porter un à quatre des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogéno, nitro, cyano, formyle, alcanoyle en C1-C4, halogénoalcanoyle en C1-C4 ou (alcoxy en C1-C4)-carbonyle ;
- un groupe naphtyle qui peut porter un à trois substituants alkyle en C1-C4 ou halogéno ;

ou bien

$R^3$, $R^4$ forment ensemble une chaîne méthylène de 4 à 7 chaînons qui peut être interrompue par l'oxygène, le soufre ou un groupe N-méthyle, ou le groupe $-(CH_2)_3$-CO-, sous réserve que, dans le cas des composés Ia à Ic, $R^3$ et $R^4$ ne peuvent représenter tous deux l'hydrogène lorsque

- $R^1$ représente l'hydrogène, un groupe méthyle ou phényle et $R^2$ représente $CONH_2$, $CO_2H$ ou $CO_2CH_3$ ou bien lorsque
- X représente l'oxygène, $R^1$ représente $CH(OCH_2CH_3)_2$ et $R^2$ un groupe $CONH_2$

et leurs sels non polluants.

**2.** Carboxamides de formule Ia, Ib, Ic, Id selon la revendication 1, pour lesquels $R^3$ représente l'hydrogène.

**3.** Carboxamides de formules Ia, Ib, Ic selon la revendication 1, pour lesquels

$R^1$ représente l'hydrogène, un groupe alkyle en C1-C4 ou cycloalkyle en C3-C6 ;

$R^2$ représente $COYR^5$ dans lequel Y représente l'oxygène et $R^5$ l'hydrogène ou un groupe $-N=CR^8R^9$ dans lequel

$R^8$ et $R^9$ représentent chacun, indépendamment l'un de l'autre, l'hydrogène, un groupe alkyle en C1-C4 ou cycloalkyle en C3-C6 ou bien ensemble une chaîne alkylène en C4-C7 ;

$R^3$ représente l'hydrogène et

$R^4$ un groupe alkyle en C1-C4 ou cycloalkyle en C3-C8.

**4.** Carboxamides de formule Ia selon les revendications 1 à 3.

**5.** Procédé de préparation des composés Ia et Ib de la revendication 1, pour lesquels $R^2$ représente $CO_2CH_3$ et X l'oxygène, caractérisé en ce que l'on fait réagir le chlorure de l'acide hydroxamique de formule II

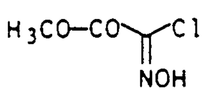

II

de manière connue en soi, en présence d'une base, avec un $\beta$-cétoester de formule III

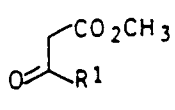

III

ce qui donne le diester diméthylique IV

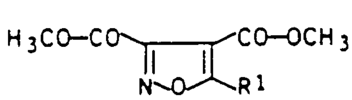

IV

qu'on soumet ensuite en premier lieu à hydrolyse à l'aide d'un équivalent d'une base aqueuse, ce qui donne les monoesters-Va et Vb

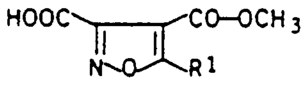

Va

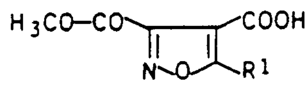

Vb

qu'on convertit d'abord, séparément ou en mélange entre eux, de manière connue en soi, en les halogénures ou d'autres formes activées des acides carboxyliques, qu'on amide ensuite à l'aide d'une amine substituée de formule VIa

$HNR^3R^4$ VIa

6. Procédé de préparation des composés Ia de la revendication 1 pour lesquels $R^2$ représente $CO_2R^5$, X l'oxygène et $R^5$ a une signification autre que l'hydrogène ou un groupe méthyle, caractérisé en ce que l'on fait réagir le chlorure d'acide hydroxamique II de manière connue en soi, en présence d'une base, avec un $\beta$-cétoester de formule IIIa

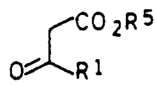

IIIa

ce qui donne le diester IVa

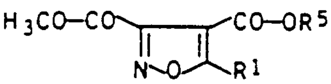

IVa

qu'on hydrolyse ensuite à l'aide d'un réactif saponifiant en le monoester Va'

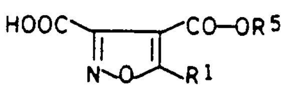

Va'

qu'on convertit de manière connue en soi en les halogénures ou d'autres formes activées des acides carboxyliques, qui sont ensuite soumises à amidation à l'aide d'une amine substituée VIa.

7. Procédé de préparation des composés Ia et Ic de la revendication 1, pour lesquels $R^1$ a une signification autre que l'hydrogène et $R^2$ représente un groupe carboxyle ou formyle et $R^3$ l'hydrogène, caractérisé en ce que l'on convertit un acide carboxylique Vc ou Vd

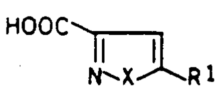

Vc

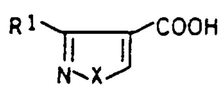

Vd

de manière connue en soi en les halogénures ou d'autres formes activées des acides carboxyliques, qu'on amide ensuite à l'aide d'une amine substituée VIa, ce qui donne les amides VIIa et VIIb

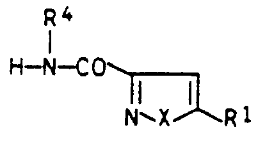

VIIa

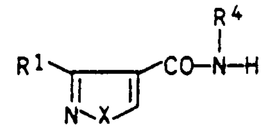

VIIb

qu'on fait réagir ensuite de manière connue en soi, en présence d'une base, avec un réactif carboxylant ou formylant.

**8.** Procédé de préparation des composés Ia et Ib de la revendication 1, pour lesquels $R^2$ représente un groupe carboxyle et X le soufre, caractérisé en ce que l'on fait réagir un anhydride d'acide isothiazole-dicarboxylique VIII

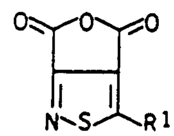

VIII

de manière connue en soi, avec une amine VIa, ce qui donne les isomères Ia et Ib qu'on sépare ensuite.

**9.** Procédé de préparation des composés Ia, Ib, Ic et Id de la revendication 1, pour lesquels $R^2$ représente $CO_2H$, caractérisé en ce que l'on hydrolyse de manière connue en soi, en présence d'une base aqueuse, un ester correspondant Ia, Ib, Ic ou Id pour lequel $R^2$ représente $CO_2R^5$ et $R^5$ un groupe alkyle en C1-C4.

**10.** Procédé de préparation des composés Ia, Ib, Ic et Id de la revendication 1, pour lesquels $R^2$ représente un groupe $COYR^5$ ou $CONR^6R^7$, caractérisé en ce que l'on convertit d'abord un acide carboxylique correspondant Ia, Ib, Ic ou respectivement Id ($R^2$ = $CO_2H$), de manière connue en soi, en les halogénures ou autres dérivés activés de ces acides, qu'on fait réagir ensuite de manière connue en soi, avec un composé IX

$HYR^5$ IX

ou avec une amine VIb

$HNR^6R^7$ VIb

**11.** Procédé de préparation des composés Ia, Ib, Ic et Id de la revendication 1, pour lesquels $R^2$ représente un groupe 4,5-dihydrooxazole-2-yle, caractérisé en ce que l'on cyclise de manière connue en soi un carboxàmide de formule Ia, Ib, Ic ou Id de la revendication 1 dans laquelle $R^2$ représente un groupe $CO_2H$ ou $CO_2R^5$ avec $R^5$ = alkyle en C1-C4, à l'aide d'un aminoalcool XV

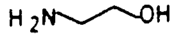

XV

**12.** Procédé de préparation des composés Ia, Ib, Ic et Id de la revendication 1, pour lesquels $R^2$ représente un groupe formyle, caractérisé en ce que l'on convertit d'abord un acide carboxylique correspondant de formule Ia, Ib, Ic ou Id ($R^2$ = $CO_2H$) en l'halogénure ou une autre forme activée de l'acide qu'on réduit ensuite de manière connue en soi.

**13.** Procédé de préparation des composés Ia, Ib, Ic et Id de la revendication 1, pour lesquels $R^1$ ou $R^{1'}$ représente un groupe alcényle en C2-C6 époxydé, caractérisé en ce que l'on époxyde de manière connue en soi, à l'aide d'un agent oxydant, un carboxamide de formule Ia, Ib, Ic ou Id de la revendication 1, dans laquelle $R^1$ ou respectivement $R^{1'}$ représente un alcényle en C2-C6.

**14.** Produits contenant des véhicules inertes et une quantité herbicide efficace d'au moins un carboxamide de formule Ia, Ib, Ic ou Id des revendications 1 à 4.

**15.** Procédé pour combattre les croissances de végétaux indésirables, caractérisé en ce que l'on fait agir sur les végétaux, leur habitat ou leurs semences, une quantité herbicide efficace d'un carboxamide Id de la revendication 1, ou d'un carboxamide de formule Ia', Ib' ou Ic', les formules Ia', Ib' et Ic' ayant les mêmes significations que Ia, Ib et respectivement Ic mais avec suppression de la réserve.

**Revendications pour l'Etat contractant suivant : ES**

**1.** Produit contenant des véhicules inertes et une quantité herbicide efficace d'au moins un carboxamide de formule Ia, Ib, Ic ou Id

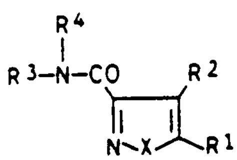
Ia

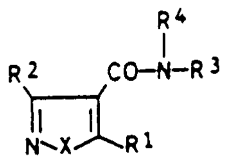
Ib

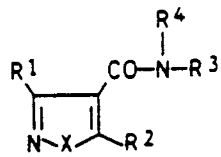
Ic

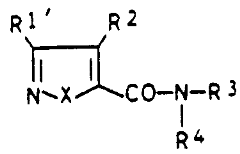
Id

dans lesquelles les symboles ont les significations suivantes :

X
- l'oxygène ou le soufre;

$R^1$
- l'hydrogène, un halogène, un groupe alkyle en C1-C6 qui peut porter un à cinq atomes d'halogènes et/ou un groupe cyano et/ou jusqu'à deux des substituants suivants : alcoxy en C1-C4, alcoxy halogéné en totalité ou en partie, alkylthio en C1-C4 ou alkylthio en C1-C4 halogéné en totalité ou en partie ;
- un groupe alcoxy en C1-C4 ou alkylthio en C1-C4, un groupe alcoxy en C1-C4 partiellement ou totalement halogéné, un groupe alkylthio en C1-C4 partiellement ou totalement halogéné ;
- le groupe benzyle qui peut être porter un à trois substituants choisis parmi les groupes alkyle en C1-C4, alkyle en C1-C4 partiellement ou totalement halogéné, alcoxy en C1-C4, alcoxy en C1-C4 partiellement ou totalement halogéné, alkylthio en C1-C4, alkylthio en C1-C4 partiellement ou totalement halogéné, les halogènes, les groupes cyano ou nitro ;
- le groupe phényle qui peut encore porter un à trois des substituants suivants : les groupes cyano, nitro, les halogènes, les groupes alkyle en C1-C6, alkyle en C1-C6 partiellement ou totalement halogéné, alcoxy en C1-C6, alcoxy en C1-C6 partiellement ou totalement halogéné, alkylthio en C1-C6 et/ou alkylthio en C1-C6 partiellement ou totalement halogéné ;
- le groupe phénoxy ou le groupe phénylthio qui peuvent tous deux porter un à trois substituants choisis parmi les groupes alkyle en C1-C4, alkyle en C1-C4 partiellement ou totalement halogénén, alcoxy en C1-C4, alcoxy en C1-C4 partiellement ou totalement halogéné, alkylthio en C1-C4, alkylthio en C1-C4 partiellement ou totalement halogéné, les halogènes, les groupes cyano ou nitro ;
- un radical hétérocyclique saturé ou aromatique de 5 à 6 chaînons contenant un ou deux hétéroatomes choisis parmi l'oxygène, le soufre et l'azote et qui peut porter un ou deux des substituants suivants : les halogènes, les groupes alkyle en C1-C3, alcoxy en C1-C3 et (alcoxy en C1-C3)-carbonyle ;
- un groupe alkyle en C1-C6 substitué par un groupe cycloalkyle en C3-C8 ;

- un groupe alcényle en C2-C6 dont la double liaison peut être époxydée ou un groupe alcynyle en C2-C6, ces deux groupes pouvant porter un à trois substituants choisis parmi les halogènes, les groupes alcoxy en C1-C3 et/ou un substituant cyclopropyle ou phényle, ce dernier pouvant en outre porter jusqu'à trois des substituants suivants : les halogènes, les groupes cyano, nitro, alkyle en C1-C4 lui-même non substitué ou partiellement ou totalement halogéné, alcoxy en C1-C4 ou alkylthio en C1-C4, tous deux non substitués ou partiellement ou totalement halogénés,
- un groupe cycloalkyle en C3-C8 ou cycloalcényle en C3-C6 qui peuvent tous deux porter un à trois substituants alkyle en C1-C4 ou halogéno ;

$R^{1'}$

- un groupe alkyle en C1-C6 substitué par un groupe cycloalkyle en C3-C8 ;
- un groupe alcényle en C2-C6 dont la double liaison peut être époxydée, ou un groupe alcynyle en C2-C6, qui peuvent tous deux porter un à trois substituants halogéno, alcoxy en C1-C3 et/ou un substituant cyclopropyle ou phényle, ce dernier pouvant en outre porter jusqu'à trois des substituants suivants : les halogènes, les groupes cyano, nitro, alkyle en C1-C4 lui-même non substitué ou partiellement ou totalement halogéné, alcoxy en C1-C4 ou alkylthio en C1-C4 tous deux non substitués ou partiellement ou totalement halogénés ;
- un groupe cycloalcényle en C3-C6 qui peut porter un à trois substituants halogéno ou alkyle en C1-C4 ;

$R^2$

- un groupe formyle, un groupe 4,5-dihydrooxazole-2-yle,
- un groupe $COYR^5$ ou $CONR^6R^7$, dans lesquels les symboles ont les significations suivantes :

Y représente l'oxygène ou le soufre ;

$R^5$

- l'hydrogène ;
- un groupe alkyle en C1-C6 qui peut porter un à cinq atomes d'halogènes et/ou jusqu'à trois groupes hydroxy et/ou alcoxy en C1-C4 et/ou l'un des substituants suivants :
- cyano,
- (alcoxy en C1-C4)-alcoxy en C2-C4,
- alkylthio en C1-C3,
- alkylamino en C1-C3, di-(alkyle en C1-C3)-amino, cycloalkylamino en C2-C6 ou di-(cycloalkyle en C3-C6)-amino,
- triméthylsilyle,
- alkylsulfinyle en C1-C3 ou alkylsulfonyle en C1-C3, carboxyle, (alcoxy en C1-C3)-carbonyle, (alcoxy en C1-C3)-carbonyl-alcoxy en C1-C3 ou (alcoxy en C1-C3)-carbonyl-(alcoxy en C1-C3)-(alcoxy en C1-C3)-carbonyle,
- di-(alkyle en C1-C3)-aminocarbonyle,
- di-(alcoxy en C1-C3)-phosphonyle,
- alcaniminoxy en C1-C6 ou cycloalcaniminoxy en C5-C6,
- N-phtalimido, N-succinimido, benzyloxy, benzoyle, ces radicaux cycliques pouvant en outre porter un à trois des substituants suivants : halogéno, alkyle en C1-C3 ou alcoxy en C1-C3,
- un radical hétérocyclique saturé de cinq ou six chaînons ou un radical hétéroaromatique de cinq ou six chaînons contenant chacun jusqu'à trois hétéroatomes choisis parmi l'oxygène, le soufre et l'azote, sous réserve que deux atomes d'oxygène et/ou de soufre ne peuvent pas être directement voisins, les hétérocycles peuvent encore porter un ou deux des substituants suivants : halogéno, alkyle en C1-C3, alcoxy en C1-C3 ou (alcoxy en C1-C3)-carbonyle ;
- un groupe phényle qui peut encore porter jusqu'à trois des substituants suivants : halogéno, nitro, cyano, alkyle en C1-C3, alkyle en C1-C3 partiellement ou totalement halogéné, alcoxy en C1-C3 ou alcoxy en C1-C3 partiellement ou totalement halogéné ;
- un groupe $CR^{10}=N-R^{11}$ dans lequel $R^{10}$ et $R^{11}$ ont les significations suivantes :
  $R^{10}$ : l'hydrogène ou un groupe alkyle en C1-C6 et
  $R^{11}$ : un groupe alcoxy en C1-C6, alcényloxy en C3-C6 ou alcynyloxy en C3-C6 qui peuvent porter chacun jusqu'à trois atomes d'halogènes et/ou un groupe phényle qui peut lui-même porter jusqu'à trois des substituants suivants : les halogènes, les

groupes nitro, cyano, alkyle en C1-C3 ou alcoxy en C1-C3 ;

un groupe phénoxy qui peut encore porter jusqu'à trois des substituants suivants : les halogènes, les groupes nitro, cyano, alkyle en C1-C3 ou alcoxy en C1-C3 ;

un groupe alkylamino en C1-C6, di-(alkyle en C1-C6)-amino ou phénylamino, la partie aromatique pouvant en outre porter jusqu'à trois des substituants suivants : halogéno, nitro, cyano, alkyle en C1-C3 ou alcoxy en C1-C3 ;

- un groupe cycloalkyle en C3-C8 ;
- un groupe alcényle en C3-C6, cycloalcényle en C5-C6, alcynyle en C3-C6, ces radicaux pouvant porter l'un des substituants suivants : hydroxy, halogéno, alcoxy en C1-C4 ou phényle qui peut lui-même porter un à trois des substituants suivants : halogéno, nitro, cyano, alkyle en C1-C4, alkyle en C1-C4 partiellement ou totalement halogéné ou alcoxy en C1-C4 ;
- un groupe phényle qui peut porter un à trois des substituants suivants : halogéno, nitro, cyano, alkyle en C1-C4, alkyle en C1-C4 partiellement ou totalement halogéné, alcoxy en C1-C4, alcoxy en C1-C4 partiellement ou totalement halogéné ou (alcoxy en C1-C4)-carbonyle ;
- un radical hétérocyclique de cinq ou six chaînons contenant jusqu'à trois hétéroatomes choisis parmi l'oxygène, le soufre et l'azote, sous réserve que deux atomes d'oxygène et/ou de soufre ne peuvent être directement voisins, les hétérocycles pouvant porter un ou deux des substituants suivants : halogéno, alcoxy en C1-C3 ou (alcoxy en C1-C3)-carbonyle ;
- un groupe benzotriazole ;
- un groupe N-phtalimido, tétrahydrophtalimido, succinimido, maléimido ;
- un groupe 2,2-diméthyl-1,3-dioxolanne-4-ylméthyle ou 1,3-dioxolanne-2-one-ylméthyle ;
- lorsque Y = 0 : un équivalent d'un cation choisi parmi les métaux alcalins et alcalino-terreux, le manganèse, le cuivre, le fer, l'ammonium contenant le cas échéant jusqu'à quatre substituants groupes alkyle en C1-C3 ; ou bien
- un groupe $-N=CR^8R^9$ dans lequel

  $R^8$, $R^9$ représentent l'hydrogène ; un groupe alkyle en C1-C4 qui peut être non substitué ou partiellement ou totalement halogéné et peut porter un groupe alcoxy en C1-C3 ou phényle, ce dernier pouvant encore lui-même porter un à trois substituants halogéno, nitro, cyano, alkyle en C1-C3, alkyle en C1-C3 partiellement ou totalement halogéné, alcoxy en C1-C3 ou alcoxy en C1-C3 partiellement ou totalement halogéné ; un groupe cycloalkyle en C3-C6 ; alcoxy en C1-C4 ; furannyle ou phényle qui peut en outre porter jusqu'à trois des substituants suivants : halogéno, nitro, cyano, alkyle en C1-C3, alkyle en C1-C3 partiellement ou totalement halogéné, alcoxy en C1-C3 ou alcoxy en C1-C3 partiellement ou totalement halogéné ; ou bien $R^8$ et $R^9$ forment ensemble une chaîne méthylène de 4 à 7 chaînons ;
- un groupe -W-Z dans lequel W représente une chaîne alkylène en C2-C4, une chaîne éthoxyéthylène, une chaîne buta-2-énylène ou buta-2-ynylène et Z représente une partie de la molécule reliée à W en position $\omega$ et qui est identique à la partie molécule reliée à W en position $\alpha$ de W ;

  $R^6$ - l'hydrogène, un groupe alkyle en C1-C6 ou cycloalkyle en C3-C8 et

  $R^7$ - l'hydrogène, un groupe alkyle en C1-C6, $-C(OR^{12}) = N-H$ ou $-C(OR^{12}) = N$-alkyle en C1-C4, $R^{12}$ représentant un groupe alkyle en C1-C4, ou bien

  $R^6$, $R^7$ forment ensemble une chaîne méthylène à 4 ou 5 chaînons ;

$R^3$

- l'hydrogène ;
- un groupe alkyle en C1-C6 qui peut porter un à trois des substituants suivants : hydroxy, halogéno, alcoxy en C1-C4, alkylthio en C1-C4 ou di-(alkyle en C1-C4)-amino ;
- cycloalkyle en C3-C8 qui peut porter un à trois substituants halogéno, alkyle en C1-C4 ou alkyle en C1-C4 partiellement ou totalement halogéné ;

$R^4$

- l'hydrogène, un groupe hydroxy, un groupe alcoxy en C1-C4 ;
- un groupe alkyle en C1-C6 qui peut porter un à trois des substituants suivants : halogéno, cyano, alcoxy en C1-C4, alcoxy en C1-C4 partiellement ou totalement halogéné, alkylthio en C1-C4, alkylthio en C1-C4 partiellement ou totalement halogéné,

di-(alkyle en C1-C4)-amino, cycloalkyle en C3-C8 ou phényle dont le noyau phényle peut lui-même porter un à trois des substituants suivants : halogéno, cyano, nitro, alkyle en C1-C4, alkyle en C1-C4 partiellement ou totalement halogéné, alcoxy en C1-C4, alcoxy en C1-C4 partiellement ou totalement halogéné, alkylthio en C1-C4 ou alkylthio en C1-C4 partiellement ou totalement halogéné ;

- un groupe cycloalkyle en C3-C8 qui peut porter un à trois des substituants suivants : halogéno, nitro, cyano, alkyle en C1-C6, alkyle en C1-C6 partiellement ou totalement halogéné, alcoxy en C1-C4 ou alcoxy en C1-C4 partiellement ou totalement halogéné ;
- un groupe alcényle en C3-C6 ou alcynyle en C3-C6 qui peuvent tous deux porter un à trois substituants halogéno et/ou un substituant phényle, lequel peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogéno, cyano ou nitro ;
- un groupe -di-(alkyle en C1-C4)-amino ;
- un radical hétérocyclique saturé ou aromatique de 5 à 6 chaînons contenant un ou deux hétéroatomes choisis parmi l'oxygène, le soufre et l'azote et qui peut porter un à trois substituants alkyle en C1-C4 ou halogéno ;
- un groupe phényle qui peut porter un à quatre des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogéno, nitro, cyano, formyle, alcanoyle en C1-C4, halogénoalcanoyle en C1-C4 ou (alcoxy en C1-C4)-carbonyle ;
- un groupe naphtyle qui peut porter un à trois substituants alkyle en C1-C4 ou halogéno ;

ou bien

$R^3$, $R^4$ forment ensemble une chaîne méthylène de 4 à 7 chaînons qui peut être interrompue par l'oxygène, le soufre ou un groupe N-méthyle, ou le groupe $-(CH_2)_3-CO-$, sous réserve que, dans le cas des composés Ia à Ic, $R^3$ et $R^4$ ne peuvent représenter tous deux l'hydrogène lorsque

- $R^1$ représente l'hydrogène, un groupe méthyle ou phényle et $R^2$ représente $CONH_2$, $CO_2H$ ou $CO_2CH_3$ ou bien lorsque
- X représente l'oxygène, $R^1$ représente $CH(OCH_2CH_3)_2$ et $R^2$ un groupe $CONH_2$

ou de leurs sels non polluants.

**2.** Produits contenant des véhicules inertes et une quantité herbicide efficace d'au moins un carboxamide de formule Ia, Ib, Ic ou Id de la revendication 1, pour lesquels $R^3$ représente l'hydrogène.

**3.** Produits contenant des véhicules inertes et une quantité herbicide efficace d'au moins un carboxamide de formule Ia, Ib eu Ic de la revendication 1, dans lesquelles les symboles ont les significations suivantes :

$R^1$ représente l'hydrogène, un groupe alkyle en C1-C4 ou cycloalkyle en C3-C6 ;

$R^2$ représente $COYR^5$ dans lequel Y représente l'oxygène et $R^5$ l'hydrogène ou un groupe $-N=CR^8R^9$ dans lequel

$R^8$ et $R^9$ représentent chacun, indépendamment l'un de l'autre, l'hydrogène, un groupe alkyle en C1-C4 ou cycloalkyle en C3-C6 ou bien ensemble une chaîne alkylène en C4-C7 ;

$R^3$ représente l'hydrogène et

$R^4$ un groupe alkyle en C1-C4 ou cycloalkyle en C3-C8.

**4.** Produits contenant des véhicules inertes et une quantité herbicide efficace d'au moins un carboxamide de formule Ia des revendications 1 à 3.

**5.** Procédé de préparation des composés Ia et Ib de la revendication 1, pour lesquels $R^2$ représente $CO_2CH_3$ et X l'oxygène, caractérisé en ce que l'on fait réagir le chlorure de l'acide hydroxamique de formule II

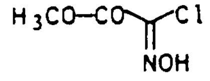 II

de manière connue en soi, en présence d'une base, avec un β-cétoester de formule III

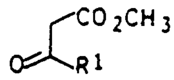

III

ce qui donne le diester diméthylique IV

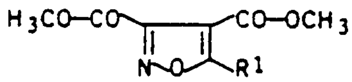

IV

qu'on soumet ensuite en premier lieu à hydrolyse à l'aide d'un équivalent d'une base aqueuse, ce qui donne les monoesters Va et Vb

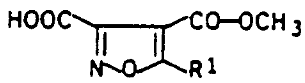

Va

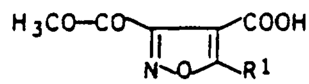

Vb

qu'on convertit d'abord, séparément ou en mélange entre eux, de manière connue en soi, en les halogénures ou d'autres formes activées des acides carboxyliques, qu'on amide ensuite à l'aide d'une amine substituée de formule VIa

$HNR^3R^4$ VIa

**6.** Procédé de préparation des composés Ia de la revendication 1, pour lesquels $R^2$ représente $CO_2R^5$, X l'oxygène et $R^5$ a une signification autre que l'hydrogène ou un groupe méthyle, caractérisé en ce que l'on fait réagir le chlorure d'acide hydroxamique II de manière connue en soi, en présence d'une base, avec un β-cétoester de formule IIIa

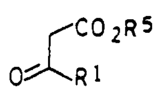

IIIa

ce qui donne le diester IVa

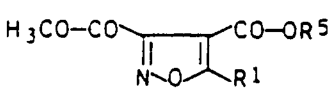

IVa

qu'on hydrolyse ensuite à l'aide d'un réactif saponifiant en le monoester Va'

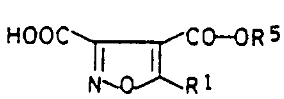

Va'

qu'on convertit de manière connue en soi en les halogénures ou d'autres formes activées des acides

carboxyliques, qui sont ensuite soumises à amidation à l'aide d'une amine substituée VIa.

**7.** Procédé de préparation des composés Ia et Ic de la revendication 1, pour lesquels $R^1$ a une signification autre que l'hydrogène et $R^2$ représente un groupe carboxyle ou formyle et $R^3$ l'hydrogène, caractérisé en ce que l'on convertit un acide carboxylique Vc ou Vd

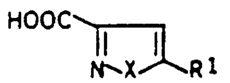

Vc

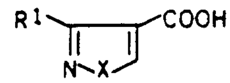

Vd

de manière connue en soi en les halogénures ou d'autres formes activées des acides carboxyliques, qu'on amide ensuite à l'aide d'une amine substituée VIa, ce qui donne les amides VIIa et VIIb

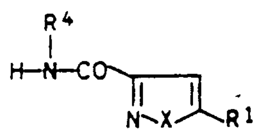

VIIa

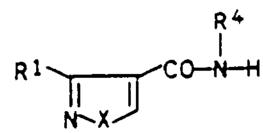

VIIb

qu'on fait réagir ensuite de manière connue en soi, en présence d'une base, avec un réactif carboxylant ou formylant.

**8.** Procédé de préparation des composés Ia et Ib de la revendication 1, pour lesquels $R^2$ représente un groupe carboxyle et X le soufre, caractérisé en ce que l'on fait réagir un anhydride d'acide isothiazole-dicarboxylique VIII

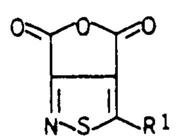

VIII

de manière connue en soi, avec une amine VIa, ce qui donne les isomères Ia et Ib qu'on sépare ensuite.

**9.** Procédé de préparation des composés Ia, Ib, Ic et Id de la revendication 1, pour lesquels $R^2$ représente $CO_2H$, caractérisé en ce que l'on hydrolyse de manière connue en soi, en présence d'une base aqueuse, un ester correspondant Ia, Ib, Ic ou Id pour lequel $R^2$ représente $CO_2R^5$ et $R^5$ un groupe alkyle en C1-C4.

**10.** Procédé de préparation des composés Ia, Ib, Ic et Id de la revendication 1, pour lesquels $R^2$ représente un groupe $COYR^5$ ou $CONR^6R^7$, caractérisé en ce que l'on convertit d'abord un acide carboxylique correspondant Ia, Ib, Ic ou respectivement Id ($R^2$ = $CO_2H$), de manière connue en soi, en les halogénures ou autres dérivés activés de ces acides, qu'on fait réagir ensuite de manière connue en soi, avec un composé IX

$HYR^5$ IX

ou avec une amine VIb

$HNR^6R^7$ VIb

**11.** Procédé de préparation des composés Ia, Ib, Ic et Id de la revendication 1, pour lesquels $R^2$ représente un groupe 4,5-dihydrooxazole-2-yle, caractérisé en ce que l'on cyclise de manière connue en soi un carboxamide de formule Ia, Ib, Ic ou Id de la revendication 1 dans laquelle $R^2$ représente un groupe $CO_2H$ ou $CO_2R^5$ avec $R^5$ = alkyle en C1-C4, à l'aide d'un aminoalcool XV

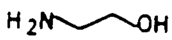

XV

**12.** Procédé de préparation des composés Ia, Ib, Ic et Id de la revendication 1, pour lesquels $R^2$ représente un groupe formyle, caractérisé en ce que l'on convertit d'abord un acide carboxylique correspondant de formule Ia, Ib, Ic ou Id ($R^2$ = $CO_2H$) en l'halogénure ou une autre forme activée de l'acide qu'on réduit ensuite de manière connue en soi.

**13.** Procédé de préparation des composés Ia, Ib, Ic et Id de la revendication 1, pour lesquels $R^1$ ou $R^{1'}$ représente un groupe alcényle en C2-C6 époxydé, caractérisé en ce que l'on époxyde de manière connue en soi, à l'aide d'un agent oxydant, un carboxamide de formule Ia, Ib, Ic ou Id de la revendication 1, dans laquelle $R^1$ ou respectivement $R^{1'}$ représente un alcényle en C2-C6.

**14.** Procédé pour combattre les croissances de végétaux indésirables, caractérisé en ce que l'on fait agir sur les végétaux, leur habitat ou leurs semences, une quantité herbicide efficace d'un carboxamide Id de la revendication 1, ou d'un carboxamide de formule Ia', Ib' ou Ic', les formules Ia', Ib' et Ic' ayant les mêmes significations que Ia, Ib et respectivement Ic mais avec suppression de la réserve.